(19) Europäisches Patentamt
European Patent Office
Office européen des brevets

(11) EP 3 974 423 A1

(12) EUROPEAN PATENT APPLICATION
published in accordance with Art. 153(4) EPC

(43) Date of publication:
**30.03.2022 Bulletin 2022/13**

(21) Application number: **20810199.8**

(22) Date of filing: **21.05.2020**

(51) International Patent Classification (IPC):
***C07D 403/04*** (2006.01) ***C07D 403/12*** (2006.01)
***C07F 9/53*** (2006.01) ***C07D 491/10*** (2006.01)
***C07D 405/14*** (2006.01) ***A61K 31/506*** (2006.01)
***A61K 31/505*** (2006.01) ***A61K 31/675*** (2006.01)
***A61P 35/00*** (2006.01)

(52) Cooperative Patent Classification (CPC):
**A61K 31/505; A61K 31/506; A61K 31/675; A61P 35/00; C07D 403/04; C07D 403/12; C07D 405/14; C07D 491/10; C07F 9/53**

(86) International application number:
**PCT/CN2020/091558**

(87) International publication number:
**WO 2020/233669 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.05.2019 CN 201910430758**
**27.09.2019 CN 201910926544**
**27.11.2019 CN 201911185460**
**17.01.2020 CN 202010054268**

(71) Applicants:
- **Shanghai Hansoh Biomedical Co., Ltd.**
**Shanghai 201203 (CN)**
- **Jiangsu Hansoh Pharmaceutical Group Co., Ltd.**
**Lianyungang, Jiangsu 222047 (CN)**

(72) Inventors:
- **WANG, Feng**
**Lianyungang, Jiangsu 222047 (CN)**
- **DENG, Haining**
**Lianyungang, Jiangsu 222047 (CN)**
- **SU, Yidong**
**Lianyungang, Jiangsu 222047 (CN)**
- **CAI, Jiaqiang**
**Lianyungang, Jiangsu 222047 (CN)**
- **BAO, Rudi**
**Lianyungang, Jiangsu 222047 (CN)**

(74) Representative: **Viganò, Elena et al**
**Dragotti & Associati S.r.l.**
**Via Nino Bixio, 7**
**20129 Milano (IT)**

# (54) INDOLE DERIVATIVE-CONTAINING INHIBITOR, PREPARATION METHOD THEREFOR AND APPLICATION THEREOF

(57) The present invention relates to an indole derivative-containing inhibitor, a preparation method therefor and an application thereof, and in particular, to a compound as represented by general formula (Ia), a preparation method therefor, a pharmaceutical composition comprising the compound, and the use thereof in treating related diseases such as cancer, inflammation, chronic liver disease, diabetes, cardiovascular disease, and AIDS as a kinase inhibitor, especially as a receptor tyrosine kinase inhibitor (TKI), more specifically, as an EGFR or HER2 inhibitor. The compound exhibits good inhibitory activity in EGFR and HER2 20 exon mutations.

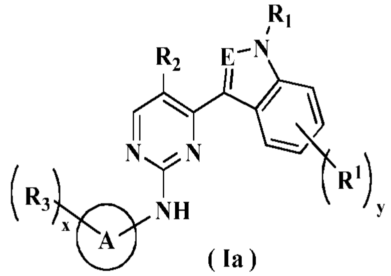

(Ia)

EP 3 974 423 A1

## Description

### TECHNICAL FIELD

The present invention belongs to the field of drug synthesis, and specifically relates to an indole-containing derivative inhibitor, a method for preparing the same, and application thereof.

### BACKGROUND

There are multiple signal pathways in cells that interact with each other to regulate the proliferation, growth, migration and apoptosis of cell. Abnormal activation of signaling pathways can lead to the occurrence of tumor. Receptor tyrosine kinases play an important role in the regulation of cells. Epidermal growth factor receptor (EGFR) is a member of the transmembrane protein tyrosine kinase ErbB receptor family (including ErbB1, ErbB2, ErbB3, ErbB4). EGFR can form homodimers on the membrane by binding to its ligand epidermal growth factor (EGF), or form heterodimers with other receptors in the ErbB family (such as ErbB2, ErbB3, ErbB4), leading to the activation of EGFR tyrosine kinase activity. Activated EGFR can phosphorylate different substrates, thereby activating the downstream PI3K-AKT pathway and RAS-MAPK pathway, etc., and it plays a role in multiple processes such as the survival, proliferation, and apoptosis of cell.

The disorder of the EGFR signaling pathway includes increased expression of ligands and receptors, EGFR gene amplification and mutation, etc., which can promote the malignant transformation of cell, leading to the occurrence of a variety of tumors. About 35% of non-small cell lung cancer (NSCLC) patients in China have EGFR mutation. The most common types of mutation are the deletion of exon 19 (Del19) and the L858R activating mutation of exon 21, which account for about 80% of the EGFR mutation. EGFR exon 20 insertion mutation is another major mutation in the EGFR mutation, accounting for 4%~10% of the EGFR mutation in NSCLC. There are dozens of types of EGFR exon 20 insertion mutations, and the common types are Ex20Ins D770_N771InsSVD, Ex20Ins V769_D770InsASV, etc.

Over the years, a large number of targeted drugs against the EGFR mutation in NSCLC have been developed, such as the first-generation reversible tyrosinase inhibitors (TKI), gefitinib and erlotinib, which is against the classic Del19 mutation and L858R mutation; the second-generation irreversible covalent binding inhibitor, afatinib; and the third-generation inhibitor, osimertinib, which is against the drug-resistant mutation EGFR T790M. They all have excellent clinical effects. However, the EGFR inhibitors currently on the market have poor effects on the EGFR exon 20 insertion mutation, and the patient's survival period is very short. This target requires more specific inhibitors, which have a large clinical demand.

HER2 is another member of the ErbB family, and its amplification and mutation are found in a variety of cancers. The HER2 mutation in NSCLC accounts for about 4%. About 90% of the HER2 mutations are exon 20 insertion mutation, of which the most common type is p.A775_G776insYVMA. The EGFR inhibitors currently on the market have a mediocre effect on the HER2 mutation.

At present, many domestic and foreign pharmaceutical companies have carried out active research on EGFR&HER2 exon 20 insertion mutations. Poziotinib of Spectrum, TAK-788 of Takeda and Tarloxotinib of Rain Therapeutics have all entered clinical studies, and compound TAS-6417 of Cullinan & Taiho has also showed good activity in preclinical experiments. Many EGFR inhibitors can cause clinical side effects such as skin rashes due to the strong inhibitory effect on wild-type EGFR, and their inhibitory activity on EGFR exon 20 insertion mutation and HER2 exon 20 insertion mutation target needs to be improved as well. Therefore, compounds that have obvious effects on EGFR and HER2 exon 20 mutations and have high selectivity for wild-type EGFR are still in great demand, and have good market prospects.

### SUMMARY OF THE INVENTION

The objective of the present invention is to provide a compound of formula (Ia), a stereoisomer thereof or a pharmaceutically acceptable salt thereof, wherein the structure of the compound of formula (Ia) is shown as following:

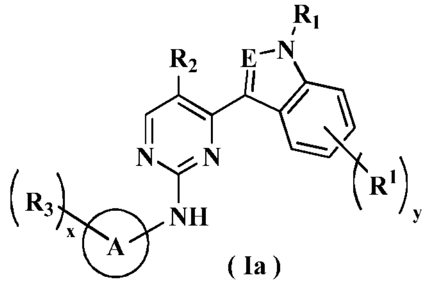

( Ia )

wherein:

E is selected from the group consisting of $CR_{aa}$ and N;
ring A is selected from the group consisting of heterocyclyl, aryl and heteroaryl;
$R_1$ is selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl can be each optionally further substituted;
$R_2$ is selected from the group consisting of hydrogen, deuterium, alkyl, deuterated alkyl, haloalkyl, alkoxy, haloalkoxy, halogen, amino, nitro, hydroxy, cyano, azido, alkoxycarbonyl, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}C(O)R_{bb}$, $-C(O)NR_{aa}(CH_2)_nR_{bb}$, $-NR_{aa}C(O)NR_{bb}R_{cc}$, $-NR_{aa}C(O)NR_{bb}(CH_2)_nR_{cc}$, $-C\equiv CR_{aa}$, $-NR_{aa}C(O)CH=CH(CH_2)_nR_{bb}$, $-NR_{aa}C(O)C\equiv CR_{bb}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$, $-(CH_2)_nNR_{aa}S(O)_mR_{bb}$ and

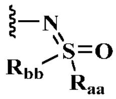

,

wherein the alkyl, haloalkyl, cycloalkyl, heterocyclyl, aryl and heteroaryl can be each optionally further substituted;
each $R_3$ is independently selected from the group consisting of hydrogen, deuterium, alkyl, deuterated alkyl, haloalkyl, alkoxy, haloalkoxy, halogen, amino, nitro, hydroxy, cyano, azido, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}(CH_2)_nR_{bb}$, $-NR_{aa}(CH_2)_nNR_{bb}R_{cc}$, $-NR_{aa}C(O)R_{bb}$, $-NR_{aa}C(O)NR_{bb}R_{cc}$, $-NR_{aa}C(O)NR_{bb}(CH_2)_nR_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}R_{bb}$, $-C(O)OR_{aa}$, $-NR_{aa}S(O)_mR_{bb}$, $-O(CH_2)_nR_{aa}$, $-O(CH_2)_nNR_{aa}R_{bb}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$, wherein the alkyl, haloalkyl, cycloalkyl, heterocyclyl, aryl and heteroaryl can be each optionally further substituted;
each $R^1$ is independently selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl can be each optionally further substituted;
or, any two of $R^1$ are bonded to form a cycloalkyl, heterocyclyl, aryl or heteroaryl, wherein the cycloalkyl, heterocyclyl, aryl and heteroaryl can be each optionally further substituted;
$R_{aa}$, $R_{bb}$ and $R_{cc}$ are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl can be each optionally further substituted;
x is an integer from 0 to 4;
y is an integer from 0 to 4;
m is an integer from 0 to 2;
n is an integer from 0 to 4.

The objective of the present invention is to provide a compound of formula (I), a stereoisomer thereof or a pharmaceutically acceptable salt thereof, wherein the structure of the compound of formula (I) is shown as following:

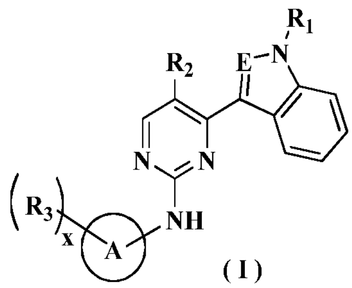

(I)

wherein:

E is selected from the group consisting of $CR_{aa}$ and N;
ring A is selected from the group consisting of heterocyclyl, aryl and heteroaryl;
$R_1$ is selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl can be each optionally further substituted;
$R_2$ is selected from the group consisting of hydrogen, deuterium, alkyl, deuterated alkyl, haloalkyl, alkoxy, haloalkoxy, halogen, amino, nitro, hydroxy, cyano, azido, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}C(O)R_{bb}$, $-C(O)NR_{aa}(CH_2)_nR_{bb}$, $-NR_{aa}C(O)NR_{bb}R_{cc}$, $-NR_{aa}C(O)NR_{bb}(CH_2)_nR_{cc}$, $-C\equiv CR_{aa}$, $-NR_{aa}C(O)CH=CH(CH_2)_nR_{bb}$, $-NR_{aa}C(O)C\equiv CR_{bb}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$, wherein the alkyl, haloalkyl, cycloalkyl, heterocyclyl, aryl and heteroaryl can be each optionally further substituted;
each $R_3$ is independently selected from the group consisting of hydrogen, deuterium, alkyl, deuterated alkyl, haloalkyl, alkoxy, haloalkoxy, halogen, amino, nitro, hydroxy, cyano, azido, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}(CH_2)_nR_{bb}$, $-NR_{aa}(CH_2)_nNR_{bb}R_{cc}$, $-NR_{aa}C(O)R_{bb}$, $-NR_{aa}C(O)NR_{bb}R_{cc}$, $-NR_{aa}C(O)NR_{bb}(CH_2)_nR_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)nS(O)_mR_{aa}$ and $-(CH_2)_nNR_{aa}S(O)_mR_{bb}$, wherein the alkyl, haloalkyl, cycloalkyl, heterocyclyl, aryl and heteroaryl can be each optionally further substituted;
$R_{aa}$, $R_{bb}$ and $R_{cc}$ are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl can be each optionally further substituted; and
x is an integer from 0 to 4;
n is an integer from 0 to 4;
m is an integer from 0 to 2.

In a preferred embodiment of the present invention, ring A is selected from the group consisting of $C_{6-14}$ aryl and 5 to 14 membered heteroaryl.
In a further preferred embodiment of the present invention, ring A is selected from the group consisting of phenyl and benzoheteroaryl.
In a further preferred embodiment of the present invention, ring A is selected from the group consisting of phenyl, quinolyl and quinoxalyl.
In a further preferred embodiment of the present invention, ring A is selected from the group consisting of 3 to 12 membered heterocyclyl and 5 to 14 membered heteroaryl; preferably selected from the group consisting of 3 to 8 membered heterocyclyl and 5 to 10 membered heteroaryl; and more preferably selected from the group consisting of 1,4-dihydropyridyl and pyridyl.
In a preferred embodiment of the present invention, $R_1$ is selected from the group consisting of hydrogen atom, deuterium atom, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-(CH_2)_nR_{aa}$.
In a further preferred embodiment of the present invention, $R_1$ is selected from the group consisting of hydrogen, $C_{1-6}$ alkyl, $C_{3-8}$ cycloalkyl and $-(CH_2)_nR_{aa}$.
In a further preferred embodiment of the present invention, $R_1$ is selected from the group consisting of methyl, cyclopropyl and $-(CH_2)_nR_{aa}$.
In a further preferred embodiment of the present invention, $R_1$ is selected from the group consisting of hydrogen,

substituted or unsubstituted $C_{1-6}$ alkyl, substituted or unsubstituted $C_{3-8}$ cycloalkyl and $-(CH_2)_nR_{aa}$, preferably selected from the group consisting of hydrogen, substituted or unsubstituted $C_{1-3}$ alkyl, substituted or unsubstituted $C_{3-6}$ cycloalkyl and $-(CH_2)_nR_{aa}$, more preferably selected from the group consisting of hydrogen, $C_{1-3}$ alkyl substituted by one or more of deuterium, fluorine, chlorine, bromine and hydroxy, $C_{3-6}$ cycloalkyl substituted by one or more of deuterium, fluorine, chlorine, bromine and hydroxy, and $-(CH_2)_nR_{aa}$, and further preferably selected from the group consisting of hydrogen, methyl, ethyl, propyl, butyl, tri-deuterated methyl, methyl substituted by one to three of fluorine, methyl substituted by one to three of chlorine, methyl substituted by one to three of bromine, cyclopropyl, cyclobutyl, cyclopentyl, cyclohexyl,

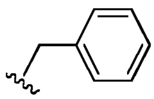 , 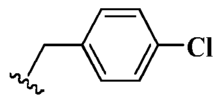

and

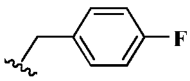 .

In a preferred embodiment of the present invention, $R_1$ is substituted or unsubstituted 3 to 12 membered heterocyclyl, preferably substituted or unsubstituted $C_{3-8}$ heterocyclyl, more preferably $C_{3-6}$ heterocyclyl, and further preferably oxetane.

In a preferred embodiment of the present invention, $R_2$ is selected from the group consisting of hydrogen, deuterium atom, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-C{\equiv}CR_{aa}$, $-NR_{aa}C(O)CH{=}CH(CH_2)_nR_{bb}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$.

In a further preferred embodiment of the present invention, $R_2$ is selected from the group consisting of hydrogen, cyano, azido, $C_{1-6}$ alkyl, $C_{2-6}$ alkenyl, $C_{2-6}$ alkynyl, 3 to 12 membered heterocyclyl, 5 to 14 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$ and $-(CH_2)_nP(O)R_{aa}R_{bb}$, which is optionally further substituted.

In a further preferred embodiment of the present invention, $R_2$ is selected from the group consisting of hydrogen, halogen, cyano, azido, 1 to 6 membered alkoxycarbonyl, $C_{1-6}$ alkyl, $C_{1-6}$ haloalkyl, $C_{2-6}$ alkenyl, $C_{2-6}$ alkynyl, substituted or unsubstituted 3 to 12 membered heterocyclyl, substituted or unsubstituted $C_{6-14}$ aryl, substituted or unsubstituted 5 to 14 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)nS(O)_mR_{aa}$ and $-(CH_2)_nP(O)R_{aa}R_{bb}$, preferably hydrogen, halogen, cyano, azido, 1 to 6 membered alkoxycarbonyl, $C_{1-3}$ alkyl, $C_{1-3}$ haloalkyl, $C_{2-5}$ alkenyl, $C_{2-5}$ alkynyl, substituted or unsubstituted 3 to 10 membered heterocyclyl, substituted or unsubstituted $C_{6-12}$ aryl, substituted or unsubstituted 5 to 12 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)_nS(O)_mR_{aa}$ and $-(CH_2)_nP(O)R_{aa}R_{bb}$, more preferably selected from the group consisting of hydrogen, halogen, cyano, azido, 1 to 3 membered alkoxycarbonyl, $C_{1-3}$ alkyl, $C_{1-3}$ haloalkyl, $C_{2-5}$ alkenyl, $C_{2-5}$ alkynyl, substituted or unsubstituted 3 to 8 membered heterocyclyl, substituted or unsubstituted $C_{6-10}$ aryl, substituted or unsubstituted 5 to 10 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)nS(O)_mR_{aa}$ and $-(CH_2)_nP(O)R_{aa}R_{bb}$, and further preferably selected from the group consisting of hydrogen, fluorine, chlorine, bromine, azido, ethynyl, propynyl, propargyl, cyano, methyl, ethyl, propyl, trifluoromethyl, $-S(O)_2CH_3$, $-S(O)_2CH(CH_3)_2$, $-P(O)(CH_3)_2$, $-C(O)N(CH_3)_2$, $-C(O)OCH_3$, $-C(O)OCH_2CH_3$, $-C(O)OCH(CH_3)_2$,

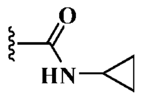 , 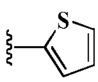 , 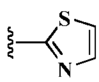 , 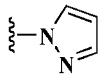 , 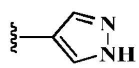 , 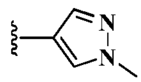 , 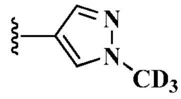 ,

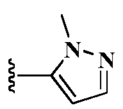 , 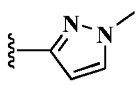 , 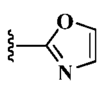 , 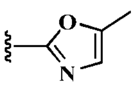 , 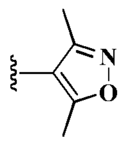 , 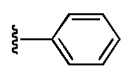 , 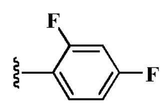 ,

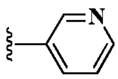 , 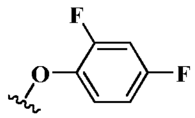 , 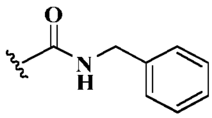 , 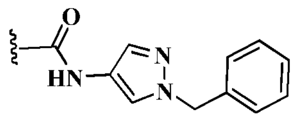 ,

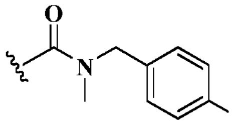 , 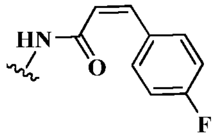 , 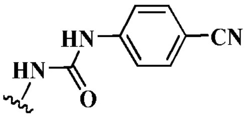

and

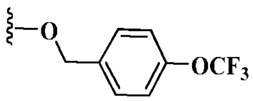 .

In a preferred embodiment of the present invention, $R_2$ is selected from the group consisting of $C_{1-6}$ hydroxyalkyl, substituted or unsubstituted 5 to 12 membered heteroaryl and

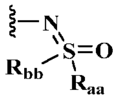

; preferably selected from the group consisting of $C_{1-3}$ hydroxyalkyl, substituted or unsubstituted 5 to 10 membered heteroaryl and

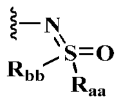 ;

and more preferably selected from the group consisting of

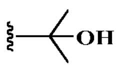 , 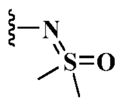 ,

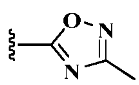

and

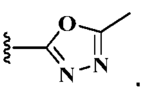 .

In a preferred embodiment of the present invention, $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}(CH_2)_nR_{bb}$, $-NR_{aa}(CH_2)_nNR_{bb}R_{cc}$, $-NR_{aa}C(O)C\equiv CR_{bb}$,$-NR_{aa}C(O)NR_{bb}(CH_2)_nR_{cc}$, $-C\equiv CR_{aa}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and

$-(CH_2)_nNR_{aa}S(O)_mR_{bb}$.

In a further preferred embodiment of the present invention, $R_3$ is selected from the group consisting of hydrogen, $C_{1-6}$ alkyl, $C_{2-6}$ alkenyl, $C_{2-6}$ alkynyl, 3 to 12 membered heterocyclyl, 5 to 14 membered heteroaryl, $-NR_{aa}(CH_2)_nR_{bb}$, $-NR_{aa}(CH_2)_nNR_{bb}R_{cc}$, $-NR_{aa}C(O)C{\equiv}CR_{bb}$,$-NR_{aa}C(O)NR_{bb}(CH_2)_nR_{cc}$, $-C{\equiv}CR_{aa}$, $-NR_{aa}C(O)CH{=}CH(CH_2)_nR_{bb}$, $-NR_{aa}C(O)CH{=}CH(CH_2)_nNR_{bb}R_{cc}$-$C(O)NR_{aa}(CH_2)_nR_{bb}$, $-C(O)OR_{aa}$, $-O(CH_2)_nR_{aa}$ and $-P(O)R_{aa}R_{bb}$.

In a preferred embodiment of the present invention, $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-6}$ cycloalkyl, 3 to 12 membered heterocyclyl, $C_{6-14}$ aryl, 5 to 14 membered heteroaryl, $-NR_eC(O)C{=}CR_f$ and $-NR_eC(O)CH{=}CH(CH_2)_nR_f$,
preferably, $R_3$ is selected from the group consisting of $C_{1-3}$ alkoxy, $C_{1-3}$ alkoxy substituted by halogen, $-NHC(O)C{=}CCH_3$, $-NHC(O)CH{=}CH_2$, $-NHC(O)CH{=}CHCH_3$, $-NHC(O)CH{=}CHCH_2N(CH_3)_2$,

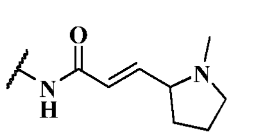 , 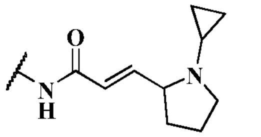 ,

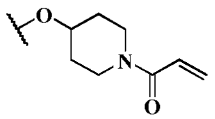 , 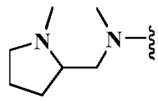 , 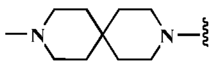 , 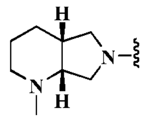 , 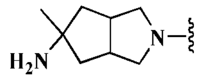 ,

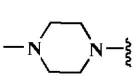 , 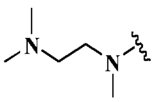 , 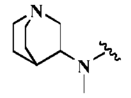 , 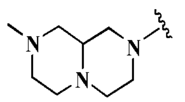 , 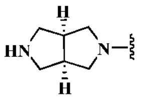 , 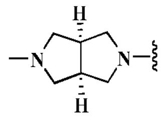 ,

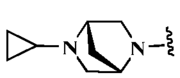 , 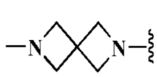 , 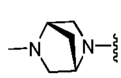 , 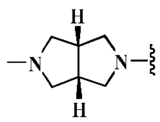 , 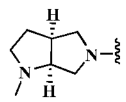 , 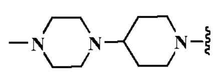 ,

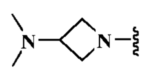 , 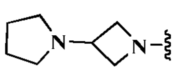 , 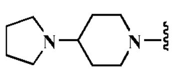 , 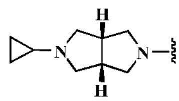 , 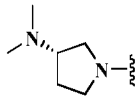 , 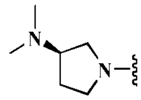 ,

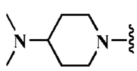 , 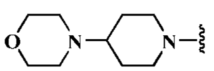

and

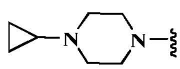 ,

which is optionally further substituted,
wherein, $R_e$ and $R_f$ are each independently selected from the group consisting of hydrogen, deuterium atom, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom.

In a preferred embodiment of the present invention, $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-6}$ cycloalkyl, 3 to 12 membered heterocyclyl, $C_{6-14}$ aryl, 5 to 14 membered heteroaryl, $-OR_e$, $-NR_e(CH_2)_nR_f$,

$-NR_eC(O)C{\equiv}CR_f$, $-NHC(O)CR_e{=}CH(CH_2)_nR_f$ and $-NR_eC(O)CH{=}CH(CH_2)_nR_f$, preferably hydrogen, deuterium, halogen, cyano, amino, nitro, hydroxy, azido, $C_{1-3}$ alkyl, $C_{2-5}$ alkenyl, $C_{2-5}$ alkynyl, $C_{1-3}$ alkoxy, $C_{1-3}$ hydroxyalkyl, $C_{3-6}$ cycloalkyl, 3 to 10 membered heterocyclyl, $C_{6-12}$ aryl, 5 to 12 membered heteroaryl, $-OR_e$, $-NR_e(CH_2)_nR_f$, $-NR_eC(O)C{\equiv}CR_f$, $-NHC(O)CR_e{=}CH(CH_2)_nR_f$ and $-NR_eC(O)CH{=}CH(CH_2)_nR_f$, more preferably hydrogen, deuterium, halogen, cyano, amino, nitro, hydroxy, azido, $C_{1-3}$ alkyl, $C_{2-5}$ alkenyl, $C_{2-5}$ alkynyl, $C_{1-3}$alkoxy, $C_{1-3}$ hydroxyalkyl, $C_{3-6}$ cycloalkyl, 3 to 8 membered heterocyclyl, $C_{6-10}$ aryl, 5 to 10 membered heteroaryl, $-OR_e$, $-NR_e(CH_2)_nR_f$, $-NR_eC(O)C{\equiv}CR_f$, $-NHC(O)CR_e{=}CH(CH_2)_nR_f$ and $-NR_eC(O)CH{=}CH(CH_2)_nR_f$, and further preferably methyl, ethyl, propyl, methoxy, ethoxy, propoxy, methoxy substituted by 1 to 3 fluorine, methoxy substituted by 1 to 3 chlorine, methoxy substituted by 1 to 3 bromine, $-NHC(O)C{=}CH$, $-NHC(O)C{=}CCH_3$, $-NHC(O)CH{=}CH_2$, $-NHC(O)CF{=}CH_2$, $-NHC(O)C(CH_3){=}CH_2$, $-NHC(O)CH{=}CHCH_3$, $-NHC(O)CH{=}CHCH_2N(CH_3)_2$,

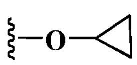 , 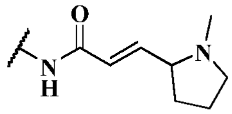 , 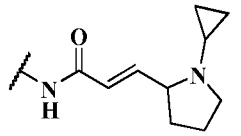 , 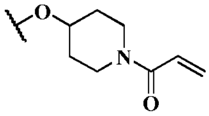 , 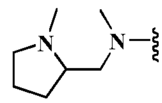 ,

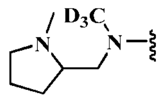 , 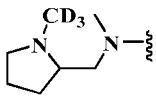 , 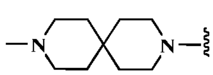 , 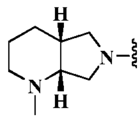 , 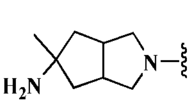 , 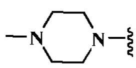 ,

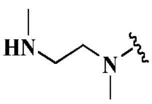 , 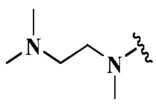 , 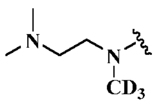 , 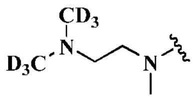 , 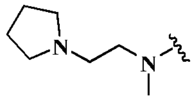 , 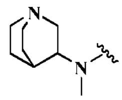 ,

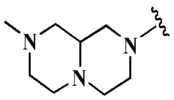 , 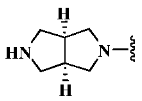 , 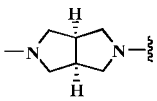 , 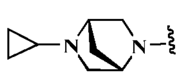 , 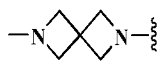 , 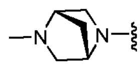 ,

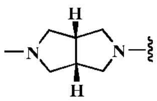 , 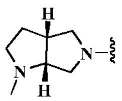 , 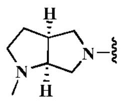 , 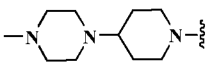 , 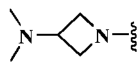 , 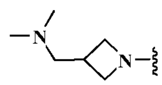 ,

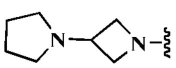 , 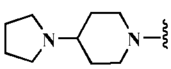 , 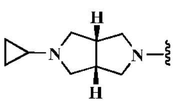 , 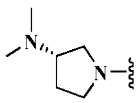 , 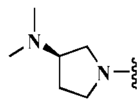 , 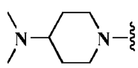 ,

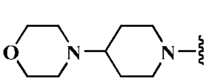 , 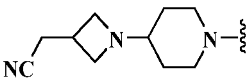 , 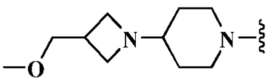

and

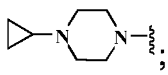 ;

wherein, $R_e$ and $R_f$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom.

In a preferred embodiment of the present invention, $R_3$ is selected from the group consisting of substituted or unsubstituted 3 to 12 membered heterocyclyl, $-O(CH_2)_nR_e$ and $-O(CH_2)_nNR_eR_f$; preferably selected from the group

consisting of substituted or unsubstituted 3 to 8 membered heterocyclyl, $-O(CH_2)_nR_e$ and $-O(CH_2)_nNR_eR_f$; and more preferably selected from the group consisting of

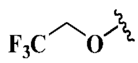, 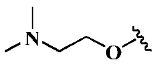

and

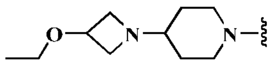;

$R_e$ and $R_f$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom; wherein the $C_{1-6}$ alkyl, $C_{1-6}$ alkoxy, $C_{3-6}$ cycloalkyl and $C_{3-6}$ heterocyclyl containing 1 to 2 nitrogen atom are each optionally substituted by one or more substituents selected from the group consisting of hydrogen, deuterium, halogen, cyano, amino, nitro, hydroxy, $C_{1-6}$ alkyl, $C_{1-6}$ alkoxy, $C_{1-6}$ hydroxyalkyl, $C_{3-6}$ cycloalkyl, $C_{3-6}$ heterocyclyl and $-C(O)CH=CR_gR_h$;

$R_g$ and $R_h$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl.

In a preferred embodiment of the present invention, $R_3$ is selected from the group consisting of substituted or unsubstituted 3 to 12 membered heterocyclyl; preferably selected from the group consisting of substituted or unsubstituted 3 to 8 membered heterocyclyl; and more preferably is

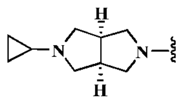.

In a preferred embodiment of the present invention, $R^1$ is selected from the group consisting of hydrogen, deuterium, halogen, amino, nitro, hydroxy, cyano, $C_{1-6}$ alkyl, $C_{2-6}$ alkenyl, $C_{2-6}$ alkynyl, deuterated $C_{1-6}$ alkyl, $C_{1-6}$ haloalkyl, $C_{1-6}$ hydroxyalkyl, $C_{1-6}$ alkoxy, $C_{1-6}$ haloalkoxy, $C_{3-8}$ cycloalkyl, 3 to 12 membered heterocyclyl, $C_{6-14}$ aryl and 5 to 14 membered heteroaryl; preferably selected from the group consisting of hydrogen, deuterium, halogen, amino, hydroxy, cyano, oxo, thioxo, $C_{1-3}$alkyl, $C_{2-5}$ alkenyl, $C_{2-5}$ alkynyl, deuterated $C_{1-3}$ alkyl, $C_{1-3}$ haloalkyl, $C_{1-3}$ hydroxyalkyl, $C_{1-3}$ alkoxy, $C_{1-3}$ haloalkoxy, $C_{3-6}$ cycloalkyl, 3 to 10 membered heterocyclyl, $C_{6-12}$ aryl and 5 to 12 membered heteroaryl; more preferably selected from the group consisting of hydrogen, deuterium, halogen, amino, hydroxy, cyano, oxo, thioxo, $C_{1-3}$ alkyl, $C_{2-5}$ alkenyl, $C_{2-5}$ alkynyl, deuterated $C_{1-3}$ alkyl, $C_{1-3}$ haloalkyl, $C_{1-3}$hydroxyalkyl, $C_{1-3}$ alkoxy, $C_{1-3}$ haloalkoxy, $C_{3-6}$ cycloalkyl, 3 to 8 membered heterocyclyl containing 1 to 3 atoms selected from the group consisting of N, O or and S, $C_{6-10}$ aryl and 5 to 10 membered heteroaryl containing 1 to 3 atoms selected from the group consisting of N, O or and S; and further preferably selected from the group consisting of hydrogen, deuterium, fluorine, chlorine, bromine, amino, hydroxy, cyano, oxo, thioxo, methyl, ethyl, propyl, vinyl, propenyl, allyl, ethynyl, propynyl, propargyl, deuterated methyl, deuterated ethyl, deuterated propyl, fluoromethyl, fluoroethyl, fluoropropyl, chloromethyl, chloroethyl, chloropropyl, bromomethyl, bromoethyl, bromopropyl, hydroxymethyl, hydroxyethyl, hydroxypropyl, methoxy, ethoxy, propoxy, fluoromethoxy, fluoroethoxy, fluoropropoxy, chloromethoxy, chloroethoxy, chloropropoxy, cyclopropyl, cyclobutyl, cyclopentyl, cyclohexyl, cycloheptyl, cyclopropoxy, cyclobutoxy, cyclopentyloxy, cyclohexyloxy, cycloheptyloxy, aziridinyl, azetidinyl, azacyclopentyl, azacyclohexyl, azacycloheptyl, thienyl, pyrrolyl, pyridyl, pyranyl, piperazinyl, phenyl and naphthyl; which is optionally further substituted by one or more substituents selected from the group consisting of deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl;

or, any two of $R^1$ are bonded to form a $C_{3-8}$ cycloalkyl, 3 to 12 membered heterocyclyl, $C_{6-14}$ aryl or 5 to 14 membered heteroaryl; preferably a 3 to 8 membered heterocyclyl or 5 to 10 membered heteroaryl; more preferably a 3 to 8 membered heterocyclyl; and further preferably

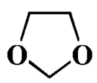

or

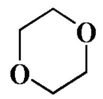

; which is optionally further substituted by one or more substituents selected from the group consisting of deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl.

In a preferred embodiment of the present invention, $R_{aa}$, $R_{bb}$ and $R_{cc}$ are each independently selected from the group consisting of hydrogen, deuterium, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-12}$ aryl and 5 to 12 membered heteroaryl are each optionally further substituted by deuterium, halogen, amino, cyano, $C_{1-6}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

In a preferred embodiment of the present invention, $R_{aa}$, $R_{bb}$ and $R_{cc}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more of deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, substituted or unsubstituted 3 to 12 membered heterocyclyl, $C_{6-14}$ aryl, 5 to 14 membered heteroaryl, $-O(CH_2)_nR_{AA}$ and $-C(O)CH=CHR_{AA}R_{BB}$;

$R_{AA}$ and $R_{BB}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

In a further preferred embodiment of the present invention, a compound of formula (IIa), a stereoisomer thereof or a pharmaceutically acceptable salt thereof is provided, wherein the specific structure thereof is shown as following:

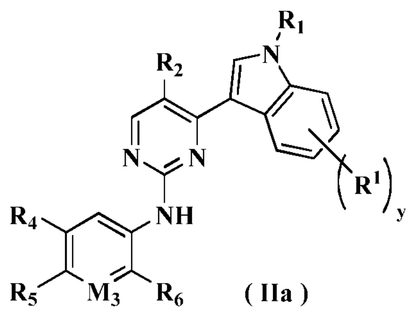
(IIa)

wherein:

$M_3$ is selected from the group consisting of $CR_{13}$ and N;

$R_4$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)R_{bb}$, $-NR_{aa}C(O)NR_{bb}(CH_2)_nR_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$; and preferably selected from the group consisting of $-NR_{aa}C(O)R_{bb}$, $-NR_{aa}C(O)C\equiv CR_{bb}$, $-NR_{aa}C(O)CH=CH(CH_2)_nR_{bb}$ and $-NR_{aa}C(O)CH=CH(CH_2)_nNR_{bb}R_{cc}$;

$R_5$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$, $-O(CH_2)_nNR_{aa}R_{bb}$ and $-NR_{aa}(CH_2)_nNR_{bb}R_{cc}$, wherein

the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of hydrogen, deuterium, halogen, hydroxy, thiol, amino, $C_{1-4}$ alkyl, $C_{1-4}$ haloalkyl, $C_{1-4}$ alkoxy, $C_{1-4}$ haloalkoxy, $C_{2-6}$ alkenylcarbonyl, $C_{1-4}$ hydroxyalkyl, $C_{3-8}$ cycloalkyl, substituted or unsubstituted 3 to 12 membered heterocyclyl and $-(CH_2)_nNR_{dd}R_{ee}$; and preferably $R_5$ is selected from the group consisting of hydrogen, deuterium, halogen, cyano, amino, nitro, hydroxy, $C_{1-3}$ alkyl, $C_{2-4}$ alkenyl, $C_{2-4}$ alkynyl, $C_{1-3}$ alkoxy, $C_{1-3}$hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$, $-O(CH_2)_nNR_{aa}R_{bb}$ and $-NR_{aa}(CH_2)_nNR_{bb}R_{cc}$, wherein the $C_{1-3}$alkyl, $C_{2-4}$ alkenyl, $C_{2-4}$ alkynyl, $C_{1-3}$ alkoxy, $C_{1-3}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of hydrogen, deuterium, halogen, hydroxy, thiol, amino, $C_{1-3}$ alkyl, $C_{1-3}$ haloalkyl, $C_{1-3}$ alkoxy, $C_{1-3}$ haloalkoxy, $C_{2-4}$ alkenylcarbonyl, $C_{1-3}$ hydroxyalkyl, $C_{3-8}$ cycloalkyl, substituted or unsubstituted 3 to 12 membered heterocyclyl and $-(CH_2)_nNR_{dd}R_{ee}$;

$R_6$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-O(CH_2)_nR_{aa}$, preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 10 membered heterocyclyl, $C_{6-12}$ aryl, 5 to 12 membered heteroaryl and $-O(CH_2)_nR_{aa}$, and more preferably selected from the group consisting of hydrogen, deuterium, halogen, cyano, amino, hydroxy, $C_{1-3}$alkyl, $C_{2-5}$ alkenyl, $C_{2-5}$ alkynyl, $C_{1-3}$ alkoxy, $C_{1-3}$ haloalkoxy, $C_{1-3}$hydroxyalkyl, $C_{3-6}$ cycloalkyl, 3 to 8 membered heterocyclyl, $C_{6-10}$ aryl, 5 to 10 membered heteroaryl and $-O(CH_2)_nR_{aa}$, which are optionally further substituted by one or more substituents selected from the group consisting of deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl;

$R_{13}$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl, preferably selected from the group consisting of hydrogen, deuterium, halogen, cyano, amino, hydroxy, $C_{1-3}$alkyl, $C_{2-5}$ alkenyl, $C_{2-5}$ alkynyl, $C_{1-3}$ alkoxy, $C_{1-3}$haloalkoxy, $C_{1-3}$ hydroxyalkyl, $C_{3-6}$ cycloalkyl, 3 to 10 membered heterocyclyl, $C_{6-12}$ aryl and 5 to 12 membered heteroaryl, and more preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 8 membered heterocyclyl, $C_{6-10}$ aryl and 5 to 10 membered heteroaryl, which are optionally further substituted by one or more substituents selected from the group consisting of deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl;

$R_{dd}$ and $R_{ee}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

In a further preferred embodiment of the present invention, a compound of formula (II), a stereoisomer thereof or a pharmaceutically acceptable salt thereof is provided, wherein the specific structure thereof is as following:

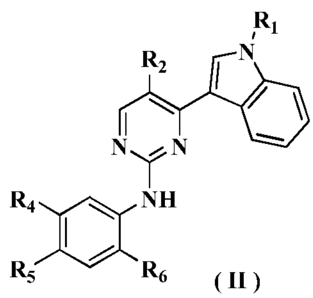
(II)

wherein:

$R_4$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{\equiv}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$;

$R_5$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of halogen, hydroxy, thiol, $C_{1-4}$ alkyl, $C_{1-4}$ haloalkyl, $C_{1-4}$ alkoxy, $C_{1-4}$ hydroxyalkyl and $C_{3-8}$ cycloalkyl;

$R_6$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl, which are optionally further substituted by one or more substituents selected from the group consisting of deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl.

In a further preferred embodiment of the present invention, $R_2$ is selected from the group consisting of hydrogen, halogen, cyano, azido, alkoxycarbonyl, $C_{1-6}$ alkyl, halo$C_{1-6}$ alkyl, $C_{2-6}$ alkenyl, $C_{2-6}$ alkynyl, 3 to 12 membered heterocyclyl, $C_{6-14}$ aryl, substituted or unsubstituted 5 to 14 membered heteroaryl, $-NR_aC(O)NR_c(CH_2)_nR_b$, $-C{\equiv}CR_a$, $-NR_aC(O)CH{=}CHR_b$, $-C(O)NR_a(CH_2)_nR_b$, $-C(O)OR_a$, $-O(CH_2)_nR_a$, $-(CH_2)_nS(O)_mR_a$, $-(CH_2)_nS(O)_mNR_aR_b$ and $-(CH_2)_nP(O)R_aR_b$, wherein the heterocyclyl is selected from 5 to 8 membered heteroaryl containing 1 to 2 atoms selected from the group consisting of nitrogen, oxygen and sulfur atom, which is optionally substituted by hydroxy, halogen, $C_{1-6}$ alkyl or $C_{1-6}$ alkoxy,

preferably, the heterocyclyl is a 5 to 6 membered heteroaryl containing 1 to 2 atoms selected from the group consisting of nitrogen, oxygen and sulfur atom, which is optionally substituted by hydroxy, halogen, $C_{1-6}$ alkyl or $C_{1-6}$ alkoxy, further selected from the group consisting of:

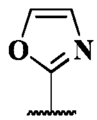 , 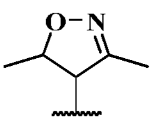 , 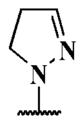 , 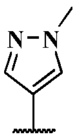 , 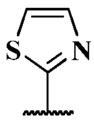 , 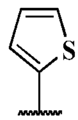 , 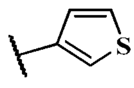 , 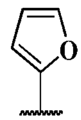 ,

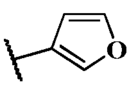 , 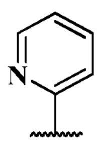 , 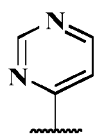

and

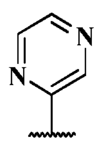 ,

, which is optionally substituted by hydroxy, halogen, $C_{1-6}$ alkyl or $C_{1-6}$ alkoxy;

wherein, $R_a$, $R_b$ and $R_c$ are each independently selected from the group consisting of hydrogen, $C_{1-6}$ alkyl, $C_{3-6}$ cycloalkyl, 3 to 12 membered heterocyclyl, $C_{6-14}$ aryl and 5 to 14 membered heteroaryl, which is optionally further substituted by one or more of halogen, hydroxy, $C_{1-6}$ alkyl, $C_{3-6}$ cycloalkyl, 3 to 12 membered heterocyclyl, $C_{6-14}$ aryl and 5 to 14 membered heteroaryl.

In a further preferred embodiment of the present invention, $R_4$ is selected from the group consisting of $-NR_cC(O)R_d$, $-NR_cC(O)C{\equiv}CR_d$ and $-NR_cC(O)CH{=}CH(CH_2)_nR_d$; wherein $R_c$ and $R_d$ are each independently selected from the group consisting of hydrogen, $C_{1-6}$ alkyl, $C_{2-6}$ alkenyl, $C_{2-6}$ alkynyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

In a further preferred embodiment of the present invention, $R_4$ is selected from the group consisting of $-NR_cC(O)R_d$, $-NR_cC(O)C{\equiv}CR_d$, $-NR_cC(O)CH{=}CH(CH_2)_nR_d$ and $-NR_cC(O)CRe{=}CH(CH_2)_nR_d$; and preferably selected from the group consisting of $-NHC(O)CH{=}CH_2$, $-NHC(O)CF{=}CH_2$, $-NHC(O)C(CH_3){=}CH_2$, $-NHC(O)C{=}CH$, $-NHC(O)C{=}CCH_3$, $-NHC(O)CH{=}CHCH_3$, $-NHC(O)CH{=}CHCH_2N(CH_3)_2$ and

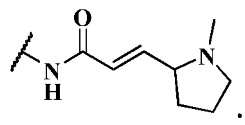.

In a further preferred embodiment of the present invention, $R_5$ is selected from the group consisting of hydrogen atom, $C_{1-6}$ alkyl, 3 to 12 membered heterocyclyl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl and 3 to 12 membered heterocyclyl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl;

$R_5$ is preferably selected from the group consisting of:

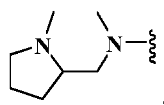, 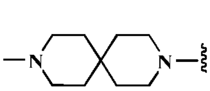, 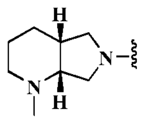, 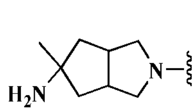, 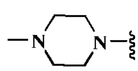, 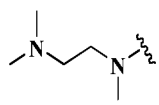,

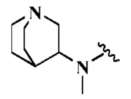, 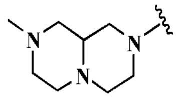, 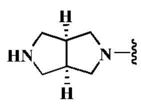, 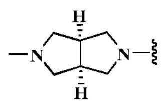, 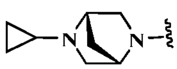, 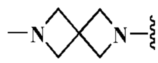,

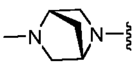, 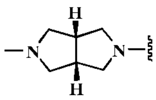, 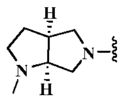, 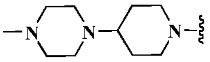, 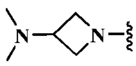, 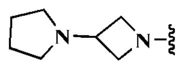,

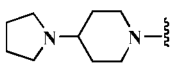, 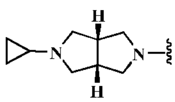, 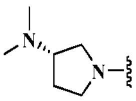, 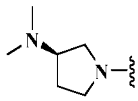, 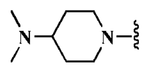, 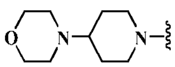

and

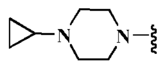.

In a further preferred embodiment of the present invention, $R_5$ is selected from the group consisting of hydrogen, $C_{1-6}$ alkyl, 3 to 12 membered heterocyclyl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl and 3 to 12 membered heterocyclyl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl; and further selected from the group consisting of methyl,

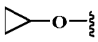, 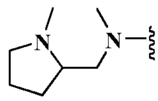, 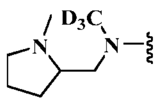, 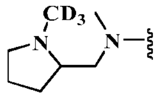, 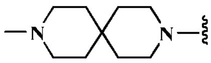,

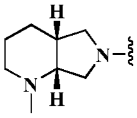, 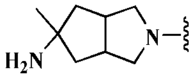, 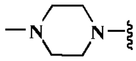, 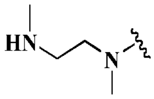, 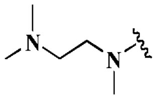, 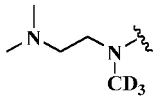,

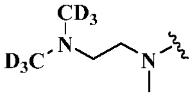, 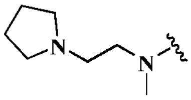, 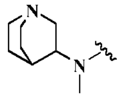, 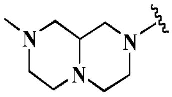, 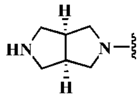, 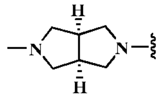,

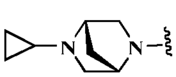, 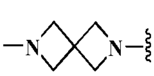, 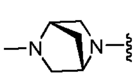, 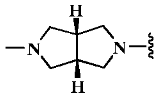, 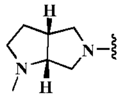, 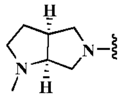,

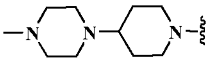, 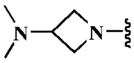, 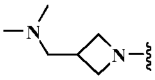, 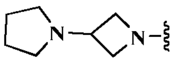, 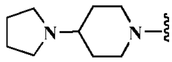, 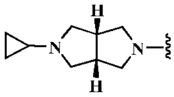,

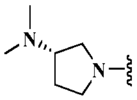, 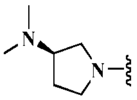, 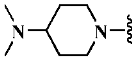, 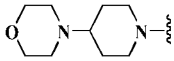, 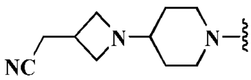,

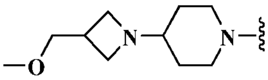, 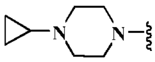, 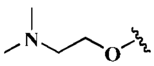

and

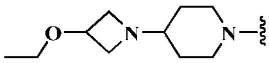.

In a preferred embodiment of the present invention, $R_5$ is selected from the group consisting of substituted or unsubstituted 3 to 12 membered heterocyclyl, and more preferably selected from the group consisting of

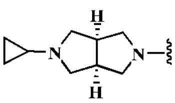

and

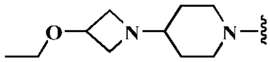.

In a further preferred embodiment of the present invention, $R_6$ is selected from the group consisting of hydrogen atom, deuterium atom, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl.

In a further preferred embodiment of the present invention, $R_6$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-O(CH_2)_nR_{aa}$, preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 10 membered heterocyclyl, $C_{6-12}$ aryl, 5 to 12 membered heteroaryl and $-O(CH_2)_nR_{aa}$, more preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 8 membered heterocyclyl, $C_{6-10}$ aryl, 5 to 10 membered heteroaryl and $-O(CH_2)_nR_{aa}$, and further preferably selected from the group consisting of hydrogen, deuterium, methyl, ethyl, propyl, methoxy, ethoxy, propoxy, $-OCH_2F$, $-OCHF_2$, $-OCF_3$ and

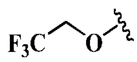.

In a further preferred embodiment of the present invention, a compound of formula (III), a stereoisomer thereof or a pharmaceutically acceptable salt thereof is provided, wherein the specific structure thereof is as following:

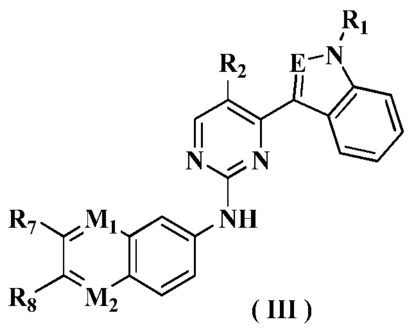

(III)

wherein:

$M_1$ and $M_2$ are each independently selected from the group consisting of $CR_9$ and N;
$R_7$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{\equiv}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)nS(O)_mR_{aa}$ and $-(CH_2)_nNR_{aa}S(O)_mR_{bb}$;
$R_8$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of halogen, hydroxy, thiol, $C_{1-6}$ alkyl, $C_{1-6}$ haloalkyl, $C_{1-6}$ alkoxy, $C_{1-6}$ hydroxyalkyl and $C_{3-8}$ cycloalkyl;
$R_9$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{\equiv}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$.

In a further preferred embodiment of the present invention, a compound of formula (IV), a stereoisomer thereof or a pharmaceutically acceptable salt thereof is provided, wherein the specific structure thereof is as following:

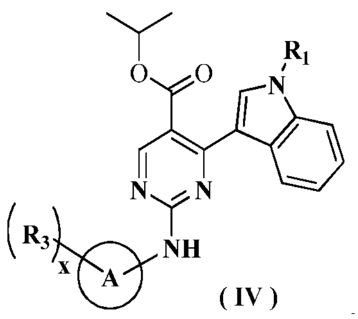

(IV)

In a further preferred embodiment of the present invention, a compound of formula (V), a stereoisomer thereof or a pharmaceutically acceptable salt thereof is provided, wherein the specific structure thereof is as following:

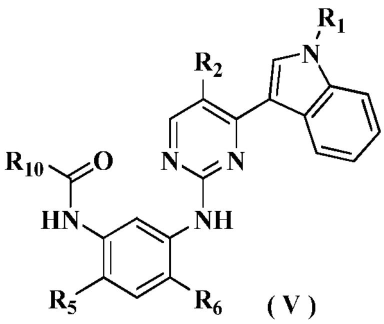

(V)

wherein:

$R_{10}$ is selected from the group consisting of hydrogen atom, deuterium atom, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-C \equiv CR_{aa}$, $-CH=CH(CH_2)_nR_{aa}$, $-CR_{aa}=CH(CH_2)_nR_{bb}$ and $-CH=CH(CH_2)_nNR_{aa}R_{bb}$, and preferably $R_{10}$ is vinyl.

In a further preferred embodiment of the present invention, a compound of formula (VI), a stereoisomer thereof or a pharmaceutically acceptable salt thereof is provided, wherein the specific structure thereof is as following:

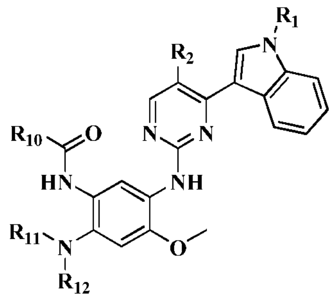

(VI)

wherein:

$R_{11}$ and $R_{12}$ are each independently selected from the group consisting of hydrogen, deuterium, halogen, cyano, amino, nitro, hydroxy, substituted or unsubstituted $C_{1-6}$ alkyl, $C_{2-6}$ alkenyl, $C_{2-6}$ alkynyl, $C_{1-6}$ alkoxy, $C_{1-6}$ hydroxyalkyl, substituted or unsubstituted $C_{3-8}$ cycloalkyl, 3 to 12 membered heterocyclyl, $C_{6-14}$ aryl, 5 to 14 membered heteroaryl, $-(CH_2)_nR_{aa}$ and $-(CH_2)_nNR_{aa}R_{bb}$, preferably selected from the group consisting of hydrogen, deuterium, halogen, cyano, amino, nitro, hydroxy, substituted or unsubstituted $C_{1-3}$ alkyl, $C_{2-5}$ alkenyl, $C_{2-5}$ alkynyl, $C_{1-3}$ alkoxy, $C_{1-3}$ hydroxyalkyl, substituted or unsubstituted $C_{3-6}$ cycloalkyl, 3 to 10 membered heterocyclyl, $C_{6-12}$ aryl, 5 to 12 membered heteroaryl, $-(CH_2)_nR_{aa}$ and $-(CH_2)_nNR_{aa}R_{bb}$, more preferably selected from the group consisting of hydrogen, deuterium, halogen, cyano, amino, nitro, hydroxy, $C_{1-3}$ alkyl substituted by one or more substituents selected from the group consisting of deuterium, fluorine, chlorine and bromine atom, $C_{2-5}$ alkenyl, $C_{2-5}$ alkynyl, $C_{1-3}$ alkoxy, $C_{1-3}$hydroxyalkyl, $C_{3-6}$ cycloalkyl substituted by one or more substituents selected from the group consisting of

deuterium, fluorine, chlorine and bromine atom, 3 to 10 membered heterocyclyl containing 1 to 3 atoms selected from the group consisting of N, O or and S, $C_{6-12}$ aryl, 5 to 12 membered heteroaryl containing 1 to 3 atoms selected from the group consisting of N, O or and S, $-(CH_2)_nR_{aa}$ and $-(CH_2)_nNR_{aa}R_{bb}$, and further preferably selected from the group consisting of hydrogen, methyl, ethyl, propyl, tri-deuterated methyl, $-CH_2F$, $-CHF_2$, $-CF_3$,

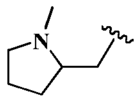 , 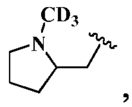 , 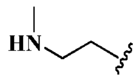 ,

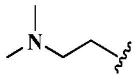 , 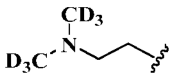 , 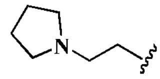

and

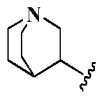 ;

or, $R_{11}$ and $R_{12}$, together with the nitrogen atom to which they are attached, form a 3 to 12 membered heterocyclyl or 5 to 10 membered heteroaryl, wherein the 3 to 12 membered heterocyclyl or 5 to 10 membered heteroaryl is optionally substituted by one or more substituents selected from the group consisting of hydrogen, deuterium, halogen, amino, hydroxy, cyano, nitro, oxo, $C_{1-6}$ alkyl, $C_{2-6}$ alkenyl, $C_{2-6}$ alkynyl, deuterated $C_{1-6}$ alkyl, $C_{1-6}$ haloalkyl, $C_{1-6}$ alkoxy, $C_{1-6}$ haloalkoxy, $C_{1-6}$ hydroxyalkyl, $C_{3-12}$ cycloalkyl, substituted or unsubstituted 3 to 12 membered heterocyclyl, $C_{6-14}$ aryl, 5 to 14 membered heteroaryl and $-(CH_2)_nNR_{aa}R_{bb}$; and preferably further form a group selected from the group consisting of:

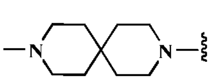 , 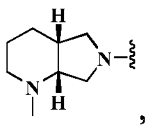 , 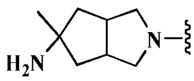 ,

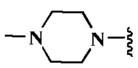 , 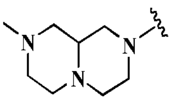 , 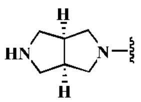 , 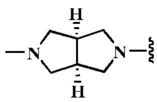 , 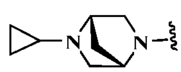 , 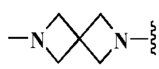 ,

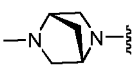 , 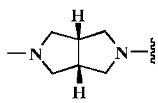 , 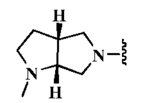 , 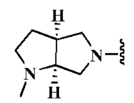 , 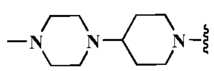 , 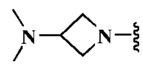 ,

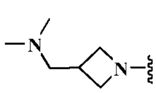 , 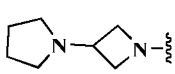 , 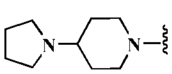 , 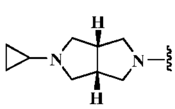 , 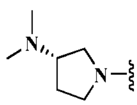 , 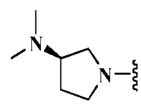 ,

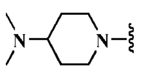 , 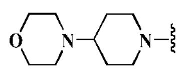 , 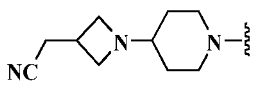 , 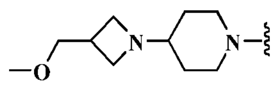

and

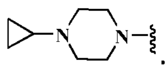

In a preferred embodiment of the present invention, $R_{11}$ and $R_{12}$, together with the nitrogen atom to which they are attached, form a 3 to 12 membered heterocyclyl, which is optionally substituted by one or more substituents selected from the group consisting of $C_{3-12}$ cycloalkyl and substituted or unsubstituted 3 to 12 membered heterocyclyl; and preferably further selected from the group consisting of

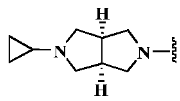

and

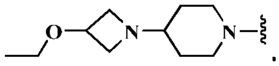

In a preferred embodiment of the present invention, a compound of formula (VII), a stereoisomer thereof or a pharmaceutically acceptable salt thereof is provided, wherein the structure thereof is as following:

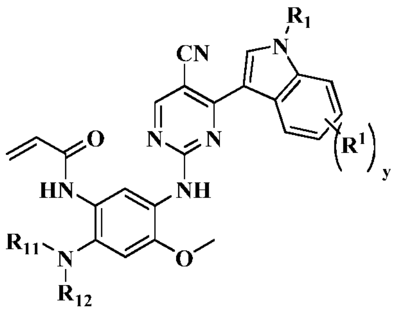

( VII)

wherein:

$R_1$ is selected from the group consisting of hydrogen, deuterium, halogen, amino, nitro, hydroxy, cyano, $C_{1-6}$ alkyl, $C_{3-8}$ cycloalkyl and 3 to 6 membered heterocyclyl, which is optionally further substituted by one or more substituents selected from the group consisting of deuterium, halogen, amino, nitro, hydroxy, cyano, $C_{1-6}$ alkyl, $C_{3-8}$ cycloalkyl and 3 to 6 membered heterocyclyl; preferably, $R_1$ is selected from the group consisting of $C_{1-3}$ alkyl, $C_{3-6}$ cycloalkyl and 3 to 6 membered heterocyclyl containing 1 to 2 atoms selected from the group consisting of N, O or and S, which is optionally further substituted by one or more substituents selected from the group consisting of deuterium, halogen, amino, nitro, hydroxy, cyano, $C_{1-3}$alkyl, $C_{3-6}$ cycloalkyl and 3 to 6 membered heterocyclyl; and more preferably, $R_1$ is selected from the group consisting of methyl, ethyl, propyl, isopropyl, cyclopropyl, cyclobutyl, cyclopentyl, cyclohexyl, cyclopropoxy, cyclobutoxy, tetrahydrofuranyl, pyranyl, aziridinyl, azetidinyl, pyrrolyl, piperidinyl and tetrahydrothienyl, which is optionally further substituted by one or more substituents selected from the group consisting of deuterium, fluorine, chlorine, bromine, amino, nitro, hydroxy, cyano, methyl, ethyl, propyl and isopropyl;

$R_{11}$ and $R_{12}$ are each independently selected from the group consisting of hydrogen, deuterium, halogen, cyano, amino, nitro, hydroxy, $C_{1-6}$ alkyl and $-(CH_2)_nNR_{aa}R_{bb}$;

$R_{aa}$ and $R_{bb}$ are each independently selected from the group consisting of hydrogen, deuterium, halogen, cyano, amino, nitro, hydroxy, $C_{1-6}$ alkyl, $C_{1-6}$ alkoxy and 3 to 6 membered heterocyclyl; and preferably, $R_{aa}$ and $R_{bb}$ are each independently selected from the group consisting of hydrogen, deuterium, halogen, cyano, amino, nitro, hydroxy, $C_{1-3}$ alkyl, $C_{1-3}$ alkoxy and 5 to 6 membered heterocyclyl containing 1 to 2 atoms selected from the group consisting of N and O;

n is an integer of 0, 1, 2 or 3;

or, $R_{11}$ and $R_{12}$, together with the nitrogen atom to which they are attached, form a 4 to 12 membered heterocyclyl,

which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;

each $R^1$ is independently selected from the group consisting of hydrogen, deuterium, halogen, amino, nitro, hydroxy, cyano, $C_{1-6}$ alkyl, $C_{1-6}$ alkoxy, $C_{1-6}$ hydroxyalkyl and $C_{3-8}$ cycloalkyl; and preferably, $R^1$ is selected from the group consisting of hydrogen, deuterium, fluorine, chlorine, bromine, amino, nitro, hydroxy, cyano, $C_{1-3}$ alkyl, $C_{1-3}$ alkoxy, $C_{1-3}$ hydroxyalkyl and $C_{3-6}$ cycloalkyl;

y is an integer of 0, 1, 2 or 3.

In a more preferred embodiment of the present invention, a compound of formula (VIII), a stereoisomer thereof or a pharmaceutically acceptable salt thereof is provided, wherein the structure thereof is as follows:

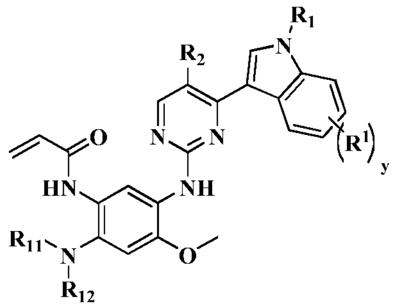

( VIII)

wherein:

$R_2$ is selected from the group consisting of $C_{6-12}$ aryl, 4 to 7 membered heterocyclyl and 4 to 7 membered heteroaryl, which is optionally further substituted by one or more substituents selected from the group consisting of hydroxy, amino, halogen, $C_{1-6}$ alkyl, $C_{1-6}$ hydroxyalkyl and $C_{1-6}$ alkoxy; preferably, $R_2$ is selected from the group consisting of phenyl, 5 to 6 membered heterocyclyl containing 1 to 2 atoms selected from the group consisting of N, O or and S and 5 to 6 membered heteroaryl containing 1 to 2 atoms selected from the group consisting of N, O or and S, which is optionally further substituted by one or more substituents selected from the group consisting of hydroxy, amino, halogen, $C_{1-3}$ alkyl, $C_{1-3}$ hydroxyalkyl and $C_{1-3}$ alkoxy; and more preferably, $R_2$ is selected from the group consisting of:

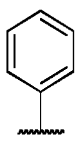, 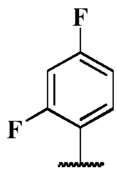, 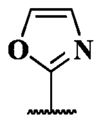, 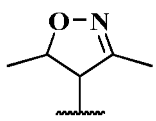, 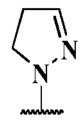, 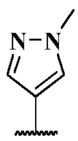, 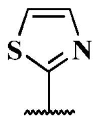, 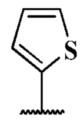, 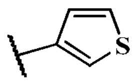,

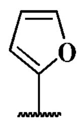, 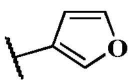, 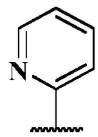, 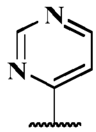

and

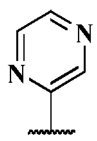,

which is optionally further substituted by one or more substituents selected from the group consisting of hydroxy, amino, halogen, methyl, ethyl, propyl, isopropyl, hydroxymethyl, hydroxyethyl, hydroxypropyl, hydroxyisopropyl, methoxy, ethoxy, propoxy and isopropoxy;

$R_1$ is selected from the group consisting of hydrogen, deuterium, halogen, amino, nitro, hydroxy, cyano, $C_{1-6}$ alkyl, $C_{3-8}$ cycloalkyl and 3 to 6 membered heterocyclyl, which is optionally further substituted by one or more substituents selected from the group consisting of deuterium, halogen, amino, nitro, hydroxy, cyano, $C_{1-6}$ alkyl, $C_{3-8}$ cycloalkyl and 3 to 6 membered heterocyclyl; preferably, $R_1$ is selected from the group consisting of $C_{1-3}$ alkyl, $C_{3-6}$ cycloalkyl and 3 to 6 membered heterocyclyl containing 1 to 2 atoms selected from the group consisting of N, O or and S, which is optionally further substituted by one or more substituents selected from the group consisting of deuterium, halogen, amino, nitro, hydroxy, cyano, $C_{1-3}$ alkyl, $C_{3-6}$ cycloalkyl and 3 to 6 membered heterocyclyl; and more preferably, $R_1$ is selected from the group consisting of methyl, ethyl, propyl, isopropyl, cyclopropyl, cyclobutyl, cyclopentyl, cyclohexyl, cyclopropoxy, cyclobutoxy, tetrahydrofuranyl, pyranyl, aziridinyl, azetidinyl, pyrrolyl, piperidinyl and tetrahydrothienyl, which is optionally further substituted by one or more substituents selected from the group consisting of deuterium, fluorine, chlorine, bromine, amino, nitro, hydroxy, cyano, methyl, ethyl, propyl and isopropyl;

$R_{11}$ and $R_{12}$ are each independently selected from the group consisting of hydrogen, deuterium, halogen, cyano, amino, nitro, hydroxy, $C_{1-6}$ alkyl and $-(CH_2)_nNR_{aa}R_{bb}$;

$R_{aa}$ and $R_{bb}$ are each independently selected from the group consisting of hydrogen, deuterium, halogen, cyano, amino, nitro, hydroxy, $C_{1-6}$ alkyl, $C_{1-6}$ alkoxy and 3 to 6 membered heterocyclyl; and preferably, $R_{aa}$ and $R_{bb}$ are each independently selected from the group consisting of hydrogen, deuterium, halogen, cyano, amino, nitro, hydroxy, $C_{1-3}$ alkyl, $C_{1-3}$ alkoxy and 5 to 6 membered heterocyclyl containing 1 to 2 atoms selected from the group consisting of N and O;

n is an integer of 0, 1, 2 or 3;

or, $R_{11}$ and $R_{12}$, together with the nitrogen atom to which they are attached, form a 4 to 12 membered heterocyclyl, which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;

each $R^1$ is independently selected from the group consisting of hydrogen, deuterium, halogen, amino, nitro, hydroxy, cyano, $C_{1-6}$ alkyl, $C_{1-6}$ alkoxy, $C_{1-6}$ hydroxyalkyl and $C_{3-8}$ cycloalkyl; and preferably, $R^1$ is selected from the group consisting of hydrogen, deuterium, fluorine, chlorine, bromine, amino, nitro, hydroxy, cyano, $C_{1-3}$ alkyl, $C_{1-3}$ alkoxy, $C_{1-3}$ hydroxyalkyl and $C_{3-6}$ cycloalkyl;

n is an integer of 0, 1, 2 or 3.

In a more preferred embodiment of the present invention, $R_{11}$ and $R_{12}$, together with the nitrogen atom to which they are attached, form a 4 to 10 membered heterocyclyl, which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;

preferably, $R_{11}$ and $R_{12}$, together with the nitrogen atom to which they are attached , form a 4 to 8 membered heterocyclyl containing 1 to 2 nitrogen atom, which is optionally substituted by one or more substituents selected from the group consisting of halogen, hydroxy, amino, $C_{1-3}$ alkyl, $C_{1-3}$ alkoxy, amino substituted by $C_{1-3}$ alkyl, $C_{3-6}$ cycloalkyl and substituted or unsubstituted 3 to 6 membered monocyclic heterocyclyl;

preferably further form a group selected from the group consisting of:

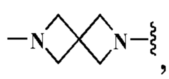,

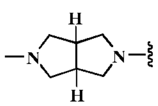, 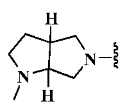, 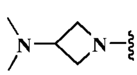, 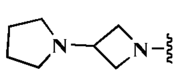, 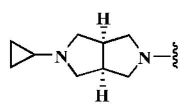

and

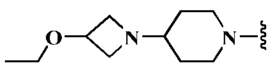,

, which are optionally substituted by one or more substituents selected from the group consisting of halogen, hydroxy,

amino, methyl, ethyl, propyl, isopropyl, methoxy, ethoxy, propoxy, isopropoxy, methylamino, dimethylamino, ethylamino, diethylamino, propylamino, dipropylamino, isopropylamino, diisopropylamino, cyclopropyl, cyclobutyl, cyclopentyl, cyclohexyl and substituted or unsubstituted 3 to 6 membered monocyclic heterocyclyl containing one nitrogen atom.

The present invention also provides a preferred embodiment relating to a method for preparing the compound of formula (V), a stereoisomer thereof or a pharmaceutically acceptable salt thereof, comprising the following steps of:

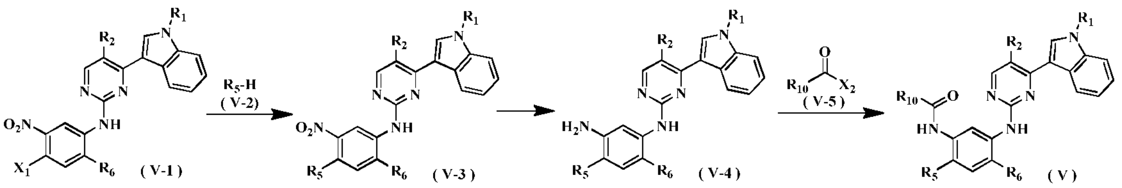


reacting a compound of formula (V-1) with a compound of formula (V-2) to obtain a compound of formula (V-3); then subjecting the compound of formula (V-3) to a reduction reaction to obtain a compound of formula (V-4); then reacting the compound of formula (V-4) with a compound of formula (V-5) to obtain the compound of formula (V), a stereoisomer thereof or a pharmaceutically acceptable salt thereof;
wherein:

$X_1$ is selected from the group consisting of halogen; preferably fluorine, chlorine, bromine or iodine; and more preferably fluorine;
$X_2$ is selected from the group consisting of halogen; preferably fluorine, chlorine, bromine or iodine; and more preferably chlorine.

The present invention also provides a preferred embodiment relating to a method for preparing the compound of formula (V), a stereoisomer thereof or a pharmaceutically acceptable salt thereof, comprising the following step of:

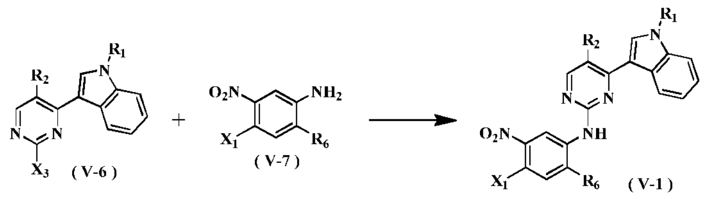

reacting a compound of formula (V-6) with a compound of formula (V-7) to obtain the compound of formula (V-1); then subjecting the compound of formula (V-1) to reactions to obtain the compound of formula (V), a stereoisomer thereof or a pharmaceutically acceptable salt thereof;
wherein:
$X_3$ is selected from the group consisting of halogen; preferably fluorine, chlorine, bromine or iodine; and more preferably chlorine.

In another aspect, the present invention provides a method for preparing the compound of formula (VII), a stereoisomer thereof or a pharmaceutically acceptable salt thereof, characterized by comprising the following steps of:

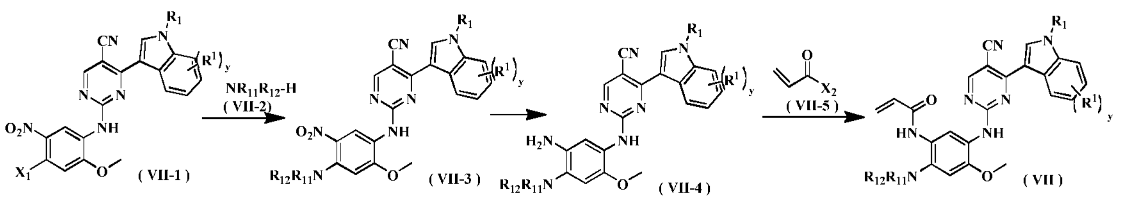


reacting a compound of formula (VII-1) with a compound of formula (VII-2) to obtain a compound of formula (VII-3); then subjecting the compound of formula (VII-3) to a reduction reaction to obtain a compound of formula (VII-4); then reacting the compound of formula (VII-4) with a compound of formula (VII-5) to obtain the compound of formula (VII), a stereoisomer thereof or a pharmaceutically acceptable salt thereof;

wherein:

$X_1$ is selected from the group consisting of halogen; preferably fluorine, chlorine, bromine or iodine; and more preferably fluorine;
$X_2$ is selected from the group consisting of halogen; preferably fluorine, chlorine, bromine or iodine; and more preferably chlorine;
$R^1$, $R_1$, $R_{11}$, $R_{12}$ and y are as defined in formula (VII).

In another aspect, the present invention provides a method for preparing the compound of formula (VIII), a stereoisomer thereof or a pharmaceutically acceptable salt thereof, characterized by comprising the following steps of:

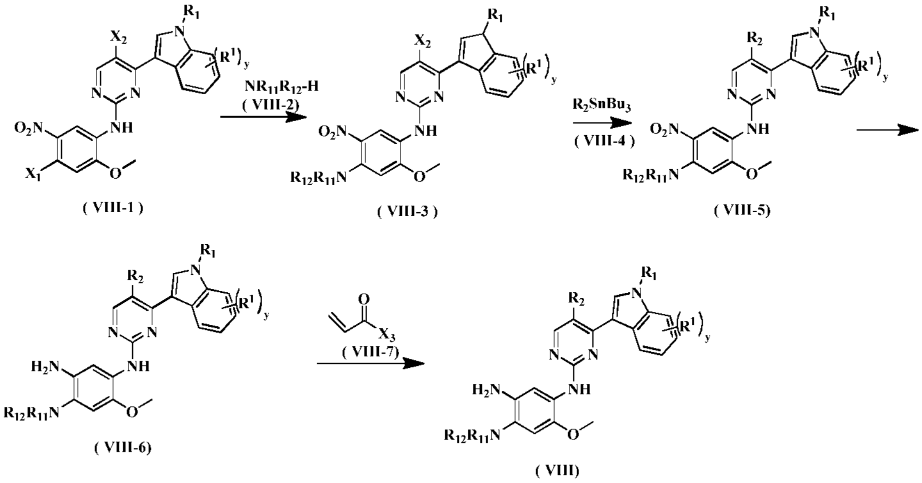


wherein:

$X_1$ is selected from the group consisting of halogen; preferably fluorine, chlorine, bromine or iodine; and more preferably fluorine;
$X_2$ and $X_3$ are each independently selected from the group consisting of halogen; preferably fluorine, chlorine, bromine or iodine; and more preferably bromine;
$R^1$, $R_1$, $R_2$, $R_{11}$, $R_{12}$ and y are as defined in claim 34.
wherein:

$X_1$ is selected from the group consisting of halogen; preferably fluorine, chlorine, bromine or iodine; and more preferably fluorine;
$X_2$ is selected from the group consisting of halogen; preferably fluorine, chlorine, bromine or iodine; and more preferably chlorine;
$R^1$, $R_1$, $R_2$, $R_{11}$, $R_{12}$ and y are as defined in formula (VIII).

The present invention also provides a pharmaceutical composition comprising a therapeutically effective dose of the compound of formula (I), a stereoisomer thereof or a pharmaceutically acceptable salt thereof, and one or more pharmaceutically acceptable carriers, diluents or excipients.

The present invention also provides a preferred embodiment relating to a use of the compound of formula (I), a stereoisomer thereof or a pharmaceutically acceptable salt thereof, or the pharmaceutical composition in the preparation of a kinase inhibitor.

The present invention also provides a preferred embodiment relating to a use of the compound of formula (I), a stereoisomer thereof or a pharmaceutically acceptable salt thereof, or the pharmaceutical composition in the preparation of a receptor tyrosine kinase inhibitor (TKI) medicament.

The present invention also provides a preferred embodiment relating to a use of the compound of formula (I), a stereoisomer thereof or a pharmaceutically acceptable salt thereof, or the pharmaceutical composition in the preparation of a HER2 inhibitor, EGFR inhibitor, EGFR monoclonal antibody or combined medicament thereof; and preferably relating to a use in the preparation of a HER2 exon 20 mutant inhibitor, EGFR exon 20 mutant inhibitor, EGFR exon 20 mutant monoclonal antibody or combined medicament thereof.

The present invention also provides a preferred embodiment relating to a use of the compound of formula (I),

a stereoisomer thereof or a pharmaceutically acceptable salt thereof, or the pharmaceutical composition in the preparation of medicaments for the treatment of cancer-related disease; and the cancer is preferably breast cancer, cervical cancer, colon cancer, lung cancer, stomach cancer, rectal cancer, pancreatic cancer, brain cancer, liver cancer, solid tumor, glioma, glioblastoma, leukemia, lymphoma, myeloma or non-small cell lung cancer.

The present invention further relates to a method for treating a cancer-related disease by the compound of formula (I), a stereoisomer thereof or a pharmaceutically acceptable salt thereof or the pharmaceutical composition thereof.

The present invention also relates to a method for treating a cancer-related disease, comprising a step of administering to a mammal a therapeutically effective amount of the compound or the pharmaceutically acceptable salt, ester, prodrug, solvate, hydrate or derivative thereof according to the present invention.

In some embodiments, the method relates to the treatment of cancer-related disease.

The treatment method provided herein comprises a step of administering to a subject a therapeutically effective amount of the compound of the present invention. In an embodiment, the present invention provides a method for treating a cancer-related disease in a mammal. The method comprises a step of administering to the mammal a therapeutically effective amount of the compound or the pharmaceutically acceptable salt, ester, prodrug, solvate, hydrate or derivative thereof according to the present invention.

The third-generation EGFR inhibitors mainly have a high inhibition against the EGFR activating mutant and T790M resistant mutant. Regarding to the EGFR and/or HER2 exon 20 insertion mutation target, the compound of the present invention shows the following significant advantages compared with the third-generation EGFR inhibitors:

1. significantly improving inhibitory activity on the Ba/F3 EGFR mutant cell line, the activity of the preferred compound is more than 10 times higher, even 20 times higher;
2. improving selectivity in inhibiting the proliferation of the Ba/F3 EGFR mutant cell line and A431 cell line, the activity of the preferred compound is more than 3 times higher, even 10 times higher;
3. showing significant advantages on the *in vivo* tumor inhibition rate in the mouse pro-B cell Ba/F3 EGFR-D770-N771ins_SVD xenograft model.

DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, *n*-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl.

The term "alkylene" refers to an alkyl of which a hydrogen atom is further substituted, for example, "methylene" refers to $-CH_2-$, "ethylene" refers to $-(CH_2)_2-$, "propylene" refers to $-(CH_2)_3-$, "butylene" refers to $-(CH_2)_4-$ and the like.

The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl group can be substituted or unsubstituted. When substituted, the substituent group(s) is preferably one or more groups independently selected from the group consisting of alkyl, alkenyl, alkynyl, alkoxy, alkylthio, alkylamino, halogen,

thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl and cycloheptyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

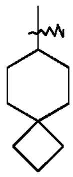 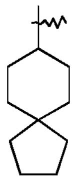 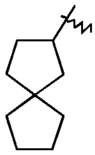 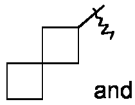 and 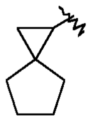

etc.;

and also include spiro cycloalkyl in which a cycloalkyl and a heterocyclyl are connected through one spiro atom, non-limiting examples thereof include:

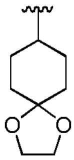 and 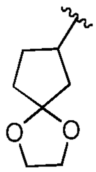

etc.

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered or 5-membered/6-membered bicyclic fused cycloalkyl. Non-limiting examples of fused cycloalkyl include:

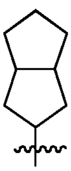 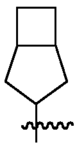 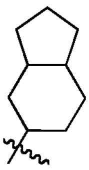 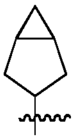 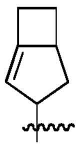 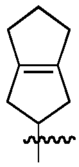 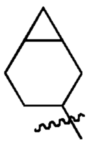 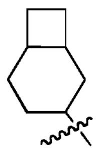

and

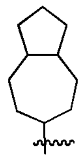

etc.

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated $\pi$-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

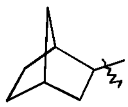 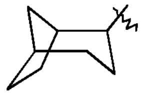 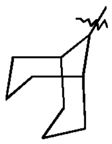 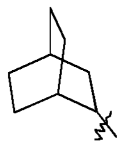 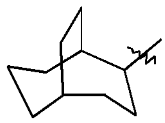

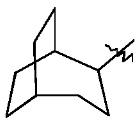 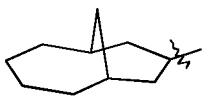 and 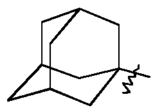 .

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of nitrogen, oxygen or $S(O)_m$ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; most preferably 3 to 8 ring atoms; and further preferably being a 3 to 8 membered heterocyclyl containing 1 to 3 nitrogen atoms. Optionally, the heterocyclyl is substituted by 1 to 2 oxygen atom, sulfur atom or oxo. The heterocyclyl includes nitrogen-containing monocyclic heterocyclyl, nitrogen-containing spiro heterocyclyl and nitrogen-containing fused heterocyclyl.

Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably pyrrolidinyl, piperidinyl and piperazinyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of nitrogen, oxygen or $S(O)_m$ (wherein m is an integer of 0 to 2), with the remaining ring atoms being carbon atoms, and the rings can contain one or more double bonds, but none of the rings has a completely conjugated $\pi$-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

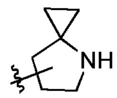 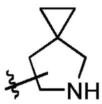 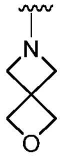 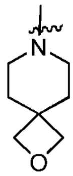 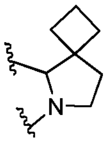 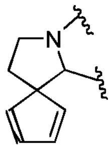 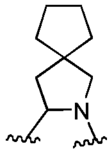 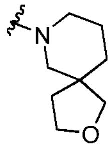

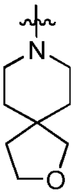 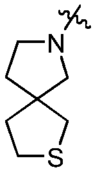 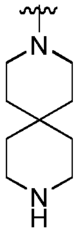 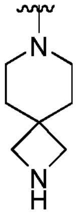 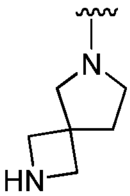 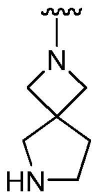 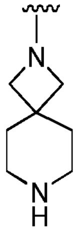

and the like.

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of nitrogen, oxygen and $S(O)_m$ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

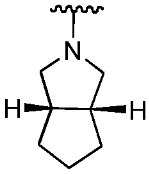 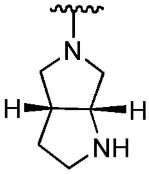 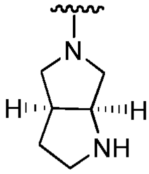 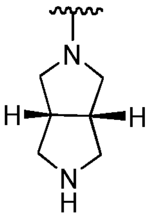 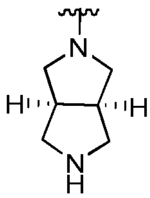 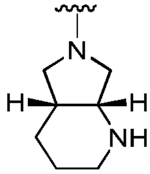 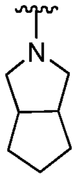

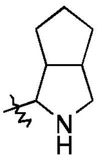 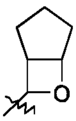 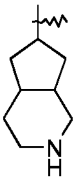 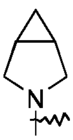 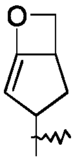 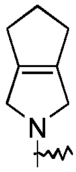 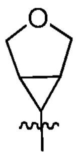 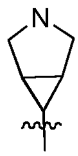 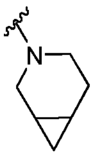

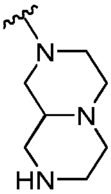 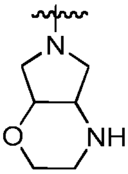 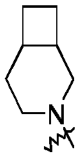 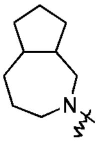 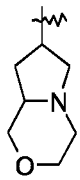 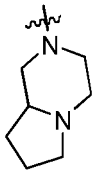 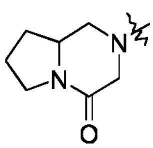

and

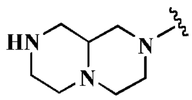

etc.

The term "bridged heterocyclyl" refers to a 5 to 14 membered polycyclic heterocyclyl group, wherein every two rings in the system share two disconnected atoms, wherein the rings can have one or more double bond(s), but none of the rings has a completely conjugated π-electron system, and one or more ring atoms are heteroatoms selected from the group consisting of nitrogen, oxygen and $S(O)_m$ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

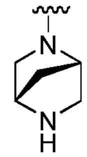 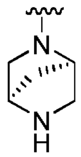 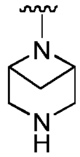 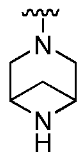 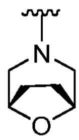 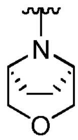 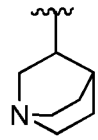 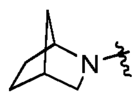 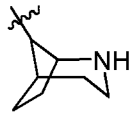

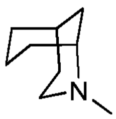 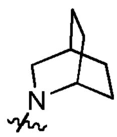 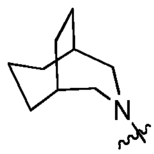 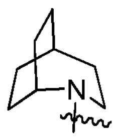 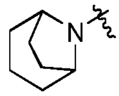 and 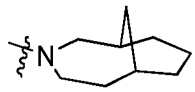

etc.

The heterocyclyl ring can be fused to the ring of aryl, heteroaryl or cycloalkyl, wherein the ring bound to the parent structure is heterocyclyl. Non-limiting examples include:

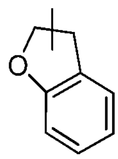 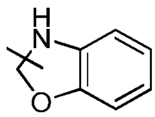 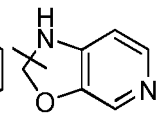 and 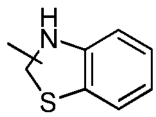

etc.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, including benzo 3 to 8 membered cycloalkyl and benzo 3 to 8 membered heterocyclyl, preferably benzo 3 to 6 membered cycloalkyl and benzo 3 to 6 membered heterocyclyl, wherein the heterocyclyl is a heterocyclyl containing 1 to 3 nitrogen atom, oxygen atom or sulfur atom; or further including nitrogen-containing tricyclic fused ring containing a benzene ring.

The ring bound to the parent structure is aryl ring. Non-limiting examples include:

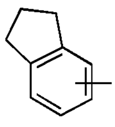 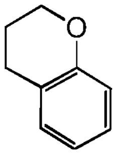 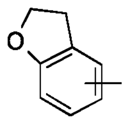 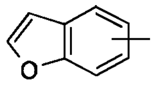 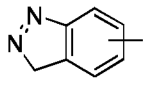 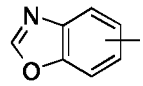 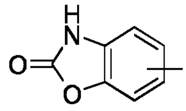

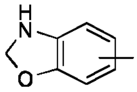 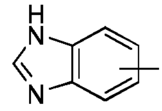 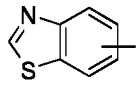 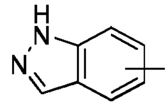 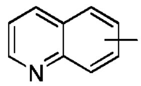 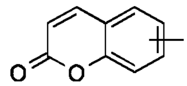

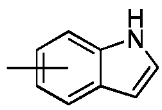 and 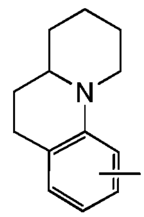

etc.

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of oxygen, sulfur and nitrogen. The heteroaryl is preferably a 5 to 10 membered heteroaryl, and more preferably a 5 or 6 membered heteroaryl, for example imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl and the like, preferably triazolyl, thienyl, imidazolyl, pyrazolyl, oxazolyl, pyrimidinyl and thiazolyl, and more preferably pyrazolyl and oxazolyl. The heteroaryl ring can be fused to the ring of aryl, heterocyclyl or cycloalkyl, wherein the ring bound to the parent structure is heteroaryl ring. Non-limiting examples include:

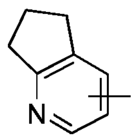 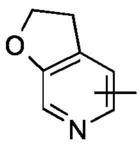 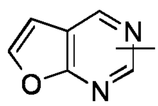 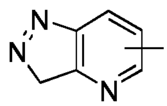 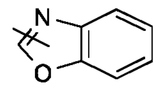 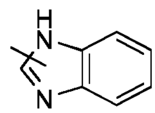

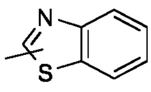 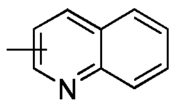 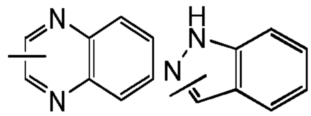

and

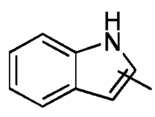

etc.

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl group substituted by one or more halogen(s), wherein the alkyl is as defined above.

"Haloalkoxy" refers to an alkoxy group substituted by one or more halogen(s), wherein the alkoxy is as defined above.

"Hydroxyalkyl" refers to an alkyl group substituted by hydroxy(s), wherein the alkyl is as defined above.

"Alkenyl" refers to a chain alkenyl, also known as alkene group. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Alkynyl" refers to (CH=C-). The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

The term "alkenylcarbonyl" refers to a -C(O)-(alkenyl), wherein the alkenyl is as defined above. Non-limiting examples of alkenylcarbonyl include vinylcarbonyl, propenylcarbonyl, butenylcarbonyl. The alkenylcarbonyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Hydroxy" refers to an -OH group.

"Halogen" refers to fluorine, chlorine, bromine or iodine.

"Amino" refers to a $-NH_2$ group.

"Cyano" refers to a -CN group.

"Nitro" refers to a $-NO_2$ group.

"Carbonyl" refers to a-C(O)- group.

"Carboxy" refers to a -C(O)OH group.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"$Et_2O$" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"$Pd_2(dba)_3$" refers to tris(dibenzylideneacetone)dipalladium.

"Dppf' refers to 1,1'-bisdiphenylphosphinoferrocene.

"HATU" refers to 2-(7-azabenzotriazol-1-yl)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to *n*-butyl lithium.

"$NaBH(OAc)_3$" refers to sodium triacetoxyborohydride.

Different expressions such as "X is selected from the group consisting of A, B or C", "X is selected from the group consisting of A, B and C", "X is A, B or C", "X is A, B and C" and the like, express the same meaning, that is, X can be any one or more of A, B and C.

The hydrogen atom of the present invention can be replaced by its isotope deuterium. Any of the hydrogen atoms in the compounds of the examples of the present invention can also be substituted by deuterium atom.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present,

and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable. Optional substituents include deuterium, halogen, amino, hydroxy, cyano, oxo, thioxo, alkyl, alkenyl, alkynyl, deuterated alkyl, haloalkyl, hydroxyalkyl, alkoxy, alkylthio, haloalkoxy, cycloalkyl, heterocyclyl, aryl and heteroaryl, and preferably deuterium, halogen, amino, hydroxy, cyano, oxo, thioxo, $C_{1-6}$ alkyl, $C_{2-6}$ alkenyl, $C_{2-6}$ alkynyl, deuterated $C_{1-6}$ alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3 to 12 membered heterocyclyl, $C_{6-14}$ aryl and 5 to 14 membered heteroaryl.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

## DETAILED DESCRIPTION

The present invention will be further described with reference to the following examples, but the examples should not be considered as limiting the scope of the present invention.

EXAMPLES

The structures of the compounds of the present invention were identified by nuclear magnetic resonance (NMR) and/or liquid chromatography-mass spectrometry (LC-MS). NMR shifts ($\delta$) are given in parts per million (ppm). NMR was determined by a Bruker AVANCE-400 instrument. The solvents for determination were deuterated-dimethyl sulfoxide (DMSO-$d_6$), deuterated-methanol ($CD_3OD$) and deuterated-chloroform ($CDCl_3$), and the internal standard was tetramethylsilane (TMS).

Liquid chromatography-mass spectrometry (LC-MS) was determined on an Agilent 1200 Infinity Series mass spectrometer. High performance liquid chromatography (HPLC) was determined on an Agilent 1200DAD high pressure liquid chromatograph (Sunfire C18 150×4.6 mm column), and a Waters 2695-2996 high pressure liquid chromatograph (Gimini C18 150×4.6 mm column).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm. Yantai Huanghai 200 to 300 mesh silica gel was generally used as a carrier for column chromatography.

The raw materials used in the examples of the present invention are known and commercially available, or can be synthesized by or according to known methods in the art.

Unless otherwise stated, all reactions of the present invention were carried out under continuous magnetic stirring under a dry nitrogen or argon atmosphere, the solvent was dry, and the reaction temperature was in degrees celsius.

### Example 1

**Isopropyl 2-((5-(but-2-ynamido)-2-methoxy-4-(methyl((1-methylpyrrolidin-2-yl)methyl)amin o)phenyl)amino)-4-(1-methyl-1H-indol-3-yl)pyrimidine-5-carboxylate**

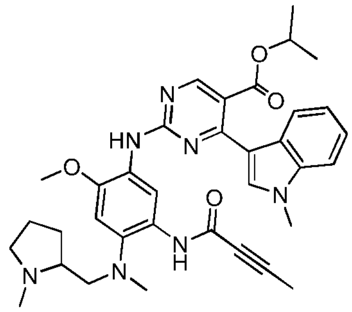

1

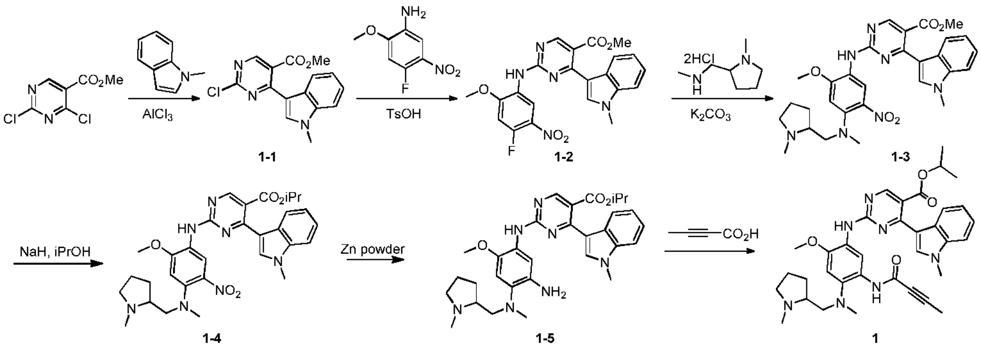


Step 1: Methyl 2-chloro-4-(1-methyl-1H-indol-3-yl)pyrimidine-5-carboxylate

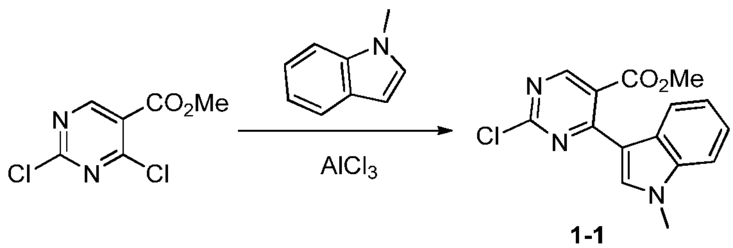


1-1

Aluminum trichloride (6.4 g, 48 mmol) was added in batches to a solution of methyl 2,4-dichloropyrimidine-5-carboxylate (5.0 g, 24 mmol) in 1,2-dichloroethane (50 mL) at 0°C. The reaction solution was stirred at room temperature for 15 minutes, followed by the addition of 1-methyl-1H-indole (3.2 g, 24 mmol). The reaction solution was heated to 55°C and stirred for 1.5 hours. The reaction solution was cooled to 0°C, to which methanol (10 mL) and water (30 mL) were added slowly. The solution was stirred at room temperature for 30 minutes, and extracted with dichloromethane (30 mL*2). The organic phases were combined, dried over anhydrous sodium sulfate, and concentrated to dryness to obtain the crude product. The resulting crude product was purified by column chromatography (petroleum ether: ethyl acetate=1:1) to obtain compound 1-1 (5.0 g, yield: 69%).

$^1H$ NMR (400 MHz, $CDCl_3$) δ 8.83 (s, 1H), 8.17 (dd, J = 6.8, 1.8 Hz, 1H), 7.97 (s, 1H), 7.48 - 7.27 (m, 3H), 3.88 (s, 3H), 3.86 (s, 3H).

Step 2: Methyl 2-((4-fluoro-2-methoxy-5-nitrophenyl)amino)-4-(1-methyl-1H-indol-3-yl)pyrimidine-5-carboxylate

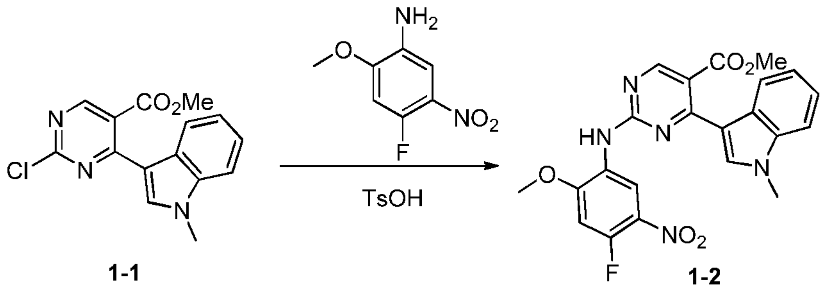


1-1 1-2

P-toluenesulfonic acid monohydrate (3.8 g, 19.9 mmol) was added to a solution of 4-fluoro-2-methoxy-5-nitroaniline (1.1 g, 5.97 mmol) and compound 1-1 (1.5 g, 4.97 mmol) in dioxane (50 mL). The reaction solution was heated to 100°C and reacted for 16 hours. The reaction solution was cooled, and concentrated to dryness to obtain the crude product. The resulting crude product was purified by column chromatography (dichloromethane: methanol: ammonia (w/w 25%) = 100:2:0.5%) to obtain compound 1-2 (1.8 g, yield: 80%).

$^1H$ NMR (400 MHz, $CDCl_3$) δ 9.34 (br, 1H), 8.80 (s, 1H), 8.27 (s, 1H), 7.85 (m, 1H), 7.64 (d, J = 8.0 Hz, 1H), 7.47 - 7.36 (m, 1H), 7.37 - 7.29 (m, 1H), 7.24 - 7.13 (m, 1H), 6.81 (d, J = 12.1 Hz, 1H), 4.01 (s, 3H), 3.94 (d, J = 2.9 Hz, 3H), 3.79 (s, 3H).

Step 3: Methyl 2-((2-methoxy-4-(methyl((1-methylpyrrolidin-2-yl)methyl)amino)-5-nitrophenyl)amino )-4-(1-methyl-1H-indol-3-yl)pyrimidine-5-carboxylate

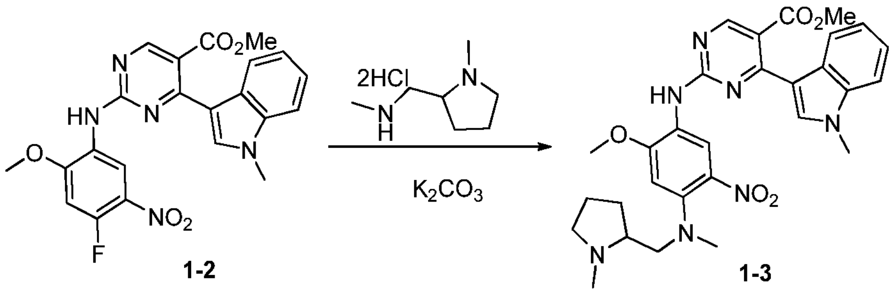


1-2 1-3

Potassium carbonate (413 mg, 2.99 mmol) was added to a solution of N-methyl-1-(1-methylpyrrolidin-2-yl)methanamine hydrochloride (180 mg, 1.19 mmol) and compound **1-2** (450 mg, 0.99 mmol) in acetonitrile (15 mL). The reaction solution was heated to 80°C and reacted for 2 hours. The reaction solution was cooled, and concentrated to dryness. The resulting residues were dissolved in dichloromethane (30 mL), and washed with brine (20 mL*3). The organic phase was dried over anhydrous sodium sulfate, and concentrated to dryness to obtain the crude product. The resulting crude product was purified by column chromatography (dichloromethane: methanol: ammonia (w/w 25%) = 100:5:0.5%) to obtain compound **1-3** (450 mg, yield: 81%).

MS m/z (ESI): 560.2[M+H]$^+$.

Step 4: Isopropyl 2-((2-methoxy-4-(methyl((1-methylpyrrolidin-2-yl)methyl)amino)-5-nitrophenyl)amino )-4-(1-methyl-1H-indol-3-yl)pyrimidine-5-carboxylate

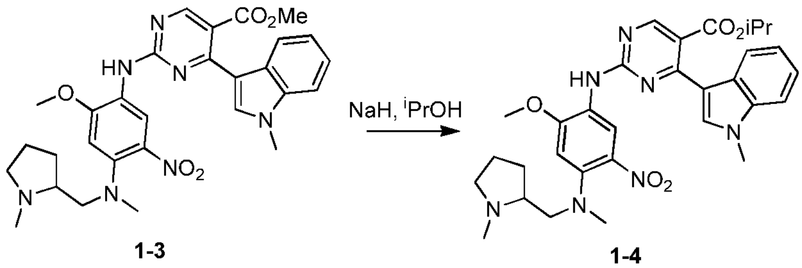


1-3 1-4

Sodium hydride (19 mg, 0.46 mmol, w/w 60%) was added to a solution of compound 1-3 (200 mg, 0.36 mmol) in isopropanol (6 mL). The reaction solution was heated to 80°C and reacted for 30 minutes. The reaction solution was concentrated to dryness to obtain the crude compound 1-4 (200 mg), which was used directly in the next step.

MS m/z (ESI): 588.3[M+H]$^+$.

Step 5: Isopropyl 2-((5-amino-2-methoxy-4-(methyl((1-methylpyrrolidin-2-yl)methyl)amino)phenyl)amin o)-4-(1-methyl-1H-indol-3 -yl)pyrimidine-5-carboxylate

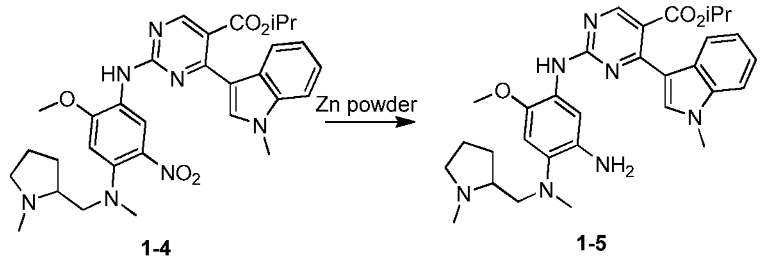


1-4 1-5

Zinc powder (111 mg, 1.7 mmol) was added to a solution of compound **1-4** (250 mg, 0.43 mmol) and ammonium chloride (227 mg, 4.25 mmol) in acetone (10 mL) and water (1 mL). The reaction solution was stirred at room temperature for 40 minutes. The reaction solution was filtered. The organic phase was added with dichloromethane (30 mL), and washed with water (15 mL*2). The solid was rinsed with dichloromethane (30 mL). The organic phases were combined, dried over anhydrous sodium sulfate, and concentrated to dryness to obtain the crude product. The resulting crude product was purified by column chromatography (dichloromethane: methanol: ammonia (w/w 25%) = 100:10:0.5%) to obtain compound **1-5** (140 mg, yield: 59%).

MS m/z (ESI): 558.3[M+H]$^+$.

Step 6: Isopropyl 2-((5-(but-2-ynamido)-2-methoxy-4-(methyl((1-methylpyrrolidin-2-yl)methyl)amino)ph enyl)amino)-4-(1-methyl-1H-indol-3-yl)pyrimidine-5-carboxylate

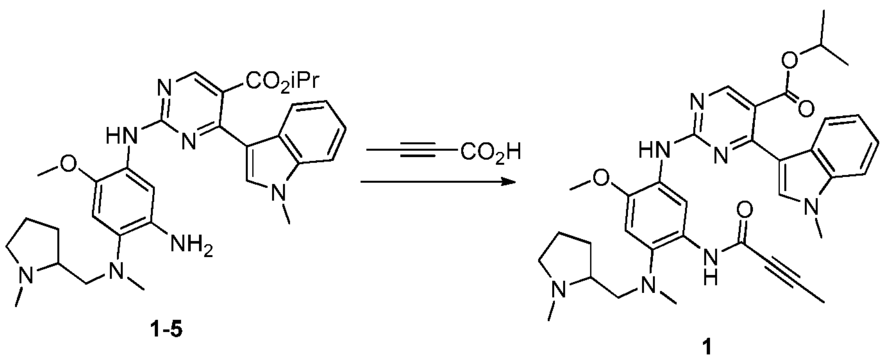

1-5 1

2-(7-Azabenzotriazol)-N,N,N',N'-tetramethyluronium hexafluorophosphate (102 mg, 0.27 mmol) was added to

a solution of compound 1-5 (100 mg, 0.18 mmol), 2-butynoic acid (23 mg, 0.27 mmol) and N,N-diisopropylethylamine (70 mg, 0.54 mmol) in dichloromethane (5 mL). The reaction solution was stirred at room temperature for 16 hours. The reaction solution was washed with water (15 mL*2). The organic phase was dried over anhydrous sodium sulfate, and concentrated to dryness to obtain the crude product. The resulting crude product was purified by thin layer chromatography (dichloromethane: methanol: ammonia (w/w 25%) = 100:5:0.5%) to obtain the product (26 mg, yield: 24%).

MS m/z (ESI): 624.3[M+H]+.

$^1$H NMR (400 MHz, Methanol-*d*4) δ 9.23 (s, 1H), 8.70 (s, 1H), 8.26 - 8.08 (m, 1H), 7.68 - 7.52 (m, 1H), 7.42 (d, J = 8.1 Hz, 1H), 7.21 (t, J = 7.5 Hz, 1H), 7.10 (t, J = 7.5 Hz, 1H), 6.90 (s, 1H), 5.05 - 4.93 (m, 1H), 4.02 - 3.79 (m, 6H), 3.24 - 3.12 (m, 1H), 3.00 - 2.84 (m, 1H), 2.83 - 2.74 (m, 1H), 2.71 (s, 3H), 2.68 - 2.58 (m, 1H), 2.49 (s, 3H), 2.43 - 2.30 (m, 1H), 2.10 - 1.93 (m, 4H), 1.83 - 1.69 (m, 2H), 1.59 - 1.41 (m, 1H), 1.16 - 0.95 (m, 6H).

**Example 2**

**Isopropyl 2-((5-acrylamido-2-methoxy-4-(9-methyl-3,9-diazaspiro[5.5]undecan-3-yl)phenyl)a mino)-4-(1-methyl-1H-indol-3-yl)pyrimidine-5-carboxylate**

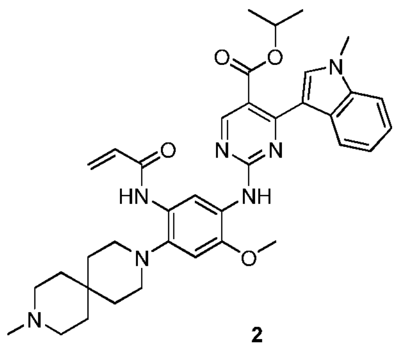

2

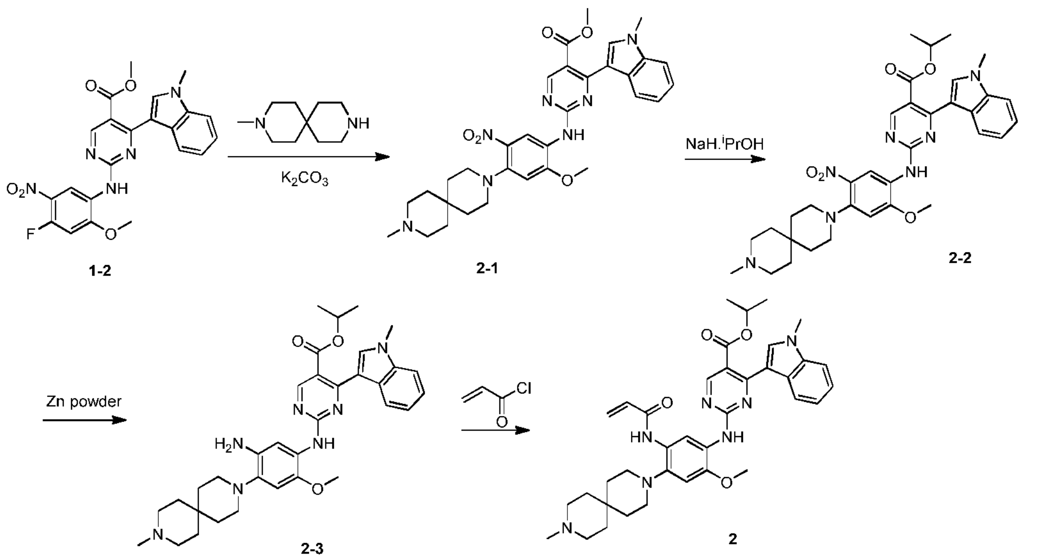


Step 1: Methyl 2-((2-methoxy-4-(9-methyl-3,9-diazaspiro[5.5]undecan-3-yl)-5-nitrophenyl)amino)-4-(1 -methyl-1H-indol-3-yl)pyrimidine-5-carboxylate

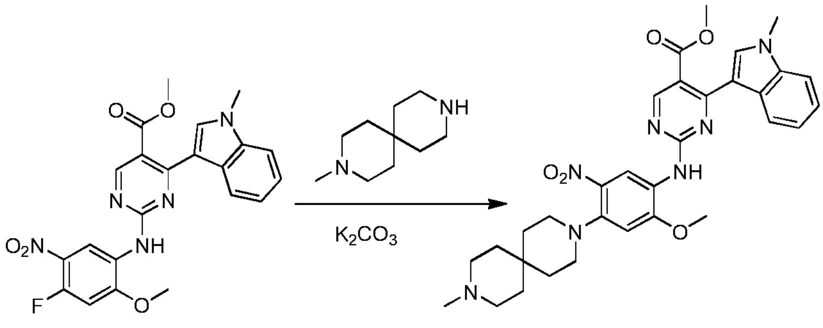


1-2 2-1

This step was carried out in accordance with Step 3 of Example 1 (260 mg, yield: 78%).

MS m/z (ESI): 600.3[M+H]$^+$.

Step 2: Isopropyl 2-((2-methoxy-4-(9-methyl-3,9-diazaspiro[5.5]undecan-3-yl)-5-nitrophenyl)amino)-4-(1 -methyl-1H-indol-3-yl)pyrimidine-5-carboxylate

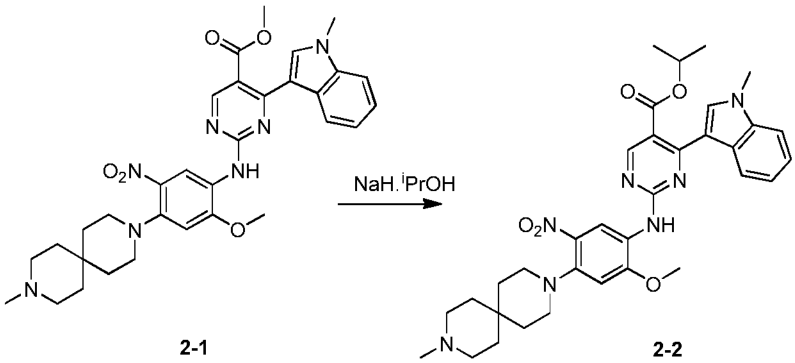


2-1 2-2

This step was carried out in accordance with Step 4 of Example 1 (260 mg, crude product).

MS m/z (ESI): 628.3[M+H]$^+$.

Step 3: Isopropyl 2-((5-amino-2-methoxy-4-(9-methyl-3,9-diazaspiro[5.5]undecan-3-yl)phenyl)amino)-4-(1-methyl-1H-indol-3-yl)pyrimidine-5-carboxylate

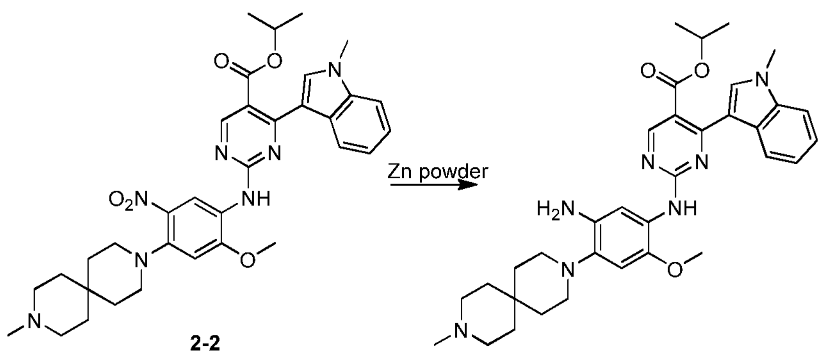


2-2 2-3

This step was carried out in accordance with Step 5 of Example 1 (220 mg, yield: 89%).

MS m/z (ESI): 598.3[M+H]$^+$.

Step 4: Isopropyl 2-((5-acrylamido-2-methoxy-4-(9-methyl-3,9-diazaspiro[5.5]undecan-3-yl)phenyl)amin o)-4-(1-methyl-1H-indol-3-yl)pyrimidine-5-carboxylate

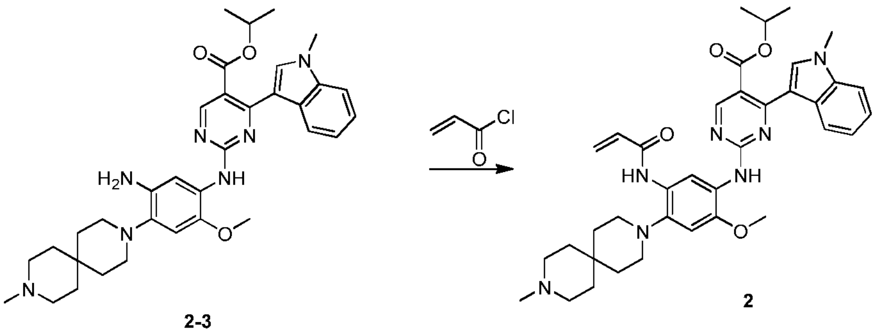

2-3

2

Acryloyl chloride (10 mg, 0.11 mmol) and triethylamine (20 mg, 0.20 mmol) were added to a solution of compound 1-5 (60 mg, 0.10 mmol) in dichloromethane (5 mL). The reaction solution was stirred at room temperature for 16 hours. The reaction solution was concentrated under reduced pressure, and the resulting residues were purified by thin layer chromatography (dichloromethane: methanol: ammonia (w/w 25%) = 100:5:0.5) to obtain the product (26 mg, yield: 40%).
MS m/z (ESI): 652.3[M+H]$^+$.

**Example 3**

**N-(5-((4-(1-Cyclopropyl-1H-indol-3-yl)-5-oxazol-2-yl)pyrimidin-2-yl)amino)-4-met hoxy-2-((4aR,7aR)-1-methyloctahydro-6H-pyrrolo[3,4-b]pyridin-6-yl)phenyl)acryl** amide

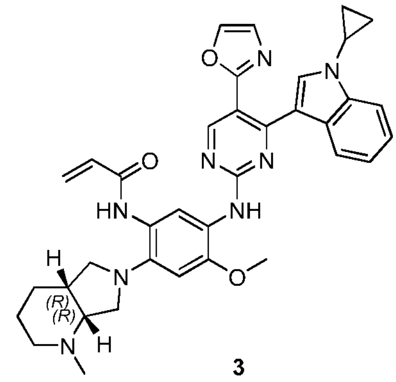

3

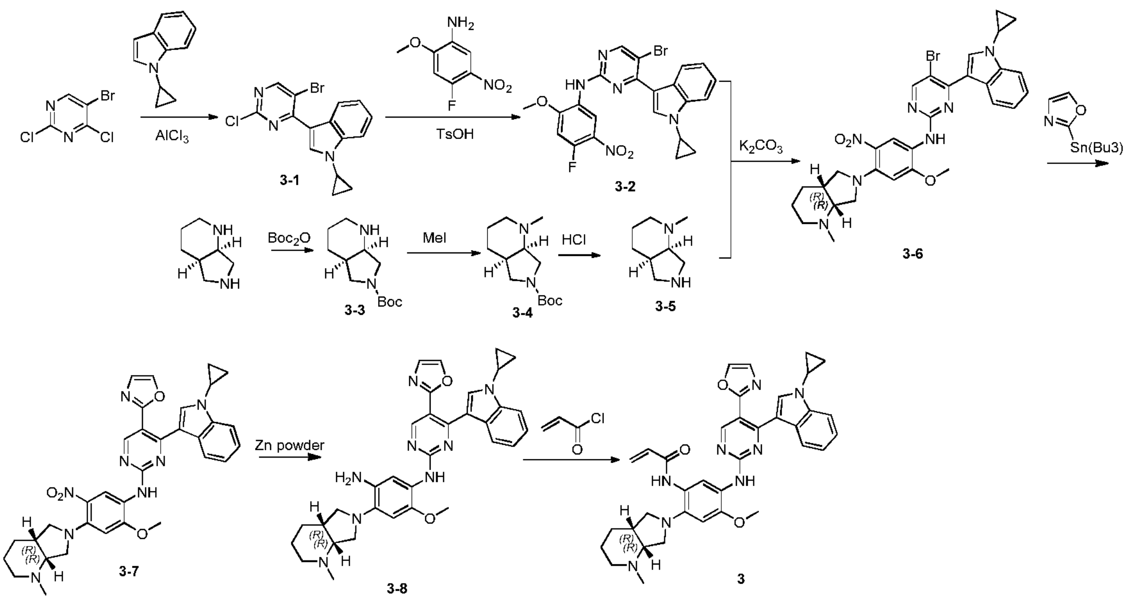


3-1 3-2 3-3 3-4 3-5 3-6 3-7 3-8 3

Step 1: 3-(5-Bromo-2-chloropyrimidin-4-yl)-1-cyclopropyl-1H-indole (intermediate **3-1**)

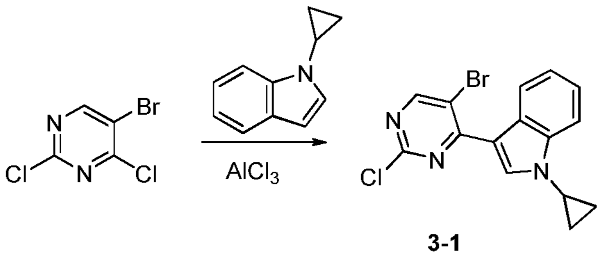


**3-1**

Aluminum trichloride (2.66 g, 20 mmol) was added in batches to a solution of 5-bromo-2,4-dichloropyrimidine (2.28 g, 10 mmol) in 1,2-dichloroethane (30 mL) at 0°C. The reaction solution was stirred at room temperature for 15 minutes, followed by the addition of 1-cyclopropyl-1H-indole (1.57 g, 10 mmol). The reaction solution was heated to 60°C and stirred for 2 hours. The reaction solution was cooled to 0°C, to which methanol (10 mL) and water (30 mL) were added slowly. The solution was stirred at room temperature for 30 minutes, and extracted with dichloromethane (20 mL*3). The organic phases were combined, dried over anhydrous sodium sulfate, and concentrated to dryness. The resulting residues were purified by column chromatography (petroleum ether: ethyl acetate = 2:3) to obtain intermediate **3-1** (2.71 g, yield: 78%).

MS m/z (ESI): 348.1$[M+H]^+$.

Step 2: 5-Bromo-4-(1-cyclopropyl-1H-indol-3-yl)-N-(4-fluoro-2-methoxy-5-nitrophenyl)pyrimi din-2-amine (intermediate **3-2)**

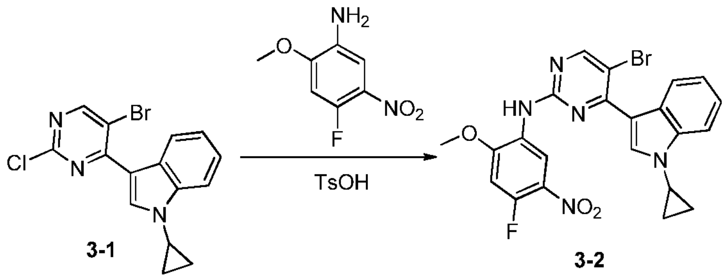


3-1 3-2

P-toluenesulfonic acid monohydrate (1.90 g, 10 mmol) was added to a solution of intermediate 3-2 (1.12 g, 3.2 mmol) and 4-fluoro-2-methoxy-5-nitroaniline (707 mg, 3.8 mmol) in dioxane (30 mL). The reaction solution was heated to 100°C and reacted for 16 hours. The reaction solution was cooled, and concentrated to dryness. The resulting residues were purified by column chromatography (dichloromethane: methanol: ammonia (w/w 25%) = 100:2:0.5) to obtain intermediate 3-2 (1.15 g, yield: 72%).
MS m/z (ESI): 498.2[M+H]$^+$.

Step 3: Tert-butyl (4aR,7aR)-octahydro-6H-pyrrolo[3,4-b]pyridine-6-carboxylate (intermediate 3-3)

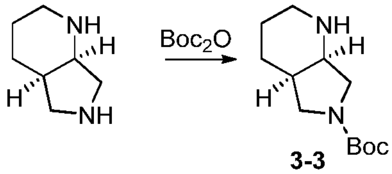


3-3

A solution of di-*tert*-butyl dicarbonate (8.29 g, 38 mmol) in dichloromethane (20 mL) was slowly added dropwise to a solution of (4aR,7aR)-octahydro-1H-pyrrolo[3,4-b]pyridine (5.00 g, 40 mmol) in dichloromethane (800 mL) under an ice-water bath. After completion of the addition, the reaction solution was reacted for 16 hours. The reaction solution was concentrated, and the resulting residues were purified by column chromatography (dichloromethane: methanol: concentrated ammonia = gradient elution from 99:1:0.1 to 90:10:1) to obtain intermediate **3-3** (7.35 g, yield: 82%).
MS m/z (ESI): 227.1[M+H]$^+$.

Step 4: Tert-butyl (4aR,7aR)-1-methyloctahydro-6H-pyrrolo[3,4-b]pyridine-6-carboxylate (intermediate **3-4**)

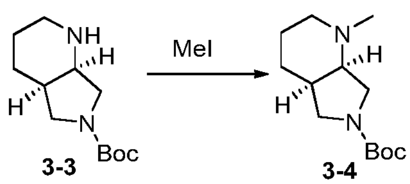


3-3 3-4

Methyl iodide (2.84 g, 20 mmol) was slowly added dropwise to a solution of intermediate **3-3** (5.00 g, 22 mmol) and triethylamine (5.06 g, 50 mmol) in dichloromethane (100 mL) under an ice-water bath. After completion of the addition, the reaction solution was slowly warmed up to room temperature and reacted for 16 hours. The reaction solution was concentrated, and the resulting residues were purified by column chromatography (dichloromethane: methanol: concentrated ammonia = gradient elution from 99:1:0.1 to 90:10:1) to obtain intermediate **3-4** (3.28 g, yield: 62%).
MS m/z (ESI): 241.1[M+H]$^+$.

Step 5: *Tert*-butyl (4aR,7aR)-1-Methyloctahydro-1H-pyrrolo[3,4-b]pyridine-6-carboxylate (intermediate **3-5)**

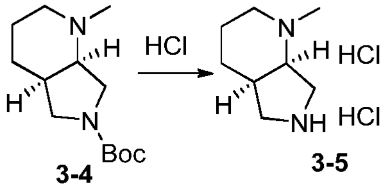


3-4 3-5

Intermediate 3-4 (3.28 g, 14 mmol) was dissolved in dichloromethane (50 mL) under a water bath, to which 2M hydrogen chloride/ether solution (25 mL, 50 mmol) was slowly added dropwise. After completion of the addition, the reaction solution was reacted for 4 hours. The reaction solution was filtered, and the solid was rinsed with anhydrous ether (10 mL*2). The solid was dried under vacuum to obtain the hydrochloride intermediate **3-5** (2.07 g, yield: 71%).
MS m/z (ESI): 141.3[M+H]$^+$.

Step 6: 5-Bromo-4-(1-cyclopropyl-1H-indol-3-yl)-N-(2-methoxy-4-((4aR,7aR)-1-methyloctahy dro-6H-pyrrolo[3,4-b]pyridin-6-yl)-5-nitrophenyl)pyrimidin-2-amine (intermediate 3-6)

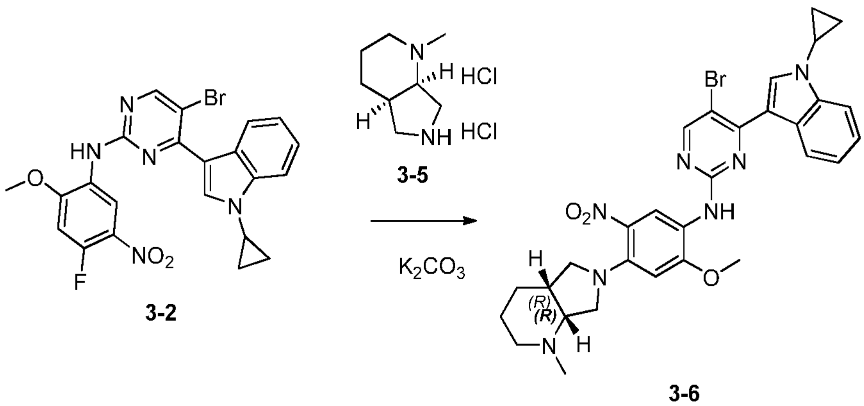


3-2 3-6

Potassium carbonate (690 mg, 5.0 mmol) was added to a solution of intermediate **3-2** (498 mg, 1.0 mmol) and intermediate **3-5** (298 mg, 1.4 mmol) in acetonitrile (30 mL). The reaction solution was reacted under reflux for 16 hours. The reaction solution was cooled, and concentrated to dryness. The resulting residues were dissolved in dichloromethane (50 mL), and washed with brine (30 mL*3). The organic phase was dried over anhydrous sodium sulfate, and concentrated to dryness. The resulting crude product was purified by column chromatography (dichloromethane: methanol: concentrated ammonia = gradient elution from 99:1:0.1 to 95:5:0.5) to obtain intermediate **3-6** (420 mg, yield: 68%).
MS m/z (ESI): 618.3 [M+H]$^+$.

Step 7: 4-(1-Cyclopropyl-1H-indol-3-yl)-N-(2-methoxy-4-((4aR,7aR)-1-methyloctahydro-6H-p yrrolo[3,4-b]pyridin-6-yl)-5-nitrophenyl)-5-(oxazol-2-yl)pyrimidin-2-amine (intermediate **3-7**)

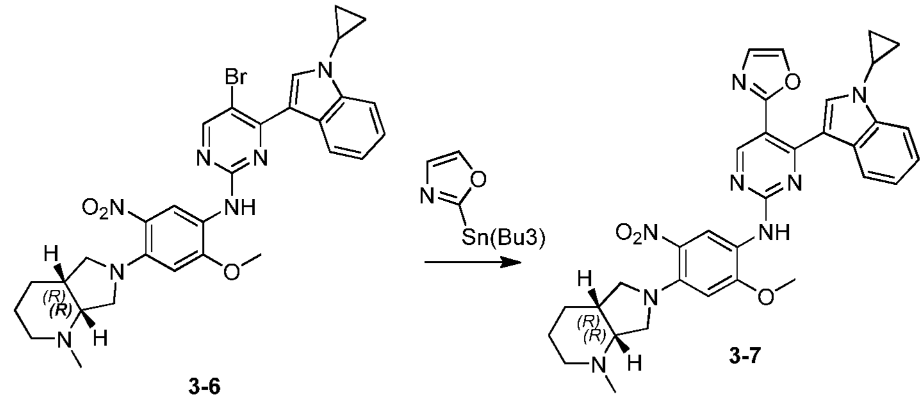


3-6 3-7

Intermediate 3-6 (200 mg, 0.3 mmol), 2-(tri-n-butylstannyl)oxazole (127 mg, 0.36 mmol) and tetrakis(triphenylphosphine) palladium (30 mg) were dissolved in toluene (3 mL) under a nitrogen atmosphere. The reaction solution was reacted at 110°C overnight. The reaction solution was concentrated under reduced pressure, and the resulting crude product was purified by column chromatography (dichloromethane: methanol: concentrated ammonia = gradient elution from 99:1:0.1 to 94:6:0.6) to obtain intermediate 3-7 (145 mg, yield: 74%).

MS m/z (ESI): 607.3 $[M+H]^+$.

Step 8: N-(5-((4-(1-Cyclopropyl-1H-indol-3-yl)-5-(oxazol-2-yl)pyrimidin-2-yl)amino)-4-metho xy-2-((4aR,7aR)-1-methyloctahydro-6H-pyrrolo[3,4-b]pyridin-1-yl)phenyl)acrylamide (compound **3**)

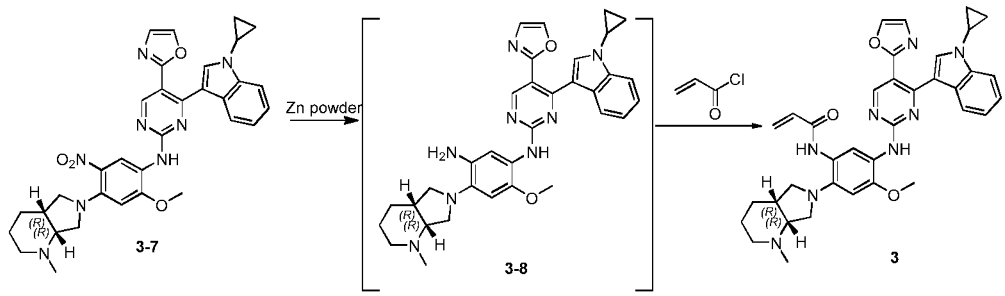


3-7 3-8 3

Zinc powder (45 mg, 0.70 mmol) was added to a solution of compound **3-7** (140 mg, 0.23 mmol) and ammonium chloride (123 mg, 2.3 mmol) in acetone (4 mL)/ water (1 mL) under stirring at room temperature. The reaction solution was reacted at room temperature for 2 hours. The reaction solution was filtered, the solid was rinsed with dichloromethane (10 mL*2) and water (5 mL*2) successively, and the filtrate was partitioned. The organic phase was washed with water (5 mL*2), dried over anhydrous sodium sulfate and filtered. The filtrate was concentrated to obtain the crude intermediate **3-8.** The crude intermediate **3-8** was dissolved in dichloromethane (5 mL), followed by the successive addition of triethylamine (50 mg, 0.50 mmol) and acryloyl chloride (23 mg, 0.25 mmol) under stirring at room temperature. The reaction solution was stirred at room temperature for 16 hours. The reaction solution was concentrated under reduced pressure, and the resulting residues were purified by column chromatography (dichloromethane: methanol: ammonia (w/w 25%) = 99:1:0.1 to 93:7:0.7) to obtain compound 3 (59 mg, yield: 40%).

MS m/z (ESI): 631.2 $[M+H]^+$.

## Example 4

**Isopropyl 2-((5-acrylamido-4-(5-amino-5-methylhexahydrocyclopenta[c]pyrrol-2(1H)-yl)-2-m ethoxyphenyl)amino)-4-(1-methyl-1H-indol-3-yl)pyrimidine-5-carboxylate**

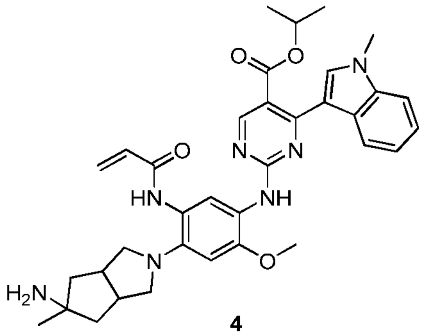

4

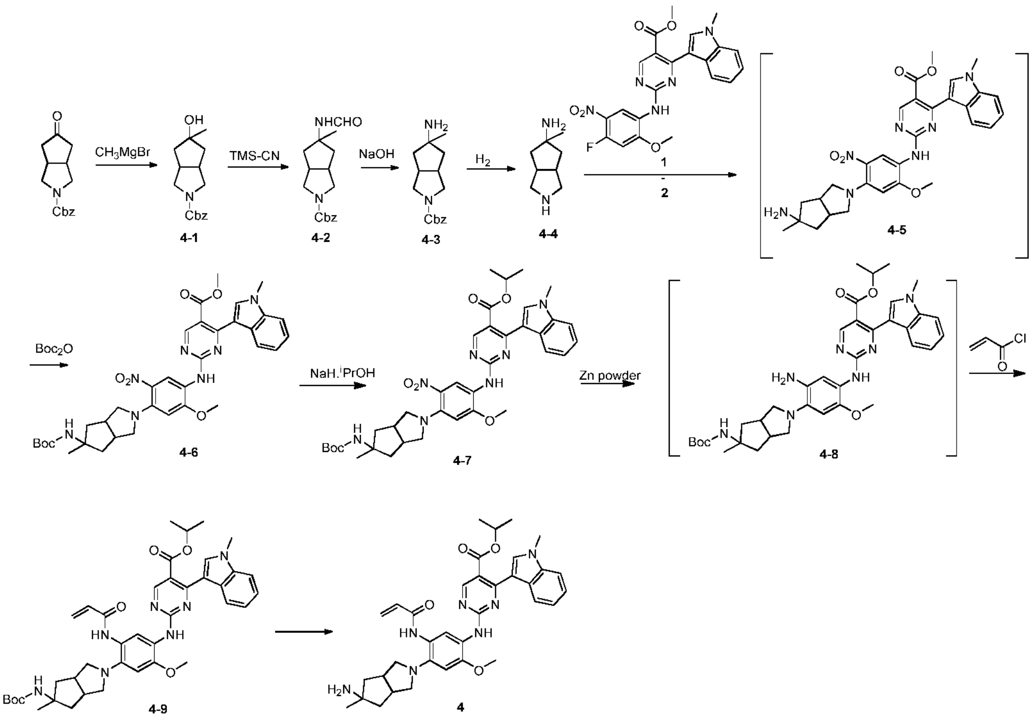


Step 1: Benzyl 5-hydroxy-5-methylhexahydrocyclopenta[c]pyrrole-2(1H)-carboxylate (intermediate **4-1)**

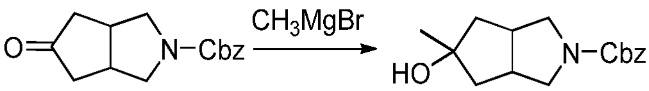


**4-1**

Benzyl 5-oxohexahydrocyclopenta[c]pyrrole-2(1H)-carboxylate (1.20 g, 4.63 mmol) was dissolved in tetrahydrofuran (20 mL) at -78°C under a nitrogen atmosphere, and methylmagnesium bromide (3.0 M ether solution, 2.5 mL, 7.5 mmol) was added dropwise. The reaction solution was stirred at -78°C for two hours, and TLC indicated that the reaction was completed. Saturated ammonium chloride solution (15 mL) was added to quench the reaction, and the solution was extracted with ethyl acetate (25 mL*3). The organic phases were combined, dried over anhydrous sodium sulfate, filtered and concentrated to dryness by rotary evaporation to obtain the crude intermediate **4-1** (1.21 g, colorless oil), which was used directly in the next step without further purification.

MS m/z (ESI):276.1 $[M+H]^+$.

Step 2: Benzyl 5-formamido-5-methylhexahydrocyclopenta[c]pyrrole-2(1H)-carboxylate (intermediate **4-2)**

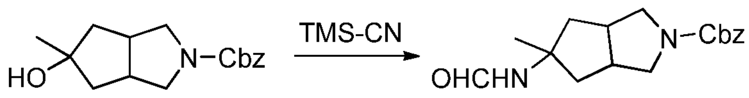


4-1 4-2

Intermediate 4-1 (1.21 g, 4.97 mmol) was dissolved in acetic acid (6 mL) at room temperature, and then the reaction system was cooled to 10°C. Trimethylsilyl cyanide (0.54 g, 5.47 mmol) was added, and then concentrated sulfuric acid (3 mL) was added dropwise. The reaction solution was kept below 10°C, and reacted for 4 hours. 10% sodium hydroxide solution was added to quench the reaction, and the reaction solution was extracted with dichloromethane (20 mL*3). The organic phases were combined, dried over anhydrous sodium sulfate, filtered and concentrated to dryness by rotary evaporation to obtain the crude intermediate **4-2** (1.10 g).

MS m/z (ESI):303.1 $[M+H]^+$.

Step 3: Benzyl 5-amino-5-methylhexahydrocyclopenta[c]pyrrole-2(1H)-carboxylate (intermediate **4-3**)

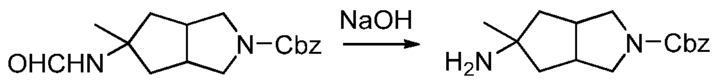


4-2 4-3

Intermediate 4-2 (1.10 g) was dissolved in ethanol (15 mL) at room temperature, followed by the addition of aqueous sodium hydroxide solution (1.50 g of sodium hydroxide dissolved in 10 mL of water). The reaction solution was heated to 75°C, and reacted for two hours. After completion of the reaction, ethanol was removed under reduced pressure, and 6N hydrochloric acid was added to adjust the pH to 2. The solution was washed with dichloromethane (25 mL*2) and partitioned. 10% sodium hydroxide solution was added to the aqueous phase to adjust the pH to 12, and the aqueous phase was extracted with dichloromethane (50 mL*3). The organic phases were combined, dried over anhydrous sodium sulfate, filtered and concentrated to dryness by rotary evaporation. The resulting residues were purified by column chromatography (dichloromethane: methanol: ammonia (w/w 25%) = 99:1:0.1 to 92:8:0.8) to obtain intermediate 4-3 (0.60 g, yield of three steps: 47%).

MS m/z (ESI): 275.1 $[M+H]^+$.

Step 4: 5-Methyloctahydrocyclopenta[c]pyrrol-5-amine (intermediate 4-4)

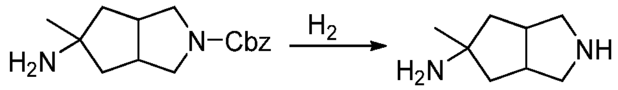


4-3 4-4

Intermediate **4-3** (600 mg) was dissolved in methanol (10 mL) at room temperature, followed by the addition of 10% palladium on carbon (120 mg). A hydrogenation reaction was carried out at room temperature for 16 hours. After completion of the reaction, the reaction solution was filtered, and the resulting solid was rinsed with dichloromethane (3 mL*2). The filtrate was concentrated under reduced pressure to obtain intermediate **4-4** (302 mg, yield: 98%).

MS m/z (ESI): 141.1 $[M+H]^+$.

Step 5: Methyl 2-((4-(5-((*tert*-butoxycarbonyl)amino)-5-methylhexahydrocyclopenta[c]pyrrol-2(1H)-yl )-2-methoxy-5-nitrophenyl)amino)-4-(1-methyl-1H-indol-3-yl)pyrimidine-5-carboxylat e (intermediate **4-6**)

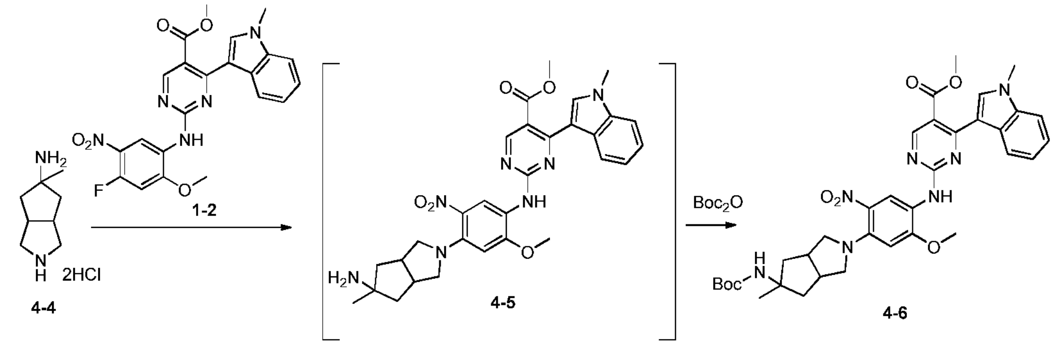


4-4 1-2 4-5 4-6

Potassium carbonate (415 mg, 3.0 mmol) was added to a solution of intermediate **1-2** (451 mg, 1.0 mmol) and intermediate **4-4** (168 mg, 1.2 mmol) in acetonitrile (15 mL). The reaction solution was reacted under reflux for 16 hours. The reaction solution was cooled and filtered, and the resulting solid was rinsed with dichloromethane (10 mL*2). The filtrates were combined and concentrated under reduced pressure to obtain the crude intermediate 4-5. The crude product was dissolved in dichloromethane (25 mL), followed by the addition of $Boc_2O$ (500 mg). The reaction solution was stirred at room temperature for 16 hours. The reaction solution was concentrated, and the resulting residues were purified by column chromatography (dichloromethane: methanol: concentrated ammonia = gradient elution from 99:1:0.1 to 95:5:0.5) to obtain intermediate **4-6** (511 mg, yield: 76%).

MS m/z (ESI): 672.4 $[M+H]^+$.

Step 6: Isopropyl 2-((4-(5-((*tert*-butoxycarbonyl)amino)-5-methylhexahydrocyclopenta[c]pyrrol-2(1H)-yl )-2-methoxy-5-nitrophenyl)amino)-4-(1-methyl-1H-indol-3-yl)pyrimidine-5-carboxylat e (intermediate 4-7)

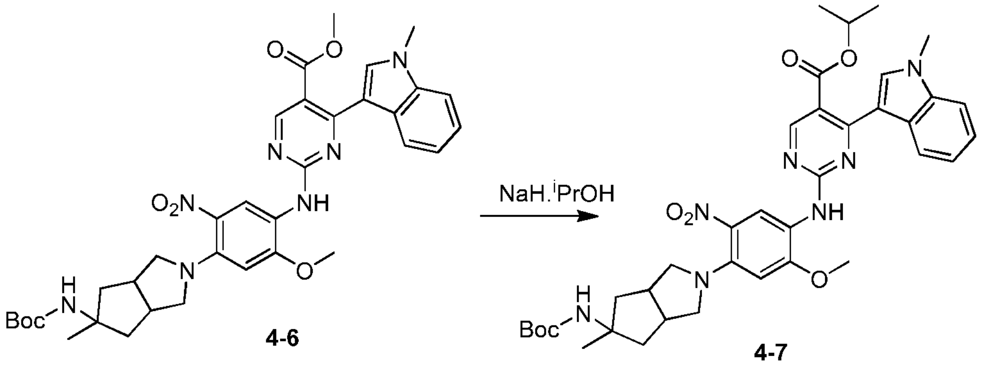


4-6 4-7

Sodium hydride (25 mg, 0.62 mmol, w/w 60%) was added to a solution of compound **4-6** (150 mg, 0.22 mmol) in isopropanol (5 mL). The reaction solution was reacted under reflux for 1 hour. The reaction solution was concentrated to dryness to obtain the crude compound **1-4** (174 mg), which was used directly in the next step.

MS m/z (ESI): 700.2$[M+H]^+$.

Step 7: Isopropyl 2-((5-acrylamido-4-(5-((*tert*-butoxycarbonyl)amino)-5-methylhexahydrocyclopenta[c]p yrrol-2(1H)-yl)-2-methoxyphenyl)amino)-4-(1-methyl-1H-indol-3-yl)pyrimidine-5-carb oxylate (intermediate **4-9**)

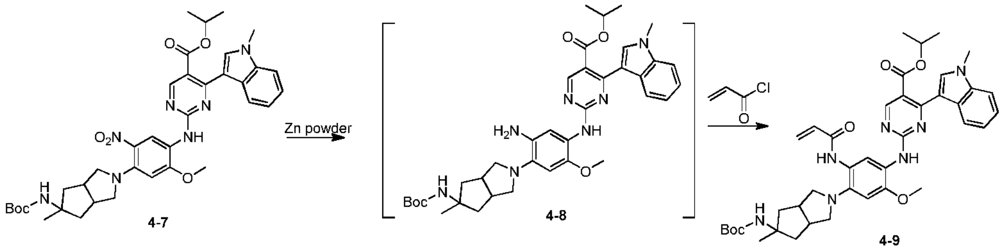


4-7 4-8 4-9

Zinc powder (100 mg, 1.5 mmol) was added to a solution of compound 4-7 (174 mg, crude product) and ammonium chloride (267 mg, 5 mmol) in acetone (10 mL)/ water (2 mL) at room temperature under stirring. The reaction solution was reacted at room temperature for 2 hours. The reaction solution was filtered, the solid was rinsed with dichloromethane (30 mL*2) and water (15 mL*2) successively, and the filtrate was partitioned. The organic phase was washed with water (10 mL*2), dried over anhydrous sodium sulfate and filtered. The filtrate was concentrated to obtain the crude intermediate 4-8. The crude intermediate 4-8 was dissolved in dichloromethane (10 mL), followed by the successive addition of triethylamine (50 mg, 0.50 mmol) and acryloyl chloride (27 mg, 0.30 mmol) under stirring at room temperature. The reaction solution was stirred at room temperature for 16 hours. The reaction solution was concentrated under reduced pressure, and the resulting residues were purified by preparative HPLC to obtain intermediate 4-9 (63 mg, yield of three steps: 39%).

MS m/z (ESI): 724.1 $[M+H]^+$.

Step 8: Isopropyl 2-((5-acrylamido-4-(5-amino-5-methylhexahydrocyclopenta[c]pyrrol-2(1H)-yl)-2-meth oxyphenyl)amino)-4-(1-methyl-1H-indol-3-yl)pyrimidine-5-carboxylate hydrochloride (hydrochloride of compound **4**)

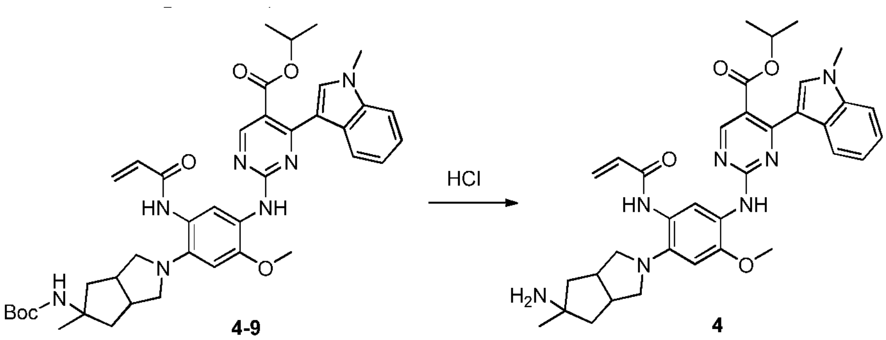


4-9 4

Intermediate 4-9 (60 mg) was dissolved in dichloromethane (2 mL) at room temperature, followed by the addition of 2M solution (1 mL) of hydrogen chloride in ether. The reaction solution was stirred at room temperature for two hours. After completion of the reaction, the reaction solution was filtered. The resulting solid was rinsed with dichloromethane (1 mL*2), and dried under vacuum to obtain the hydrochloride of compound 4 (39 mg, yield: 71%).

MS m/z (ESI): 624.3 $[M+H]^+$.

## Example 5

**N-(4-Fluorobenzyl)-2-((5-methacrylamido-2-methyl-4-(4-methylpiperazin-1-yl)phe nyl)amino)-N-methyl-4-(1-methyl-1H-indol-3-yl)pyrimidine-5-carboxamide**

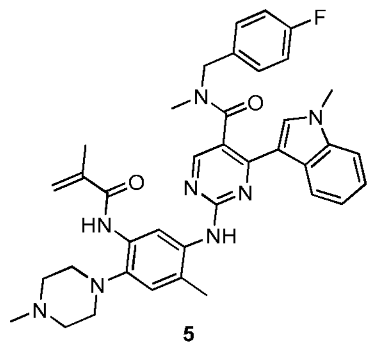

5

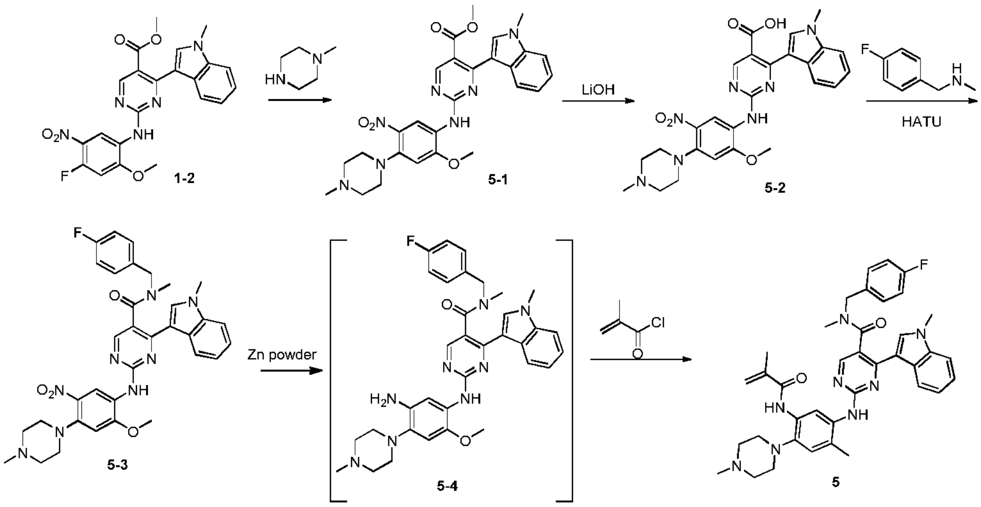


1-2 5-1 5-2 5-3 5-4 5

Step 1: Methyl 2-((2-methoxy-4-(4-methylpiperazin-1-yl)-5-nitrophenyl)amino)-4-(1-methyl-1H-indol-3-yl)pyrimidine-5-carboxylate (intermediate 5-1)

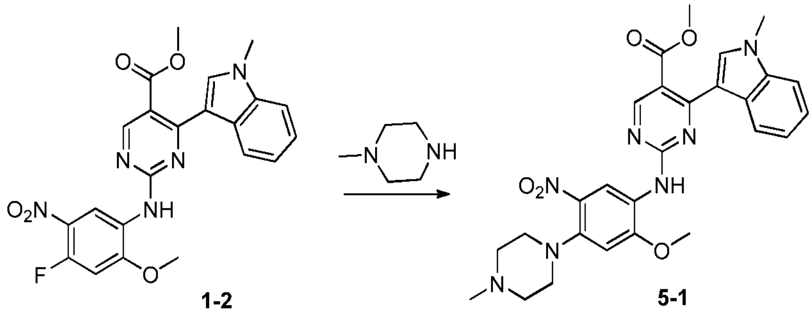

1-2 5-1

Potassium carbonate (415 mg, 3.0 mmol) was added to a solution of intermediate 1-2 (451 mg, 1.0 mmol) and methylpiperazine (120 mg, 1.2 mmol) in acetonitrile (15 mL). The reaction solution was reacted under reflux for 16 hours. The reaction solution was cooled and filtered, and the resulting solid was rinsed with dichloromethane (10 mL*2). The filtrates were combined and concentrated under reduced pressure. The resulting residues were purified by column chromatography (dichloromethane: methanol: concentrated ammonia = gradient elution from 99:1:0.1 to 95:5:0.5) to obtain intermediate 5-1 (401 mg, yield: 75%).

MS m/z (ESI): 532.3[M+H]$^+$.

Step 2: 2-((2-Methoxy-4-(4-methylpiperazin-1-yl)-5-nitrophenyl)amino)-4-(1-methyl-1H-indol-3-yl)pyrimidine-5-carboxylic acid (intermediate 5-2)

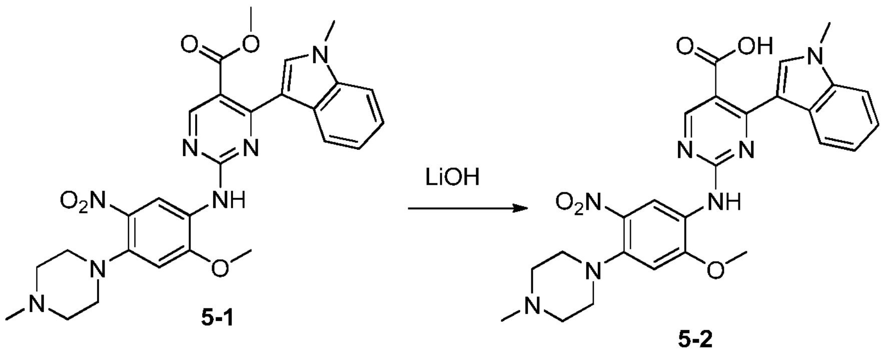


5-1

5-2

Intermediate 5-1 (400 mg, 0.75 mmol) was dissolved in tetrahydrofuran (5 mL) at room temperature, followed by the addition of lithium hydroxide monohydrate (42 mg, 1.0 mmol). The reaction solution was stirred at room temperature for 16 hours. The reaction solution was directly purified by preparative HPLC to obtain compound 5-2 (286 mg, yield: 73%).
MS m/z (ESI): 518.3[M+H]+.

Step 3: N-(4-Fluorobenzyl)-2-((2-methoxy-4-(4-methylpiperazin-1-yl)-5-nitrophenyl)amino)-N -methyl-4-(1-methyl-1H-indol-3-yl)pyrimidine-5-carboxamide

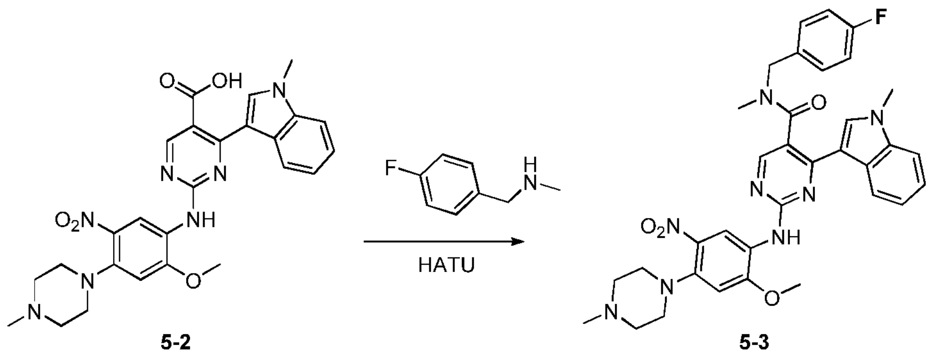


5-2

5-3

Intermediate 5-2 (200 mg, 0.39 mmol), 1-(4-fluorophenyl)-N-methylmethanamine (60 mg, 0.43 mmol), triethylamine (101 mg, 1.0 mmol) and 2-(7-azabenzotriazol-1-yl)-N,N,N',N'-tetramethyluronium hexafluorophosphate (190 mg, 0.50 mmol) were dissolved in dichloromethane (10 mL) at room temperature. The reaction solution was stirred at room temperature for 16 hours. The reaction solution was concentrated, and the resulting residues were purified by column chromatography (dichloromethane: methanol: concentrated ammonia = gradient elution from 99:1:0.1 to 94:6:0.6) to obtain intermediate 5-3 (143 mg, yield: 58%).
MS m/z (ESI): 639.1[M+H]+.

Step 4: N-(4-Fluorobenzyl)-2-((5-methacrylamido-2-methyl-4-(4-methylpiperazin-1-yl)phenyl) amino)-N-methyl-4-(1-methyl-1H-indol-3-yl)pyrimidine-5-carboxamide (compound **5**)

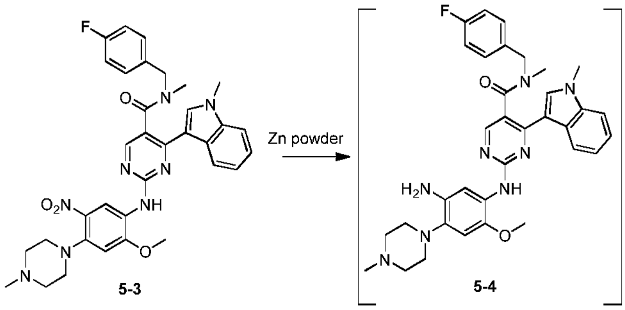


Zinc powder (40 mg, 0.62 mmol) was added to a solution of compound 5-3 (140 mg, 0.22 mmol) and ammonium chloride (118 mg, 2.2 mmol) in acetone (3 mL)/ water (1 mL) under stirring at room temperature. The reaction solution was reacted at room temperature for 2 hours. The reaction solution was filtered, the solid was rinsed with dichloromethane (10 mL*2) and water (5 mL*2) successively, and the filtrate was partitioned. The organic phase was washed with water (5 mL*2), dried over anhydrous sodium sulfate and filtered. The filtrate was concentrated to obtain the crude intermediate 5-4. The crude intermediate 5-4 was dissolved in dichloromethane (3 mL), followed by the successive addition of triethylamine (50 mg, 0.50 mmol) and methacryloyl chloride (31 mg, 0.30 mmol) under stirring at room temperature. The reaction solution was stirred at room temperature for 16 hours. The reaction solution was concentrated under reduced pressure, and the resulting residues were purified by preparative HPLC to obtain compound 5 (45 mg, yield: 31%).

MS m/z (ESI): 661.3 $[M+H]^+$.

**Example 6**

**Trans-N-(2-((2-(dimethylamino)ethyl)(methyl)amino)-5-((5-(3-(4-fluorophenyl)acry lamido)-4-(1-methyl-1H-indol-3-yl)pyrimidin-2-yl)amino)-4-(trifluoromethoxy)phe nyl)but-2-ynamide**

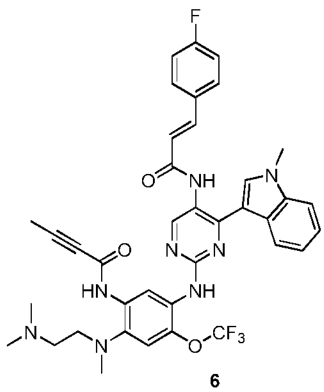

6

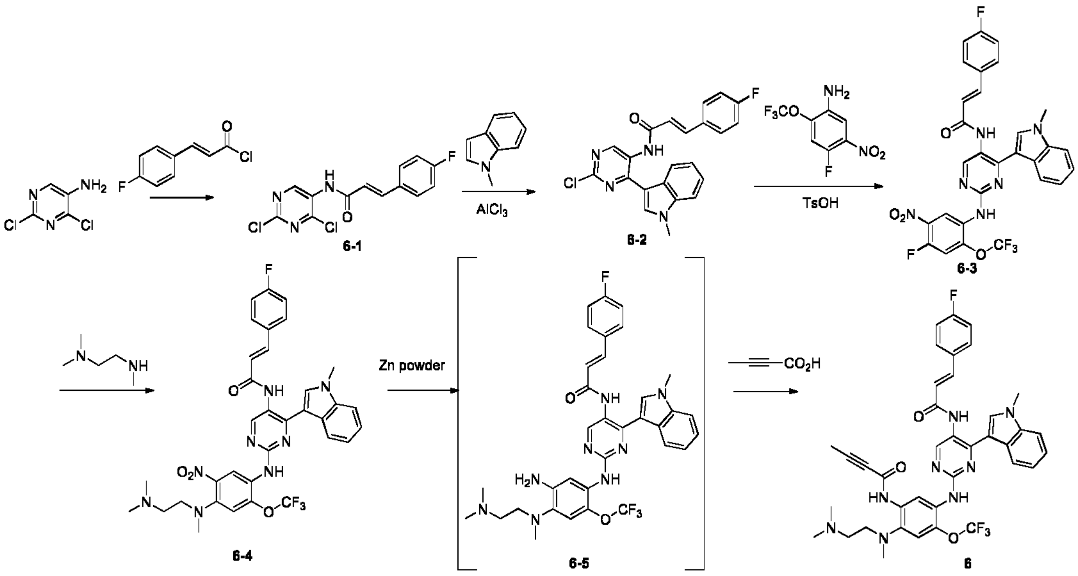

6-1 6-2 6-3 6-4 6-5 6

Step 1: Trans-N-(2,4-dichloropyrimidin-5-yl)-3-(4-fluorophenyl)acrylamide

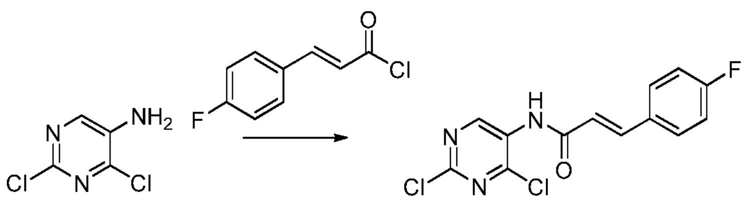
6-1

2,4-Dichloropyrimidin-5-amine (1.64 g, 10 mmol) and triethylamine (1.52 g, 15 mmol) were dissolved in dichloromethane (50 mL), followed by the addition of *p*-fluorocinnamyl chloride (1.84 g, 10 mmol) at room temperature. The reaction solution was stirred at room temperature for 2 hours. The reaction solution was concentrated, and the resulting residues were purified by column chromatography (petroleum ether: ethyl acetate = 90: 10 to 20: 80) to obtain intermediate 6-1 (2.14 g, yield: 68%).

MS m/z (ESI): 312.2[M+H]+.

Step 2: Trans-N-(2-chloro-4-(1-methyl-1H-indol-3-yl)pyrimidin-5-yl)-3-(4-fluorophenyl)acryla mide

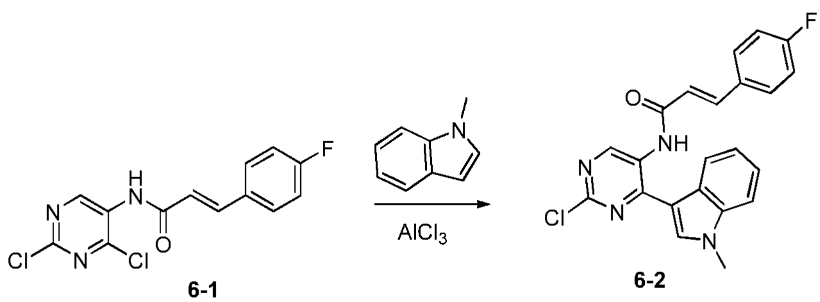

6-1 6-2

Aluminum trichloride (853 mg, 6.4 mmol) was added in batches to a solution of methyl 2,4-dichloropyrimidine-5-carboxylate (1.00 g, 3.2 mmol) in 1,2-dichloroethane (20 mL) at 0°C. The reaction solution was stirred at room temperature for 15 minutes, followed by the addition of 1-methyl-1H-indole (420 mg, 3.2 mmol). The reaction solution was

heated to 55°C and stirred for 1.5 hours. The reaction solution was cooled to 0°C, to which methanol (5 mL) and water (20 mL) were added slowly. The solution was stirred at room temperature for 30 minutes, and extracted with dichloromethane (20 mL*2). The organic phases were combined, dried over anhydrous sodium sulfate, and concentrated to dryness to obtain the crude product. The resulting crude product was purified by column chromatography (petroleum ether: ethyl acetate = 1:1) to obtain compound **6-2** (814 mg, yield: 62%).

MS m/z (ESI): 407.1[M+H]$^+$.

Step 3: Trans-N-(2-((4-fluoro-5-nitro-2-(trifluoromethoxy)phenyl)amino)-4-(1-methyl-1H-indol -3-yl)pyrimidin-5-yl)-3-(4-fluorophenyl)acrylamide

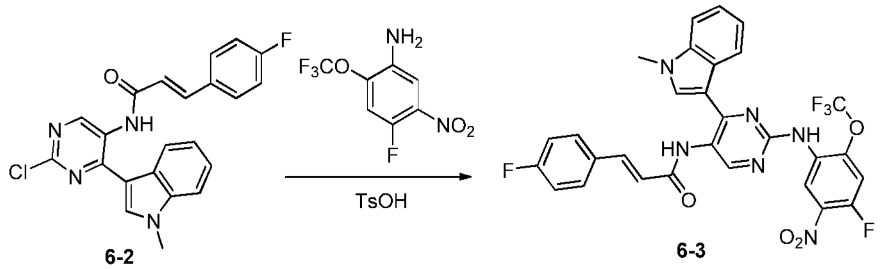


6-2

6-3

P-toluenesulfonic acid monohydrate (1.52 g, 8 mmol) was added to a solution of 4-fluoro-2-trifluoromethoxy-5-nitroaniline (576 mg, 2.40 mmol) and compound **6-2** (814 mg, 2.00 mmol) in dioxane (20 mL). The reaction solution was heated to 100°C and reacted for 16 hours. The reaction solution was cooled, and concentrated to dryness to obtain the crude product. The resulting crude product was purified by column chromatography (dichloromethane: methanol: concentrated ammonia = 100:0:0 to 97:3:0.3) to obtain compound **6-3** (722 mg, yield: 59%).

MS m/z (ESI): 611.1[M+H]$^+$.

Step 4: Trans-N-(2-((4-((2-(dimethylamino)ethyl)(methyl)amino)-5-nitro-2-(trifluoromethoxy)p henyl)amino)-4-(1-methyl-1H-indol-3-yl)pyrimidin-5-yl)-3-(4-fluorophenyl)acrylamide

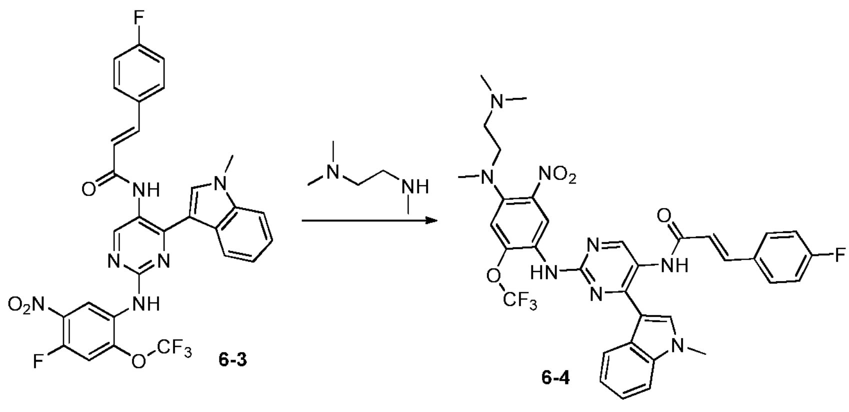

6-3

6-4

Potassium carbonate (138 mg, 1.0 mmol) was added to a solution of N,N,N'-trimethylethylenediamine (61 mg, 0.60 mmol) and compound 6-3 (305 mg, 0.50 mmol) in acetonitrile (10 mL). The reaction solution was heated to 80°C and reacted for 2 hours. The reaction solution was cooled, and concentrated to dryness. The resulting residues were dissolved in dichloromethane (20 mL), and washed with brine (15 mL*3). The organic phase was dried over anhydrous sodium sulfate, and concentrated to dryness to obtain the crude product. The resulting crude product was purified by column chromatography (dichloromethane: methanol: concentrated ammonia = 100:0:0 to 95:5:0.5) to obtain compound **6-4** (267 mg, yield: 77%).

MS m/z (ESI): 693.5[M+H]$^+$.

Step 5: Trans-N-(2-((2-(dimethylamino)ethyl)(methyl)amino)-5-((5-(3-(4-fluorophenyl)acrylam ido)-4-(1-methyl-1H-indol-3 -yl)pyrimidin-2-yl)amino)-4-(trifluoromethoxy)phenyl)but-2-ynamide

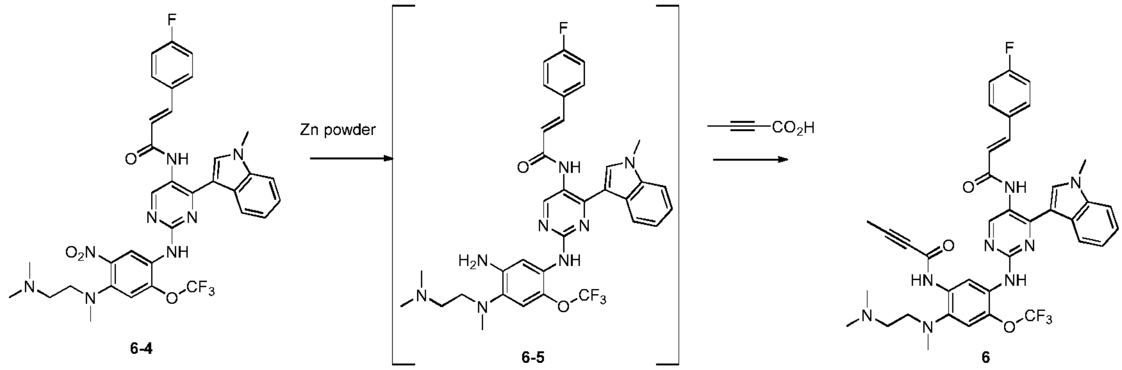


6-4 6-5 6

Zinc powder (37 mg, 0.57 mmol) was added to a solution of compound 6-4 (100 mg, 0.14 mmol) and ammonium chloride (77 mg, 1.4 mmol) in acetone (5 mL) and water (1 mL). The reaction solution was stirred at room temperature for 40 minutes. The reaction solution was filtered. Dichloromethane (10 mL) was added to the organic phase, and washed with water (5 mL*2). The solid was rinsed with dichloromethane (10 mL). The organic phases were combined, dried over anhydrous sodium sulfate, and concentrated to dryness to obtain the crude intermediate 6-5. The crude intermediate 6-5 was dissolved in dichloromethane (5 mL), followed by the successive addition of 2-butynoic acid (17 mg, 0.20 mmol), N,N-diisopropylethylamine (37 mg, 0.29 mmol) and 2-(7-azabenzotriazol-1-yl)-N,N,N',N'-tetramethyluronium hexafluorophosphate (76 mg, 0.20 mmol). The reaction solution was reacted at room temperature for 16 hours. The reaction solution was concentrated under reduced pressure, and the resulting residues were purified by preparative HPLC to obtain compound 6 (34.2 mg, yield: 32%).

MS m/z (ESI): 729.3[M+H]$^+$.

**Example 7**

**N-(6-((4-(1-Methyl-1H-indol-3-yl)pyrimidin-2-yl)amino)quinolin-4-yl)acrylamide**

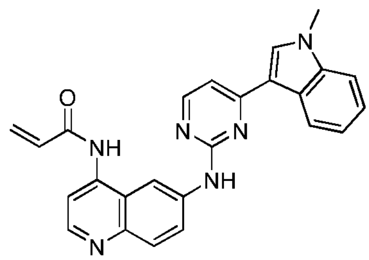

**7**

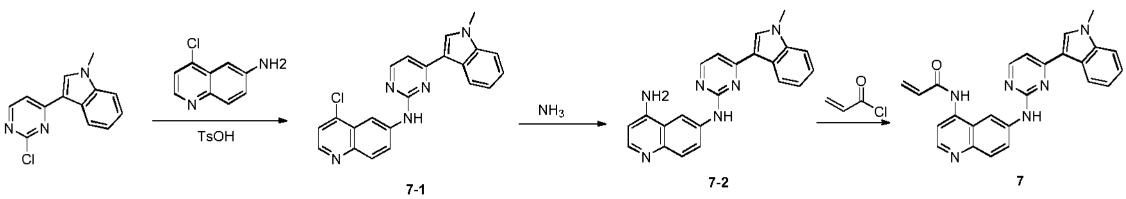


7-1 7-2 7

Step 1: 4-Chloro-N-(4-(1-methyl-1H-indol-3-yl)pyrimidin-2-yl)quinolin-6-amine (intermediate 7-1)

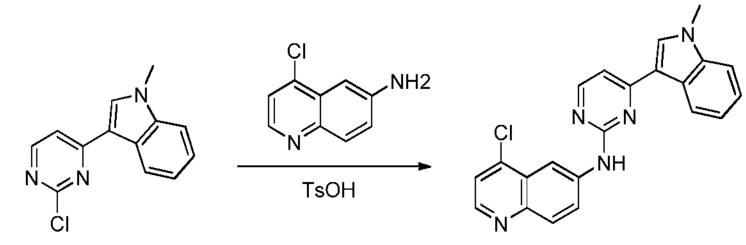


7-1

P-toluenesulfonic acid monohydrate (5.70 g, 30 mmol) was added to a solution of 4-chloroquinolin-6-amine (1.78 g, 10 mmol) and 3-(2-chloropyrimidin-4-yl)-1-methyl-1H-indole (2.43 g, 10 mmol) in dioxane (80 mL). The reaction solution was heated to 100°C and reacted for 16 hours. The reaction solution was cooled, and concentrated to dryness to obtain the crude product. The resulting crude product was purified by column chromatography (dichloromethane: methanol: concentrated ammonia = 100:0:0 to 97:3:0.3) to obtain compound 7-1 (1.73 g, yield: 45%).

MS m/z (ESI): 386.1[M+H]$^+$.

Step 2: *N*6-(4-(1-Methyl-1H-indol-3-yl)pyrimidin-2-yl)quinoline-4,6-diamine (intermediate **7-2**)

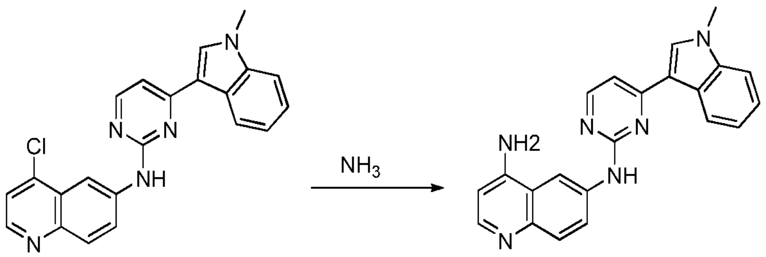


7-1 7-2

A mixture of intermediate 7-1 (1.00 g, 2.6 mmol) and phenol (10 g) was heated to 150°C. Ammonia gas was introduced and reacted for 2 hours. The reaction solution was cooled, to which water (100 mL) was added. 6M sodium hydroxide solution was added to adjust the pH to above 11, and the solution was extracted with dichloromethane (50 mL*3). The organic phases were combined, dried over anhydrous sodium sulfate, and concentrated under reduced pressure. The resulting residues were purified by column chromatography (dichloromethane: methanol: concentrated ammonia = 100:0:0 to 90:10:1) to obtain compound 7-3 (433 mg, yield: 46%).

MS m/z (ESI): 367.1[M+H]$^+$.

Step 3: N-(6-((4-(1-Methyl-1H-indol-3-yl)pyrimidin-2-yl)amino)quinolin-4-yl)acrylamide (compound 7)

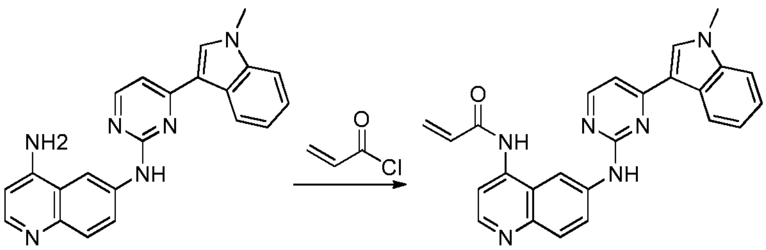

7-2 7

Intermediate 7-2 (100 mg, 0.27 mmol) and triethylamine (50 mg, 0.5 mmol) were dissolved in dichloromethane (5 mL), followed by the addition of acryloyl chloride (30 mg, 0.33 mmol) at room temperature. The reaction solution was stirred at room temperature for 2 hours. The reaction solution was concentrated, and the resulting residues were purified

by preparative HPLC to obtain compound 7 (21 mg, yield: 18%).

MS m/z (ESI): 421.2[M+H]$^+$.

**Example 8**

**N-(5-((4-(1-Cyclopropyl-1H-indol-3-yl)-5-(dimethylphosphono)pyrimidin-2-yl)ami no)-4-methoxy- 2-(9-methyl-3,9-diazaspiro [5.5] undec-3-yl)phenyl)acrylamide**

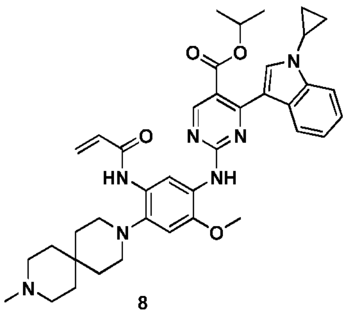

8

The compound of Example 8 was prepared by referring to the method of Example 2.

MS m/z (ESI): 678.2 [M+H]$^+$.

Example 9

**N-(5-((4-(1-Cyclopropyl-1H-indol-3-yl)-5-(dimethylphosphoryl)pyrimidin-2-yl)ami no)-4-methoxy-2-(9-methyl-3,9-diazaspiro[5.5]undecan-3-yl)phenyl)acrylamide**

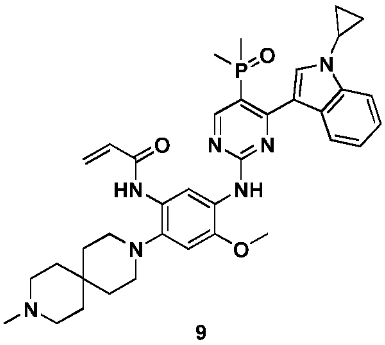

9

The compound of Example 9 was prepared by referring to the method of Example 2.

MS m/z (ESI): 668.3 [M+H]$^+$.

**Example 10**

**N-(5-((5-Chloro-4-(1-cyclopropyl-1H-indol-3-yl)pyrimidin-2-yl)amino)-2-((2-(dimet hylamino)ethyl)(methyl)amino)-4-methoxyphenyl)but-2-ynamide**

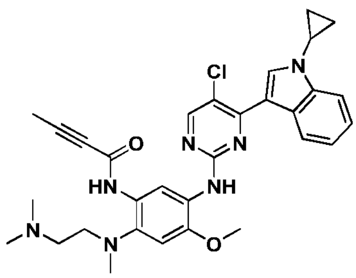

**10**

The compound of Example 10 was prepared by referring to the method of Example 1.

MS m/z (ESI): 572.3 $[M+H]^+$.

**Example 11**

**N-(5-((5-Azido-4-(1-methyl-1H-indol-3-yl)pyrimidin-2-yl)amino)-4-methoxy-2-(9-methyl-3,9-diazaspiro[5.5]undecan-3-yl)phenyl)acrylamide**

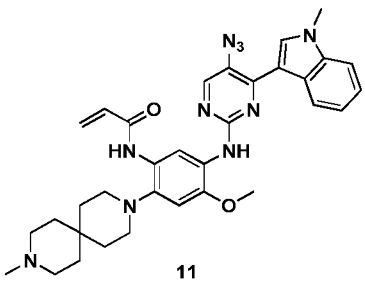

**11**

The compound of Example 11 was prepared by referring to the method of Example 3.

MS m/z (ESI): 607.1 $[M+H]^+$.

Example 12

Isopropyl **2-((5-acrylamido-2-methoxy-4-(methyl(quinuclidin-3-yl)amino)phenyl)amino)-4-(1 -methyl-1H-indol-3-yl)pyrimidine-5-carboxylate**

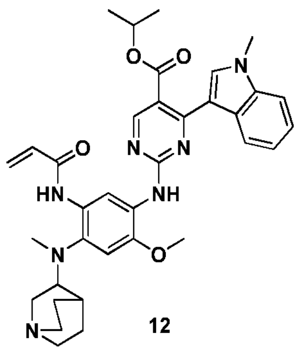

**12**

The compound of Example 12 was prepared by referring to the method of Example 4.

MS m/z (ESI): 624.1 $[M+H]^+$.

**Example 13**

**Isopropyl (E)-2-((3-(3-(1-cyclopropylpyrrolidin-2-yl)acrylamido)quinoxalin-6-yl)amino)-4-(1-methyl-1H-indol-3-yl)pyrimidine-5-carboxylate**

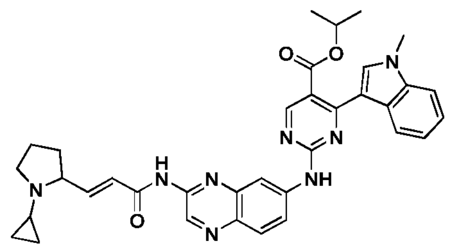

13

The compound of Example 13 was prepared by referring to the method of Example 7.
MS m/z (ESI): 617.3 [M+H]+.

**Example 14**

**2-((2-((1-Acryloylpiperidin-4-yl)oxy)quinolin-7-yl)amino)-4-(1-methyl-1H-indazol-3-yl)pyrimidine-5-carbonitrile**

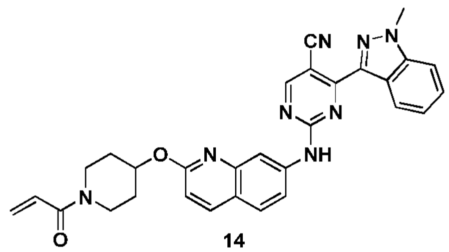

14

The compound of Example 14 was prepared by referring to the method of Example 7.
MS m/z (ESI): 531.2 [M+H]+.

**Example 15**

**Isopropyl (E)-2-((3-(4-(dimethylamino)but-2-enamido)-2-methylquinolin-6-yl)amino)-4-(1-m ethyl-1H-indol-3-yl)pyrimidine-5-carboxylate**

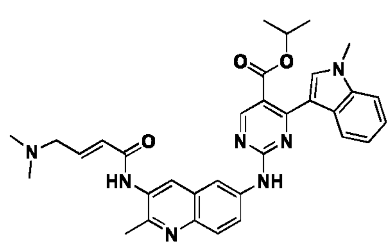

15

The compound of Example 15 was prepared by referring to the method of Example 7.
MS m/z (ESI): 578.1 [M+H]+.

**Example 16**

**Isopropyl 2-((5-acrylamido-4-(5-amino-5-methylhexahydrocyclopenta[c]pyrrol-2(1H)-yl)-2-m ethoxyphenyl)amino)-4-(1-cyclopropyl-1H-indol-pyridinyl-3-yl)pyrimidine-5-carbo xylate**

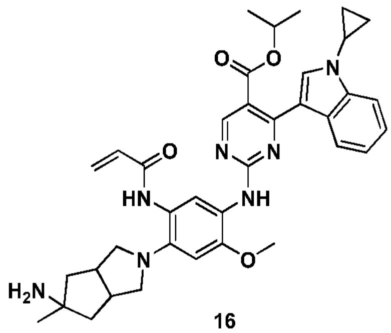

16

The compound of Example 16 was prepared by referring to the method of Example 4.
MS m/z (ESI): 650.2 $[M+H]^+$.

**Example 17**

**N-(5-((4-(1-Cyclopropyl-1Hthiazol-2-yl)pyrimidin-2-yl)amino)-4-me thoxy-2-(8-methyloctahydro-2H-pyrazino[1,2-a]pyrazin-2-yl)phenyl)acrylamide**

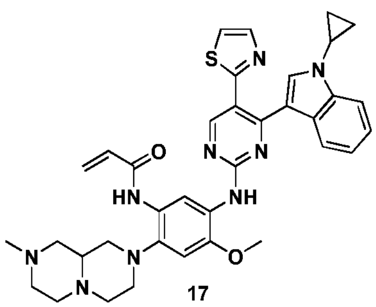

17

The compound of Example 17 was prepared by referring to the method of Example 3.
MS m/z (ESI): 662.2 $[M+H]^+$.

**Example 18**

**N-(2-(5-Amino-5-methylhexahydrocyclopenta[c]pyrrol-2(1H)-yl)-5-((4-(1-cyclopro pyl-1H-indol-3-yl)-5-(1H-pyrazol-1-yl)pyrimidin-2-yl)amino)-4-methoxyphenyl)acr ylamide**

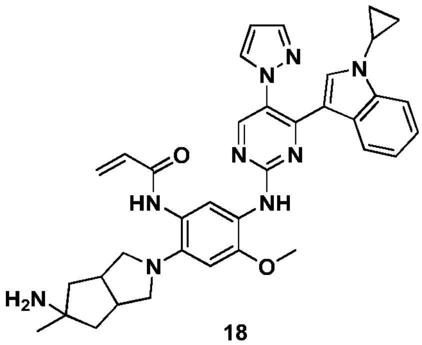

18

The compound of Example 18 was prepared by referring to the methods of Examples 3 and 4.

MS m/z (ESI): 630.1 $[M+H]^+$.

**Example 19**

**Isopropyl 2-((5-acrylamido-4-((3aR,6aS)-hexahydropyrrolo[3,4-c]pyrrol-2(1H)-yl)-2-methoxy phenyl)amino)-4-(1-cyclopropyl-1H-indol-3-yl)pyrimidine-5-carboxylate**

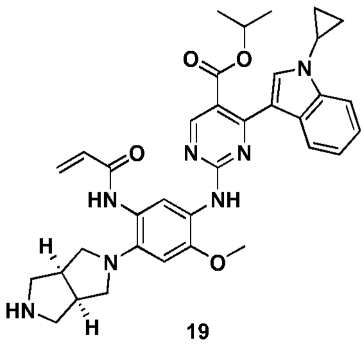

19

The compound of Example 19 was prepared by referring to the method of Example 2.

MS m/z (ESI): 622.3 $[M+H]^+$.

**Example 20**

**N-(5-((5-(Dimethylphosphoryl)-4-(1-(4-fluorobenzyl)-1H-indol-3-yl)pyrimidin-2-yl) amino)-4-ethoxy-2-(9-methyl-3,9-diazaspiro[5.5]undecan-3-yl)phenyl)acrylamide**

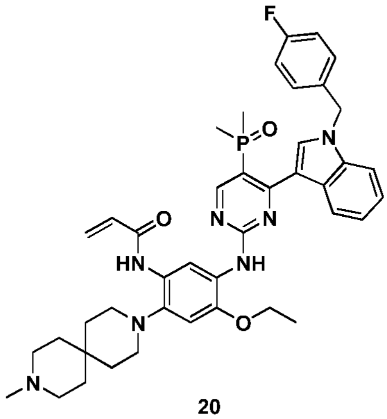

20

The compound of Example 20 was prepared by referring to the method of Example 3.

MS m/z (ESI): 750.4 $[M+H]^+$.

**Example 21**

**(E)-N-(5-((5-(3-(4-Cyanophenyl)ureido)-4-(1-cyclopropyl-1H-indol-3-yl)pyrimidin-2-yl)amino)-2-cyclopropoxyphenyl)-3-(1-methylpyrrolidin-2-yl)acrylamide**

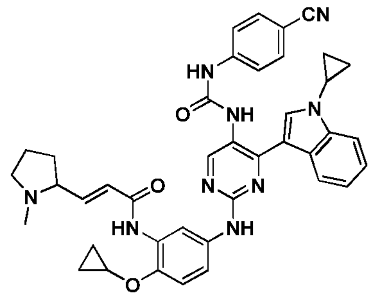

21

The compound of Example 21 was prepared by referring to the method of Example 5.

MS m/z (ESI): 694.1 $[M+H]^+$.

**Example 22**

**(E)-N-(5-((4-(1-Cyclopropyl-1H-indol-3-yl)-5-((4-(trifluoromethoxy)benzyl)oxy)pyr imidin-2-yl)amino)-2-methylphenyl)-3-(1-methylpyrrolidin-2-yl)acrylamide**

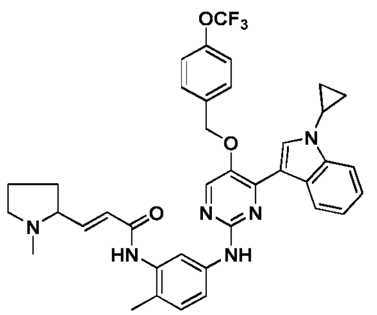

22

The compound of Example 22 was prepared by referring to the method of Example 5.
MS m/z (ESI): 683.3 [M+H]$^+$.

**Example 23**

**N-(5-((4-(1-Cyclopropyl-1H-indol-3-yl)-5-(prop-1-yn-1-yl)pyrimidin-2-yl)amino)-2-((1S,4S)-5-cyclopropyl-2,5-diazabicyclo[2.2.1]heptan-2-yl)-4-methoxyphenyl)acryla** mide

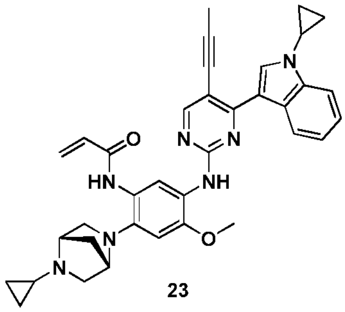

**23**

The compound of Example 23 was prepared by referring to the method of Example 3.
MS m/z (ESI): 600.2 [M+H]$^+$.

**Example 24**

**Isopropyl 2-((5-acrylamido-2-methoxy-4-(5-methylhexahydropyrrolo[3,4-c]pyrrol-2(1H)-yl)p henyl)amino)-4-(1-methyl-1H-indol-3-yl)pyrimidine-5-carboxylate**

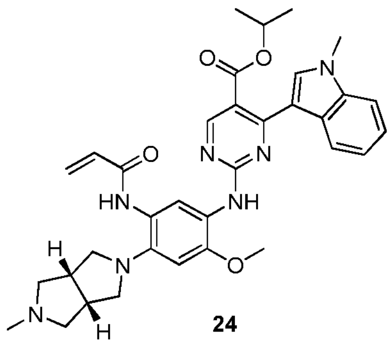

**24**

The compound of Example 24 was prepared by referring to the method of Example 1.
MS m/z (ESI): 610.3[M+H]$^+$.
$^1$H NMR (400 MHz, DMSO-*d*6) δ 8.66 - 8.58 (m, 2H), 8.24 (s, 1H), 8.08 (s, 1H), 7.78 (br, 1H), 7.52-7.46 (m, 1H), 7.25-7.15 (m, 1H), 7.15 - 7.02 (m, 1H), 6.78 (s, 1H), 6.66 (br, 1H), 6.39 - 6.19 (m, 1H), 5.83 - 5.76 (m, 1H), 5.12 - 4.91 (m, 1H), 3.87 (s, 3H), 3.82 (s, 3H), 3.15 - 3.01 (m, 9H), 2.81-2.70 (m, 5H), 1.22 - 0.98 (m, 6H).

**Example 25**

**Methyl 2-((5-acrylamido-2-methoxy-4-((1S,4S)-5-methyl-2,5-diazabicyclo[2.2.1]heptan-2-yl )phenyl)amino)-4-(1-methyl-1H-indol-3-yl)pyrimidine-5-carboxylate**

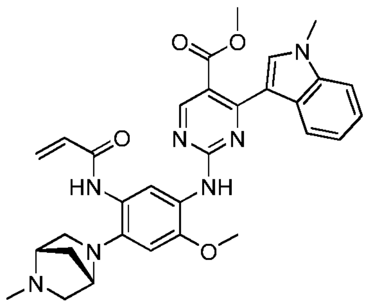

25

The compound of Example 25 was prepared by referring to the method of Example 2, and the synthetic route is as follows:

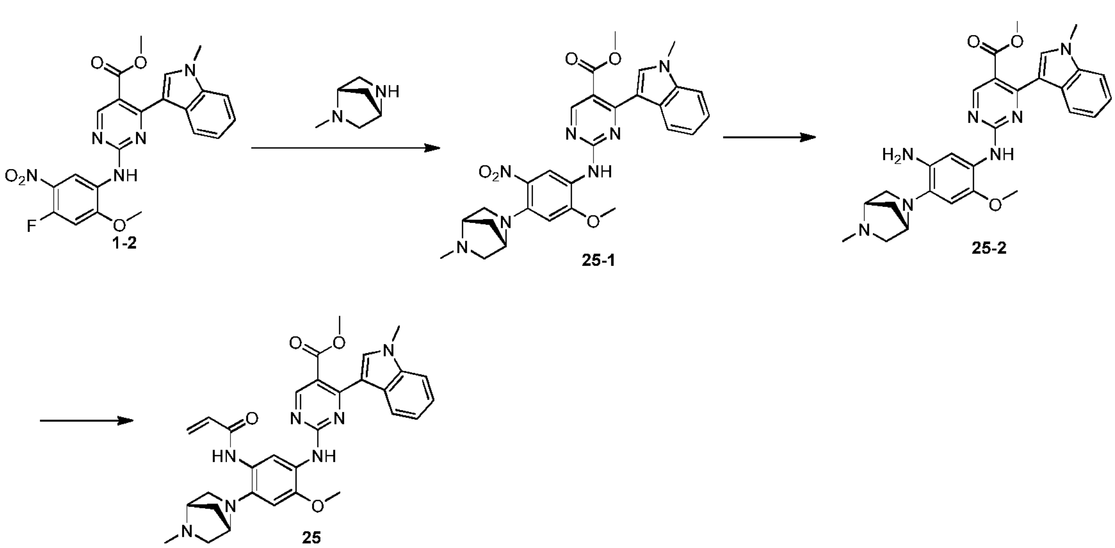

1-2 25-1 25-2

25

MS m/z (ESI): 568.1 $[M+H]^+$.

$^1H$ NMR (400 MHz, Methanol-*d*4) δ 8.74 (s, 1H), 8.68 - 8.31 (m, 3H), 7.99 (s, 1H), 7.86 - 7.71 (m, 1H), 7.51 - 7.38 (m, 1H), 7.28 - 7.18 (m, 1H), 7.18 - 7.05 (m, 1H), 6.61 (s, 1H), 6.54 - 6.41 (m, 1H), 6.41 - 6.27 (m, 1H), 5.86 - 5.73 (m, 1H), 4.48 - 4.32 (m, 1H), 4.29 - 4.16 (m, 1H), 3.96 (s, 3H), 3.89 (s, 3H), 3.82 - 3.66 (m, 4H), 3.66 - 3.55 (m, 1H), 3.38 - 3.34 (m, 1H), 3.27 - 3.21 (m, 1H), 2.90 (s, 3H), 2.35 - 2.19 (m, 2H).

## Example 26

**Isopropyl 2-((5-acrylamido-2-methoxy-4-(6-methyl-2,6-diazaspiro[3.3]heptan-2-yl)phenyl)am ino)-4-(1-methyl-1H-indol-3-yl)pyrimidine-5-carboxylate**

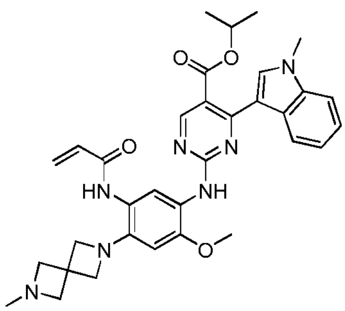

26

The compound of Example 26 was prepared by referring to the method of Example 2.

MS m/z (ESI): 596.4 $[M+H]^+$.

$^1$H NMR (400 MHz, Methanol-*d*4) δ 8.69 (s, 1H), 8.40 - 8.20 (m, 1H), 8.00 - 7.83 (m, 1H), 7.83 - 7.70 (m, 1H), 7.50 - 7.36 (m, 1H), 7.30 - 7.17 (m, 1H), 7.18 - 7.04 (m, 1H), 6.59 - 6.18 (m, 3H), 5.86 - 5.71 (m, 1H), 5.55 - 5.41 (m, 1H), 5.10 - 4.98 (m, 1H), 4.68 - 4.63 (m, 1H), 4.46 - 4.40 (m, 1H), 4.20 - 4.02 (m, 3H), 4.00 - 3.76 (m, 6H), 3.70 - 3.54 (m, 2H), 3.12 (s, 3H), 2.55 - 2.40 (m, 2H), 1.22 - 0.98 (m, 6H).

**Example 27**

**N-(5-((4-(1-Cyclopropyl-1H-indol-3-yl)-5-(trifluoromethyl)pyrimidin-2-yl)amino)-4 -methoxy-2-((3aR,6aS)-5-methylhexahydropyrrolo[3,4-c]pyrrol-2(1H)-yl)phenyl)ac** rylamide

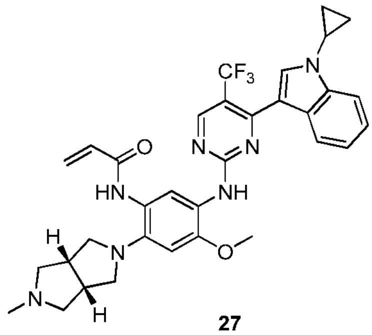

**27**

The compound of Example 27 was prepared by referring to the method of Example 2.

MS m/z (ESI): 618.2 $[M+H]^+$.

**Example 28**

**N-(5-((5-Bromo-4-(1-cyclopropyl-1H-indol-3-yl)pyrimidin-2-yl)amino)-4-methoxy-2-((3aR,6aS)-5-methylhexahydropyrrolo[3,4-c]pyrrol-2(1H)-yl)phenyl)acrylamide**

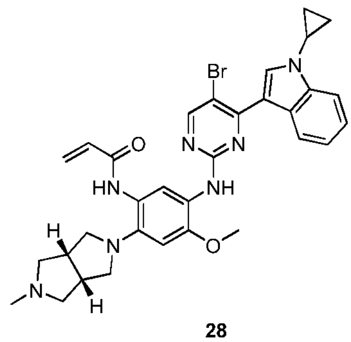

**28**

The compound of Example 28 was prepared by referring to the method of Example 2, and the synthetic route is as follows:

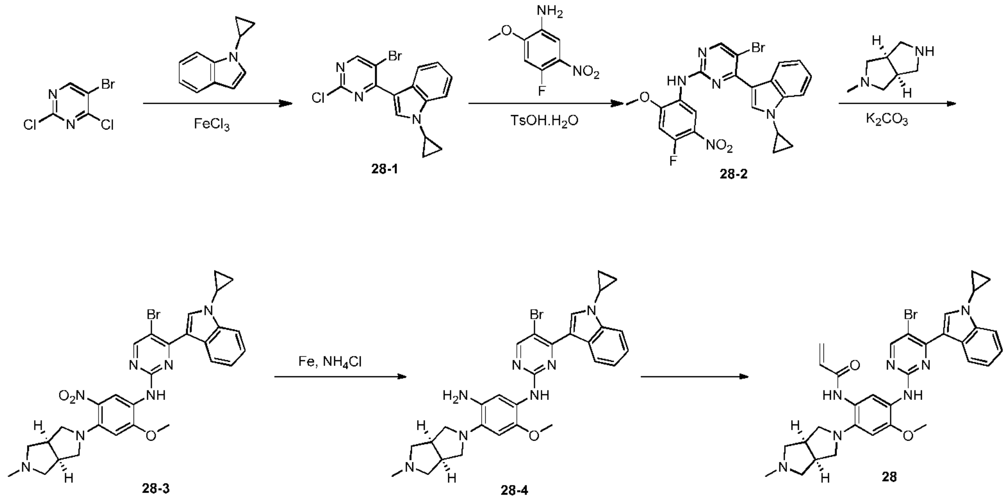


Step 1: Preparation of 3-(5-bromo-2-chloropyrimidm-4-yl)-1-cyclopropyl-1H-indole

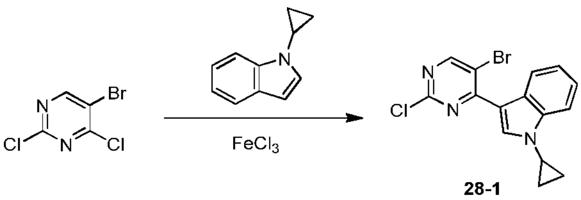


5-Bromo-2,4-dichloropyrimidine (1.2 g, 6.3 mmol) was dissolved in 1,2-dichloroethane (10 mL), and the solution was cooled to 0°C. Ferric chloride (1.7 g, 10.6 mmol) was added, and the reaction solution was stirred at room temperature for half an hour. 1-Cyclopropyl-1H-indole (0.83 g, 5.3 mmol) was added, and the reaction solution was stirred at 60°C for 1 hour. Water (30 mL) was added, and the solution was filtered. The filtrate was extracted with dichloromethane (20 mL × 3). The organic phases were combined, dried over anhydrous sodium sulfate, filtered and concentrated to dryness by rotary evaporation. The resulting crude product was purified by column chromatography (petroleum ether/ethyl acetate: 100/1 ~ dichloromethane/ethyl acetate: 5/1) to obtain 3-(5-bromo-2-chloropyrimidin-4-yl)-1-cyclopropyl-1H-indole (0.7 g, yield: 38%) as a brown solid.

Step 2: Preparation of 5-bromo-4-(1-cyclopropyl-1H-indol-3-yl)-N-(4-fluoro-2-methoxy-5-nitrophenyl)pyrimi din-2-amine

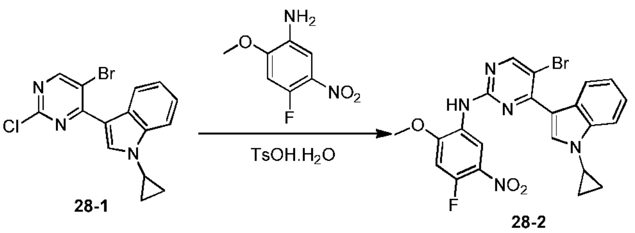


3-(5-Bromo-2-chloropyrimidin-4-yl)-1-cyclopropyl-1H-indole (0.4 g, 1.2 mmol), 4-fluoro-2-methoxy-5-nitroaniline (0.32 g, 1.7 mmol) andp-toluenesulfonic acid (1.14 g, 6.0 mmol) were dissolved in dioxane (10 mL). The reaction solution was stirred at 100°C overnight. The reaction solution was cooled to room temperature, to which water (10 mL) was added. The solution was extracted with dichloromethane (10 mL × 2). The organic phase was dried over anhydrous sodium sulfate, filtered and concentrated to dryness by rotary evaporation. The resulting crude product was purified by column chromatography (dichloromethane/ethyl acetate: 100/1 ~3/1) to obtain 5-bromo-4-(1-cyclopropyl-1H-indol-3-yl)-N-(4-fluoro-2-methoxy-5-nitrophenyl)pyrimi din-2-amine (0.3 g, 53%) as a yellow solid.

MS m/z (ESI): 498.1 $[M+H]^+$.

Step 3: Preparation of 5-bromo-4-(1-cyclopropyl-1H-indol-3-yl)-N-(2-methoxy-4-((3aR,6aS)-5-methylhexahy dropyrrolo[3,4-c]pyrrol-2(1H)-yl)-5-nitrophenyl)pyrimidin-2-amine

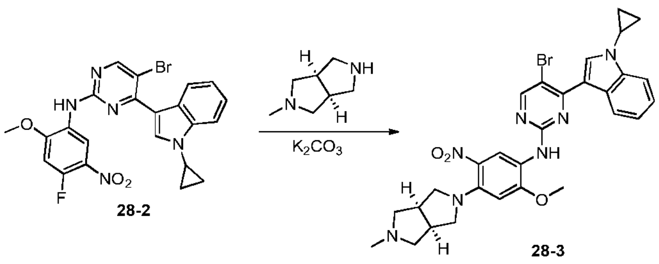


5-Bromo-4-(1-cyclopropyl-1H-indol-3-yl)-N-(4-fluoro-2-methoxy-5-nitrophenyl)p yrimidin-2-amine (100 mg, 0.2 mmol) was dissolved in acetonitrile (10 mL), followed by the addition of potassium carbonate (83 mg, 0.6 mmol) and (3aR,6aS)-2-methyloctahydropyrrolo[3,4-c]pyrrole (30 mg, 0.24 mmol). The reaction solution was stirred at 80°C for 1 hour. The reaction solution was cooled to room temperature and filtered. The filtrate was concentrated to dryness by rotary evaporation to obtain the crude 5-bromo-4-(1-cyclopropyl-1H-indol-3-yl)-N-(2-methoxy-4-((3aR,6aS)-5-methylhexahy dropyrrolo[3,4-c]pyrrol-2(1H)-yl)-5-nitrophenyl)pyrimidin-2-amine (100 mg, yield: 83%) as a red gel.
MS m/z (ESI): 604.1 $[M+H]^+$.

Step 4: Preparation of N1-(5-bromo-4-(1-cyclopropyl-1H-indol-3-yl)pyrimidin-2-yl)-6-methoxy-4-((3aR,6aS)-5-methylhexahydropyrrolo[3,4-c]pyrrol-2(1H)-yl)benzene-1,3-diamine

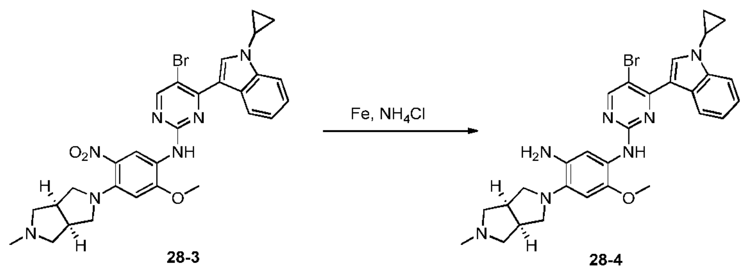


5-Bromo-4-(1-cyclopropyl-1H-indol-3-yl)-N-(2-methoxy-4-((3aR,6aS)-5-methylhe xahydropyrrolo[3,4-c]pyrrol-2(lH)-yl)-5-nitrophenyl)pyrimidin-2-amine (100 mg, 0.17 mmol) was dissolved in ethanol (5 mL), followed by the addition of saturated ammonium chloride solution (2.5 mL) and iron powder (46 mg, 0.83 mmol). The reaction solution was stirred at 70°C for 1 hour. The reaction solution was cooled to room temperature and filtered. The filtrate was extracted with dichloromethane (20 mL × 2). The organic phase was dried over anhydrous sodium sulfate, filtered and concentrated to dryness by rotary evaporation to obtain N1-(5-bromo-4-(1-cyclopropyl-1H-indol-3-yl)pyrimidin-2-yl)-6-methoxy-4-((3aR,6aS)-5-methylhexahydropyrrolo[3,4-c]pyrrol-2(1H)-yl)benzene-1,3-diamine (100 mg, yield: 100% crude) as a yellow solid.
MS m/z (ESI): 474.1 $[M+H]^+$.

Step 5: Preparation of N-(5-((5-bromo-4-(1-cyclopropyl-1H-indol-3-yl)pyrimidin-2-yl)amino)-4-methoxy-2-(( 3aR,6aS)-5-methylhexahydropyrrolo[3,4-c]pyrrol-2(1H)-yl)phenyl)acrylamide

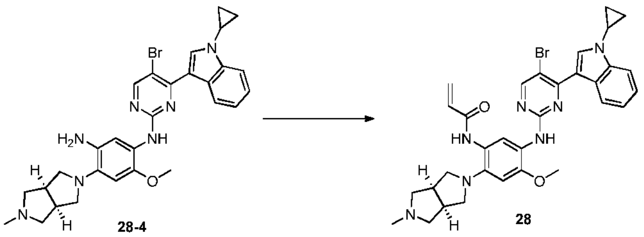

28-4 28

N1-(5-Bromo-4-(1-cyclopropyl-1H-indol-3-yl)pyrimidin-2-yl)-6-methoxy-4-((3aR, 6aS)-5-methylhexahydropyrrolo[3,4-c]pyrrol-2(1H)-yl)benzene-1,3-diamine (30 mg, 0.05 mmol) was dissolved in tetrahydrofuran (1 mL), and the solution was cooled to 0°C. Triethylamine (10.6 mg, 0.1 mmol) and 3-chloropropionyl chloride (6.6 mg, 0.05 mmol) were added successively. The reaction solution was stirred at 0°C for 1 hour. Water (5 mL) was added, and the solution was extracted with dichloromethane (5 mL × 2). The organic phase was dried over anhydrous sodium sulfate, filtered and concentrated to dryness by rotary evaporation. The resulting crude product was dissolved in acetonitrile (3 mL), followed by the addition of sodium hydroxide (18 mg, 0.5 mmol) in water (0.5 mL). The reaction solution was stirred at room temperature for 1 hour. Water (5 mL) was added, and the solution was extracted with dichloromethane (10 mL × 2). The organic phase was dried over anhydrous sodium sulfate, filtered and concentrated to dryness by rotary evaporation. The resulting crude product was purified by prep-TLC to obtain N-(5-((5-bromo-4-(1-cyclopropyl-1H-indol-3-yl)pyrimidin-2-yl)amino)-4-methoxy-2-(( 3aR,6aS)-5-methylhexahydropyrrolo[3,4-c]pyrrol-2(1H)-yl)phenyl)acrylamide (5.4 mg, yield: 19%) as a pale yellow solid.

MS m/z (ESI): 628.1 $[M+H]^+$.

## Example 29

### N-(5-((4-(1-Cyclopropyl-1H-indol-3-yl)pyrimidin-2-yl)amino)-4-methoxy-2-((3aR,6 aS)-5-methylhexahydropyrrolo [3,4-c] pyrrol-2(1H)-yl)phenyl)acrylamide

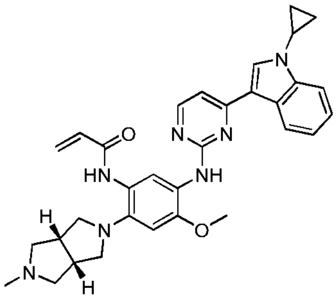

29

The compound of Example 29 was prepared by referring to the method of Example 2.

MS m/z (ESI): 550.3 $[M+H]^+$.

$^1$H NMR (400 MHz, $CDCl_3$) δ 9.60 *(br,* 1H), 9.03-8.83 *(br,* 1H), 8.50 (s, 1H), 8.40-8.37 (m, 1H), 8.14-8.11 (m, 1H), 7.65-7.60 (m, 2H), 7.35-7.28 (m, 2H), 7.16 (d, *J* = 5.2 Hz, 1H), 6.76 (s, 1H), 6.45-6.37 (m, 2H), 5.76-5.70 (m, 1H), 3.88 (s, 3H), 3.48-3.40 (m, 1H), 3.05-2.81 (m, 8H),2.68-2.55 (m, 2H), 2.48 (s, 3H), 1.25-1.15 (m, 4H).

## Example 30

### N-(5-((4-(1-Cyclopropyl-1H-indol-3-yl)-5-(trifluoromethyl)pyrimidin-2-yl)amino)-4 -methoxy-2-((3aR,6aS)-5-methylhexahydropyrrolo[3,4-c]pyrrol-2(1H)-yl)phenyl)b ut-2-ynamide

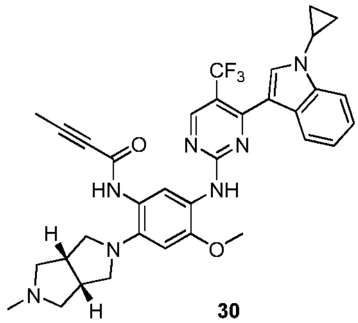

30

The compound of Example 30 was prepared by referring to the method of Example 1.

MS m/z (ESI): 630.4 $[M+H]^+$.

$^1$H NMR (400 MHz, $CDCl_3$) δ 9.19-9.08 *(br,* 1H), 8.72 (s, 1H), 8.54-8.40 *(br,* 1H), 8.24 (d, *J* = 8.0 Hz, 1H), 7.74-7.70 (m, 2H), 7.60 (d, *J* = 8.0 Hz, 1H), 7.32-7.24 (m, 2H), 6.74 (s, 1H), 3.85 (s, 3H), 3.47-3.42 (m, 1H), 3.08-2.86 (m, 8H), 2.56-2.48 (m, 2H), 2.47 (s, 3H), 2.01 (s, 3H), 1.15-1.03 (m, 4H).

**Example 31**

**2-((5-Acrylamido-2-methoxy-4-((3aR,6aS)-5-methylhexahydropyrrolo[3,4-c]pyrrol -2(1H)-yl)phenyl)amino)-N,N-dimethyl-4-(1-methyl-1H-indol-3-yl)pyrimidine-5-ca rboxamide**

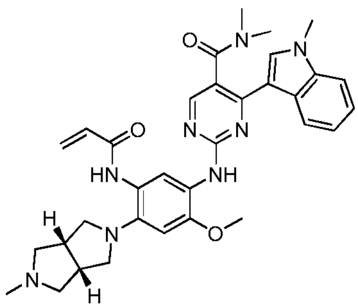

31

The compound of Example 31 was prepared by referring to the method of Example 5 with intermediate 1-2 as the starting material, and the specific synthetic route is as follows:

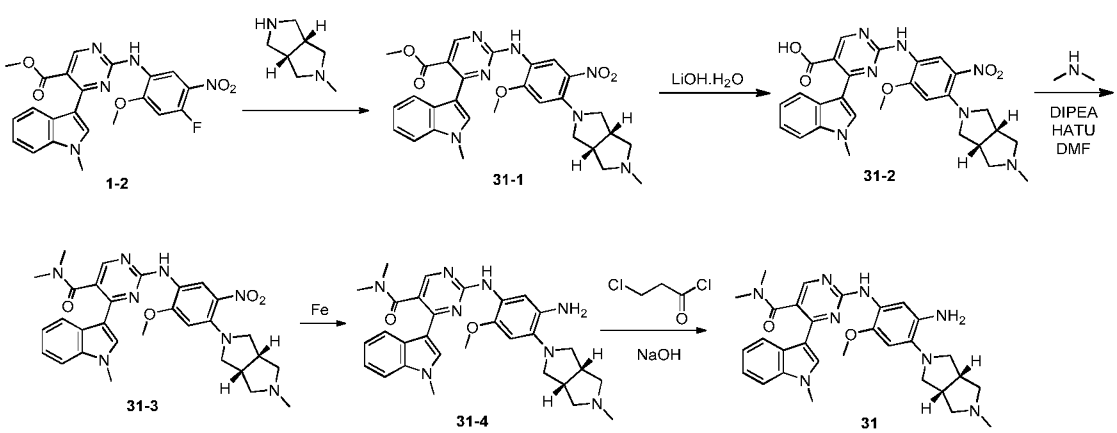


1-2 31-1 31-2 31-3 31-4 31

MS m/z (ESI): 595.2 $[M+H]^+$.

$^1$H NMR (400 MHz, DMSO-*d*6) δ 9.18 - 9.07 (m, 1H), 8.45 - 8.35 (m, 1H), 8.28 - 8.20 (m, 1H), 8.21 - 8.11 (m, 1H), 8.11 - 8.00 (m, 1H), 7.90 - 7.80 (m, 1H), 7.47 - 7.35 (m, 1H), 7.20 - 7.07 (m, 1H), 7.07 - 6.93 (m, 1H), 6.70 (s, 1H),

6.57 - 6.39 (m, 1H), 6.19 - 6.03 (m, 1H), 5.73 - 5.55 (m, 1H), 3.89 - 3.64 (m, 6H), 3.45-3.38 (m, 4H), 3.20 - 3.13 (m, 8H), 2.86 - 2.66 (m, 4H), 2.20 (s, 3H).

**Example 32**

**2-((5-Acrylamido-2-methoxy-4-((3aR,6aS)-5-methylhexahydropyrrolo[3,4-c]pyrrol -2(1H)-yl)phenyl)amino)-N-cyclopropyl-4-(1-methyl-1H-indol-3-yl)pyrimidine-5-ca** rboxamide

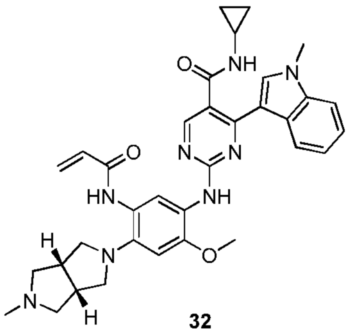

**32**

The compound of Example 32 was prepared by referring to the method of Example 5.
MS m/z (ESI): 607.1 $[M+H]^+$.
$^1H$ NMR (400 MHz, DMSO-*d*6) δ 9.18 - 9.07 (m, 1H), 8.45 - 8.35 (m, 1H), 8.28 - 8.20 (m, 1H), 8.21 - 8.11 (m, 2H), 8.11 - 8.00 (m, 1H), 7.90 - 7.80 (m, 1H), 7.47 - 7.35 (m, 1H), 7.20 - 7.07 (m, 1H), 7.07 - 6.93 (m, 1H), 6.70 (s, 1H), 6.57 - 6.39 (m, 1H), 6.19 - 6.03 (m, 1H), 5.73 - 5.55 (m, 1H), 3.89 - 3.64 (m, 6H), 3.45-3.38 (m, 5H), 3.18 - 3.06 (m, 2H), 2.86 - 2.66 (m, 4H), 2.20 (s, 3H), 0.70 - 0.47 (m, 2H), 0.47 - 0.24 (m, 2H).

**Example 33**

**2-((5-Acrylamido-4-((2-(dimethylamino)ethyl)(methyl)amino)-2-methoxyphenyl)a mino)-N-benzyl-4-(1-methyl-1H-indol-3-yl)pyrimidine-5-carboxamide**

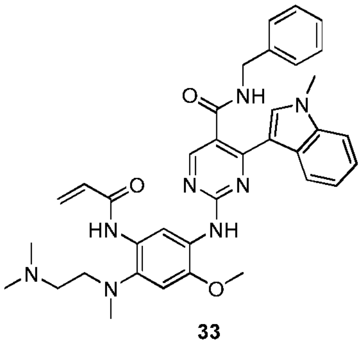

**33**

The compound of Example 33 was prepared by referring to the method of Example 5.
MS m/z (ESI): 633.3 $[M+H]^+$.

**Example 34**

**2-((5-Acrylamido-4-((2-(dimethylamino)ethyl)(methyl)amino)-2-methoxyphenyl)a mino)-N-(1-benzyl-1H-pyrazol-4-yl)-4-(1-methyl-1H-indol-3-yl)pyrimidine-5-carbo xamide**

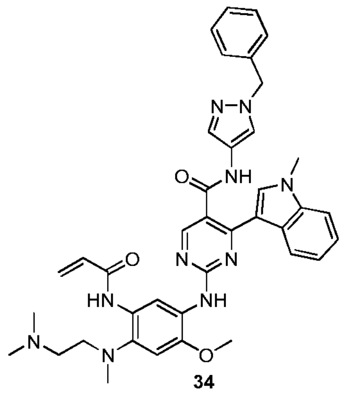

34

The compound of Example 34 was prepared by referring to the method of Example 5.

MS m/z (ESI): 699.1 $[M+H]^+$.

$^1H$ NMR (400 MHz, DMSO-*d6*) δ 10.55 - 10.42 (m, 1H), 10.18 - 10.06 (m, 1H), 8.81 - 8.67 (m, 1H), 8.54 - 8.43 (m, 1H), 8.43 - 8.34 (m, 1H), 8.19 - 7.99 (m, 2H), 8.00 - 7.90 (m, 1H), 7.53 - 7.40 (m, 2H), 7.40 - 7.26 (m, 3H), 7.27 - 7.19 (m, 2H), 7.21 - 7.12 (m, 1H), 7.11 - 7.01 (m, 1H), 7.01 - 6.91 (m, 1H), 6.48 - 6.34 (m, 1H), 6.31 - 6.12 (m, 1H), 5.88 - 5.67 (m, 1H), 5.31 (s, 2H), 3.99 - 3.62 (m, 6H), 2.98 - 2.82 (m, 2H), 2.74 (s, 3H), 2.42 - 2.27 (m, 2H), 2.29 - 2.02 (m, 6H).

**Example 35**

**N-(5-((5-Cyano-4-(1-cyclopropyl-1H-indol-3-yl)pyrimidin-2-yl)amino)-4-methoxy-2 -((3aR,6aS)-5-methylhexahydropyrrolo[3,4-c]pyrrol-2(1H)-yl)phenyl)acrylamide**

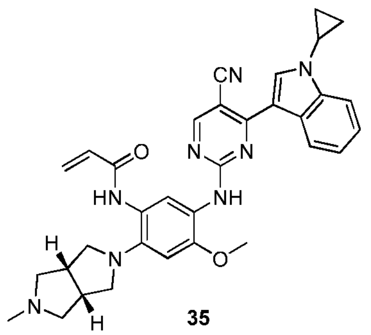

35

The compound of Example 35 was prepared by referring to the method of Example 2, and the synthetic route is as follows:

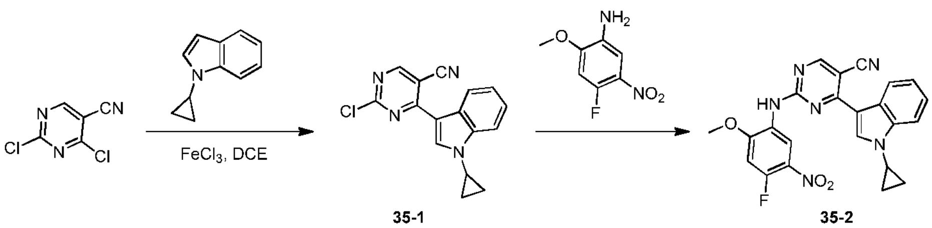


35-1

35-2

Step 1: Preparation of 2-chloro-4-(1-cyclopropyl-1H-indol-3-yl)pyrimidine-5-carbonitrile

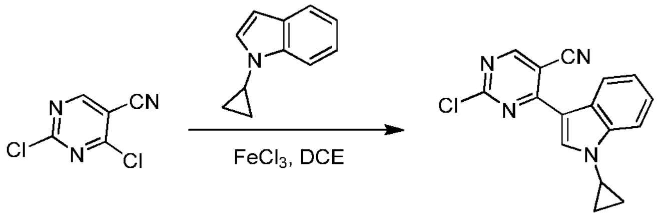


35-1

2,4-Dichloropyrimidine-5-carbonitrile (2 g, 12 mmol) was dissolved in dichloroethane (30 mL), and the solution was cooled to 0°C. Ferric chloride (3.9 g, 24 mmol) was added, and the reaction solution was stirred at room temperature for half an hour. 1-Cyclopropyl-1H-indole (2.17 g, 14 mmol) was added, and the reaction solution was stirred at 60°C for 2 hours. Water (30 mL) was added, and the solution was filtered. The filtrate was extracted with dichloromethane (30 mL x 2). The organic phases were combined, dried over anhydrous sodium sulfate, filtered and concentrated to dryness by rotary evaporation. The resulting crude product was purified by column chromatography (petroleum ether/ethyl acetate: 100/1 ~ 3/1) to obtain 2-chloro-4-(1-cyclopropyl-1H-indol-3-yl)pyrimidine-5-carbonitrile (1.6 g, yield: 47%) as a brown solid.

Step 2: Preparation of 4-(1-cyclopropyl-1H-indol-3-yl)-2-((4-fluoro-2-methoxy-5-nitrophenyl)amino)pyrimidi ne-5-carbonitrile

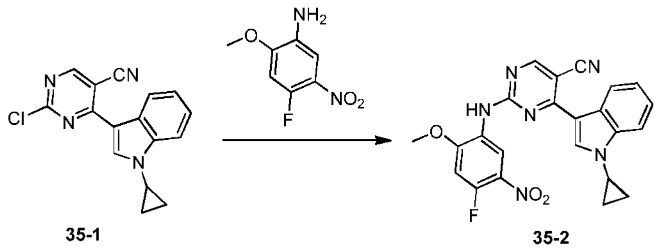

35-1 35-2

2-Chloro-4-(1-cyclopropyl-1H-indol-3-yl)pyrimidine-5-carbonitrile (1 g, 3.4 mmol), 4-fluoro-2-methoxy-5-nitroaniline (0.7 g, 3.7 mmol) and *p*-toluenesulfonic acid (0.7 g, 3.7 mmol) were dissolved in 2-pentanol (40 mL). The reaction solution was stirred at 100°C overnight. The reaction solution was cooled to room temperature, to which water (50 mL) was added. The solution was extracted with dichloromethane (50 mL x 2). The organic phase was dried over anhydrous sodium sulfate, filtered and concentrated to dryness by rotary evaporation. The resulting crude product was purified by column chromatography (petroleum ether/ethyl acetate: 100/1 ~1/1 ~ dichloromethane/ethyl acetate: 100/1 ~10/1) to obtain 4-(1-cyclopropyl-1H-indol-3-yl)-2-((4-fluoro-2-methoxy-5 -nitrophenyl)amino)pyrimidi ne-5-carbonitrile (1 g, 67%) as a brown solid.

Steps 3 to 5 of Example 35 were carried out by referring to Steps 3 to 5 of Example 28.

MS m/z (ESI): 575.2 $[M+H]^+$.

$^1$H NMR (400 MHz, $CDCl_3$) δ 9.20 - 9.05 (m, 1H), 8.88 - 8.73 (m, 1H), 8.64 (s, 1H), 8.56 - 8.41 (m, 2H), 7.73 (s, 1H), 7.66 - 7.58 (m, 1H), 7.34 - 7.28 (m, 1H), 6.74 (s, 1H), 6.46 - 6.30 (m, 2H), 5.76 - 5.69 (m, 1H), 3.88 (s, 3H), 3.52 - 3.41 (m, 1H), 3.05 - 2.90 (m, 6H), 2.88 - 2.59 (m, 5H), 2.56 - 2.44 (m, 3H), 1.19 - 1.09 (m, 4H).

**Example 36**

**N-(5-((4-(1-Cyclopropyl-1H-indol-3-yl)-5-(oxazol-2-yl)pyrimidin-2-yl)amino)-4-met hoxy-2-((3aR,6aS)-5-methylhexahydropyrrolo [3,4-c]pyrrol-2(1H)-yl)phenyl)acryla mide**

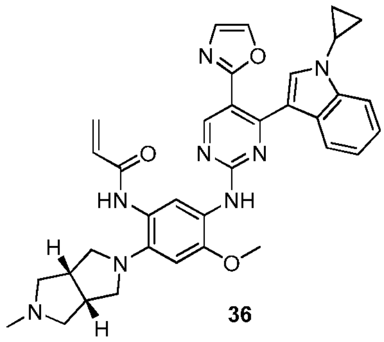

36

The compound of Example 36 was prepared by referring to the method of Example 3, and the synthetic route is as follows:

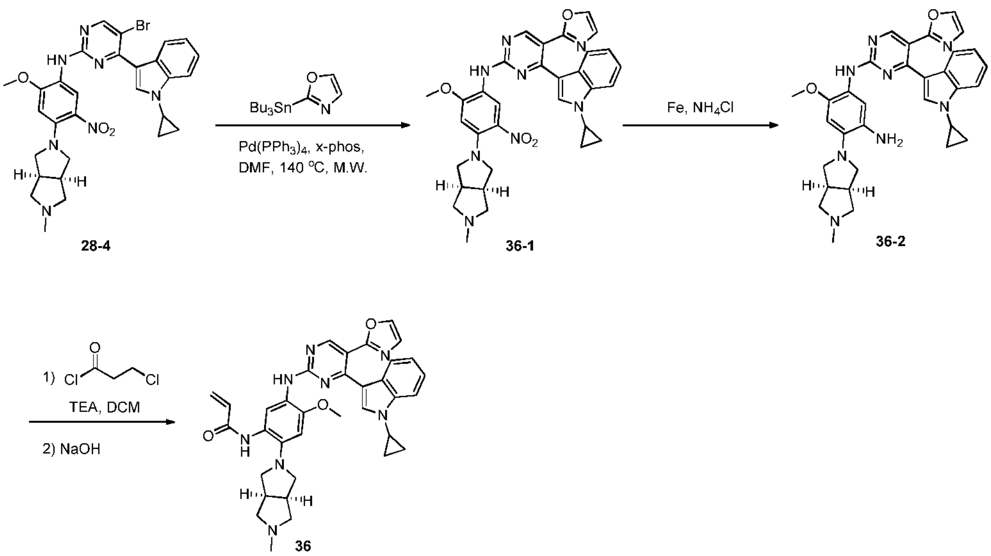


Step 1: Preparation of 4-(1-cyclopropyl-1H-indol-3-yl)-N-(2-methoxy-4-((3aR,6aS)-5-methylhexahydropyrrol o[3,4-c]pyrrol-2(1H)-yl)-5-nitrophenyl)-5-(oxazol-2-yl)pyrimidin-2-amine

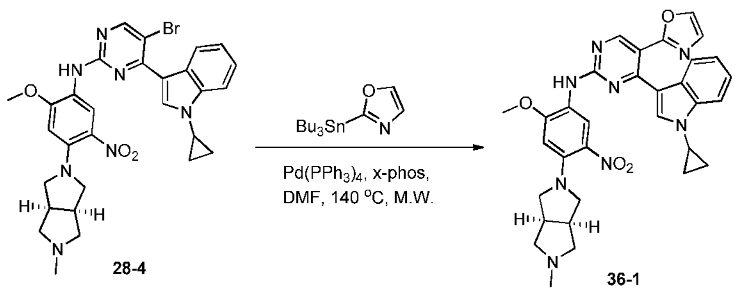


28-4

36-1

5-Bromo-4-(1-cyclopropyl-1H-indol-3-yl)-N-(2-methoxy-4-((3aR,6aS)-5-methylhe xahydropyrrolo[3,4-c]pyrrol-2(1H)-yl)-5-nitrophenyl)pyrimidin-2-amine (200 mg, 0.33 mmol), 2-(tributylstannyl)oxazole (178 mg, 0.5 mmol) and 2-dicyclohexylphosphorus-2,4,6-triisopropylbiphenyl (157 mg, 0.33 mmol) were dissolved in N,N-dimethylformamide (5 mL), followed by the addition of tetrakis(triphenylphosphine)palladium (191 mg, 0.17 mmol). The reaction solution was purged with nitrogen, and stirred at 140°C under microwave for 2 hours. The reaction solution was cooled to room temperature, to which water (20 mL) was added. The solution was extracted with dichloromethane (20 mL x 2). The organic phase was dried over anhydrous sodium sulfate, filtered and concentrated to dryness by rotary evaporation. The resulting crude product was purified by prep-TLC to obtain 4-(1-cyclopropyl-1H-indol-3-yl)-N-(2-methoxy-4-((3aR,6aS)-5-methylhexahydropyrrol o[3,4-c]pyrrol-2(1H)-yl)-5-nitrophenyl)-5-(oxazol-2-yl)pyrimidin-2-amine (200 mg, yield: 100% crude) as a yellow solid.

MS m/z (ESI): 593.1 [M+H]+.

Step 2: Preparation of N1-(4-(1-cyclopropyl-1H-indol-3-yl)-5-(oxazol-2-yl)pyrimidin-2-yl)-6-methoxy-4-((3a R,6aS)-5-methylhexahydropyrrolo[3,4-c]pyrrol-2(1H)-yl)benzene-1,3-diamine

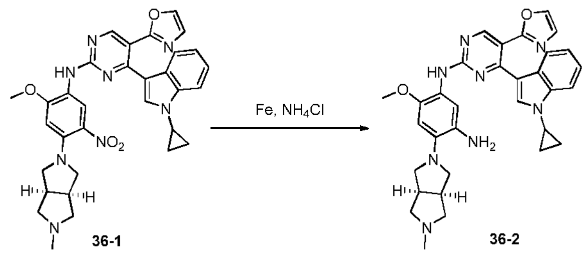


36-1 36-2

4-(1-Cyclopropyl-1H-indol-3-yl)-N-(2-methoxy-4-((3aR,6aS)-5-methylhexahydro pyrrolo[3,4-c]pyrrol-2(1H)-yl)-5-nitrophenyl)-5-(oxazol-2-yl)pyrimidin-2-amine (200 mg, 0.34 mmol) was dissolved in ethanol (10 mL), followed by the addition of saturated ammonium chloride solution (5 mL) and iron powder (190 mg, 3.4 mmol). The reaction solution was stirred at 80°C for 2 hours. The reaction solution was cooled to room temperature and filtered. The filtrate was extracted with dichloromethane (20 mL x 2). The organic phase was dried over anhydrous sodium sulfate, filtered and concentrated to dryness by rotary evaporation to obtain N1-(4-(1-cyclopropyl-1H-indol-3-yl)-5-(oxazol-2-yl)pyrimidin-2-yl)-6-methoxy-4-((3a R,6aS)-5-methylhexahydropyrrolo[3,4-c]pyrrol-2(1H)-yl)benzene-1,3-diamine (200 mg, yield: 100% crude) as a yellow solid.

MS m/z (ESI): 563.1 [M+H]+.

Step 3: Preparation of N-(5-((4-(1-cyclopropyl-1H-indol-3-yl)-5-(oxazol-2-yl)pyrimidin-2-yl)amino)-4-metho xy-2-((3aR,6aS)-5-methylhexahydropyrrolo[3,4-c]pyrrol-2(1H)-yl)phenyl)acrylamide

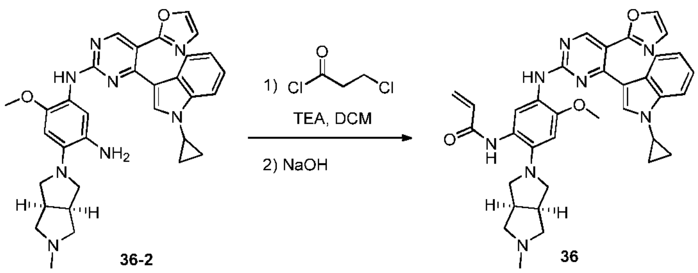


36-2 36

N1-(4-(1-Cyclopropyl-1H-indol-3-yl)-5-(oxazol-2-yl)pyrimidin-2-yl)-6-methoxy-4 -((3aR,6aS)-5-methylhexahydropyrrolo[3,4-c]pyrrol-2(lH)-yl)benzene-1,3-diamine (200 mg, 0.36 mmol) was dissolved in tetrahydrofuran (10 mL), and the solution was cooled to 0°C. Triethylamine (54 mg, 0.53 mmol) and 3-chloropropionyl chloride (68 mg, 0.53 mmol) were added successively. The reaction solution was stirred at 0°C for 1 hour. Sodium hydroxide (142 mg, 3.5 mmol) in water (1 mL) was added, and the reaction solution was stirred at room temperature for 1 hour. Water (10 mL) was added, and the solution was extracted with dichloromethane (10 mL x 2). The organic phase was dried over anhydrous sodium sulfate, filtered and concentrated to dryness by rotary evaporation. The resulting crude product was purified by prep-HPLC to obtain N-(5-((4-(1-cyclopropyl-1H-indol-3-yl)-5-(oxazol-2-yl)pyrimidin-2-yl)amino)-4-metho xy-2-((3aR,6aS)-5-methylhexahydropyrrolo[3,4-c]pyrrol-2(1H)-yl)phenyl)acrylamide (15.3 mg, yield: 7%) as a pale yellow solid.

MS m/z (ESI): 617.3 [M+H]+.

$^1$H NMR (400 MHz, $CDCl_3$) δ 9.30 - 9.18 (m, 1H), 8.87 - 8.77 (m, 1H), 7.95 - 7.87 (m, 1H), 7.81 - 7.75 (m, 1H), 7.69 - 7.59 (m, 2H), 7.59 - 7.50 (m, 2H), 7.23 - 7.17 (m, 1H), 7.08 (s, 1H), 6.70 (s, 1H), 6.64 - 6.54 (m, 1H), 6.52 - 6.40 (m, 1H), 5.78 - 5.70 (m, 1H), 5.34 - 5.26 (m, 1H), 3.88 (s, 3H), 3.42 (s, 1H), 6.70 (s, 1H), 6.64 Ccy4H), 2.98 - 2.90 (m, 2H), 2.75 - 2.66 (m, 2H), 2.59 - 2.53 (m, 1H), 2.36 - 2.31 (m, 1H), 1.70 - 1.61 (m, 3H), 1.13 - 1.08 (m, 2H), 0.95 - 0.93 (m, 2H).

**Example 37**

**N-(5-((4-(1-Cyclopropyl-1H-indol-3-yl)-5-(oxazol-2-yl)pyrimidin-2-yl)amino)-2-((2-(dimethylamino)ethyl)(methyl)amino)-4-methoxyphenyl)acrylamide**

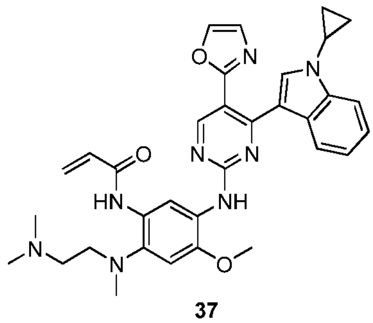

**37**

The compound of Example 37 was prepared by referring to the method of Example 3, and the synthetic route is as follows:

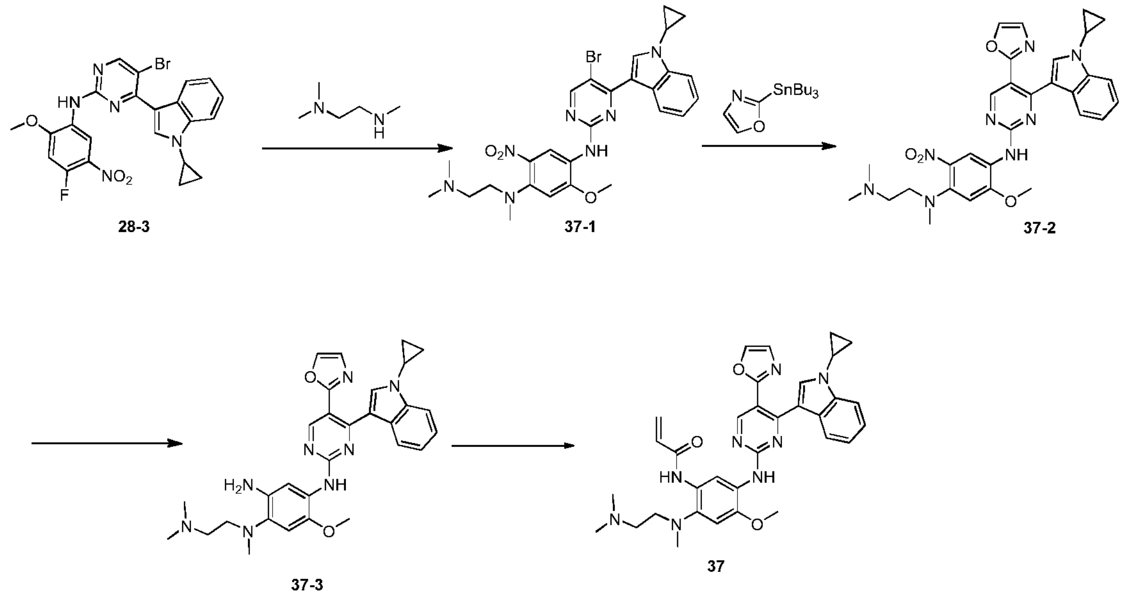

28-3 37-1 37-2

37-3 37

MS m/z (ESI): 593.1 $[M+H]^+$.

$^1$H NMR (400 MHz, $CDCl_3$) δ9.65 (s, 1H), 8.86 (s, 1H), 8.14 - 7.98 (m, 1H), 7.76 (s, 1H), 7.57 - 7.44 (m, 2H), 7.21 - 7.12 (m, 2H), 7.02 - 6.94 (m, 1H), 6.73 (s, 1H), 6.47 (d, *J*= 17.2 Hz, 1H), 5.73 (d, *J*= 11.7 Hz, 1H), 5.40 - 5.22 (m, 1H), 3.88 (s, 3H), 3.43 - 3.36 (m, 1H), 3.22 - 3.04 (m, 2H), 2.88 (s, 2H), 2.73 (s, 3H), 2.70 - 2.35 (m, 6H), 2.25 - 2.18 (m, 1H), 2.05 - 1.96 (m, 1H), 1.15 - 1.01 (m, 4H).

**Example 38**

**N-(5-((4-(1-Cyclopropyl-1H-indol-3-yl)-5-(oxazol-2-yl)pyrimidin-2-yl)amino)-4-met hoxy-2-(methyl((1-methylpyrrolidin-2-yl)methyl)amino)phenyl)acrylamide**

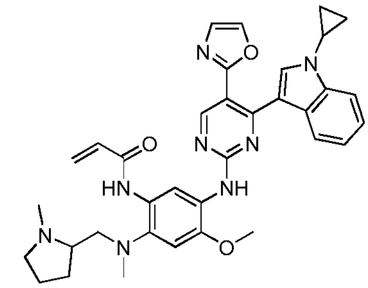

38

The compound of Example 38 was prepared by referring to the method of Example 3.

MS m/z (ESI): 619.2[M+H]+.

$^1$H NMR (400 MHz, $CDCl_3$) $\delta$9.75 (s, 1H), 8.86 (s, 1H), 7.79 - 7.65 (m, 2H), 7.55 - 7.45 (m, 2H), 7.21 - 7.12 (m, 2H), 7.02 - 6.93 (m, 1H), 6.72 (s, 1H), 6.49 (d, *J*= 15.9 Hz, 1H), 5.78 - 5.71 (m, 1H), 5.34 - 5.22 (m, 1H), 3.88 (s, 3H), 3.44 - 3.35 (m, 1H), 3.32 - 3.03 (m, 4H), 2.75 (s, 3H), 2.56 (s, 3H), 2.25 - 2.19 (m, 1H), 2.05 - 1.98 (m, 1H), 1.37 - 1.29 (m, 5H), 1.16 - 1.02 (m, 4H).

**Example 39**

**N-(5-((4-(1-Cyclopropyl-1H-indol-3-yl)-5-(oxazol-2-yl)pyrimidin-2-yl)amino)-4-met hoxy-2-(8-methyloctahydro-2H-pyrazino[1,2-a]pyrazin-2-yl)phenyl)acrylamide**

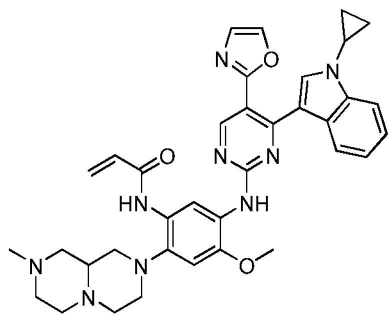

39

The compound of Example 39 was prepared by referring to the method of Example 3.

MS m/z (ESI): 646.1[M+H]+.

$^1$H NMR (400 MHz, $CDCl_3$) $\delta$ 9.63 (s, 1H), 8.86 (s, 1H), 8.55 - 8.46 (m, 1H), 7.77 (s, 1H), 7.73 - 7.63 (m, 2H), 7.55 - 7.53 (m, 1H), 7.50 - 7.48 (m, 1H), 7.21 - 7.13 (m, 2H), 7.02 - 6.94 (m, 1H), 6.74 (s, 1H), 6.47 - 6.31 (m, 2H), 5.80 (d, *J* = 9.5 Hz, 1H), 5.37 - 5.24 (m, 1H), 3.88 (s, 3H), 3.46 - 3.39 (m, 1H), 3.09 - 2.78 (m, 8H), 2.71 - 2.51 (m, 5H), 2.26 - 2.19 (m, 1H), 2.04 - 1.98 (m, 1H), 1.16 - 1.02 (m, 4H).

**Example 40**

**cis N-(5-((4-(1-Cyclopropyl-1H-indol-3-yl)-5-(oxazol-2-yl)pyrimidin-2-yl)amino)-4-met hoxy-2-(1-methylhexahydropyrrolo[3,4-b]pyrrol-5(1H)-yl)phenyl)acrylamide**

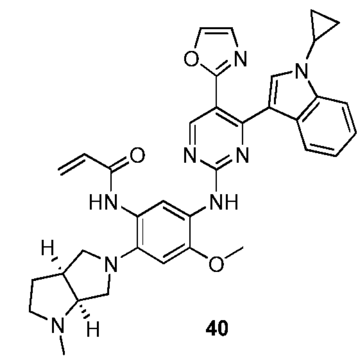

40

The compound of Example 40 was prepared by referring to the method of Example 3.

MS m/z (ESI): 617.3[M+H]+.

$^1$H NMR (400 MHz, $CDCl_3$) δ 9.33-9.19 (m, 2H), 8.82 (s, 1H), 8.06-7.86 *(br,* 1H), 7.72 (s, 1H), 7.54-7.50 (m, 2H), 7.42-7.32 *(br,* 1H), 7.19-7.14 (m, 2H), 7.03-7.00 (m, 1H), 6.66-6.51 (m, 2H), 6.48-6.43 (m, 1H), 5.71 (d, *J* = 11.6 Hz, 1H), 3.86 (s, 3H), 3.42-3.22 (m, 3H), 3.16-3.04 *(br,* 1H), 2.98-2.91 (m, 2H), 2.90-2.81 (m, 2H), 2.76-2.66 *(br,* 1H), 2.62-2.49 *(br,* 3H), 2.32-2.29 *(br,* 1H), 2.28-2.17 (m, 1H), 1.11-1.05 (m, 4H).

**Example 41**

**N-(5-((4-(1-Cyclopropyl-1H-indol-3-yl)-5-(3,5-dimethylisoxazol-4-yl)pyrimidin-2-yl )amino)-4-methoxy-2-((3aR,6aS)-5-methylhexahydropyrrolo[3,4-c]pyrrol-2(1H)-yl) phenyl)acrylamide**

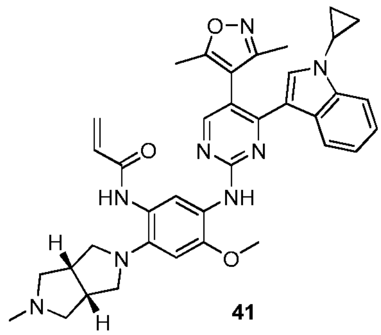

41

The compound of Example 41 was prepared by referring to the method of Example 3.

MS m/z (ESI): 645.2[M+H]+.

$^1$H NMR (400 MHz, $CDCl_3$) δ8.96 (s, 1H), 8.37 - 8.28 (m, 1H), 8.19 (s, 1H), 7.66 - 7.50 (m, 2H), 6.99 (s, 1H), 6.68 (s, 1H), 6.38 (d, *J* = 16.6 Hz, 1H), 5.69 (d, *J* = 10.3 Hz, 1H), 5.34 - 5.26 (m, 1H), 3.90 (s, 3H), 3.34 - 3.08 (m, 6H), 3.00 - 2.91 (m, 2H), 2.85 - 2.73 (m, 3H), 2.30 - 2.17 (m, 3H), 2.06 - 1.71 (m, 9H), 1.10 - 1.01 (m, 2H), 0.91 - 0.84 (m, 2H).

**Example 42**

**N-(5-((4-(1-Cyclopropyl-1H-indol-3-yl)-5-(1H-pyrazol-1-yl)pyrimidin-2-yl)amino)-4-methoxy-2-((3aR,6aS)-5-methylhexahydropyrrolo[3,4-c]pyrrol-2(1H)-yl)phenyl)a crylamide**

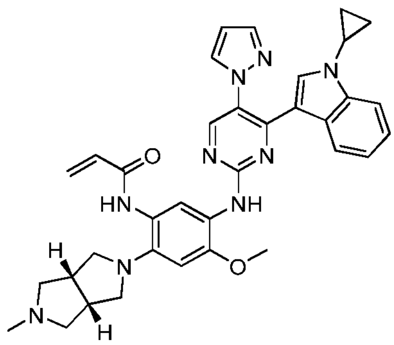

**42**

The compound of Example 42 was prepared by referring to the method of Example 3.

MS m/z (ESI): 616.4[M+H]$^+$.

**Example 43**

**N-(5-((4-(1-Cyclopropyl-1H-indol-3-yl)-5-(1-methyl-1H-pyrazol-4-yl)pyrimidin-2-y l)amino)-4-methoxy-2-((3aR,6aS)-5-methylhexahydropyrrolo[3,4-c]pyrrol-2(1H)-yl )phenyl)acrylamide**

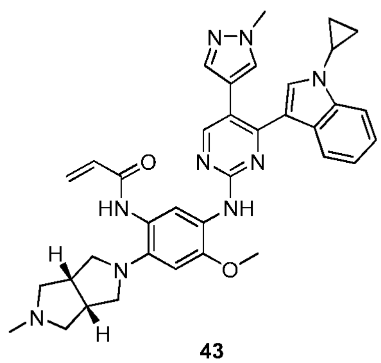

**43**

The compound of Example 43 was prepared by referring to the method of Example 3.

MS m/z (ESI): 630.3[M+H]$^+$.

$^1$H NMR (400 MHz, $CDCl_3$) δ 9.41 (s, 1H), 8.81 (s, 1H), 8.41 (s, 1H), 7.70 (d, *J* = 7.2 Hz, 1H), 7.56-7.52 (m, 3H), 7.45 (s, 1H), 7.20-7.11 (m, 2H),7.09-7.05 (m, 1H), 6.73 (s, 1H), 6.45-6.32 (m, 2H), 5.71 (d, *J* = 11.2 Hz, 1H), 3.86 (s, 3H), 3.84 (s, 3H), 3.38-3.31 (m, 1H), 2.99-2.83 (m, 8H), 2.65-2.53 *(br,* 2H), 2.45 (s, 3H), 1.06-0.99 (m, 4H).

**Example 44**

**N-(5-((4-(1-Cyclopropyl-1H-indol-3-yl)-5-(5-methyloxazol-2-yl)pyrimidin-2-yl)ami no)-4-methoxy-2-((3aR,6aS)-5-methylhexahydropyrrolo[3,4-c]pyrrol-2(1H)-yl)phe nyl)acrylamide**

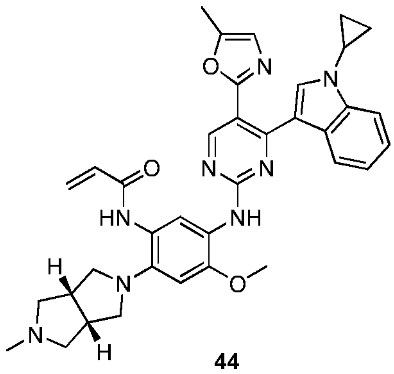

**44**

The compound of Example 44 was prepared by referring to the method of Example 3.

MS m/z (ESI): 631.1[M+H]+.

**Example 45**

**N-(5-((4-(1-Cyclopropyl-1H-indol-3-yl)-5-(thiazol-2-yl)pyrimidin-2-yl)amino)-4-me thoxy-2-((3aR,6aS)-5-methylhexahydropyrrolo[3,4-c]pyrrol-2(1H)-yl)phenyl)acryl amide**

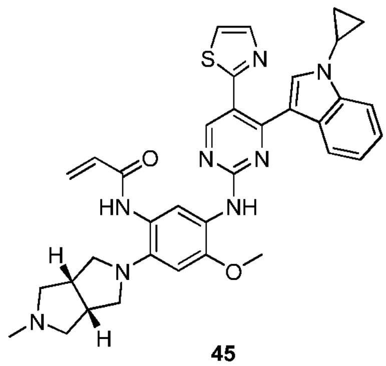

**45**

The compound of Example 45 was prepared by referring to the method of Example 3.

MS m/z (ESI): 633.1[M+H]+.

$^1$H NMR (400 MHz, $CDCl_3$) δ 9.36-9.18 *(br,* 1H), 8.76 (s, 1H), 7.84-7.81 (m, 1H), 7.74-7.70 (m, 2H), 7.55-7.50 (m, 2H), 7.18-7.15 (m, 2H), 7.06-6.98 (m, 2H), 6.70 (s, 1H), 6.46-6.41 (m, 2H), 5.77-5.72 (m, 1H), 3.87 (s, 3H), 3.44-3.33 (m, 1H), 2.98-2.80 (m, 8H), 2.67-2.57 *(br,* 5H), 0.99-0.90 (m, 4H).

**Example 46**

**N-(5-((4-(1-Cyclopropyl-1H-indol-3-yl)-5-(thiophen-2-yl)pyrimidin-2-yl)amino)-4-methoxy-2-((3aR,6aS)-5-methylhexahydropyrrolo[3,4-c]pyrrol-2(1H)-yl)phenyl)ac rylamide**

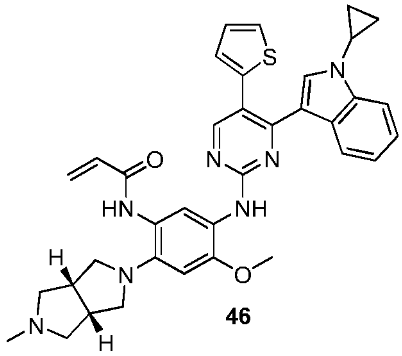

46

The compound of Example 46 was prepared by referring to the method of Example 3.
MS m/z (ESI): 632.2[M+H]$^+$.

**Example 47**

**N-(5-((4-(1-Cyclopropyl-1H-indol-3-yl)-5-phenylpyrimidin-2-yl)amino)-4-methoxy-2-((3aR,6aS)-5-methylhexahydropyrrolo[3,4-c]pyrrol-2(1H)-yl)phenyl)acrylamide**

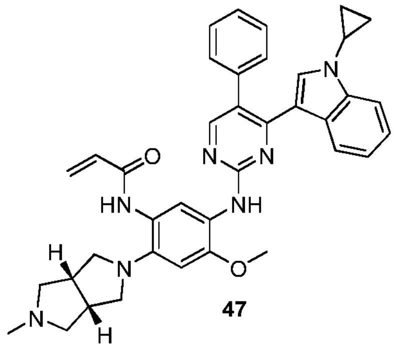

47

The compound of Example 47 was prepared by referring to the method of Example 3.
MS m/z (ESI): 626.4[M+H]$^+$.

**Example 48**

**N-(5-((4-(1-Cyclopropyl-1H-indol-3-yl)-5-(pyridin-2-yl)pyrimidin-2-yl)amino)-4-m ethoxy-2-((3aR,6aS)-5-methylhexahydropyrrolo[3,4-c]pyrrol-2(1H)-yl)phenyl)acryl amide**

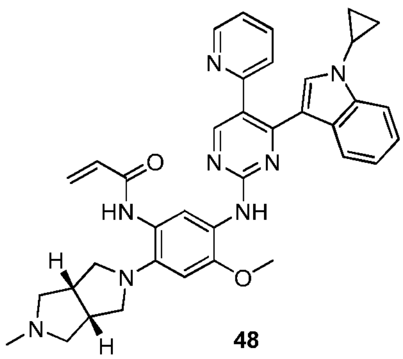

48

The compound of Example 48 was prepared by referring to the method of Example 3.
MS m/z (ESI): 627.1 $[M+H]^+$.

**Example 49**

**N-(5-((4-(1-Cyclopropyl-1H-indol-3-yl)-5-(2,4-difluorophenoxy)pyrimidin-2-yl)ami no)-4-methoxy-2-((3aR,6aS)-5-methylhexahydropyrrolo[3,4-c]pyrrol-2(1H)-yl)phe nyl)acrylamide**

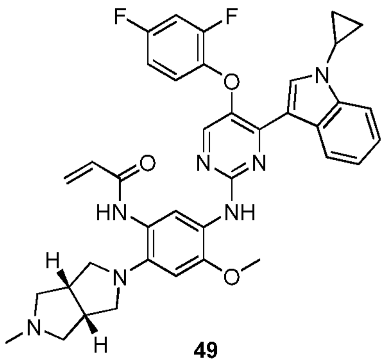

**49**

The compound of Example 49 was prepared by referring to the method of Example 3.
MS m/z (ESI): 678.2$[M+H]^+$.

**Example 50**

**N-(5-((4-(1-Cyclopropyl-1H-indol-3-yl)-5-(oxazol-2-yl)pyrimidin-2-yl)amino)-4-met hoxy-2-(4-methylpiperazin-1-yl)phenyl)acrylamide**

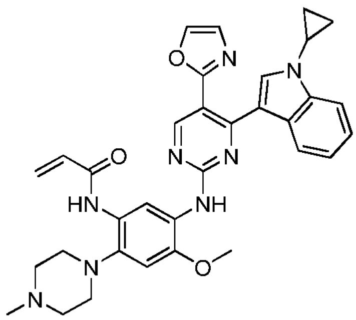

**50**

The compound of Example 50 was prepared by referring to the method of Example 3.
MS m/z (ESI): 591.1$[M+H]^+$.
$^1$H NMR (400 MHz, $CDCl_3$) $\delta$ 9.59 (s, 1H), 8.85 (s, 1H), 8.42-8.35 *(br,* 1H), 8.08-7.94 *(br,* 1H), 7.81 (s, 1H), 7.54 (d, *J* = 8.0 Hz, 1H), 7.51 (s, 1H), 7.40-7.31 *(br,* 1H),7.20-7.15 (m, 2H), 7.04-6.94 (m, 1H), 6.81 (s, 1H), 6.42 (d, *J* = 6.0 Hz, 1H), 6.34-6.27 (m, 1H), 5.77 (d, *J* = 8.8 Hz, 1H), 3.89 (s, 3H), 3.43-3.37 (m, 1H), 3.22-2.80 (m, 8H), 2.62 (s, 3H), 1.13-1.05 (m, 4H).

**Example 51**

**N-(5-((4-(1-Cyclopropyl-1H-indol-3-yl)-5-(oxazol-2-yl)pyrimidin-2-yl)amino)-4-met hoxy-2-(4-(4-methylpiperazin-1-yl)piperidin-1-yl)phenyl)acrylamide**

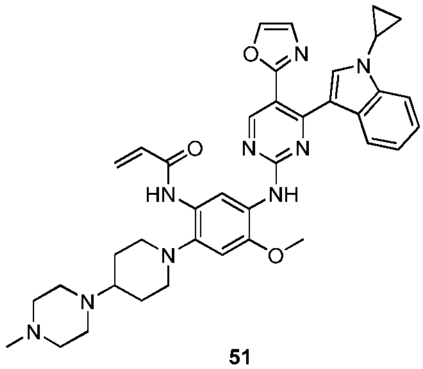

**51**

The compound of Example 51 was prepared by referring to the method of Example 3.

MS m/z (ESI): 674.2$[M+H]^+$.

$^1$H NMR (400 MHz, $CDCl_3$) δ 9.60 (s, 1H), 8.85 (s, 1H), 8.52 (s, 1H), 8.11-8.02 (*br,* 1H), 7.76 (s, 1H), 7.53 (d, *J* = 8.4 Hz, 1H), 7.49 (s, 1H), 7.30-7.28 (m, 1H), 7.19 (s, 1H), 7.15 (t, *J* = 7.6 Hz, 1H),7.00-6.94 (m, 1H), 6.73 (s, 1H), 6.44-6.40 (m, 1H), 6.34-6.27 (m, 1H), 5.77 (d, *J*= 10.0 Hz, 1H), 3.88 (s, 3H), 3.44-3.36 (m, 1H), 3.08-2.70 (m, 11H), 2.52-2.48(m, 4H), 2.10-2.06 (m, 2H), 1.65-1.41(m, 3H), 1.15-1.05 (m, 4H).

**Example 52**

**N-(5-((4-(1-Cyclopropyl-1H-indol-3-yl)-5-(oxazol-2-yl)pyrimidin-2-yl)amino)-2-(3-( dimethylamino)azetidin-1-yl)-4-methoxyphenyl)acrylamide**

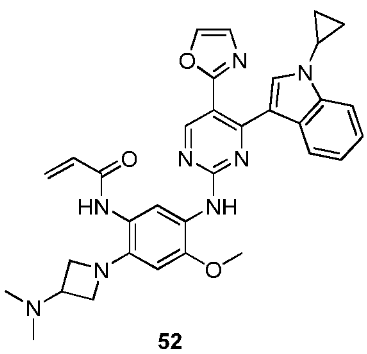

**52**

The compound of Example 52 was prepared by referring to the method of Example 3.

MS m/z (ESI): 591.2$[M+H]^+$.

$^1$H NMR (400 MHz, $CDCl_3$) δ 8.86 (s, 1H), 8.76 (s, 1H), 7.70 -7.64 (m, 3H), 7.58 -7.51 (m, 2H), 7.41-7.28 (m, 2H), 7.18-7.22 (m, 2H), 7.08 - 7.04 (m, 1H), 6.35 -6.23 (m, 2H), 5.74 (d, J = 10.0 Hz, 1H), 3.96-3.88 (m, 5H), 3.74 -3.67 (m, 2H), 3.42-3.33 (m, 1H), 3.23 - 3.17 (m, 1H), 2.27 (s, 6H), 1.05 -0.97 (s, 4H).

**Example 53**

**N-(5-((4-(1-Cyclopropyl-1H-indol-3-yl)-5-(oxazol-2-yl)pyrimidin-2-yl)amino)-4-met hoxy-2-(3-(pyrrolidin-1-yl)azetidin-1-yl)phenyl)acrylamide**

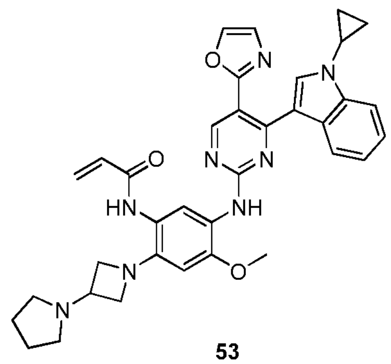

**53**

The compound of Example 53 was prepared by referring to the method of Example 3.

MS m/z (ESI): 617.3[M+H]$^+$.

$^1$H NMR (400 MHz, $CDCl_3$) δ 8.86 - 8.66 (m, 2H), 7.70 - 7.51 (m, 5H), 7.36 - 7.29 (m, 1H), 7.23 - 7.18 (m, 1H), 7.10 - 7.02 (m, 1H), 6.44 - 6.28 (m, 3H), 5.73 (d, *J* = 10.7 Hz, 1H), 3.94 - 3.87 (m, 5H), 3.80 - 3.72 (m, 2H), 3.40 - 3.33 (m, 2H), 2.63 - 2.50 (m, 5H), 1.88 - 1.82 (m, 4H), 1.11 - 1.01 (m, 4H).

**Example 54**

**N-(5-((4-(1-Cyclopropyl-1H-indol-3-yl)-5-(oxazol-2-yl)pyrimidin-2-yl)amino)-4-met hoxy-2-(4-(pyrrolidin-1-yl)piperidin-1-yl)phenyl)acrylamide**

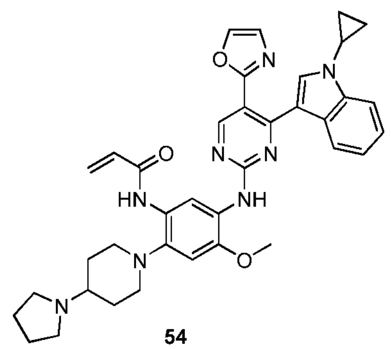

**54**

The compound of Example 54 was prepared by referring to the method of Example 3.

MS m/z (ESI): 645.4[M+H]$^+$.

$^1$H NMR (400 MHz, $CDCl_3$) δ 9.59 (s, 1H), 8.85 (s, 1H), 8.55 (s, 1H), 8.11 (s, 1H), 7.75 (s, 1H), 7.54 (d, J = 8.3 Hz, 1H), 7.48 (s, 1H), 7.20 - 7.12 (m, 2H), 7.00 - 6.93 (m, 1H), 6.72 (s, 1H), 6.45 - 6.41 (m, 1H), 5.78 (d, J = 8.7 Hz, 1H), 3.87 (s, 3H), 3.44 - 3.37 (m, 1H), 3.16 - 3.06 (m, 2H), 2.80 - 2.65 (m, 3H), 2.22 - 2.01 (m, 6H), 1.45 - 1.19 (m, 5H), 1.17 - 1.03 (m, 4H), 0.95 - 0.89 (m, 1H).

**Example 55**

**N-(5-((4-(1-Cyclopropyl-1H-indol-3-yl)-5-(oxazol-2-yl)pyrimidin-2-yl)amino)-2-((3a R,6aS)-5-cyclopropylhexahydropyrrolo[3,4-c]pyrrol-2(1H)-yl)-4-methoxyphenyl)ac rylamide**

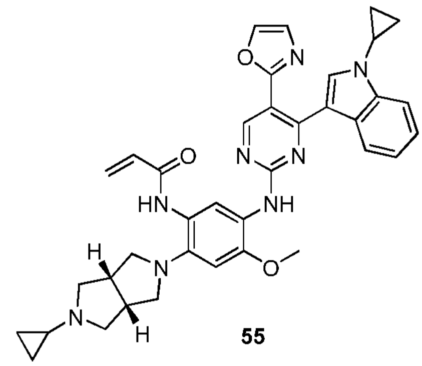

**55**

The compound of Example 55 was prepared by referring to the method of Example 3.
MS m/z (ESI): 643.2[M+H]+.

**Example 56**

**(S)-N-(5-((4-(1-Cyclopropyl-1H-indol-3-yl)-5-(oxazol-2-yl)pyrimidin-2-yl)amino)-2-(3-(dimethylamino)pyrrolidin-1-yl)-4-methoxyphenyl)acrylamide**

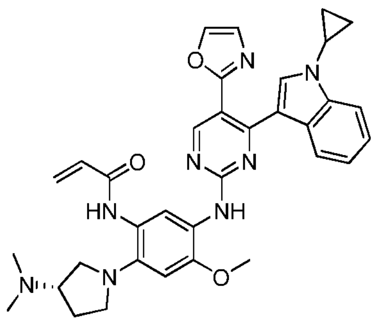

**56**

The compound of Example 56 was prepared by referring to the method of Example 3.
MS m/z (ESI): 605.1[M+H]+.
$^1$H NMR (400 MHz, $CDCl_3$) δ 9.39 (s, 1H), 8.83 (s, 1H), 8.69 - 8.51 (m, 1H), 8.09 - 7.84 (m, 1H), 7.73 (s, 1H), 7.58 - 7.47 (m, 2H), 7.44 - 7.32 (m, 1H), 7.23 - 7.13 (m, 2H), 7.08 - 6.94 (m, 1H), 6.82 - 6.61 (m, 2H), 6.43 (d, *J* = 16.9 Hz, 1H), 5.73 (d, *J* = 10.6 Hz, 1H), 3.87 (s, 3H), 3.44 - 3.28 (m, 3H), 3.23 - 2.99 (m, 3H), 2.55 (s, 6H), 2.32 - 2.16 (m, 2H), 1.17 - 1.01 (m, 4H).

**Example 57**

**(R)-N-(5-((4-(1-Cyclopropyl-1H-indol-3-yl)-5-(oxazol-2-yl)pyrimidin-2-yl)amino)-2 -(3-(dimethylamino)pyrrolidin-1-yl)-4-methoxyphenyl)acrylamide**

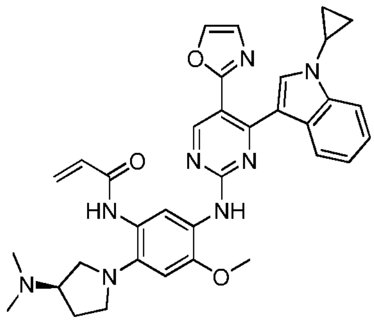

**57**

The compound of Example 57 was prepared by referring to the method of Example 3.

MS m/z (ESI): 605.3[M+H]+.

$^1$H NMR (400 MHz, $CDCl_3$) δ 9.39 (s, 1H), 8.83 (s, 1H), 8.66 - 8.51 (m, 1H), 8.06 - 7.87 (m, 1H), 7.73 (s, 1H), 7.64 - 7.47 (m, 2H), 7.37 (mi, 1H), 7.22 - 7.10 (m, 2H), 7.05 - 6.96 (m, 1H), 6.80 - 6.60 (m, 2H), 6.43 (d, *J* = 17.0 Hz, 1H), 5.73 (d, *J* = 9.8 Hz, 1H), 3.87 (s, 3H), 3.47 - 3.24 (m, 3H), 3.23 - 2.98 (m, 3H), 2.52 (s, 6H), 2.29 - 2.19 (m, 2H), 1.17 - 0.97 (m, 4H).

**Example 58**

**N-(5-((4-(1-Cyclopropyl-1H-indol-3-yl)-5-(oxazol-2-yl)pyrimidin-2-yl)amino)-2-(4-( dimethylamino)piperidin-1-yl)-4-methoxyphenyl)acrylamide**

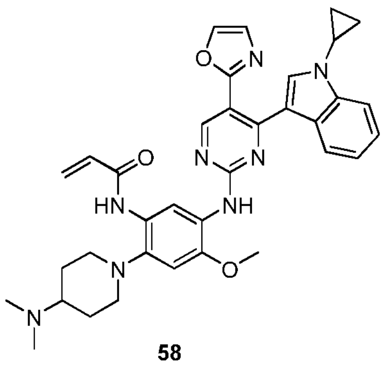

**58**

The compound of Example 58 was prepared by referring to the method of Example 3.

MS m/z (ESI): 619.1 [M+H]+.

**Example 59**

**N-(5-((4-(1-Cyclopropyl-1H-indol-3-yl)-5-(oxazol-2-yl)pyrimidin-2-yl)amino)-4-met hoxy-2-(4-morpholinopiperidin-1-yl)phenyl)acrylamide**

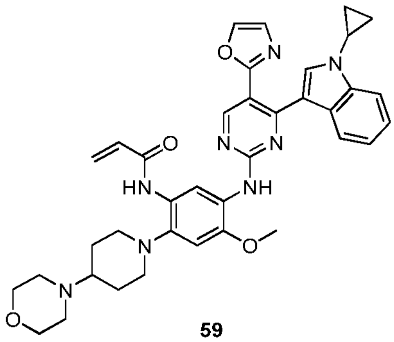

59

The compound of Example 59 was prepared by referring to the method of Example 3.
MS m/z (ESI): 661.1[M+H]+.

**Example 60**

**N-(5-((4-(1-Cyclopropyl-1H-indol-3-yl)-5-(oxazol-2-yl)pyrimidin-2-yl)amino)-2-(4-c yclopropylpiperazin-1-yl)-4-methoxyphenyl)acrylamide**

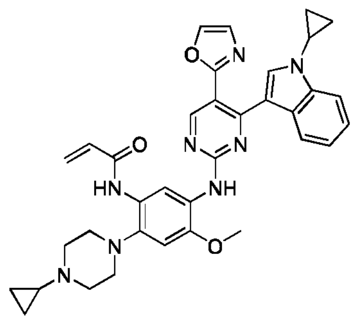

60

The compound of Example 60 was prepared by referring to the method of Example 3.
MS m/z (ESI): 617.2[M+H]+.

**Example 61**

**N-(5-((4-(1-Cyclopropyl-1H-indol-3-yl)-5-(methylsulfonyl)pyrimidin-2-yl)amino)-4-methoxy-2-((3aR,6aS)-5-methylhexahydropyrrolo[3,4-c]pyrrol-2(1H)-yl)phenyl)ac rylamide**

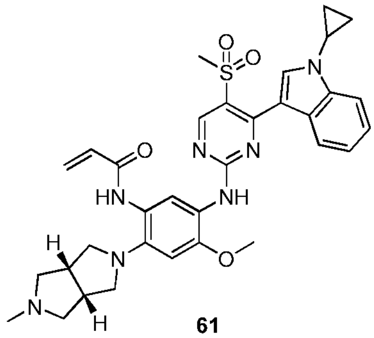

61

The compound of Example 61 was prepared by referring to the method of Example 3.

MS m/z (ESI): 628.3$[M+H]^+$.

**Example 62**

**N-(5-((4-(1-Cyclopropyl-1H-indol-3-yl)-5-(dimethylphosphoryl)pyrimidin-2-yl)ami no)-4-methoxy-2-((3aR,6aS)-5-methylhexahydropyrrolo[3,4-c]pyrrol-2(1H)-yl)phe nyl)acrylamide**

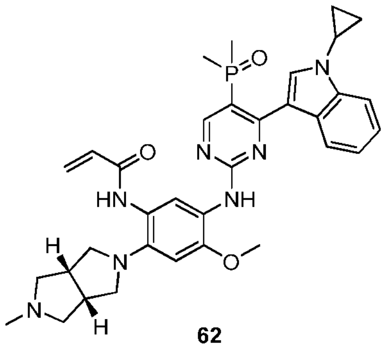

**62**

The compound of Example 62 was prepared by referring to the method of Example 3.
MS m/z (ESI): 626.2$[M+H]^+$.

**Example 63**

**Isopropyl 2-((5-acrylamido-2-methoxy-4-((3aR,6aS)-5-methylhexahydropyrrolo[3,4-c]pyrrol-2(1H)-yl)phenyl)amino)-4-(1-methyl-1H-indol-3-yl)pyrimidine-5-carboxylate**

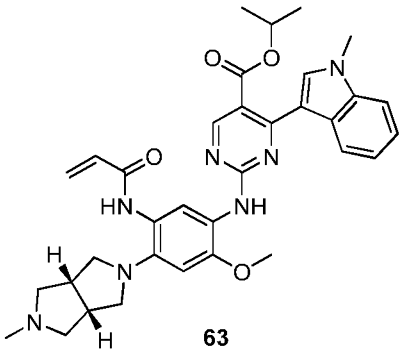

**63**

The compound of Example 63 was prepared by referring to the method of Example 2.
MS m/z (ESI): 610.2 $[M+H]^+$.
1H NMR (400 MHz, DMSO-d6) δ 8.66 - 8.58 (m, 2H), 8.24 (s, 1H), 8.08 (s, 1H), 7.78 (br, 1H), 7.49 (d, J = 8.2 Hz, 1H), 7.20 (t, J = 7.6 Hz, 1H), 7.15 - 7.02 (m, 1H), 6.78 (s, 1H), 6.66 (br, 1H), 6.39 - 6.19 (m, 1H), 5.83 - 5.76 (m, 1H), 5.12 - 4.91 (m, 1H), 3.87 (s, 3H), 3.82 (s, 3H), 3.15 - 3.01 (m, 9H), 2.76 (s, 5H), 1.13 (d, J = 6.2 Hz, 6H).

**Example 64**

**N-(5-((4-(1-Cyclopropyl-1H-indol-3-yl)-5-(pyridin-3-yl)pyrimidin-2-yl)amino)-4-m ethoxy-2-((3aR,6aS)-5-methylhexahydropyrrolo[3,4-c]pyrrol-2(1H)-yl)phenyl)acryl amide**

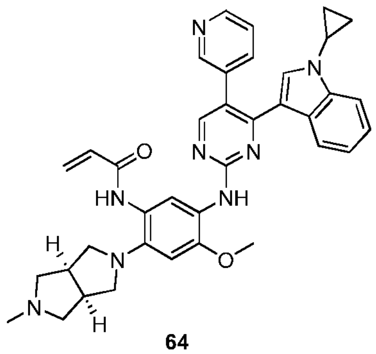

64

The compound of Example 64 was prepared by referring to the method of Example 3.

MS m/z (ESI): 627.2 [M+H]+.

$^1$H NMR (400 MHz, $CDCl_3$) $\delta$ 9.39 *(br,* 1H), 8.94-8.73 *(br,* 1H), 8.60 (d, *J* = 1.6 Hz, 1H), 8.51 (d, *J*= 4.0 Hz, 1H),8.41 (s, 1H), 7.78-7.60 (m, 3H), 7.49 (d, *J* = 8.0 Hz, 1H), 7.23-7.15 (m, 2H), 7.05-7.02 (m, 1H),7.09-7.05 (m, 1H), 6.75 (s, 1H), 6.45-6.35 (m, 2H), 5.74-5.72 (m, 1H), 3.88 (s, 3H), 3.33-3.25 (m, 1H), 2.96-2.86 (m, 8H), 2.65-2.50 *(br,* 2H), 2.48 (s, 3H), 0.99-0.90 (m, 4H).

## Example 65

**N-(5-((4-(1-Cyclopropyl-1H-indol-3-yl)-5-(1-methyl-1H-pyrazol-4-yl)pyrimidin-2-y l)amino)-4-methoxy-2-((3aS,6aS)-1-methylhexahydropyrrolo[3,4-b]pyrrol-5(1H)-yl )phenyl)acrylamide**

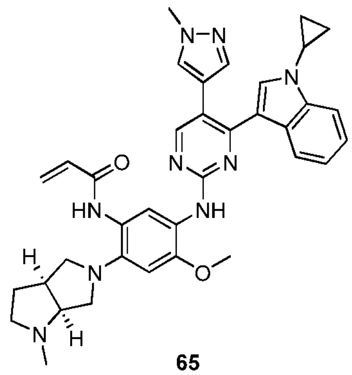

65

The compound of Example 65 was prepared by referring to the method of Example 3.

MS m/z (ESI): 630.3 [M+H]+.

$^1$H NMR (400 MHz, Chloroform-*d*) $\delta$ 9.24 - 9.06 (br, 1H), 8.42 - 8.26 (m, 1H), 7.85 - 7.64 (m, 1H), 7.60 - 7.49 (m, 2H), 7.49 - 7.35 (m, 2H), 7.24 - 7.16 (m, 2H), 7.15 - 7.06 (m, 1H), 6.93 - 6.68 (br, 1H), 6.63 (s, 1H), 6.49 - 6.35 (m, 1H), 5.74 - 5.59 (m, 1H), 3.93 - 3.76 (m, 7H), 3.53 - 3.40 (m, 1H), 3.38 - 3.25 (m, 2H), 3.08 - 2.98 (m, 1H), 2.98 - 2.88 (m, 1H), 2.88 - 2.76 (m, 3H), 2.69 (s, 3H), 2.60 - 2.44 (m, 1H), 2.38 - 2.25 (m, 1H), 2.05 - 1.91 (m, 1H), 1.09 - 0.93 (m, 4H).

## Example 66

**N-(5-((4-(1-Cyclopropyl-1H-indol-3-yl)-5-(oxazol-2-yl)pyrimidin-2-yl)amino)-2-(3-( dimethylamino)azetidin-1-yl)-4-methoxyphenyl)-2-fluoroacrylamide**

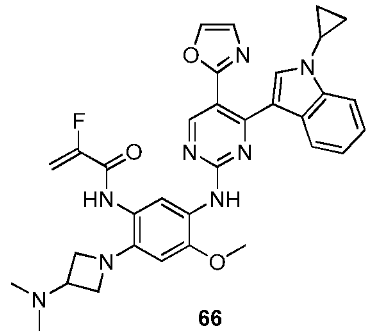

66

The compound of Example 66 was prepared by referring to the method of Example 3.

MS m/z (ESI): 609.2[M+H]$^+$.

$^1$H NMR (400 MHz, $CDCl_3$) MR (400 MHz, *br,* 1H), 8.76 (s, 1H), 7.84-7.81 (m, 1H), 7.74-7.70 (m, 2H), 7.55-7.50 (m, 2H), 7.18-7.15 (m, 2H), 7.06-6.98 (m, 2H), 6.70 (s, 1H), 6.46-6.41 (m, 2H), 3.87 (s, 3H), 3.44-3.33 (m, 1H), 2.98-2.80 (m, 8H), 2.67-2.57 *(br,* 3H), 0.99-0.90 (m, 4H).

### Example 67

**N-(5-((5-Cyano-4-(1-cyclopropyl-1H-indol-3-yl)pyrimidin-2-yl)amino)-2-((2-(dimet hylamino)ethyl)(methyl)amino)-4-methoxyphenyl)acrylamide**

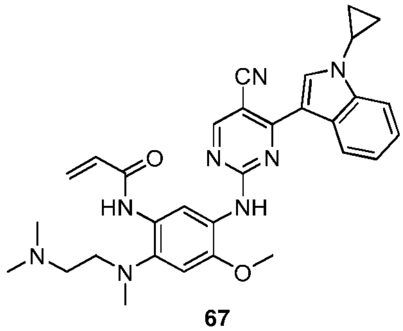

67

The compound of Example 67 was prepared by referring to the method of Example 2, and the specific synthetic route is as follows:

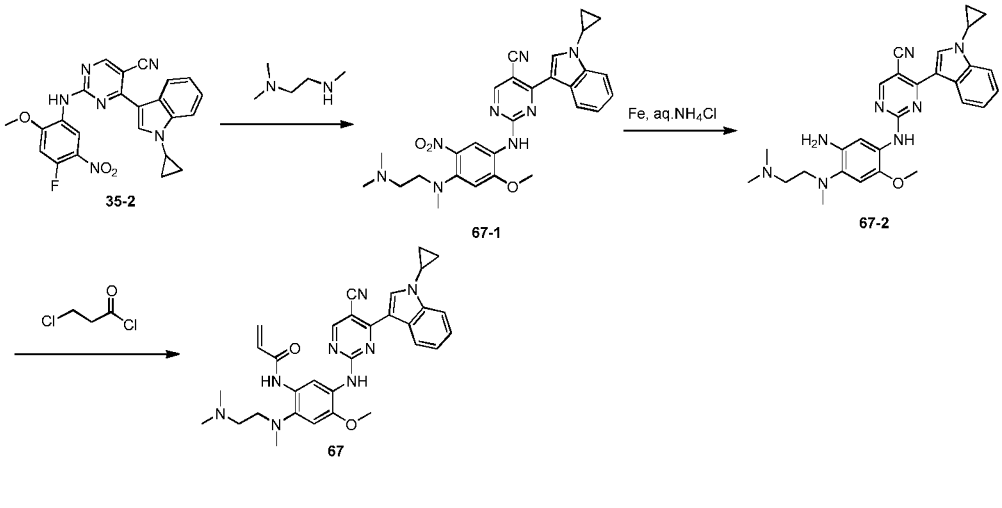


35-2 67-1 67-2 67

Step 1: Preparation of 4-(1-cyclopropyl-1H-indol-3-yl)-2-((4-((2-(dimethylamino)ethyl)(methyl)amino)-2-met hoxy-5-nitrophenyl)amino)pyrimidine-5-carbonitrile

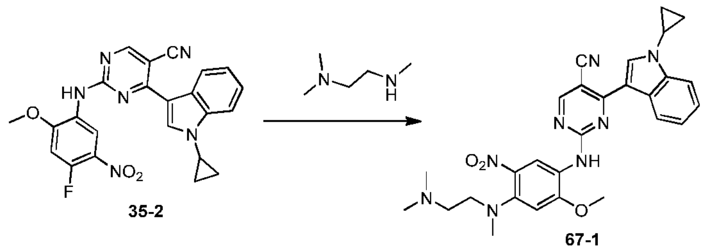

35-2 67-1

4-(1-Cyclopropyl-1H-indol-3-yl)-2-((4-fluoro-2-methoxy-5-nitrophenyl)amino)pyr imidine-5-carbonitrile (0.4 g, 0.9 mmol) was dissolved in acetonitrile (20 mL), followed by the addition of potassium carbonate (0.37 g, 2.7 mmol) and N1,N1,N2-trimethylethane-1,2-diamine (0.11 g, 1.1 mmol). The reaction solution was stirred at 80°C for 1 hour. The reaction solution was cooled to room temperature and filtered. The filtrate was concentrated to dryness by rotary evaporation to obtain the crude product 4-(1-cyclopropyl-1H-indol-3-yl)-2-((4-((2-(dimethylamino)ethyl)(methyl)amino)-2-met hoxy-5-nitrophenyl)amino)pyrimidine-5-carbonitrile (0.48 g, yield: 100% crude) as a yellow solid.

Step 2: Preparation of 2-((5-amino-4-((2-(dimethylamino)ethyl)(methyl)amino)-2-methoxyphenyl)amino)-4-(1 -cyclopropyl-1H-indol-3-yl)pyrimidine-5-carbonitrile

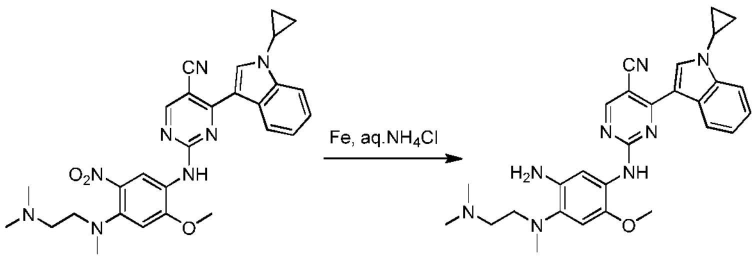


67-1 67-2

4-(1-Cyclopropyl-1H-indol-3-yl)-2-((4-((2-(dimethylamino)ethyl)(methyl)amino)-2-methoxy-5-nitrophenyl)amino)pyrimidine-5-carbonitrile (0.48 g, 0.97 mmol) was dissolved in ethanol (20 mL), followed by the addition of saturated sodium bicarbonate solution (10 mL) and iron powder (0.54 g, 9.7 mmol). The reaction solution was stirred at 80°C for 2 hours. The reaction solution was cooled to room temperature and filtered. The filtrate was extracted with dichloromethane (20 mL x 2). The organic phase was dried over anhydrous sodium sulfate, filtered and concentrated to dryness by rotary evaporation to obtain 2-((5-amino-4-((2-(dimethylamino)ethyl)(methyl)amino)-2-methoxyphenyl)amino)-4-(1 -cyclopropyl-1H-indol-3-yl)pyrimidine-5-carbonitrile (0.4 g, yield: 88%) as a yellow solid.

Step 3: Preparation of N-(5-((5-cyano-4-(1-cyclopropyl-1H-indol-3-yl)pyrimidin-2-yl)amino)-2-((2-(dimethyl amino)ethyl)(methyl)amino)-4-methoxyphenyl)acrylamide

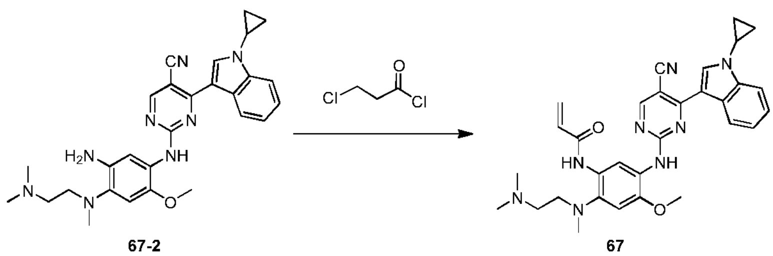

67-2 67

2-((5-Amino-4-((2-(dimethylamino)ethyl)(methyl)amino)-2-methoxyphenyl)amino )-4-(1-cyclopropyl-1H-indol-3-yl)pyrimidine-5-carbonitrile (0.4 g, 0.8 mmol) was dissolved in tetrahydrofuran (10 mL), and the solution was cooled to 0°C. Triethylamine (0.12 g, 1.2 mmol) and 3-chloropropionyl chloride (0.12 g, 0.97 mmol) were added successively. The reaction solution was stirred at 0°C for 1 hour. 3M sodium hydroxide solution (3 mL) was added, and the reaction solution was stirred at room temperature for 1 hour. Water (30 mL) was added, and the solution was extracted with dichloromethane (30 mL x 2). The organic phase was dried over anhydrous sodium sulfate, filtered and concentrated to dryness by rotary evaporation. The resulting crude product was purified by prep-TLC (dichloromethane/MeOH: 20/1) to obtain N-(5-((5-cyano-4-(1-cyclopropyl-1H-indol-3-yl)pyrimidin-2-yl)amino)-2-((2-(dimethyl amino)ethyl)(methyl)amino)-4-methoxyphenyl)acrylamide (0.15 g, yield: 34%) as a yellow solid.

MS m/z (ESI): 551.4 $[M+H]^+$.

$^1$H NMR (400 MHz, $CDCl_3$) δ 10.08 (s, 1H), 9.42 (s, 1H), 8.68 (s, 1H), 8.49-8.47 (m, 2H), 7.76 (s, 1H), 7.61 (d, *J* = 8.0 Hz, 1H), 7.30-7.26 (m, 2H), 6.81 (s, 1H), 6.39 (s, 2H), 5.69-5.67 (m, 1H), 3.89 (s, 3H), 3.48-3.44 (m, 1H), 2.93-2.91 (m, 2H), 2.73 (s, 3H), 2.35-2.30 (m, 8H), 1.17-1.11 (m, 4H).

## Example 68

**N-(5-((4-(1-Cyclopropyl-1H-indol-3-yl)-5-(1-methyl-1H-pyrazol-4-yl)pyrimidin-2-y l)amino)-4-methoxy-2-((3aR,6aR)-1-hexahydropyrrolo[3,4-b]pyrrol-5(1H)-yl)phen yl)-2-fluoroacrylamide**

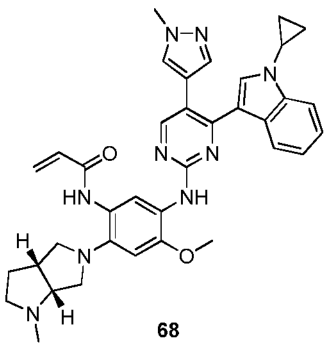

68

The compound of Example 68 was prepared by referring to the method of Example 3.

MS m/z (ESI): 630.4$[M+H]^+$.

$^1$H NMR (400 MHz, Chloroform-*d*) δ 9.39 (s, 1H), 9.07 - 8.84 (m, 1H), 8.40 (s, 1H), 7.69 (d, *J* = 7.9 Hz, 1H), 7.60 - 7.41 (m, 4H), 7.23 - 7.14 (m, 2H), 7.06 (t, *J* = 7.5 Hz, 1H), 6.68 (s, 1H), 6.54 - 6.28 (m, 2H), 5.79 - 5.60 (m, 1H), 3.85 (s, 3H), 3.83 (s, 3H), 3.41 - 3.28 (m, 1H), 3.20 - 3.02 (m, 2H), 3.00 - 2.86 (m, 3H), 2.86 - 2.72 (m, 1H), 2.70 - 2.56 (m, 1H), 2.44 (s, 3H), 2.38 - 2.25 (m, 1H), 2.24 - 2.07 (m, 1H), 1.83 - 1.61 (m, 1H), 1.12 - 0.87 (m, 4H).

## Example 69

**N-(5-((4-(1-Cyclopropyl-1H-indol-3-yl)-5-(2,4-difluorophenyl)pyrimidin-2-yl)amin o)-4-methoxy-2-((3aR,6aS)-5-methylhexahydropyrrolo[3,4-c]pyrrol-2(1H)-yl)phen yl)acrylamide**

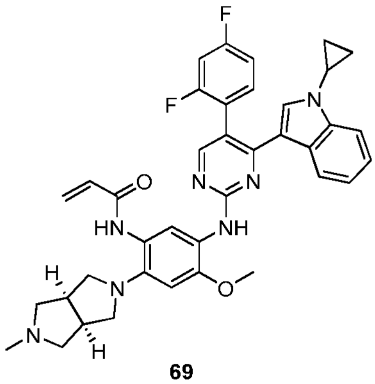

69

The compound of Example 69 was prepared by referring to the method of Example 3.

MS m/z (ESI): 662.0[M+H]$^+$.

$^1$H NMR (400 MHz, Chloroform-*d*) δ 9.37 (s, 1H), 8.75 (br s, 1H), 8.35 (s, 1H), 7.97 (s, 1H), 7.60 (s, 1H), 7.49 (d, *J* = 8.1 Hz, 1H), 7.24 - 7.17 (m, 2H), 7.14 - 6.98 (m, 2H), 6.88 - 6.83 (m, 2H), 6.74 (s, 1H), 6.43 - 6.39 (m, 2H), 5.77 - 5.65 (m, 1H), 3.88 (s, 3H), 3.29 - 3.26 (m, 1H), 2.96 - 2.86 (m, 8H), 2.70 - 2.54 (m, 2H), 2.47 (s, 3H), 1.01 - 0.96 (m, 2H), 0.89 - 0.78 (m, 2H).

**Example 70**

**N-(5-((4-(1-Cyelopropyl-1H-indol-3-yl)-5-(1-methyl-1H-pyrazol-5-yl)pyrimidin-2-y l)amino)-4-methoxy-2-((3aR,6aS)-5-methylhexahydropyrrolo[3,4-c]pyrrol-2(1H)-yl )phenyl)acrylamide**

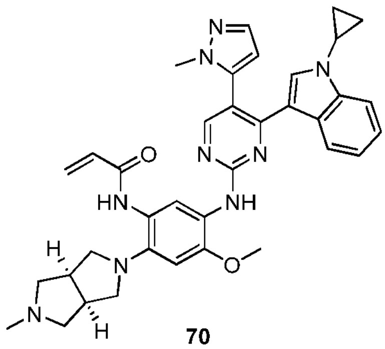

70

The compound of Example 70 was prepared by referring to the method of Example 3.

MS m/z (ESI): 630.2[M+H]$^+$.

$^1$H NMR (400 MHz, $CDCl_3$) δ 9.11 (s, 1H), 8.53 - 8.47 (m, 1H), 8.31 (s, 1H), 7.66 - 7.59 (m, 2H), 7.56 - 7.49 (m, 1H), 7.35 - 7.27 (m, 2H), 6.73 (s, 1H), 6.44 - 6.34 (m, 3H), 5.70 (d, *J* = 11.8 Hz, 1H), 3.91 (s, 3H), 3.40 (s, 3H), 3.32 - 3.22 (m, 2H), 3.21 - 2.89 (m, 9H), 2.76 - 2.60 (m, 3H), 1.03 - 0.97 (m, 2H), 0.83 - 0.77 (m, 2H).

**Example 71**

**N-(5-((4-(1-Cyclopropyl-1H-indol-3-yl)-5-(1-methyl-1H-pyrazol-3-yl)pyrimidin-2-y l)amino)-4-methoxy-2-((3aR,6aS)-5-methylhexahydropyrrolo[3,4-c]pyrrol-2(1H)-yl )phenyl)acrylamide**

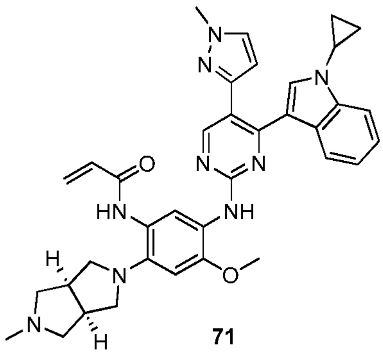

71

The compound of Example 71 was prepared by referring to the method of Example 3.
MS m/z (ESI): 630.1[M+H]+.
$^1$H NMR (400 MHz, Chloroform-*d*) δ 9.26 (s, 1H), 8.94 - 8.71 (m, 1H), 8.62 (s, 1H), 7.71 - 7.58 (m, 2H), 7.51 (d, *J* = 8.5 Hz, 1H), 7.39 - 7.32 (m, 1H), 7.23 (s, 1H), 7.19 - 7.12 (m, 1H), 7.07 - 6.98 (m, 1H), 6.67 (s, 1H), 6.48 - 6.30 (m, 2H), 5.97 (s, 1H), 5.73 - 5.70 (m, 1H), 3.94 (s, 3H), 3.86 (s, 3H), 3.43 - 3.27 (m, 2H), 3.11 - 2.65 (m, 10H), 2.57 - 2.49 (m, 2H), 1.08 - 0.92 (m, 4H).

**Example 72**

**N-(5-((5-Cyano-4-(1-cyclopropyl-1H-indol-3-yl)pyrimidin-2-yl)amino)-4-methoxy-2 -(methyl((1-methylpyrrolidin-2-yl)methyl)amino)phenyl)acrylamide**

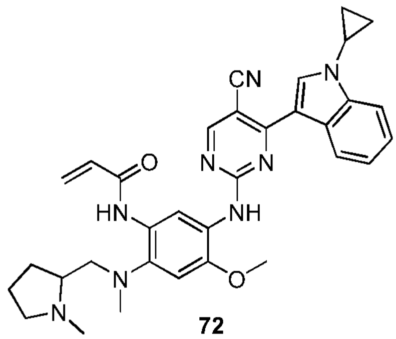

72

The compound of Example 72 was prepared by referring to the method of Example 2.
MS m/z (ESI): 577.1 [M+H]+.

**Example 73**

**N-(5-((5-Cyano-4-(1-cyclopropyl-1H-indol-3-yl)pyrimidin-2-yl)amino)-4-methoxy-2 -(methyl(2-(pyrrolidin-1-yl)ethyl)amino)phenyl)acrylamide**

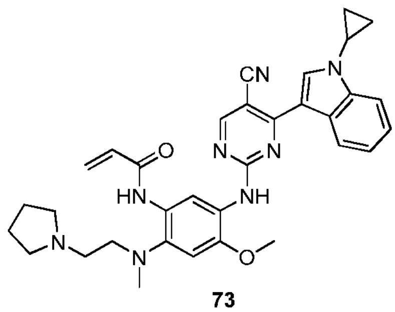

73

The compound of Example 73 was prepared by referring to the method of Example 2.

MS m/z (ESI): 577.4 $[M+H]^+$.

**Example 74**

**N-(5-((5-Cyano-4-(1-cyclopropyl-1H-indol-3-yl)pyrimidin-2-yl)amino)-2-(3-(dimeth ylamino)azetidin-1-yl)-4-methoxyphenyl)acrylamide**

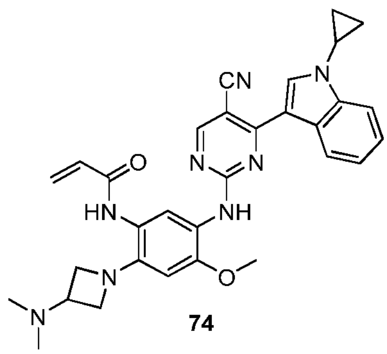

74

The compound of Example 74 was prepared by referring to the method of Example 2.

MS m/z (ESI): 549.2 $[M+H]^+$.

$^1$H NMR (400 MHz, Chloroform-*d*) δ 8.90 - 8.72 (m, 1H), 8.59 (s, 1H), 8.54 - 8.36 (m, 2H), 7.76 (s, 1H), 7.67 - 7.56 (m, 1H), 7.39 - 7.27 (m, 3H), 6.86 - 6.58 (m, 1H), 6.56 - 6.29 (m, 2H), 5.81 - 5.62 (m, 1H), 4.42 - 4.22 (m, 2H), 4.10 - 3.99 (m, 2H), 3.96 (s, 3H), 3.86 - 3.76 (m, 1H), 3.56 - 3.39 (m, 1H), 2.81 (s, 6H), 1.27 - 1.20 (m, 4H).

**Example 75**

**N-(5-((5-Cyano-4-(1-cyclopropyl-1H-indol-3-yl)pyrimidin-2-yl)amino)-4-methoxy-2 -(3-(pyrrolidin-1-yl)azetidin-1-yl)phenyl)acrylamide**

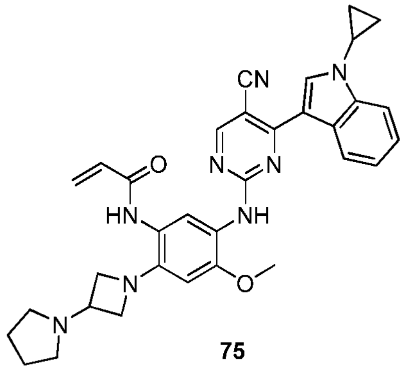

75

The compound of Example 75 was prepared by referring to the method of Example 2.
MS m/z (ESI): 575.1 $[M+H]^+$.

**Example 76**

**N-(5-((5-Cyano-4-(1-cyclopropyl-1H-indol-3-yl)pyrimidin-2-yl)amino)-2-(2-((dimet hylamino)methyl)azetidin-1-yl)-4-methoxyphenyl)acrylamide**

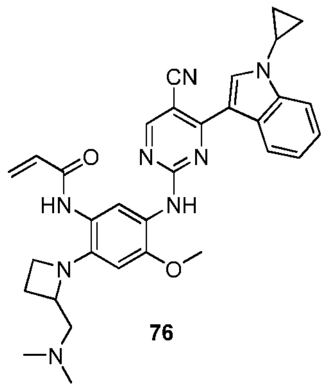

76

The compound of Example 76 was prepared by referring to the method of Example 2.
MS m/z (ESI): 563.5 $[M+H]^+$.

**Example 77**

**(*S*)-N-(5-((5-cyano-4-(1-cyclopropyl-1H-indol-3-yl)pyrimidin-2-yl)amino)-2-(3-(dim ethylamino)pyrrolidin-1-yl)-4-methoxyphenyl)acrylamide**

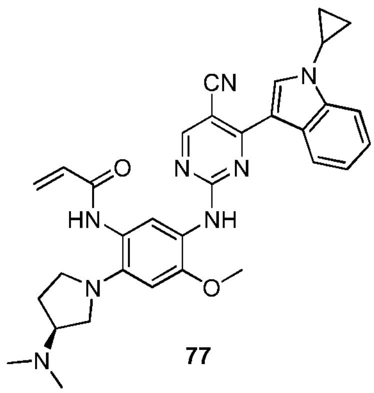

77

The compound of Example 77 was prepared by referring to the method of Example 2.

MS m/z (ESI): 563.3 $[M+H]^+$.

$^1$H NMR (400 MHz, Chloroform-*d*) δ 9.08 (s, 1H), 8.73 - 8.59 (m, 1H), 8.56 - 8.39 (m, 2H), 8.14 (br s, 1H), 7.71 (s, 1H), 7.62 (d, J = 8.1 Hz, 1H), 7.34 - 7.20 (m, 2H), 6.72 (s, 1H), 6.46 - 6.28 (m, 2H), 5.78 - 5.66 (m, 1H), 3.89 (s, 3H), 3.49 - 3.44 (m, 1H), 3.24 - 3.15 (m, 4H), 2.97 - 2.89 (m, 1H), 2.35 (s, 6H), 2.24 - 2.16 (m, 1H), 2.04 - 1.96 (m, 1H), 1.22 - 1.05 (m, 4H).

## Example 78

**(*R*)-N-(5-((5-Cyano-4-(1-cyclopropyl-1H-indol-3-yl)pyrimidin-2-yl)amino)-2-(3-(di methylamino)pyrrolidin-1-yl)-4-methoxyphenyl)acrylamide**

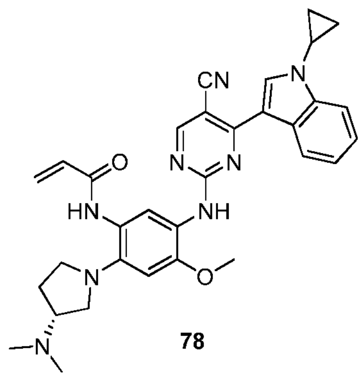

78

The compound of Example 78 was prepared by referring to the method of Example 2.

MS m/z (ESI): 563.3 $[M+H]^+$.

$^1$H NMR (400 MHz, Chloroform-d) δ 9.08 (s, 1H), 8.63 (s, 1H), 8.55 - 8.46 (m, 2H), 8.24 - 8.11 (m, 1H), 7.71 (s, 1H), 7.62 (d, J = 8.1 Hz, 1H), 7.37 - 7.27 (m, 2H), 6.71 (s, 1H), 6.37 (s, 2H), 5.79 - 5.66 (m, 1H), 3.89 (s, 3H), 3.50 - 3.44 (m, 1H), 3.26 - 3.09 (m, 4H), 2.99 - 2.89 (m, 1H), 2.37 (s, 6H), 2.25 - 2.17 (m, 1H), 2.05 - 1.97 (m, 1H), 1.18 - 1.07 (m, 4H).

## Example 79

**N-(5-((5-Cyano-4-(1-cyclopropyl-1H-indol-3-yl)pyrimidin-2-yl)amino)-2-(4-(3-(cya nomethyl)azetidin-1-yl)piperidin-1-yl)-4-methoxyphenyl)acrylamide**

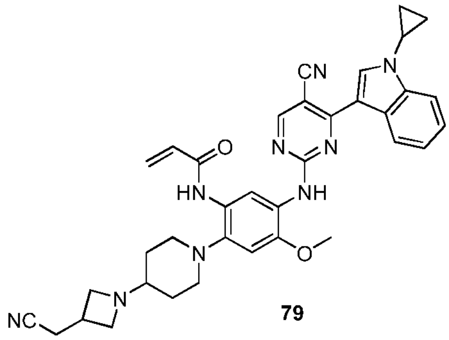

79

The compound of Example 79 was prepared by referring to the method of Example 2.
MS m/z (ESI): 628.2 [M+H]+.

**Example 80**

**N-(5-((5-Cyano-4-(1-cyclopropyl-1H-indol-3-yl)pyrimidin-2-yl)amino)-4-methoxy-2 -(4-(3-(methoxymethyl)azetidin-1-yl)piperidin-1-yl)phenyl)acrylamide**

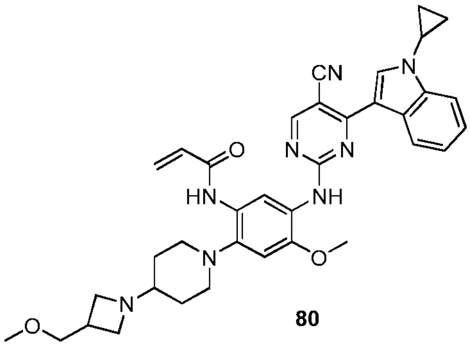

80

The compound of Example 80 was prepared by referring to the method of Example 2.
MS m/z (ESI): 633.3 [M+H]+.

**Example 81**

**N-(2-((2-(Dimethylamino)ethyl)(methyl)amino)-4-methoxy-5-((4-(1-methyl-1H-ind ol-3-yl)-5-(oxazol-2-yl)pyrimidin-2-yl)amino)phenyl)acrylamide**

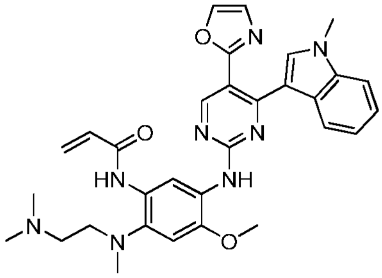

81

The compound of Example 81 was prepared by referring to the method of Example 3.

MS m/z (ESI): 567.1 [M+H]+.

$^1$H NMR (400 MHz, $CDCl_3$) δ 10.16-9.96 *(br,* 1H), 9.78 (s, 1H), 8.86 (s, 1H), 8.77-8.56 *(br,* 1H), 7.88 (s, 1H), 7.47 (s, 1H), 7.28 (d, *J* = 8.0 Hz, 1H), 7.19 (s, 1H), 7.13 (t, *J* = 8.0 Hz, 1H), 7.01-6.88 (m, 2H), 6.78 (s, 1H), 6.45(d, *J* = 13.2 Hz, 2H), 5.73-6.69 (m, 1H), 3.94(s, 3H), 3.88 (s, 3H), 3.00-2.87 *(br,* 2H), 2.71 (s, 3H), 2.42-2.21 *(br,* 8H).

**Example 82**

**N-(4-Methoxy-2-(methyl((1-methylpyrrolidin-2-yl)methyl)amino)-5-((4-(1-methyl-1 H-indol-3-yl)-5-(oxazol-2-yl)pyrimidin-2-yl)amino)phenyl)acrylamide**

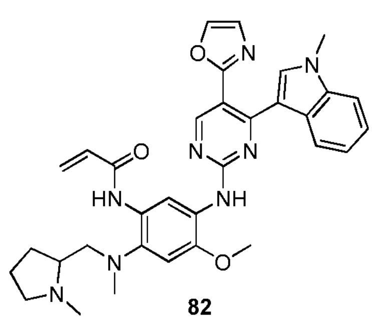

**82**

The compound of Example 82 was prepared by referring to the method of Example 3.

MS m/z (ESI): 593.4 [M+H]+.

**Example 83**

**N-(2-((2-(Dimethylamino)ethyl)(methyl)amino)-4-methoxy-5-((4-(1-methyl-1H-ind ol-3-yl)-5-(1-methyl-1H-pyrazol-4-yl)pyrimidin-2-yl)amino)phenyl)acrylamide**

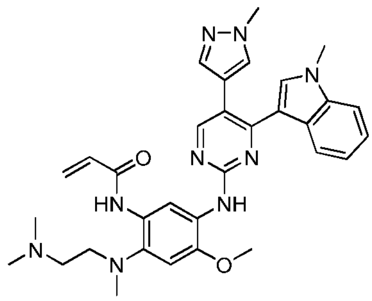

**83**

The compound of Example 83 was prepared by referring to the method of Example 3.

MS m/z (ESI): 580.5 [M+H]+.

**Example 84**

**N-(4-Methoxy-2-(methyl((1-methylpyrrolidin-2-yl)methyl)amino)-5-((4-(1-methyl-1 H-indol-3-yl)-5-(1-methyl-1H-pyrazol-4-yl)pyrimidin-2-yl)amino)phenyl)acrylamid** e

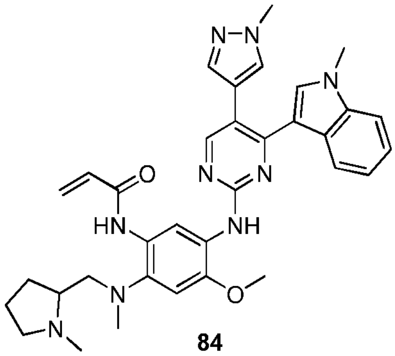

**84**

The compound of Example 84 was prepared by referring to the method of Example 3.

MS m/z (ESI): 606.2 $[M+H]^+$.

**Example 85**

**N-(5-((4-(1-Cyclopropyl-1H-indol-3-yl)-5-(1-methyl-1H-pyrazol-4-yl)pyimidin-2-y l)amino)-2-((2-(dimethylamino)ethyl)(methyl)amino)-4-methoxyphenyl)acrylamide**

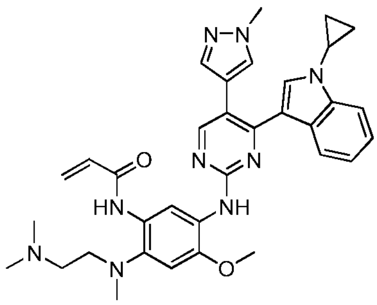

**85**

The compound of Example 85 was prepared by referring to the method of Example 3.

MS m/z (ESI): 606.3 $[M+H]^+$.

$^1$H NMR (400 MHz, $CDCl_3$) $\delta$ 10.25-10.01(*br*, 1H), 9.62 (s, 1H), 8.44 (s, 1H), 7.66-7.61 (m, 2H), 7.56 (s, 1H), 7.52 (d, *J* = 8.0 Hz, 1H), 7.41 (s, 1H), 7.19-7.16 (m, 2H), 7.05(t, *J* = 7.2 Hz, 1H), 6.77 (s, 1H), 6.48-6.21 (m, 2H), 5.71-5.68 (m, 1H), 3.87 (s, 3H), 3.83 (s, 3H), 3.38-3.30 (m, 1H),3.00-2.84 (m, 2H), 2.71 (s, 3H), 2.40-2.16 (m, 8H), 1.04-1.00 (m, 4H).

**Example 86**

**N-(5-((4-(1-Cyclopropyl-1H-indol-3-yl)-5-(1-methyl-1H-pyrazol-4-yl)pyrimidin-2-y l)amino)-4-methoxy-2-(methyl((1-methylpyrrolidin-2-yl)methyl)amino)phenyl)acry lamide**

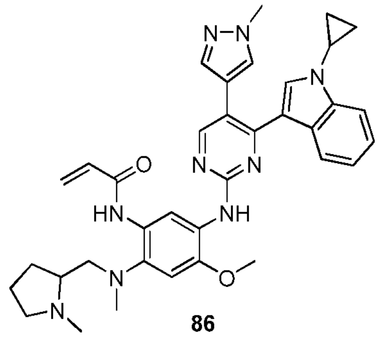

86

The compound of Example 86 was prepared by referring to the method of Example 3.
MS m/z (ESI): 632.1 [M+H]$^+$.

**Example 87**

**N-(2-((2-(Dimethylamino)ethyl)(methyl)amino)-4-methoxy-5-((4-(1-methyl-1H-ind ol-3-yl)-5-(pyridin-3-yl)pyrimidin-2-yl)amino)phenyl)acrylamide**

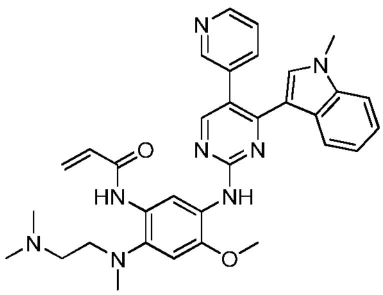

87

The compound of Example 87 was prepared by referring to the method of Example 3.
MS m/z (ESI): 577.5 [M+H]$^+$.

**Example 88**

**N-(4-Methoxy-2-(methyl((1-methylpyrrolidin-2-yl)methyl)amino)-5-((4-(1-methyl-1 H-indol-3-yl)-5-(pyridin-3-yl)pyrimidin-2-yl)amino)phenyl)acrylamide**

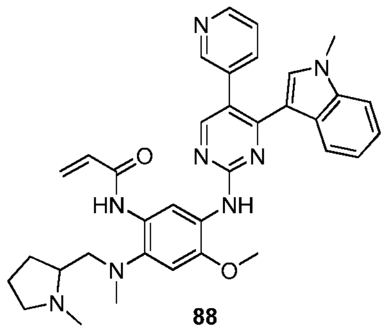

88

The compound of Example 88 was prepared by referring to the method of Example 3.
MS m/z (ESI): 603.2 [M+H]$^+$.

**Example 89**

**N-(5-((4-(1-Cyclopropyl-1H-indol-3-yl)-5-(pyridin-3-yl)pyrimidin-2-yl)amino)-2-((2 -(dimethylamino)ethyl)(methyl)amino)-4-methoxyphenyl)acrylamide**

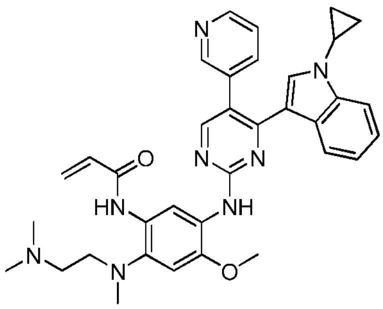

**89**

The compound of Example 89 was prepared by referring to the method of Example 3.

MS m/z (ESI): 603.1 $[M+H]^+$.

**Example 90**

**N-(5-((4-(1-Cyclopropyl-1H-indol-3-yl)-5-(pyridin-3-yl)pyrimidin-2-yl)amino)-4-m ethoxy-2-(methyl((1-methylpyrrolidin-2-yl)methyl)amino)phenyl)acrylamide**

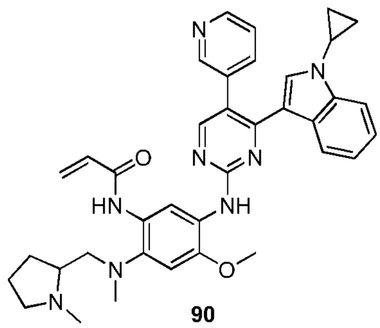

**90**

The compound of Example 90 was prepared by referring to the method of Example 3.

MS m/z (ESI): 629.3 $[M+H]^+$.

**Example 91**

**N-(2-((2-(Bis(methyl-*d*3)amino)ethyl)(methyl)amino)-5-((5-cyano-4-(1-cyclopropyl-1H-indol-3-yl)pyrimidin-2-yl)amino)-4-methoxyphenyl)acrylamide**

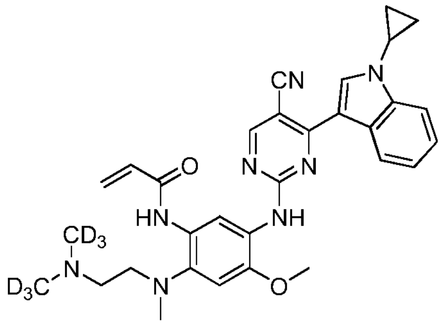

**91**

The compound of Example 91 was prepared by referring to the method of Example 2.
MS m/z (ESI): 557.1 [M+H]$^+$.

**Example 92**

**N-(5-((5-Cyano-4-(1-cyclopropyl-1H-indol-3-yl)pyrimidin-2-yl)amino)-4-methoxy-2 -(methyl((1-(methyl-*d*3)pyrrolidin-2-yl)methyl)amino)phenyl)acrylamide**

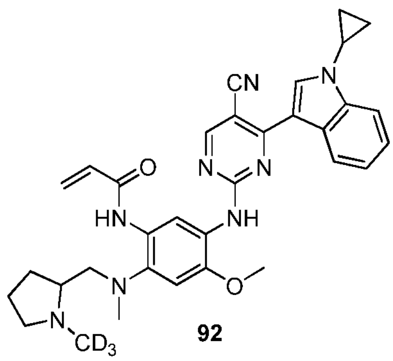

**92**

The compound of Example 92 was prepared by referring to the method of Example 2.
MS m/z (ESI): 580.4 [M+H]$^+$.

**Example 93**

**N-(5-((5-Cyano-4-(1-cyclopropyl-1H-indol-3-yl)pyrimidin-2-yl)amino)-2-((2-(dimet hylamino)ethyl)(methyl-*d*3)amino)-4-methoxyphenyl)acrylamide**

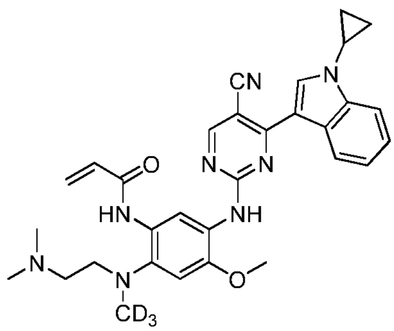

**93**

The compound of Example 93 was prepared by referring to the method of Example 2.
MS m/z (ESI): 554.2 $[M+H]^+$.

### Example 94

**N-(5-((5-Cyano-4-(1-cyclopropyl-1H-indol-3-yl)pyrimidin-2-yl)amino)-4-methoxy-2 -((methyl-*d*3)((1-methylpyrrolidin-2-yl)methyl)amino)phenyl)acrylamide**

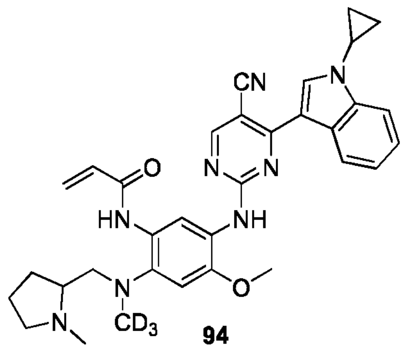

**94**

The compound of Example 94 was prepared by referring to the method of Example 2.
MS m/z (ESI): 580.5 $[M+H]^+$.

### Example 95

**N-(5-((4-(1-Cyclopropyl-1H-indol-3-yl)-5-(1-(methyl-*d*3)-1H-pyrazol-4-yl)pyrimidi n-2-yl)amino)-2-((2-(dimethylamino)ethyl)(methyl)amino)-4-methoxyphenyl)acryla mide**

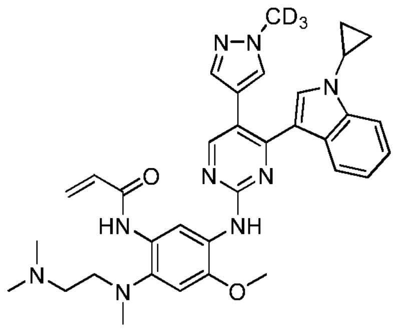

**95**

The compound of Example 95 was prepared by referring to the method of Example 3.
MS m/z (ESI): 609.4 $[M+H]^+$.

### Example 96

**N-(5-((4-(1-Cyclopropyl-1H-indol-3-yl)-5-(1-(methyl-*d*3)-1H-pyrazol-4-yl)pyrimidi n-2-yl)amino)-4-methoxy-2-(methyl((1-methylpyrrolidin-2-yl)methyl)amino)phenyl )acrylamide**

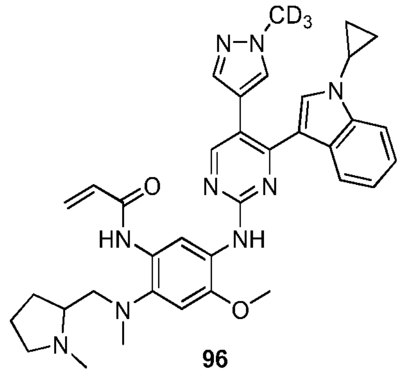

96

The compound of Example 96 was prepared by referring to the method of Example 3.

MS m/z (ESI): 635.2 [M+H]+.

**Example 97**

**N-(5-((4-(1-Cyclopropyl-1H-indol-3-yl)-5-(1H-pyrazol-4-yl)pyrimidin-2-yl)amino)-2-((2-(dimethylamino)ethyl)(methyl)amino)-4-methoxyphenyl)acrylamide**

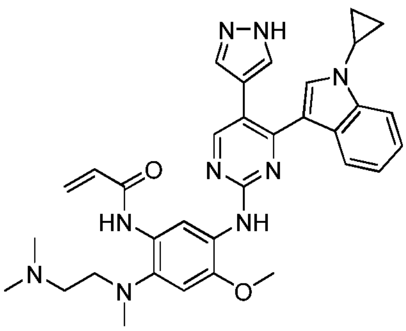

97

The compound of Example 97 was prepared by referring to the method of Example 3.

MS m/z (ESI): 592.1 [M+H]+.

**Example 98 N-(2-((2-(Dimethylamino)ethyl)(methyl)amino)-4-methoxy-5-((5-(1-methyl-1H-pyr azol-4-yl)-4-(1-(methyl-*d*3)-1H-indol-3-yl)pyrimidin-2-yl)amino)phenyl)acrylamide**

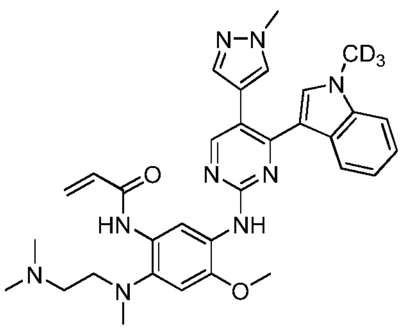

98

The compound of Example 98 was prepared by referring to the method of Example 3.

MS m/z (ESI): 583.3 [M+H]$^+$.

**Example 99**

**N-(5-((4-(1H-Indol-3-yl)-5-(1-methyl-1H-pyrazol-4-yl)pyrimidin-2-yl)amino)-2-((2-(dimethylamino)ethyl)(methyl)amino)-4-methoxyphenyl)acrylamide**

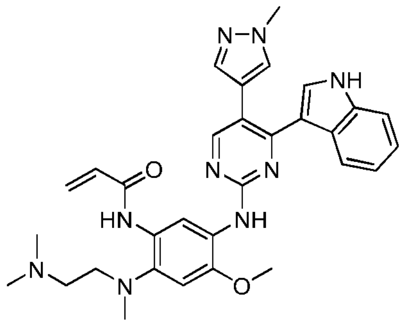

**99**

The compound of Example 99 was prepared by referring to the method of Example 3.

MS m/z (ESI): 566.4 [M+H]$^+$.

**Example 100**

**N-(4-Methoxy-2-(methyl(2-(methylamino)ethyl)amino)-5-((4-(1-methyl-1H-indol-3-yl)-5-(1-methyl-1H-pyrazol-4-yl)pyrimidin-2-yl)amino)phenyl)acrylamide**

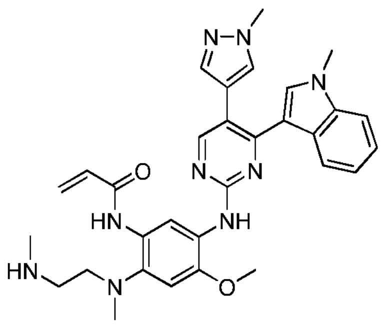

**100**

The compound of Example 100 was prepared by referring to the method of Example 3.

MS m/z (ESI): 566.2 [M+H]$^+$.

**Example 101**

**N-(5-((4-(1-Cyclopropyl-1H-indol-3-yl)-5-(isopropylsulfonyl)pyrimidin-2-yl)amino) -2-((2-(dimethylamino)ethyl)(methyl)amino)-4-methoxyphenyl)acrylamide**

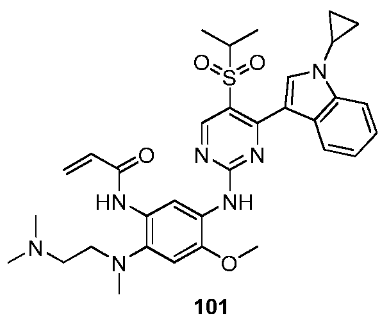

**101**

The compound of Example 101 was prepared by referring to the method of Example 2.

MS m/z (ESI): 632.3[M+H]$^+$.

**Example 102**

**N-(5-((4-(1-Cyclopropyl-1H-indol-3-yl)-5-(isopropylsulfonyl)pyrimidin-2-yl)amino) -4-methoxy-2-(methyl((1-methylpyrrolidin-2-yl)methyl)amino)phenyl)acrylamide**

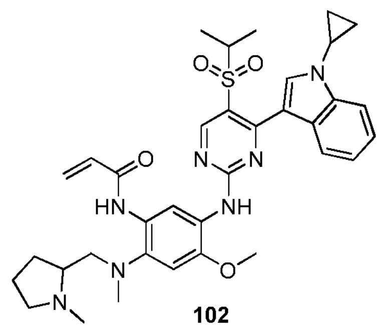

**102**

The compound of Example 102 was prepared by referring to the method of Example 2.

MS m/z (ESI): 658.1[M+H]$^+$.

**Example 103**

**N-(2-((2-(Dimethylamino)ethyl)(methyl)amino)-4-methoxy-5-((4-(1-methyl-1H-ind ol-3-yl)-5-(methylsulfonyl)pyrimidin-2-yl)amino)phenyl)acrylamide**

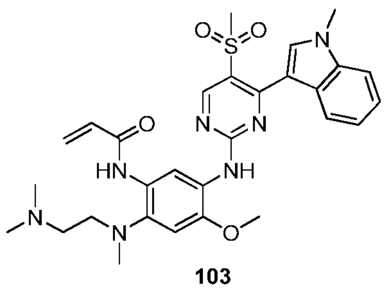

**103**

The compound of Example 103 was prepared by referring to the method of Example 2.

MS m/z (ESI): 578.4[M+H]$^+$.

**Example 104**

**N-(4-Methoxy-2-(methyl((1-methylpyrrolidin-2-yl)methyl)amino)-5-((4-(1-methyl-1 H-indol-3-yl)-5-(methylsulfonyl)pyrimidin-2-yl)amino)phenyl)acrylamide**

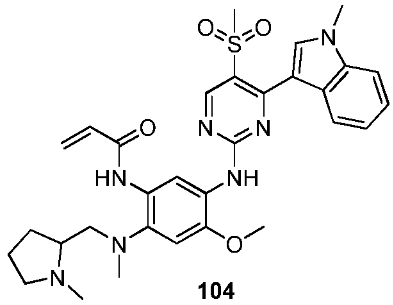

**104**

The compound of Example 104 was prepared by referring to the method of Example 2.
MS m/z (ESI): 604.5[M+H]+.

**Example 105**

**N-(2-((2-(Dimethylamino)ethyl)(methyl)amino)-5-((5-(dimethylphosphoryl)-4-(1-m ethyl-1H-indol-3-yl)pyrimidin-2-yl)amino)-4-methoxyphenyl)acrylamide**

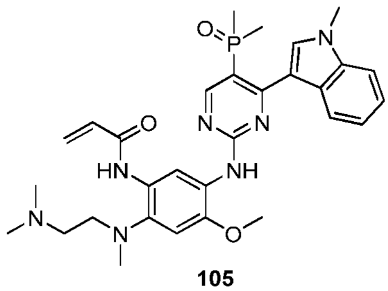

**105**

The compound of Example 105 was prepared by referring to the method of Example 3.
MS m/z (ESI): 576.1 [M+H]+.

**Example 106**

**N-(5-((5-(Dimethylphosphoryl)-4-(1-methyl-1H-indol-3-yl)pyrimidin-2-yl)amino)-4 -methoxy-2-(methyl((1-methylpyrrolidin-2-yl)methyl)amino)phenyl)acrylamide**

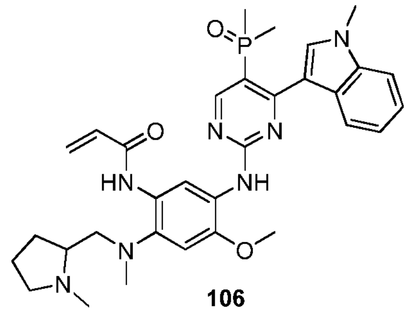

106

The compound of Example 106 was prepared by referring to the method of Example 3.

MS m/z (ESI): 602.2[M+H]$^+$.

**Example 107**

**N-(5-((4-(1-Cyclopropyl-1H-indol-3-yl)-5-(dimethylphosphoryl)pyrimidin-2-yl)ami no)-2-((2-(dimethylamino)ethyl)(methyl)amino)-4-methoxyphenyl)acrylamide**

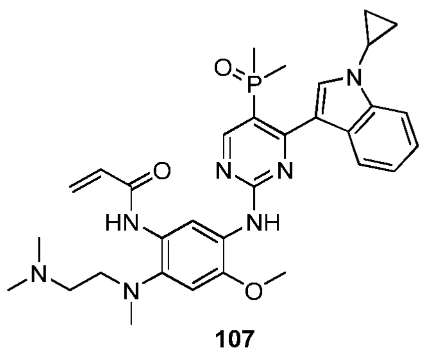

107

The compound of Example 107 was prepared by referring to the method of Example 3.

MS m/z (ESI): 602.3[M+H]$^+$.

**Example 108**

**N-(5-((4-(1-Cyclopropyl-1H-indol-3-yl)-5-(dimethylphosphoryl)pyrimidin-2-yl)ami no)-4-methoxy-2-(methyl((1-methylpyrrolidin-2-yl)methyl)amino)phenyl)acrylami de**

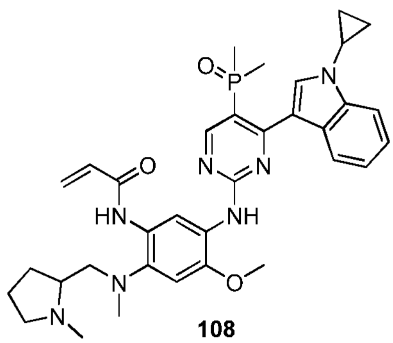

108

The compound of Example 108 was prepared by referring to the method of Example 3.

MS m/z (ESI): 628.1 [M+H]$^+$.

**Example 109**

**N-(5-((5-Cyano-4-(1-cyclopropyl-1H-indol-3-yl)pyrimidin-2-yl)amino)-4-methoxy-2 -((3aR,6aR)-1-methylhexahydropyrrolo[3,4-b]pyrrol-5(1H)-yl)phenyl)acrylamide**

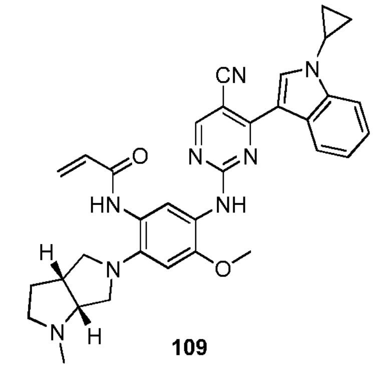

**109**

The compound of Example 109 was prepared by referring to the method of Example 2.

MS m/z (ESI): 575.2[M+H]+.

$^1$H NMR (400 MHz, $CDCl_3$) δ 10.10(*br,* 1H), 9.21-9.00 *(br,* 1H), 8.64 (s, 1H), 8.55-8.38 (m, 2H), 7.69 *(br,* 1H), 7.60 (d, *J* = 8.0 Hz, 1H), 7.32-7.29 (m, 2H), 6.77 (s, 1H), 6.45-6.32 (m, 2H), 5.70-5.67 (m, 1H), 3.88 (s, 3H), 3.48-3.35 (m, 2H),3.08-3.01(m, 2H), 2.98-2.81 (m, 4H), 2.72-2.23 (m, 6H), 1.17-1.11 (m, 4H).

**Example 110**

**N-(5-((5-Cyano-4-(1-cyclopropyl-1H-indol-3-yl)pyrimidin-2-yl)amino)-4-methoxy-2 -((3aS,6aS)-1-methylhexahydropyrrolo[3,4-b]pyrrol-5(1H)-yl)phenyl)acrylamide**

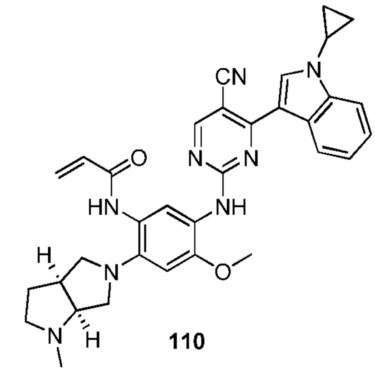

**110**

The compound of Example 110 was prepared by referring to the method of Example 2.

MS m/z (ESI): 575.3 [M+H]+.

$^1$H NMR (400 MHz, Chloroform-*d*) δ 8.90 - 8.72 (m, 1H), 8.56 (s, 1H), 8.51 - 8.30 (m, 2H), 7.64 (s, 1H), 7.59 - 7.45 (m, 1H), 7.40 - 7.21 (m, 3H), 6.70 - 6.47 (m, 1H), 6.47 - 6.10 (m, 2H), 5.73 - 5.45 (m, 1H), 3.81 (s, 3H), 3.71 - 3.48 (m, 1H), 3.50 - 3.33 (m, 1H), 3.22 - 1.92 (m, 9H), 1.33 - 0.65 (m, 7H).

**Example 111**

**N-(5-((5-Cyano-4-(1-(oxetan-3-yl)-1H-indol-3-yl)pyrimidin-2-yl)amino)-2-((2-(dime thylamino)ethyl)(methyl)amino)-4-methoxyphenyl)acrylamide**

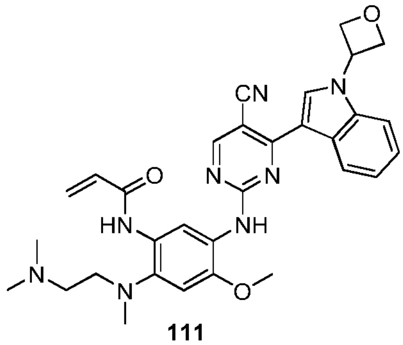

**111**

Method 1:

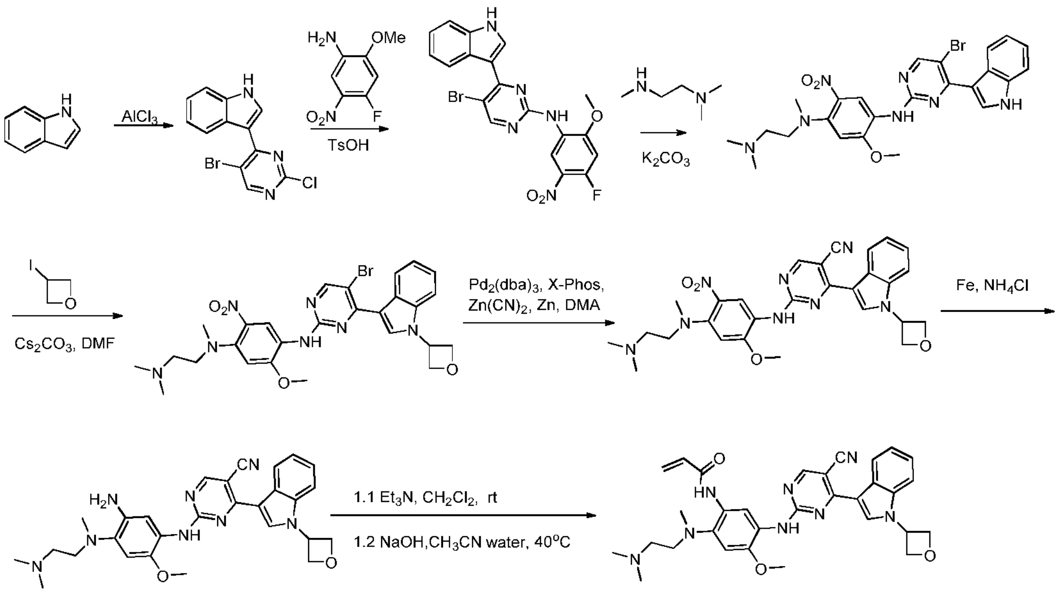


111

Step 1: Preparation of 3-(5-bromo-2-chloropyrimidin-4-yl)-1H-indole

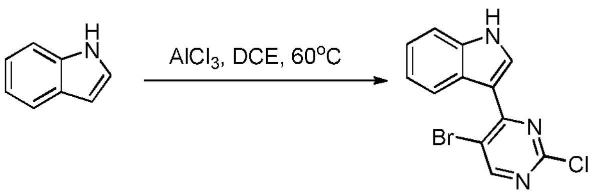


2,4-Dichloro-5-bromopyrimidine (3.0 g, 13.17 mmol) was dissolved in 1,2-dichloroethane (50 mL), and the solution was cooled to 0°C. Aluminum trichloride (3.88 g, 23.94 mmol) was added, and the reaction solution was stirred at room temperature for half an hour. Indole (1.4 g, 11.97 mmol) was added, and the reaction solution was heated to 60°C and reacted for 14 hours. The reaction solution was cooled to room temperature, neutralized with saturated sodium bicarbonate solution, and stirred at room temperature for 20 minutes. The solution was filtered, and the filter cake was rinsed with dichloromethane. The filter cake was dried by rotary evaporation to obtain the crude product 3-(5-bromo-2-chloropyrimidin-4-yl)-1H-indole (1.7 g, yield: 41.8%, yellow solid).

MS m/z (ESI): 308.0, 310.0 $[M+H]^+$.

Step 2: Preparation of 5-bromo-N-(4-fluoro-2-methoxy-5-nitrophenyl)-4-(1H-indol-3-yl)pyrimidin-2-amine

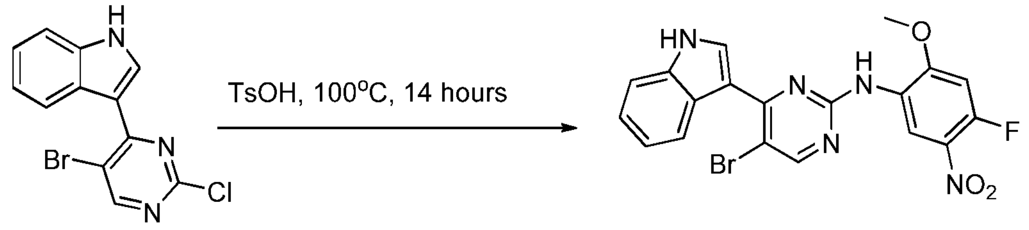


3-(5-Bromo-2-chloropyrimidin-4-yl)-1H-indole (0.50 g, 1.62 mmol), 4-fluoro-2-methoxy-5-nitroaniline (0.33 g, 1.78 mmol) andp-toluenesulfonic acid (0.34 g, 1.78 mmol) were dissolved in 2-pentanol (25 mL). The reaction solution was reacted at 100°C for 14 hours. The reaction solution was cooled to room temperature, neutralized with saturated sodium bicarbonate solution, and stirred at room temperature for 20 minutes. The solution was filtered, and the filter cake was rinsed with 2-pentanol followed by petroleum ether. The filter cake was dried to obtain 5-bromo-N-(4-fluoro-2-methoxy-5-nitrophenyl)-4-(1H-indol-3-yl)pyrimidin-2-amine (0.40 g, 54%, khaki solid).

MS m/z (ESI): 458.0, 460.0 $[M+H]^+$.

Step 3: Preparation of $N^1$-(5-bromo-4-(1H-indol-3-yl)pyrimidin-2-yl)-$N^4$-(2-(dimethylamino)ethyl)-2-methoxy -$N^4$-methyl-5-nitrobenzene-1,4-diamine

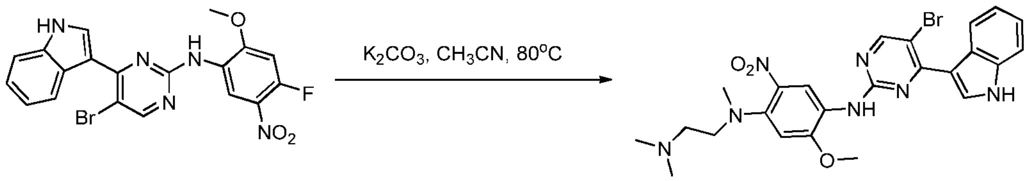


5-Bromo-N-(4-fluoro-2-methoxy-5-nitrophenyl)-4-(1H-indol-3-yl)pyrimidin-2-ami ne (0.40 g, 0.87 mmol) was dissolved in acetonitrile (20 mL), followed by the addition of potassium carbonate (0.36 g, 2.61 mmol) and N1,N1,N2-trimethylethane-1,2-diamine (0.10 g, 0.96 mmol). The reaction solution was stirred at 80°C for 2 hours. The reaction solution was cooled to room temperature, diluted with ethyl acetate, and washed with saturated brine. The organic phase was dried over anhydrous sodium sulfate, filtered and concentrated to dryness by rotary evaporation to obtain $N^1$-(5-bromo-4-(1H-indol-3-yl)pyrimidin-2-yl)-$N^4$-(2-(dimethylamino)ethyl)-2-methoxy -$N^4$-methyl-5-nitrobenzene-1,4-diamine (0.40 g, yield: 87%, red solid).

MS m/z (ESI): 540.2, 542.2 $[M+H]^+$.

Step 4: Preparation of $N^1$-(5-bromo-4-(1-(oxetan-3-yl)-1H-indol-3-yl)pyrimidin-2-yl)-$N^4$-(2-(dimethylamino)e thyl)-2-methoxy-$N^4$-methyl-5-nitrobenzene-1,4-diamine

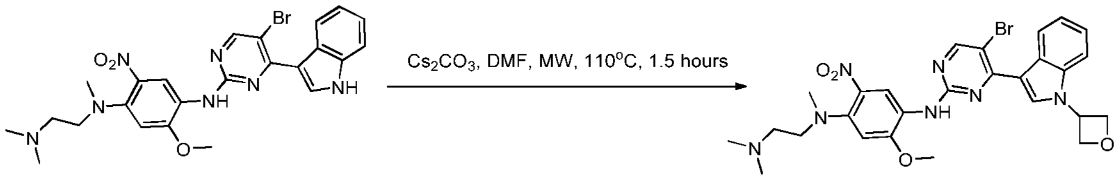


$N^1$-(5-Bromo-4-(1H-indol-3-yl)pyrimidin-2-yl)-$N^4$-(2-(dimethylamino)ethyl)-2-me thoxy-$N^4$-methyl-5-nitrobenzene-1,4-diamine (0.30 g, 0.56 mmol), cesium carbonate (0.13 g, 0.72 mmol) and 3-iodooxetane (0.36 g, 1.11 mmol) were dissolved in N,N-dimethylformamide (8 mL) at room temperature. The reaction solution was reacted under microwave at 110°C for 1.5 hours, followed by cooling to room temperature. The reaction solution was diluted with ethyl

acetate and filtered. The organic phase was washed with saturated brine, dried over anhydrous sodium sulfate, filtered and concentrated to dryness by rotary evaporation. The resulting crude product was purified by preparative thin layer chromatography (dichloromethane: methanol= 20:1) to obtain $N^1$-(5-bromo-4-(1-(oxetan-3-yl)-1H-indol-3-yl)pyrimidin-2-yl)-$N^4$-(2-(dimethylamino)e thyl)-2-methoxy-$N^4$-methyl-5-nitrobenzene-1,4-diamine (0.12 g, yield: 36%, red solid).

MS m/z (ESI): 596.0, 598.0 $[M+H]^+$.

Step 5: Preparation of 2-((4-((2-(dimethylamino)ethyl)(methyl)amino)-2-methoxy-5-nitrophenyl)amino)-4-(1-( oxetan-3-yl)-1H-indol-3-yl)pyrimidine-5-carbonitrile

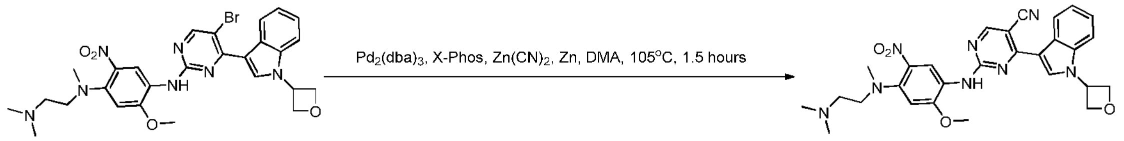


$N^1$-(5-Bromo-4-(1-(oxetan-3-yl)-1H-indol-3-yl)pyrimidin-2-yl)-$N^4$-(2-(dimethylam ino)ethyl)-2-methoxy-$N^4$-methyl-5-nitrobenzene-1,4-diamine (0.12 g, 0.20 mmol), $Pd_2(dba)_3$ (18.4 mg, 0.02 mmol), X-Phos (19 mg, 0.04 mmol), zinc cyanide (23.5 mg, 0.20 mmol) and zinc powder (13 mg, 0.20 mmol) were dissolved in N,N-dimethylacetamide (3 mL) at room temperature. The reaction solution was purged with nitrogen, and reacted under microwave at 110°C for 1.5 hours. The reaction solution was cooled to room temperature and filtered, and the resulting solid was rinsed with ethyl acetate. The organic phase was dried by an oil pump, and the resulting crude product was purified by preparative thin layer chromatography (dichloromethane: methanol= 20:1) to obtain 2-((4-((2-(dimethylamino)ethyl)(methyl)amino)-2-methoxy-5-nitrophenyl)amino)-4-(1-( oxetan-3-yl)-1H-indol-3-yl)pyrimidine-5-carbonitrile (0.096 g, yield: 88%, yellow solid).

MS m/z (ESI): 543.1 $[M+H]^+$.

Step 6: Preparation of 2-((5-amino-4-((2-(dimethylamino)ethyl)(methyl)amino)-2-methoxyphenyl)amino)-4-(1 -(oxetan-3-yl)-1H-indol-3-yl)pyrimidine-5-carbonitrile

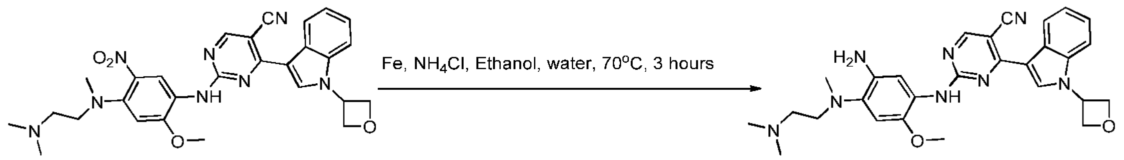


2-((4-((2-(Dimethylamino)ethyl)(methyl)amino)-2-methoxy-5-nitrophenyl)amino)-4-(1-(oxetan-3-yl)-1H-indol-3-yl)pyrimidine-5-carbonitrile (0.12 g, 0.22 mmol) was dissolved in ethanol (10 mL), followed by the addition of aqueous ammonium chloride solution (0.06 g, 1.1 mmol, water, 2 mL) and iron powder (0.12 g, 2.2 mmol). The reaction solution was stirred at 80°C for 2 hours. The reaction solution was cooled to room temperature and filtered. The filter cake was rinsed with dichloromethane, and the organic solvent was removed under reduced pressure. The residues were dissolved in dichloromethane and water, and the resulting solution was partitioned. The aqueous phase was extracted with dichloromethane (20 mL x 2). The organic phases were combined, dried over anhydrous sodium sulfate, filtered and concentrated to dryness by rotary evaporation to obtain 2-((5-amino-4-((2-(dimethylamino)ethyl)(methyl)amino)-2-methoxyphenyl)amino)-4-(1 -(oxetan-3-yl)-1H-indol-3-yl)pyrimidine-5-carbonitrile (0.095 g, yield: 83.8%, yellow oil).

MS m/z (ESI): 513.2 $[M+H]^+$.

Step 7: Preparation of N-(5-((5-cyano-4-(1-(oxetan-3-yl)-1H-indol-3-yl)pyrimidin-2-yl)amino)-2-((2-(dimethy lamino)ethyl)(methyl)amino)-4-methoxyphenyl)acrylamide

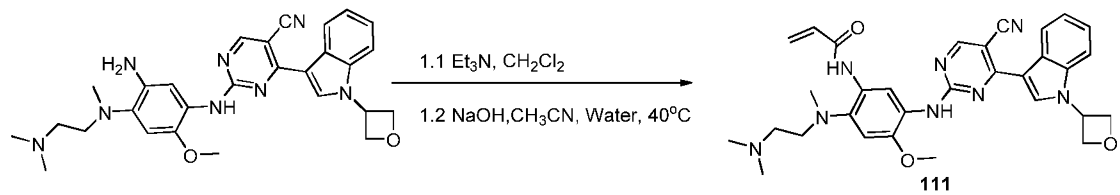


111

2-((5-Amino-4-((2-(dimethylamino)ethyl)(methyl)amino)-2-methoxyphenyl)amino )-4-(1-(oxetan-3-yl)-1H-indol-3-yl)pyrimidine-5-carbonitrile (0.075 g, 0.146 mmol) was dissolved in dichloromethane (5 mL), and the solution was cooled to 0°C. Triethylamine (0.03 g, 0.292 mmol) and 3-chloropropionyl chloride (0.024 g, 0.19 mmol) were added successively. The reaction solution was stirred at 0°C for 1 hour. After completion of the reaction, the reaction solution was concentrated to dryness by rotary evaporation. The resulting crude product was dissolved in dichloromethane, and washed with saturated sodium bicarbonate solution and saturated brine. The organic phase was dried over anhydrous sodium sulfate, filtered and concentrated to dryness by rotary evaporation. The resulting crude product was dissolved in acetonitrile (5 mL), followed by the addition of aqueous sodium hydroxide solution (0.058 g, 1.46 mmol, water, 0.5 mL). The reaction solution was reacted at 40°C for two hours. The reaction solution was cooled to room temperature, diluted with ethyl acetate, and washed with saturated brine. The organic phase was dried over anhydrous sodium sulfate, filtered and concentrated to dryness by rotary evaporation. The resulting crude product was purified by preparative chromatography to obtain N-(5-((5-cyano-4-(1-(oxetan-3-yl)-1H-indol-3-yl)pyrimidin-2-yl)amino)-2-((2-(dimethy lamino)ethyl)(methyl)amino)-4-methoxyphenyl)acrylamide (0.04 g, yield: 48%, yellow solid).

Method 2:

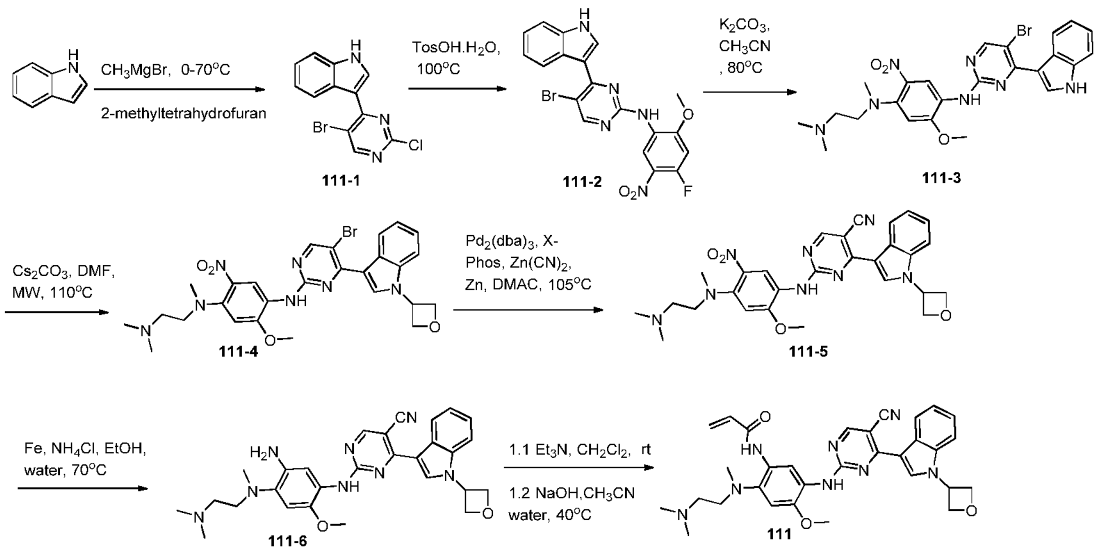


## Step 1: Preparation of 3-(5-bromo-2-chloropyrimidin-4-yl)-1H-indole

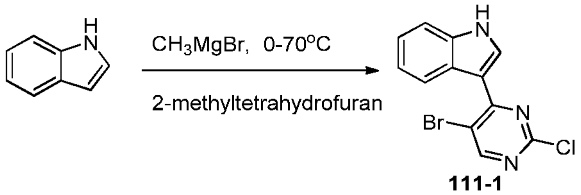


111-1

Indole (5.0 g, 54.86 mmol) was dissolved in 2-methyltetrahydrofuran (50 mL) under an ice bath. Methyl magnesium bromide (3.0M 2-methyltetrahydrofuran solution, 18.3 mL, 54.86 mmol) was added dropwise, and the reaction solution was kept at below 30°C. After completion of the addition, the reaction solution was stirred at room temperature for 30 minutes. A solution of 2,4-dichloro-5-bromopyrimidine in 2-methyltetrahydrofuran (5.0 g, 21.94 mmol, 10 mL of solvent) was added dropwise. After completion of the addition, the reaction solution was stirred at room temperature for half an hour, and then heated to 70°C for 14 hours. The reaction solution was cooled to room temperature, and poured into saturated aqueous ammonium chloride solution. A solid precipitated out, which was collected by filtration. The resulting solid was resuspended in water, sonicated and filtered. The resulting solid was dried to obtain the product 3-(5-bromo-2-chloropyrimidin-4-yl)-1H-indole (4.0 g, yield: 59%, yellow solid).

MS m/z (ESI): 308.0 $[M+H]^+$.

$^1H$ NMR (400 MHz, DMSO-*d6*) $\delta$ 12.21 (s, 1H), 8.86 (s, 1H), 8.80 (s, 1H), 8.46 (d, *J* = 7.2 Hz, 1H), 7.55 (d, *J*= 7.2 Hz, 1H), 7.30-7.23 (m, 2H).

Step 2: Preparation of 5-bromo-N-(4-fluoro-2-methoxy-5-nitrophenyl)-4-(1H-indol-3-yl)pyrimidin-2-amine

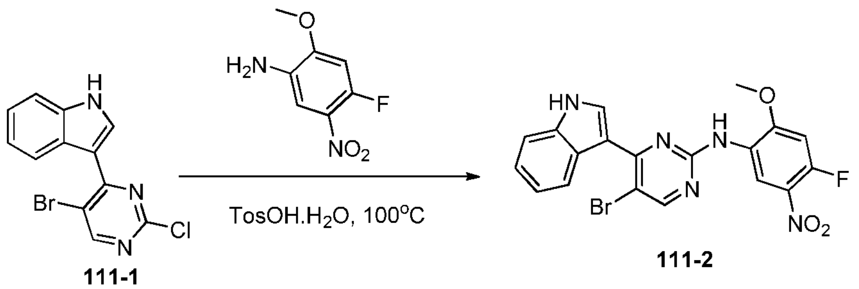


111-1 111-2

3-(5-Bromo-2-chloropyrimidin-4-yl)-1H-indole (0.50 g, 1.62 mmol), 4-fluoro-2-methoxy-5-nitroaniline (0.33 g, 1.78 mmol) andp-toluenesulfonic acid (0.34 g, 1.78 mmol) were dissolved in 2-pentanol (25 mL). The reaction solution was reacted at 100°C for 14 hours. The reaction solution was cooled to room temperature, neutralized with saturated sodium bicarbonate solution, and stirred at room temperature for 20 minutes. The solution was filtered, and the filter cake was rinsed with 2-pentanol followed by petroleum ether. The filter cake was dried to obtain 5-bromo-N-(4-fluoro-2-methoxy-5-nitrophenyl)-4-(1H-indol-3-yl)pyrimidin-2-amine (0.40 g, 54%, khaki solid).

MS m/z (ESI): 458.0, 460.0 $[M+H]^+$.

Step 3: Preparation of N1-(5-bromo-4-(1H-indol-3-yl)pyrimidin-2-yl)-N4-(2-(dimethylamino)ethyl)-2-methox y-N4-methyl-5-nitrobenzene-1,4-diamine

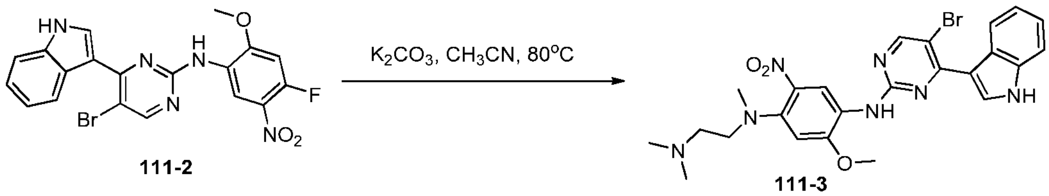


111-2 111-3

5-Bromo-N-(4-fluoro-2-methoxy-5-nitrophenyl)-4-(1H-indol-3-yl)pyrimidin-2-ami ne (0.40 g, 0.87 mmol) was dissolved in acetonitrile (20 mL), followed by the addition of potassium carbonate (0.36 g, 2.61 mmol) and N1,N1,N2-trimethylethane-1,2-diamine (0.10 g, 0.96 mmol). The reaction solution was stirred at 80°C for 2 hours. The reaction solution was cooled to room temperature, diluted with ethyl acetate, and washed with saturated brine. The organic phase was dried over anhydrous sodium sulfate, filtered and concentrated to dryness by rotary evaporation to obtain N1-(5-bromo-4-(1H-indol-3-yl)pyrimidin-2-yl)-N4-(2-(dimethylamino)ethyl)-2-methox y-N4-methyl-5-nitrobenzene-1,4-diamine (0.40 g, yield: 87%, red solid).

MS m/z (ESI): 540.2, 542.2 $[M+H]^+$.

Step 4: Preparation of N1-(5-bromo-4-(1-(oxetan-3-yl)-1H-indol-3-yl)pyrimidin-2-yl)-N4-(2-(dimethylamino) ethyl)-2-methoxy-N4-methyl-5-nitrobenzene-1,4-diamine

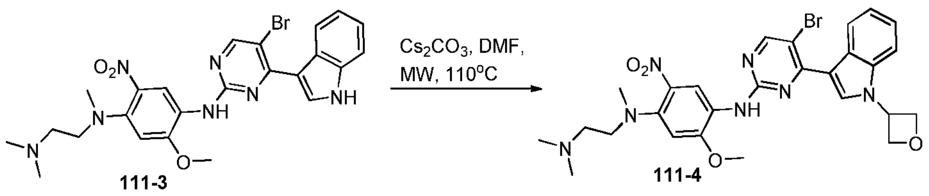


N1-(5-Bromo-4-(1H-indol-3-yl)pyrimidin-2-yl)-N4-(2-(dimethylamino)ethyl)-2-m ethoxy-N4-methyl-5-nitrobenzene-1,4-diamine (0.30 g, 0.56 mmol), cesium carbonate (0.13 g, 0.72 mmol) and 3-iodo-oxetane (0.36 g, 1.11 mmol) were dissolved in N,N-dimethylformamide (8 mL) at room temperature. The reaction solution was reacted under microwave at 110°C for 1.5 hours, followed by cooling to room temperature. The reaction solution was diluted with ethyl acetate and filtered. The organic phase was washed with saturated brine, dried over anhydrous sodium sulfate, filtered and concentrated to dryness by rotary evaporation. The resulting crude product was purified by silica gel preparative thin layer chromatography (dichloromethane: methanol= 20:1) to obtain N1-(5-bromo-4-(1-(oxetan-3-yl)-1H-indol-3-yl)pyrimidin-2-yl)-N4-(2-(dimethylamino) ethyl)-2-methoxy-N4-methyl-5-nitrobenzene-1,4-diamine (0.12 g, yield: 36%, red solid).

MS m/z (ESI): 596.0 [M+H]+.

Step 5: Preparation of 2-((4-((2-(dimethylamino)ethyl)(methyl)amino)-2-methoxy-5-nitrophenyl)amino)-4-(1-( oxetan-3-yl)-1H-indol-3-yl)pyrimidine-5-carbonitrile

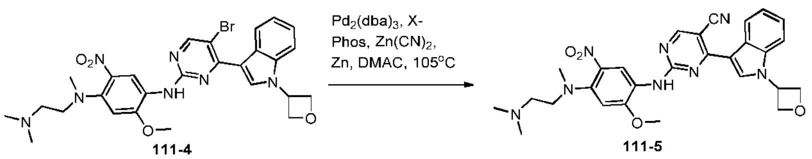


N1-(5-Bromo-4-(1-(oxetan-3-yl)-1H-indol-3-yl)pyrimidin-2-yl)-N4-(2-(dimethyla mino)ethyl)-2-methoxy-N4-methyl-5-nitrobenzene-1,4-diamine (0.12 g, 0.20 mmol), $Pd_2(dba)_3$ (18.4 mg, 0.02 mmol), X-Phos (19 mg, 0.04 mmol), zinc cyanide (23.5 mg, 0.20 mmol) and zinc powder (13 mg, 0.20 mmol) were dissolved in N,N-dimethylacetamide (3 mL) at room temperature. The reaction solution was purged with nitrogen, and reacted under microwave at 110°C for 1.5 hours. The reaction solution was cooled to room temperature and filtered, and the resulting solid was rinsed with ethyl acetate. The organic phase was concentrated under reduced pressure, and the resulting crude product was purified by preparative thin layer chromatography (dichloromethane: methanol= 20:1) to obtain 2-((4-((2-(dimethylamino)ethyl)(methyl)amino)-2-methoxy-5-nitrophenyl)amino)-4-(1-( oxetan-3-yl)-1H-indol-3-yl)pyrimidine-5-carbonitrile (0.096 g, yield: 88%, yellow solid).

MS m/z (ESI): 543.1 [M+H]+.

Step 6: Preparation of 2-((5-amino-4-((2-(dimethylamino)ethyl)(methyl)amino)-2-methoxyphenyl)amino)-4-(1 -(oxetan-3-yl)-1H-indol-3-yl)pyrimidine-5-carbonitrile

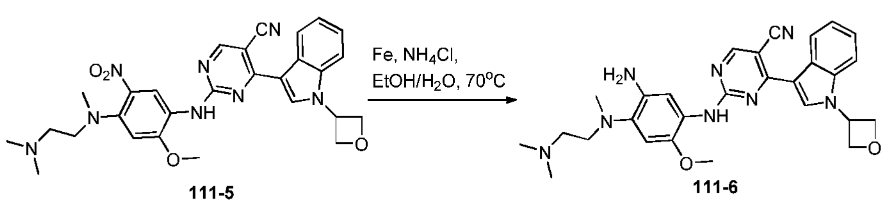


2-((4-((2-(Dimethylamino)ethyl)(methyl)amino)-2-methoxy-5-nitrophenyl)amino)-4-(1-(oxetan-3-yl)-1H-indol-3-yl)pyrimidine-5-carbonitrile (0.12 g, 0.22 mmol) was dissolved in ethanol (10 mL), followed by the addition of aqueous ammonium chloride solution (0.06 g, 1.1 mmol, water, 2 mL) and iron powder (0.12 g, 2.2 mmol). The reaction solution was stirred at 80°C for 2 hours. The reaction solution was cooled to room temperature and filtered. The filter cake was rinsed with

dichloromethane, and the organic solvent was removed under reduced pressure. The residues were dissolved in dichloromethane and water, and the resulting solution was partitioned. The aqueous phase was extracted with dichloromethane (20 mL × 2). The organic phases were combined, dried over anhydrous sodium sulfate, filtered and concentrated to dryness by rotary evaporation to obtain 2-((5-amino-4-((2-(dimethylamino)ethyl)(methyl)amino)-2-methoxyphenyl)amino)-4-(1 -(oxetan-3-yl)-1H-indol-3-yl)pyrimidine-5-carbonitrile (0.095 g, yield: 83.8%, yellow oil).

MS m/z (ESI): 513.2 $[M+H]^+$.

Step 7: Preparation of N-(5-((5-cyano-4-(1-(oxetan-3-yl)-1H-indol-3-yl)pyrimidin-2-yl)amino)-2-((2-(dimethy lamino)ethyl)(methyl)amino)-4-methoxyphenyl)acrylamide

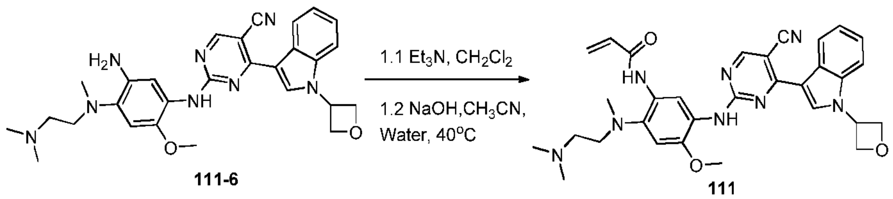


111-6 111

2-((5-Amino-4-((2-(dimethylamino)ethyl)(methyl)amino)-2-methoxyphenyl)amino )-4-(1-(oxetan-3-yl)-1H-indol-3-yl)pyrimidine-5-carbonitrile (0.075 g, 0.146 mmol) was dissolved in dichloromethane (5 mL), and the solution was cooled to 0°C. Triethylamine (0.03 g, 0.292 mmol) and 3-chloropropionyl chloride (0.024 g, 0.19 mmol) were added successively. The reaction solution was stirred at 0°C for 1 hour. After completion of the reaction, the reaction solution was concentrated to dryness by rotary evaporation. The resulting crude product was dissolved in dichloromethane, and washed with saturated sodium bicarbonate solution and saturated brine. The organic phase was dried over anhydrous sodium sulfate, filtered and concentrated to dryness by rotary evaporation. The resulting crude product was dissolved in acetonitrile (5 mL), followed by the addition of aqueous sodium hydroxide solution (0.058 g, 1.46 mmol, water, 0.5 mL). The reaction solution was reacted at 40°C for two hours. The reaction solution was cooled to room temperature, diluted with ethyl acetate, and washed with saturated brine. The organic phase was dried over anhydrous sodium sulfate, filtered and concentrated to dryness by rotary evaporation. The resulting crude product was purified by preparative chromatography to obtain N-(5-((5-cyano-4-(1-(oxetan-3-yl)-1H-indol-3-yl)pyrimidin-2-yl)amino)-2-((2-(dimethy lamino)ethyl)(methyl)amino)-4-methoxyphenyl)acrylamide (0.04 g, yield: 48%, yellow solid).

MS m/z (ESI): 567.2$[M+H]^+$.

$^1H$ NMR (400 MHz, $CDCl_3$) δ 10.15-9.82 (*br,* 1H), 9.45 (s, 1H), 8.69 (s, 2H),8.55-8.38(*br*, 1H) 7.80 (s, 1H), 7.61 (d, *J*= 8.0 Hz, 1H), 7.33-7.29 (m, 2H), 6.78 (s, 1H), 6.42-6.38 (m, 1H), 5.72-5.66 (m, 2H), 5.28-5.13(m, 5H), 3.90 (s, 3H), 3.13-2.95 (*br,* 2H),2.74 (s, 3H), 2.61-2.24 (*br,* 6H), 1.85-1.53 (m, 2H).

## Example 112

**N-(5-((5-Cyano-4-(1-cyclopropyl-6-methoxy-1H-indol-3-yl)pyrimidin-2-yl)amino)-2 -((2-(dimethylamino)ethyl)(methyl)amino)-4-methoxyphenyl)acrylamide**

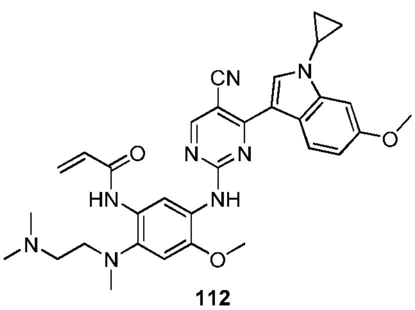

112

The compound of Example 112 was prepared by referring to the method of Example 2.

MS m/z (ESI): 581.4 $[M+H]^+$.

$^1$H NMR (400 MHz, $CDCl_3$) δ 10.04 (s, 1H), 9.38 (s, 1H), 8.66 (s, 1H), 8.40-8.38 (m, 2H), 7.71 (s, 1H), 7.06 (s, 1H), 6.92 (s, 1H), 6.77 (s, 1H), 6.42-6.38 (m, 1H), 5.71-5.68 (m, 1H), 3.91-3.89 (m, 6H), 3.41-3.40 (m, 1H), 3.10-2.90 (m, 3H), 2.74 (s, 3H), 2.70-2.30 (m, 8H), 1.16-1.10 (m, 4H).

**Example 113**

**N-(5-((5-Cyano-4-(1-cyclopropyl-1H-indol-3-yl)pyrimidin-2-yl)amino)-2-(2-(dimeth ylamino)ethoxy)-4-methoxyphenyl)acrylamide**

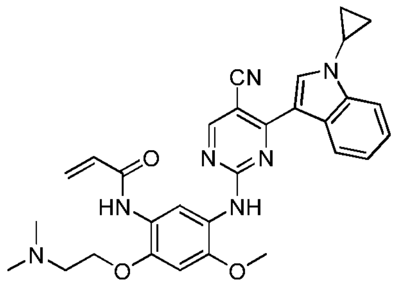

**113**

The compound of Example 113 was prepared by referring to the method of Example 2.

MS m/z (ESI): 538.4[M+H]$^+$.

$^1$H NMR (400 MHz, $CDCl_3$) δ 9.76 (s, 1H), 9.26 (s, 1H), 8.64 (s, 1H), 8.47 (s, 1H), 7.77 - 7.63 (m, 1H), 7.61 (d, J = 8.1 Hz, 1H), 7.34 - 7.27 (m, 1H), 6.65 (s, 1H), 6.35 (t, J = 26.3 Hz, 2H), 5.69 (d, J = 11.4 Hz, 1H), 4.16 (t, J = 5.1 Hz, 2H), 3.87 (s, 3H), 3.52 - 3.39 (m, 1H), 2.74 - 2.59 (m, 2H), 2.40 (s, 6H), 1.21 - 1.04 (m, 4H).

**Example 114**

**N-(5-((5-Cyano-4-(1-cyclopropyl-6-fluoro-1H-indol-3-yl)pyrimidin-2-yl)amino)-2-(( 2-(dimethylamino)ethyl)(methyl)amino)-4-methoxyphenyl)acrylamide**

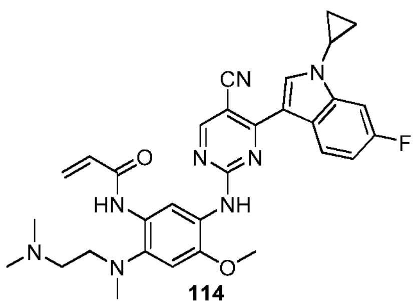

**114**

The compound of Example 114 was prepared by referring to the method of Example 2.

MS m/z (ESI): 569.4 [M+H]$^+$.

$^1$H NMR (400 MHz, $CDCl_3$) δ 10.03 - 9.64 (m, 1H), 9.48 - 9.26 (m, 1H), 8.66 (s, 1H), 8.54 - 8.33 (m, 2H), 7.73 (s, 1H), 7.12 - 6.92 (m, 1H), 6.76 (s, 1H), 6.38 (d, *J* = 17.0 Hz, 1H), 5.70 (d, *J* = 11.4 Hz, 1H), 3.90 (s, 3H), 3.48 - 3.35 (m, 1H), 3.18 - 2.98 (m, 2H), 2.74 (s, 3H), 2.71 - 2.31 (m, 6H), 2.12 - 1.47 (m, 4H), 1.22 - 1.02 (m, 4H).

**Example 115**

**N-(5-((5-Cyano-4-(6-cyano-1-cyclopropyl-1H-indol-3-yl)pyrimidin-2-yl)amino)-2-(( 2-(dimethylamino)ethyl)(methyl)amino)-4-methoxyphenyl)acrylamide**

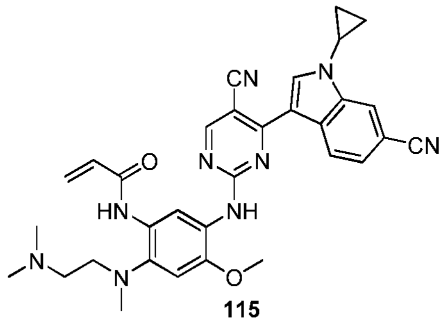

115

The compound of Example 115 was prepared by referring to the method of Example 2.

MS m/z (ESI): 576.4[M+H]+.

$^1$H NMR (400 MHz, $CDCl_3$) δ 12.53 (s, 1H), 9.48 (s, 1H), 9.29 (s, 1H), 8.68 (s, 1H), 8.61 (s, 1H), 8.56 - 8.43 (m, 1H), 8.03 - 7.92 (m, 1H), 7.91 - 7.79 (m, 1H), 7.55 - 7.44 (m, 1H), 6.83 - 6.69 (m, 1H), 6.44 - 6.32 (m, 1H), 5.76 (d, J = 10.2 Hz, 1H), 3.95 (s, 3H), 3.58 - 3.30 (m, 3H), 3.25 - 3.06 (m, 2H), 2.83 (s, 6H), 1.65 (s, 3H), 1.30 - 1.20 (m, 2H), 1.17 - 1.12 (s, 2H).

## Example 116

**N-(5-((5-Cyano-4-(1-cyclopropyl-1H-indol-3-yl)pyrimidin-2-yl)amino)-2-((2-(dimet hylamino)ethyl)(methyl)amino)-6-methoxypyridin-3-yl)acrylamide**

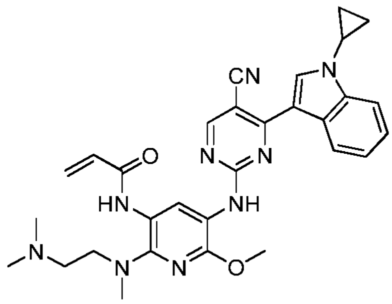

116

Method 1:

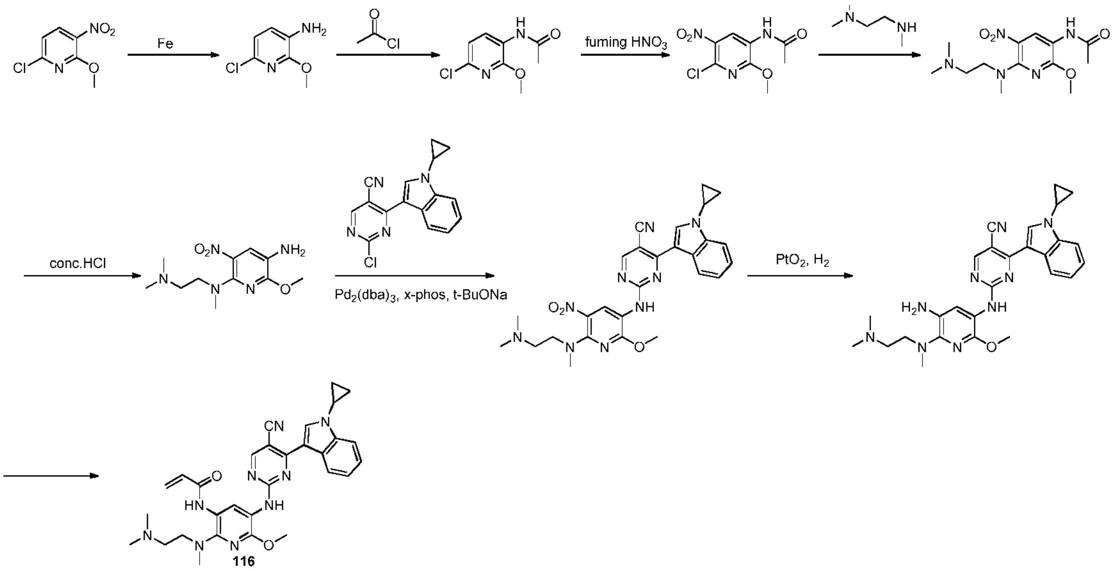

116

Step 1: Preparation of 6-chloro-2-methoxypyridin-3-amine

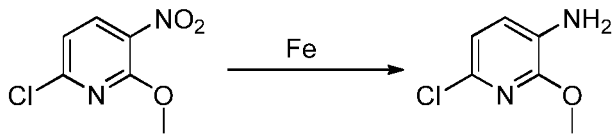


6-Chloro-2-methoxy-3-nitropyridine (5 g, 26.6 mmol) was dissolved in ethanol (100 mL) and water (30 mL). Ammonium chloride (7.0 g, 133 mmol) was added, and iron powder (7.5 g, 133 mmol) was added in batches. The reaction solution was stirred at 80°C for 2 hours. The reaction solution was cooled to room temperature, and filtered through celite. Ethyl acetate (150 mL) and saturated brine (120 mL) were added to the filtrate, and the organic layer was separated. The organic layer was dried over anhydrous sodium sulfate, filtered and concentrated to dryness by rotary evaporation to obtain 6-chloro-2-methoxypyridin-3-amine (4 g, yield: 95%) as a brown solid.
MS m/z (ESI): 159.1 $[M+H]^+$.

Step 2: Preparation of N-(6-chloro-2-methoxypyridin-3-yl)acetamide

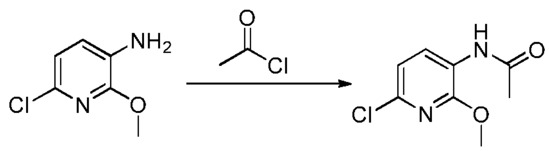

6-Chloro-2-methoxypyridin-3-amine (2.0 g, 12.7 mmol) was dissolved in dichloromethane (100 mL), followed by the addition of diisopropylethylamine (3.5 mL, 19.0 mmol). The reaction solution was cooled to 0°C, to which acetyl chloride (1.2 g, 15.2 mmol) was added and stirred for 2 hours. The reaction solution was washed successively with 80 mL of water, 80 mL of 1N hydrochloric acid and 80 mL of saturated brine, dried over anhydrous sodium sulfate, filtered and concentrated to dryness by rotary evaporation to obtain N-(6-chloro-2-methoxypyridin-3-yl)acetamide (2.0 g, yield: 79%) as a brown solid.
MS m/z (ESI): 201.1 $[M+H]^+$.

Step 3: Preparation of N-(6-chloro-2-methoxy-5-nitropyridin-3-yl)acetamide

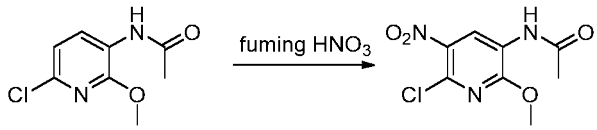


6-Chloro-2-methoxypyridin-3-amine (2.0 g, 10.0 mmol) was dissolved in trifluoroacetic anhydride (20 mL), and cooled to -10°C. Fuming nitric acid (0.5 mL, 10 mmol) was added dropwise, and the reaction solution was stirred for 2 hours. Crushed ice was added to the reaction solution, and the solution was extracted with dichloromethane (50 mL). The organic phase was dried over anhydrous sodium sulfate, filtered and concentrated to dryness by rotary evaporation to obtain N-(6-chloro-2-methoxy-5-nitropyridin-3-yl)acetamide (1.6 g, yield: 65%) as a brown solid.
MS m/z (ESI): 244.1 $[M-H]^+$.

Step 4: Preparation of N-(6-((2-(dimethylamino)ethyl)(methyl)amino)-2-methoxy-5-nitropyridin-3-yl)acetami de

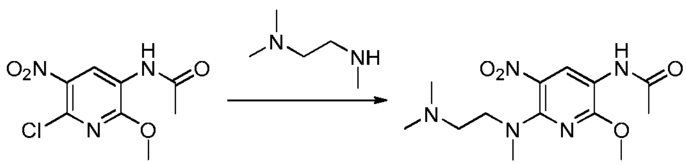

N-(6-chloro-2-methoxy-5-nitropyridin-3-yl)acetamide (1.6 g, 6.5 mmol) was dissolved in acetonitrile (30 mL), followed by the addition of N1,N1,N2-trimethylethane-1,2-diamine (1 g, 9.8 mmol). The reaction solution was stirred at 80°C for 3 hours. The solvent was removed by rotary evaporation, and the resulting crude product was purified by column chromatography (dichloromethane/methanol: 100/1 ~ 10/1) to obtain N-(6-((2-(dimethylamino)ethyl)(methyl)amino)-2-methoxy-5-nitropyridin-3-yl)acetami de (0.9 g, yield: 45%) as a brown solid.
MS m/z (ESI): 312.1 $[M+H]^+$.

Step 5: Preparation of N2-(2-(dimethylamino)ethyl)-6-methoxy-N2-methyl-3-nitropyridine-2,5-diamine

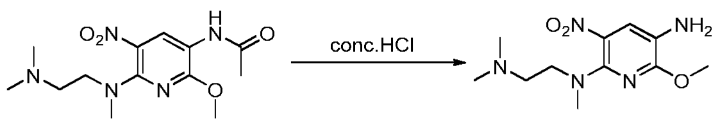


N-(6-((2-(dimethylamino)ethyl)(methyl)amino)-2-methoxy-5-nitropyridin-3-yl)ace tamide (0.9 g, 2.9 mmol) was dissolved in methanol (30 mL) and concentrated hydrochloric acid (5 mL). The reaction solution was stirred at 60°C for 3 hours. The solvent was removed by rotary evaporation, and dichloromethane (50 mL) and saturated sodium bicarbonate solution (50 mL) were added to the resulting residues and stirred until no bubbles generation. The organic layer was separated, dried over anhydrous sodium sulfate, filtered and concentrated to dryness by rotary evaporation. The resulting crude product was purified by column chromatography (dichloromethane/methanol: 100/1 ~ 10/1) to obtain N2-(2-(dimethylamino)ethyl)-6-methoxy-N2-methyl-3-nitropyridine-2,5-diamine (0.15 g, yield: 19%) as a brown solid.
MS m/z (ESI): 270.1 $[M+H]^+$.

Step 6: Preparation of 4-(1-cyclopropyl-1H-indol-3-yl)-2-((6-((2-(dimethylamino)ethyl)(methyl)amino)-2-met hoxy-5-nitropyridin-3-yl)amino)pyrimidine-5-carbonitrile

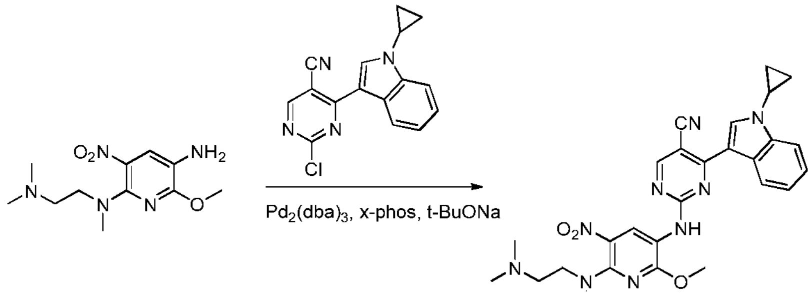


N2-(2-(Dimethylamino)ethyl)-6-methoxy-N2-methyl-3-nitropyridine-2,5-diamine (0.11 g, 0.41 mmol), 2-chloro-4-(1-cyclopropyl-1H-indol-3-yl)pyrimidine-5-carbonitrile (0.12 g, 0.41 mmol), tris(dibenzylideneacetone)dipalladium (0.18 g, 0.2 mmol), x-phos (0.2 g, 0.41 mmol) and sodium *tert*-butoxide (0.12 g, 1.2 mmol) were dissolved in dioxane (5 mL). The reaction solution was purged with nitrogen, and stirred under microwave at 140°C for 1 hour. The reaction solution was cooled to room temperature and filtered. The filtrate was concentrated to dryness by rotary evaporation, and the resulting crude product was purified by prep-TLC (dichloromethane/methanol: 20/1) to obtain 4-(1-cyclopropyl-1H-indol-3-yl)-2-((4-((2-(dimethylamino)ethyl)(methyl)amino)-2-met hoxy-5-nitrophenyl)amino)pyrimidine-5-carbonitrile (0.1 g, yield: 47%) as a yellow solid.

MS m/z (ESI): 528.1 $[M+H]^+$.

Step 7: Preparation of 2-((5-amino-6-((2-(dimethylamino)ethyl)(methyl)amino)-2-methoxypyridin-3-yl)amino )-4-(1-cyclopropyl-1H-indol-3-yl)pyrimidine-5-carbonitrile

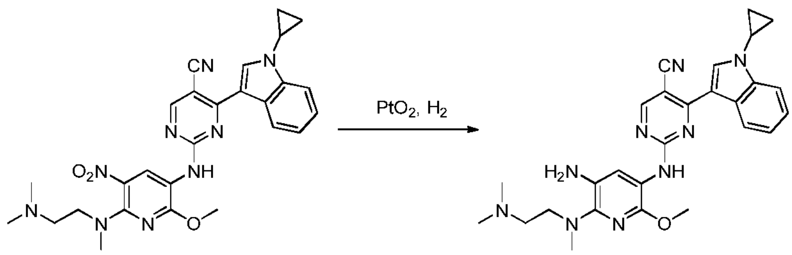


4-(1-Cyclopropyl-1H-indol-3-yl)-2-((4-((2-(dimethylamino)ethyl)(methyl)amino)-2-methoxy-5-nitropphenyl)amino)pyrimidine-5-carbonitrile (0.1 g, 0.19 mmol) and platinum dioxide (0.05 g) were dissolved in tetrahydrofuran (5 mL). The reaction solution was purged with hydrogen, and stirred at room temperature overnight. The reaction solution was filtered, and the filtrate was concentrated to dryness by rotary evaporation to obtain 2-((5-amino-6-((2-(dimethylamino)ethyl)(methyl)amino)-2-methoxypyridin-3-yl)amino )-4-(1-cyclopropyl-1H-indol-3-yl)pyrimidine-5-carbonitrile (85 mg, yield: 90%) as a brown solid.

MS m/z (ESI): 498.1 $[M+H]^+$.

Step 8: Preparation of N-(5-((5-cyano-4-(1-cyclopropyl-1H-indol-3-yl)pyrimidin-2-yl)amino)-2-((2-(dimethyl amino)ethyl)(methyl)amino)-6-methoxypyridin-3-yl)acrylamide

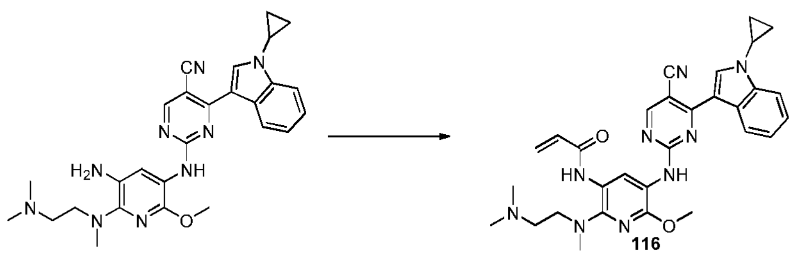

116

2-((5-Amino-6-((2-(dimethylamino)ethyl)(methyl)amino)-2-methoxypyridin-3-yl)a mino)-4-(1-cyclopropyl-1H-indol-3-yl)pyrimidine-5-carbonitrile (85 mg, 0.17 mmol) was dissolved in tetrahydrofuran (5 mL), and the solution was cooled to 0°C. Triethylamine (26 mg, 0.26 mmol) and 3-chloropropionyl chloride (33 mg, 0.26 mmol) were added successively. The reaction solution was stirred at 0°C for 1 hour. 3M sodium hydroxide solution (2 mL) was added, and the reaction solution was stirred at 40°C for 1 hour. Water (20 mL) was added, and the solution was extracted with dichloromethane (20 mL × 2). The organic phase was dried over anhydrous sodium sulfate, filtered and concentrated to dryness by rotary evaporation. The resulting crude product was purified by prep-TLC (dichloromethane/MeOH: 20/1) to obtain N-(5-((5-cyano-4-(1-cyclopropyl-1H-indol-3-yl)pyrimidin-2-yl)amino)-2-((2-(dimethyl amino)ethyl)(methyl)amino)-6-methoxypyridin-3-yl)acrylamide (0.03 g, yield: 32%) as a yellow solid.

MS m/z (ESI): 552.1 [M+H]+.

Method 2:

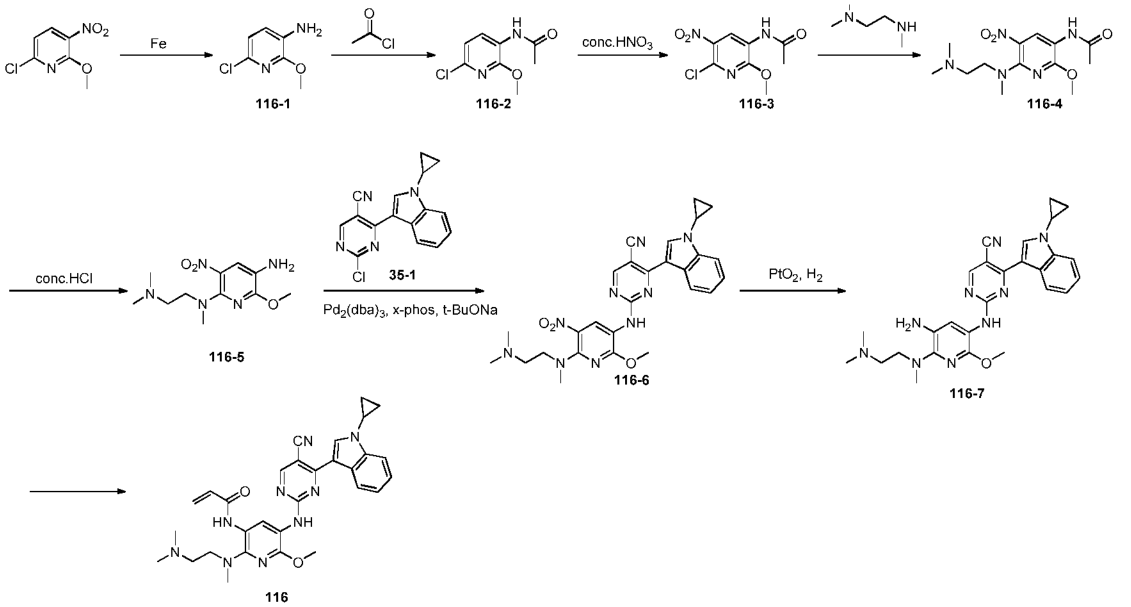


Step 1: Preparation of 6-chloro-2-methoxypyridin-3-amine

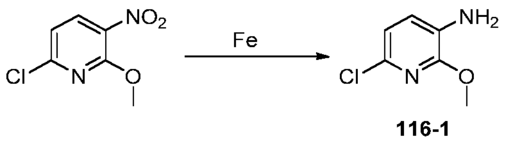


116-1

6-Chloro-2-methoxy-3-nitropyridine (5 g, 26.6 mmol) was dissolved in ethanol (100 mL) and water (30 mL). Ammonium chloride (7.0 g, 133 mmol) was added, and iron powder (7.5 g, 133 mmol) was added in batches. The reaction solution was stirred at 85°C for 2 hours. The reaction solution was cooled to room temperature, and filtered through celite. Ethyl acetate (150 mL) and saturated brine (120 mL) were added to the filtrate, and the organic layer was separated. The organic layer was dried over anhydrous sodium sulfate, filtered and concentrated to dryness by rotary evaporation to obtain 6-chloro-2-methoxypyridin-3-amine (4 g, yield: 95%) as a brown solid.

MS m/z (ESI): 159.1 [M+H]+.

Step 2: Preparation of N-(6-chloro-2-methoxypyridin-3-yl)acetamide

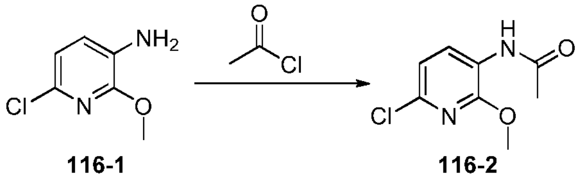

116-1 116-2

6-Chloro-2-methoxypyridin-3-amine (4.0 g, 25.0 mmol) was dissolved in dichloromethane (100 mL), followed by the addition of diisopropylethylamine (4.8 g, 37.5 mmol). The reaction solution was cooled to 0°C, to which acetyl chloride (2.4 g, 30.0 mmol) was added and stirred for 2 hours. The reaction solution was washed successively with 80 mL of water, 80 mL of 1N hydrochloric acid and 80 mL of saturated brine, dried over anhydrous sodium sulfate, filtered and concentrated to dryness by rotary evaporation to obtain N-(6-chloro-2-methoxypyridin-3-yl)acetamide (4.0 g, yield: 79%) as a brown solid.

MS m/z (ESI): 201.1 $[M+H]^+$.

Step 3: Preparation of N-(6-chloro-2-methoxy-5-nitropyridin-3-yl)acetamide

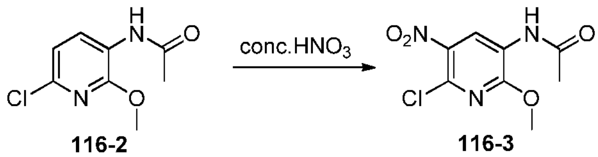


116-2 116-3

6-Chloro-2-methoxypyridin-3-amine (2.0 g, 10.0 mmol) was dissolved in trifluoroacetic anhydride (20 mL), and cooled to -10°C. Fuming nitric acid (0.5 mL, 10 mmol) was added dropwise, and the reaction solution was stirred for 2 hours. Crushed ice was added to the reaction solution, and the solution was extracted with dichloromethane (50 mL). The organic phase was dried over anhydrous sodium sulfate, filtered and concentrated to dryness by rotary evaporation to obtain N-(6-chloro-2-methoxy-5-nitropyridin-3-yl)acetamide (1.6 g, yield: 65%) as a brown solid.

MS m/z (ESI): 244.1 $[M-H]^+$.

Step 4: Preparation of N-(6-((2-(dimethylamino)ethyl)(methyl)amino)-2-methoxy-5-nitropyridin-3-yl)acetami de

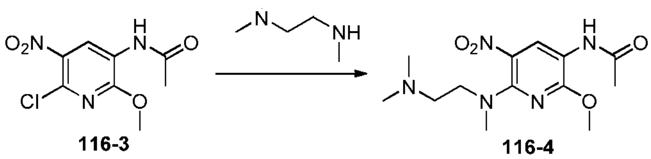

116-3 116-4

N-(6-chloro-2-methoxy-5-nitropyridin-3-yl)acetamide (1.6 g, 6.5 mmol) was dissolved in acetonitrile (30 mL), followed by the addition of N1,N1,N2-trimethylethane-1,2-diamine (1 g, 9.8 mmol). The reaction solution was stirred at 80°C for 3 hours. The solvent was removed by rotary evaporation, and the resulting crude product was purified by column chromatography (dichloromethane/methanol: 100/1 ~ 10/1) to obtain N-(6-((2-(dimethylamino)ethyl)(methyl)amino)-2-methoxy-5-nitropyridin-3-yl)acetami de (0.9 g, yield: 45%) as a brown solid.

MS m/z (ESI): 312.1 $[M+H]^+$.

Step 5: Preparation of N2-(2-(dimethylamino)ethyl)-6-methoxy-N2-methyl-3-nitropyridine-2,5-diamine

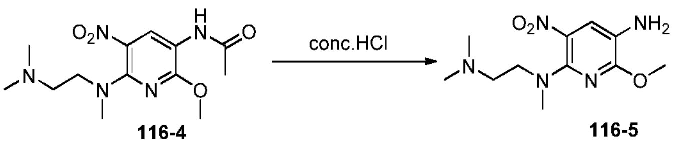


116-4 116-5

N-(6-((2-(dimethylamino)ethyl)(methyl)amino)-2-methoxy-5-nitropyridin-3-yl)ace tamide (0.9 g, 2.9 mmol) was dissolved in methanol (30 mL) and concentrated hydrochloric acid (5 mL). The reaction solution was stirred at 60°C for 3 hours. The solvent was removed by rotary evaporation, and dichloromethane (50 mL) and saturated sodium bicarbonate solution (50 mL) were added to the resulting residues and stirred until no bubbles generation. The organic layer was separated, dried over anhydrous sodium sulfate, filtered and concentrated to dryness by rotary evaporation. The resulting crude product was purified by column chromatography (dichloromethane/methanol: 100/1 ~ 10/1) to obtain N2-(2-(dimethylamino)ethyl)-6-methoxy-N2-methyl-3-nitropyridine-2,5-diamine (0.15 g, yield: 19%) as a brown solid.
MS m/z (ESI): 270.1 $[M+H]^+$.

Step 6: Preparation of 4-(1-cyclopropyl-1H-indol-3-yl)-2-((6-((2-(dimethylamino)ethyl)(methyl)amino)-2-met hoxy-5-nitropyridin-3-yl)amino)pyrimidine-5-carbonitrile

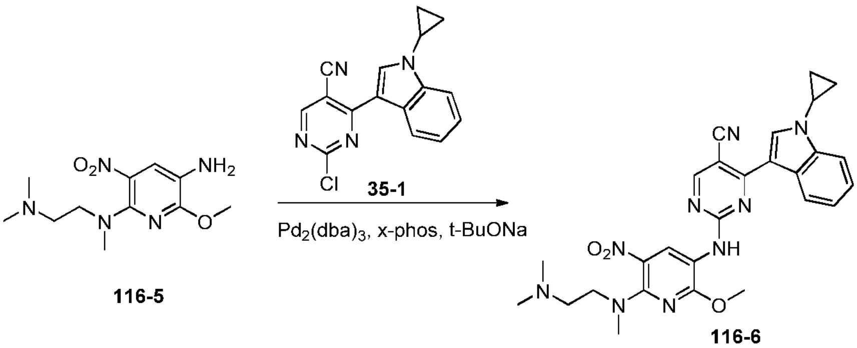

116-5
35-1
116-6

N2-(2-(Dimethylamino)ethyl)-6-methoxy-N2-methyl-3-nitropyridine-2,5-diamine (0.11 g, 0.41 mmol), 2-chloro-4-(1-cyclopropyl-1H-indol-3-yl)pyrimidine-5-carbonitrile (0.12 g, 0.41 mmol), tris(dibenzylideneacetone)dipalladium (0.18 g, 0.2 mmol), x-phos (0.2 g, 0.41 mmol) and sodium tert-butoxide (0.12 g, 1.2 mmol) were dissolved in dioxane (5 mL). The reaction solution was purged with nitrogen, and stirred under microwave at 140°C for 1 hour. The reaction solution was cooled to room temperature and filtered. The filtrate was concentrated to dryness by rotary evaporation, and the resulting crude product was purified by prep-TLC (dichloromethane/methanol: 20/1) to obtain 4-(1-cyclopropyl-1H-indol-3-yl)-2-((4-((2-(dimethylamino)ethyl)(methyl)amino)-2-met hoxy-5-nitrophenyl)amino)pyrimidine-5-carbonitrile (0.1 g, yield: 47%) as a yellow solid.
MS m/z (ESI): 528.1 $[M+H]^+$.

Step 7: Preparation of 2-((5-amino-6-((2-(dimethylamino)ethyl)(methyl)amino)-2-methoxypyridin-3-yl)amino )-4-(1-cyclopropyl-1H-indol-3-yl)pyrimidine-5-carbonitrile

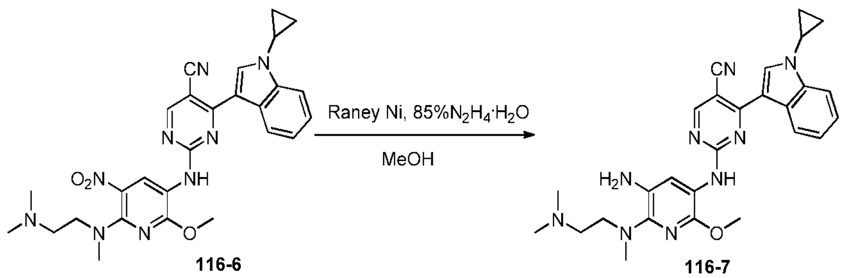

116-6
116-7

4-(1-Cyclopropyl-1H-indol-3-yl)-2-((4-((2-(dimethylamino)ethyl)(methyl)amino)-2-methoxy-5-nitrophenyl)amino)pyrimidine-5-carbonitrile (80 mg, 0.15 mmol) was dissolved in methanol (10 mL), followed by the addition of Raney nickel (80 mg) and 85% hydrazine hydrate (90 mg, 1.5 mmol) at 0°C. The reaction solution was stirred at room temperature for 2 hours. The reaction solution was filtered, and the filtrate was concentrated to dryness by rotary evaporation. Water (15 mL) was added to the resulting residues, and the solution was extracted with dichloromethane (15 mL × 2). The organic phase was dried over anhydrous sodium sulfate, filtered and concentrated to dryness by rotary evaporation to

obtain 2-((5-amino-6-((2-(dimethylamino)ethyl)(methyl)amino)-2-methoxypyridin-3-yl)amino )-4-(1-cyclopropyl-1H-indol-3-yl)pyrimidine-5-carbonitrile (80 mg, yield: 100% crude) as a brown solid.

MS m/z (ESI): 498.1 $[M+H]^+$.

Step 8: Preparation of N-(5-((5-cyano-4-(1-cyclopropyl-1H-indol-3-yl)pyrimidin-2-yl)amino)-2-((2-(dimethyl amino)ethyl)(methyl)amino)-6-methoxypyridin-3-yl)acrylamide

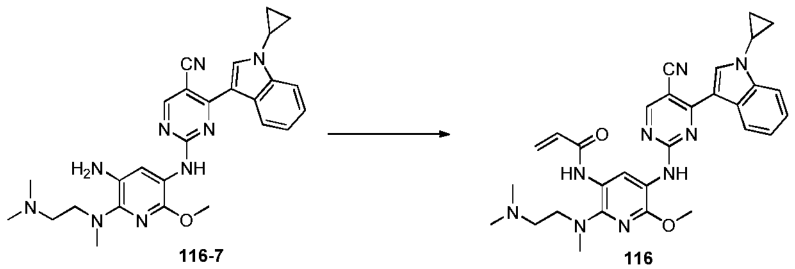

2-((5-Amino-6-((2-(dimethylamino)ethyl)(methyl)amino)-2-methoxypyridin-3-yl)a mino)-4-(1-cyclopropyl-1H-indol-3-yl)pyrimidine-5-carbonitrile (80 mg, 0.16 mmol) was dissolved in dichloromethane (10 mL), and the solution was cooled to 0°C. Triethylamine (24 mg, 0.24 mmol) and 3-chloropropionyl chloride (25 mg, 0.19 mmol) were added successively. The reaction solution was stirred at 0°C for 1 hour. Water (10 mL) was added, and the solution was extracted with dichloromethane (10 mL × 2). The organic phase was dried over anhydrous sodium sulfate, filtered and concentrated to dryness by rotary evaporation. Tetrahydrofuran (5 mL) and a solution of sodium hydroxide (64 mg, 1.6 mmol) in water (0.5 mL) were added, and the reaction solution was stirred at 40°C overnight. Water (10 mL) was added, and the solution was extracted with ethyl acetate (10 mL × 2). The organic phase was dried over anhydrous sodium sulfate, filtered and concentrated to dryness by rotary evaporation. The resulting crude product was purified by prep-HPLC to obtain N-(5-((5-cyano-4-(1-cyclopropyl-1H-indol-3-yl)pyrimidin-2-yl)amino)-2-((2-(dimethyl amino)ethyl)(methyl)amino)-6-methoxypyridin-3-yl)acrylamide (0.9 mg, yield: 0.7%) as a yellow solid. MS m/z (ESI): 552.1 $[M+H]^+$.

**Example 117**

**N-(5-((5-Cyano-4-(1-cyclopropyl-4-methoxy-1H-indol-3-yl)pyrimidin-2-yl)amino)-2 -((2-(dimethylamino)ethyl)(methyl)amino)-4-methoxyphenyl)acrylamide**

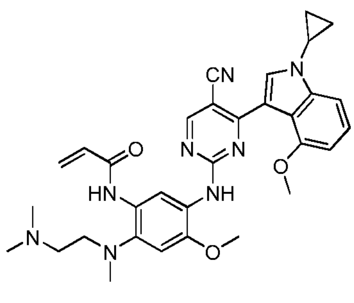

**117**

The compound of Example 117 was prepared by referring to the method of Example 2.

MS m/z (ESI): 581.1 $[M+H]^+$.

$^1H$ NMR (400 MHz, DMSO-$d_6$) δ 10.14 (s, 1H), 9.10-8.91 (*br,* 2H), 8.72 (s, 1H), 7.83 (s, 1H), 7.29-7.21 (m, 2H), 7.02 (s, 1H), 6.72 (d, *J* = 7.6 Hz, 1H), 6.44-6.37 (m, 1H), 6.32-6.27 (m, 1H), 5.81-5.77 (m, 1H), 3.83 (s, 3H), 3.80 (s, 3H), 3.59-3.50 (m, 1H), 2.85-2.75 (m, 2H), 2.70 (s, 3H), 2.34-2.25 (m, 2H), 2.22 (s, 6H), 1.12-1.06 (m, 4H).

**Example 118**

**N-(5-((5-Cyano-4-(1-cyclopropyl-5-methoxy-1H-indol-3-yl)pyrimidin-2-yl)amino)-2 -((2-(dimethylamino)ethyl)(methyl)amino)-4-methoxyphenyl)acrylamide**

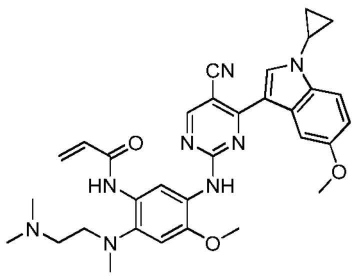

**118**

The compound of Example 118 was prepared by referring to the method of Example 2.

MS m/z (ESI): 581.4[M+H]+.

$^1$H NMR (400 MHz, $CDCl_3$) δ 10.08 - 9.55 (m, 1H), 9.42 (s, 1H), 8.68 (s, 1H), 8.44 (s, 1H), 8.03 (s, 1H), 7.76 (s, 1H), 7.51 (d, *J* = 8.6 Hz, 1H), 6.97 (d, *J* = 8.6 Hz, 1H), 6.77 (s, 1H), 6.39 (d, *J* = 15.7 Hz, 1H), 5.69 (d, *J* = 10.5 Hz, 1H), 3.89 (s, 6H), 3.51 - 3.38 (m, 1H), 3.03 (s, 2H), 2.73 (s, 3H), 2.65 - 2.18 (m, 6H), 1.89 - 1.54 (m, 3H), 1.23 - 1.04 (m, 4H).

**Example 119**

**N-(5-((5-Cyano-4-(1-cyclopropyl-7-methoxy-1H-indol-3-yl)pyrimidin-2-yl)amino)-2 -((2-(dimethylamino)ethyl)(methyl)amino)-4-methoxyphenyl)acrylamide**

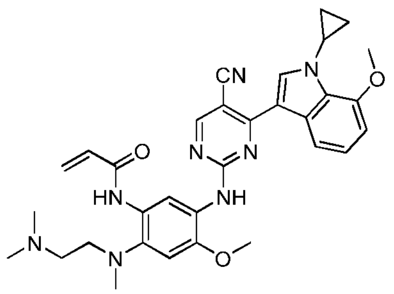

**119**

The compound of Example 119 was prepared by referring to the method of Example 2.

MS m/z (ESI): 581.3 [M+H]+.

$^1$H NMR (400 MHz, Chloroform-*d*) δ 10.32 - 9.79 (m, 1H), 9.56 - 9.18 (m, 1H), 8.80 - 8.55 (m, 1H), 8.47 - 8.27 (m, 1H), 8.24 - 7.97 (m, 1H), 7.88 - 7.61 (m, 1H), 7.22 - 7.04 (m, 1H), 6.89 - 6.68 (m, 2H), 6.56 - 6.21 (m, 2H), 5.83 - 5.55 (m, 1H), 4.20 - 3.77 (m, 7H), 3.16 - 2.81 (m, 2H), 2.73 (s, 3H), 2.59 - 2.15 (m, 8H), 1.19 - 1.02 (m, 4H).

**Example 120**

**N-(5-((5-Cyano-4-(1-cyclopropyl-4-fluoro-1H-indol-3-yl)pyrimidin-2-yl)amino)-2-(( 2-(dimethylamino)ethyl)(methyl)amino)-4-methoxyphenyl)acrylamide**

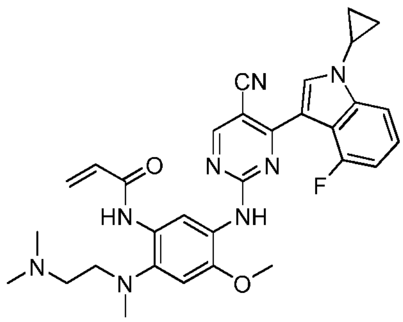

**120**

The compound of Example 120 was prepared by referring to the method of Example 2.

MS m/z (ESI): 569.2 $[M+H]^+$.

$^1$H NMR (400 MHz, DMSO-$d_6$) $\delta$ 10.22 (s, 1H), 8.90 (s, 1H), 8.75 (s, 1H), 8.69(s, 1H), 8.27(s, 1H), 7.48 (d, *J* = 7.6 Hz, 1H), 7.28-7.23 (m, 1H), 7.09 (s, 1H),6.92-6.87(m, 1H), 6.46-6.39 (m, 1H), 6.30-6.26 (m, 1H), 5.78 (d, *J* = 9.6 Hz, 1H), 3.84 (s, 3H),3.61-3.52 (m, 1H), 2.96-2.92(m, 2H), 2.75 (s, 3H), 2.37-2.29 (m, 2H), 2.22(s, 6H), 1.16-1.04 (m, 4H).

**Example 121**

**N-(5-((5-Cyano-4-(1-cyclopropyl-5-fluoro-1H-indol-3-yl)pyrimidin-2-yl)amino)-2-(( 2-(dimethylamino)ethyl)(methyl)amino)-4-methoxyphenyl)acrylamide**

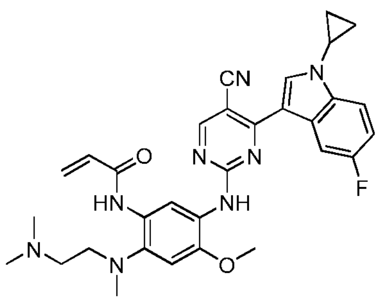

**121**

The compound of Example 121 was prepared by referring to the method of Example 2.

MS m/z (ESI): 569.1 $[M+H]^+$.

**Example 122**

**N-(5-((5-Cyano-4-(1-cyclopropyl-7-fluoro-1H-indol-3-yl)pyrimidin-2-yl)amino)-2-(( 2-(dimethylamino)ethyl)(methyl)amino)-4-methoxyphenyl)acrylamide**

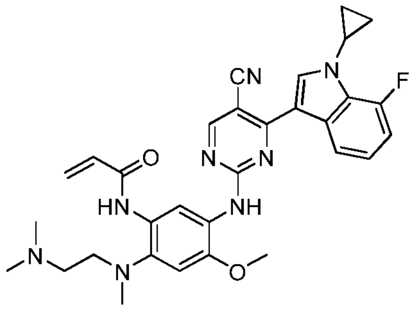

**122**

The compound of Example 122 was prepared by referring to the method of Example 2.
MS m/z (ESI): 569.4$[M+H]^+$.

**Example 123**

**N-(5-((5-Cyano-4-(4-cyano-1-cyclopropyl-1H-indol-3-yl)pyrimidin-2-yl)amino)-2-(( 2-(dimethylamino)ethyl)(methyl)amino)-4-methoxyphenyl)acrylamide**

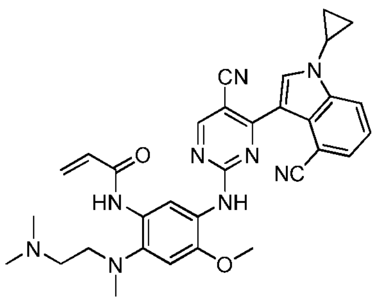

**123**

The compound of Example 123 was prepared by referring to the method of Example 2.
MS m/z (ESI): 576.3$[M+H]^+$.

**Example 124**

**N-(5-((5-Cyano-4-(5-cyano-1-cyclopropyl-1H-indol-3-yl)pyrimidin-2-yl)amino)-2-(( 2-(dimethylamino)ethyl)(methyl)amino)-4-methoxyphenyl)acrylamide**

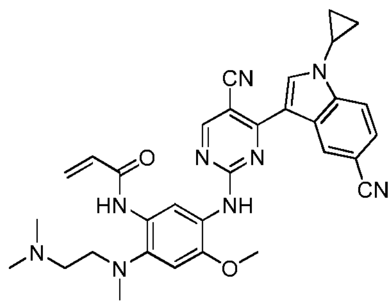

**124**

The compound of Example 124 was prepared by referring to the method of Example 2.

MS m/z (ESI): 576.2[M+H]+.

**Example 125**

**N-(5-((5-Cyano-4-(7-cyano-1-cyclopropyl-1H-indol-3-yl)pyrimidin-2-yl)amino)-2-(( 2-(dimethylamino)ethyl)(methyl)amino)-4-methoxyphenyl)acrylamide**

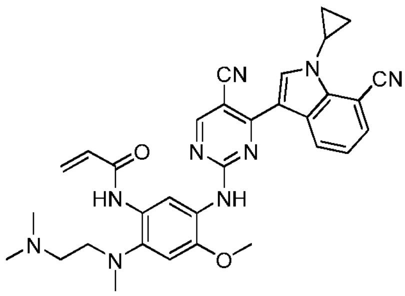

**125**

The compound of Example 125 was prepared by referring to the method of Example 2.
MS m/z (ESI): 576.2[M+H]+.

**Example 126**

**N-(5-((5-Cyano-4-(1-cyclopropyl-5,6-dimethoxy-1H-indol-3-yl)pyrimidin-2-yl)amin o)-2-((2-(dimethylamino)ethyl)(methyl)amino)-4-methoxyphenyl)acrylamide**

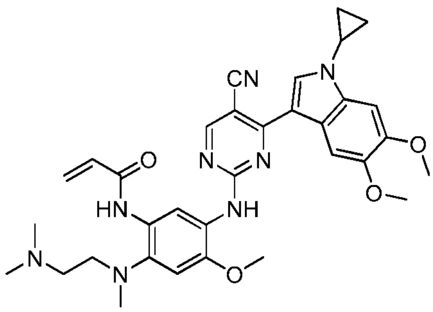

**126**

The compound of Example 126 was prepared by referring to the method of Example 2.
MS m/z (ESI): 611.1[M+H]+.

**Example 127**

**N-(5-((5-Cyano-4-(1-cyclopropyl-5-fluoro-6-methoxy-1H-indol-3-yl)pyrimidin-2-yl) amino)-2-((2-(dimethylamino)ethyl)(methyl)amino)-4-methoxyphenyl)acrylamide**

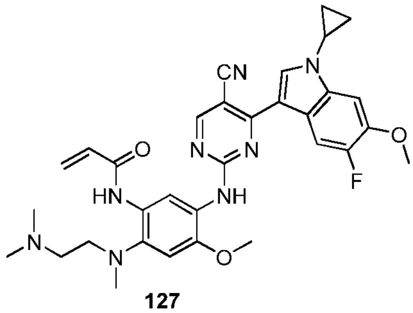

**127**

The compound of Example 127 was prepared by referring to the method of Example 2.

MS m/z (ESI): 599.2[M+H]$^+$.

$^1$H NMR (400 MHz, DMSO-$d_6$) δ 10.06 (s, 1H), 9.54 (s, 1H), 8.66 (s, 1H), 8.38 (s, 1H), 8.32 (s, 1H), 7.75 - 7.55 (m, 1H), 7.27 - 7.25 (m, 1H), 7.05 (s, 1H), 6.41 - 6.34 (m, 1H), 6.17 (d, *J* = 16.9 Hz, 1H), 5.72 (d, *J* = 10.3 Hz, 1H), 3.91 (s, 3H), 3.72 (s, 3H), 3.63 - 3.54 (m, 1H), 2.90 - 2.88 (m, 2H), 2.75 (s, 3H), 2.63 - 2.56 (m, 1H), 2.37 - 2.35 (m, 2H), 2.21 (s, 5H), 1.18 - 1.16 (m, 2H), 1.04 - 0.93 (m, 2H).

**Example 128**

**N-(5-((5-Cyano-4-(6-cyclopropyl-2,3-dihydro-6H-[1,4]dioxino[2,3-f]indol-8-yl)pyri midin-2-yl)amino)-2-((2-(dimethylamino)ethyl)(methyl)amino)-4-methoxyphenyl)a crylamide**

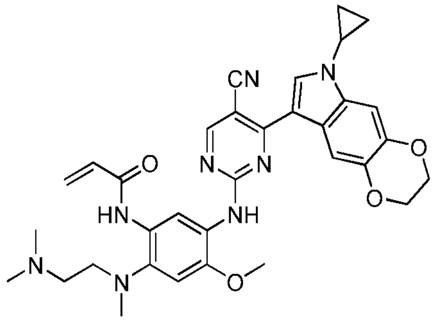

**128**

The compound of Example 128 was prepared by referring to the method of Example 2.

MS m/z (ESI): 609.3[M+H]$^+$.

**Example 129**

**N-(5-((5-Cyano-4-(5-cyclopropyl-5H-[1,3]dioxolo[4,5-f]indol-7-yl)pyrimidin-2-yl)a mino)-2-((2-(dimethylamino)ethyl)(methyl)amino)-4-methoxyphenyl)acrylamide**

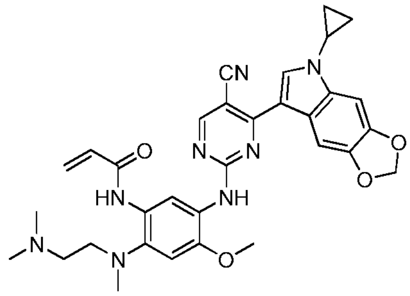

**129**

The compound of Example 129 was prepared by referring to the method of Example 2.
MS m/z (ESI): 595.1 $[M+H]^+$.

**Example 130**

**N-(2-((2-(Dimethylamino)ethyl)(methyl)amino)-4-methoxy-5-((5-(3-methyl-1,2,4-ox adiazol-5-yl)-4-(1-methyl-1H-indol-3-yl)pyrimidin-2-yl)amino)phenyl)acrylamide**

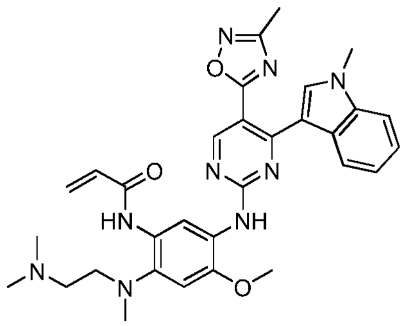

**130**

The compound of Example 130 was prepared by referring to the method of Example 2.
MS m/z (ESI): 582.4$[M+H]^+$.

**Example 131**

**N-(2-((2-(Dimethylamino)ethyl)(methyl)amino)-4-methoxy-5-((5-(5-methyl-1,3,4-ox adiazol-2-yl)-4-(1-methyl-1H-indol-3-yl)pyrimidin-2-yl)amino)phenyl)acrylamide**

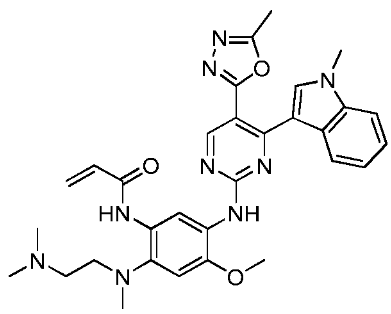

**131**

The compound of Example 131 was prepared by referring to the method of Example 2.

MS m/z (ESI): 582.2[M+H]+.

**Example 132**

**N-(5-((5-Cyano-4-(1-cyclopropyl-1H-indazol-3-yl)pyrimidin-2-yl)amino)-2-((2-(dim ethylamino)ethyl)(methyl)amino)-4-methoxyphenyl)acrylamide**

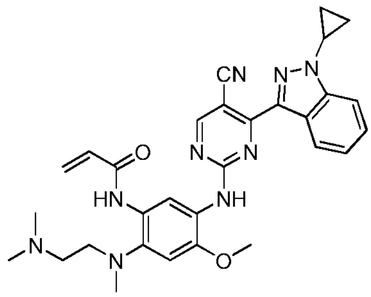

**132**

The compound of Example 132 was prepared by referring to the method of Example 2.

MS m/z (ESI): 552.1 [M+H]+.

**Example 133**

**N-(5-((5-Cyano-4-(1-cyclopropyl-1H-indol-3-yl)pyrimidin-2-yl)amino)-2-((2-(dimet hylamino)ethyl)(methyl)amino)-6-(2,2,2-trifluoroethoxy)pyridin-3-yl)acrylamide**

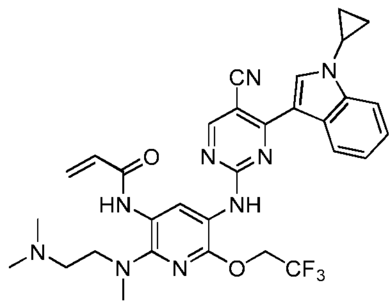

**133**

The compound of Example 133 was prepared by referring to the method of Example 116.

MS m/z (ESI): 620.3[M+H]+.

**Example 134**

**N-(5-((5-Cyano-4-(1-methyl-1H-indol-3-yl)pyrimidin-2-yl)amino)-2-((2-(dimethyla mino)ethyl)(methyl)amino)-6-(2,2,2-trifluoroethoxy)pyridin-3-yl)acrylamide**

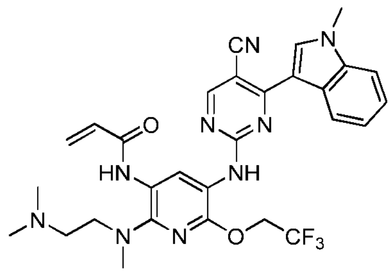

134

The compound of Example 134 was prepared by referring to the method of Example 116.
MS m/z (ESI): 594.0[M+H]+.

**Example 135**

**N-(5-((5-Cyano-4-(1-methyl-1H-indol-3-yl)pyrimidin-2-yl)amino)-2-((2-(dimethyla mino)ethyl)(methyl)amino)-6-methoxypyridin-3-yl)acrylamide**

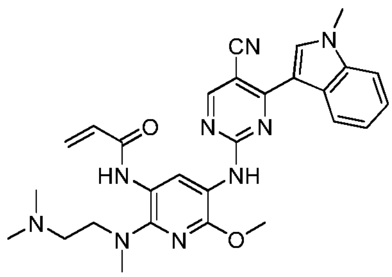

135

The compound of Example 135 was prepared by referring to the method of Example 116.
MS m/z (ESI): 526.3[M+H]+.

**Example 136**

**N-(5-((5-((Dimethyl(oxo)-λ$^6$-sulfaneylidene)amino)-4-(1-methyl-1H-indol-3-yl)pyri midin-2-yl)amino)-2-((2-(dimethylamino)ethyl)(methyl)amino)-4-methoxyphenyl)a crylamide**

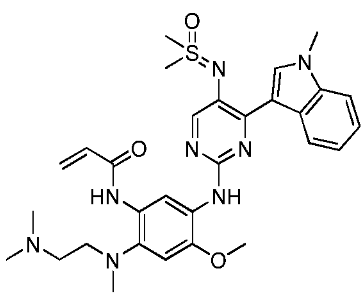

136

The compound of Example 136 was prepared by referring to the method of Example 36.
MS m/z (ESI): 591.2 [M+H]+.

**Example 137**

**N-(5-((5-Cyano-4-(6-methoxy-1-(oxetan-3-yl)-1H-indol-3-yl)pyrimidin-2-yl)amino)-2-((2-(dimethylamino)ethyl)(methyl)amino)-4-methoxyphenyl)acrylamide**

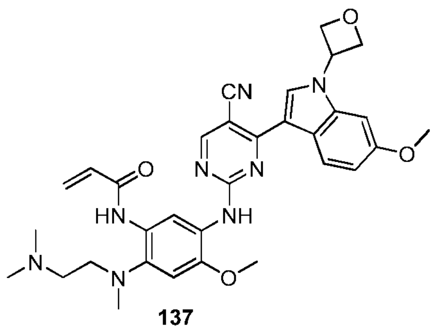

**137**

The compound of Example 137 was prepared by referring to the method of Example 111.
MS m/z (ESI): 597.2 $[M+H]^+$.
$^1$H NMR (400 MHz, DMSO-$d_6$) δ 10.29-10.22 (*br,* 1H), 9.42 (s, 1H), 8.67 (s, 1H),8.59 (m, 1H), 8.34 (s, 1H), 7.77 (s, 1H), 7.08 (s, 1H), 6.97-6.88 *(br,* 1H), 6.79 (s, 1H), 6.39 (d, *J* = 14.4 Hz, 1H), 5.72-5.69 (m, 1H), 5.61-5.58 (m, 1H), 5.20 (d, *J* = 6.4 Hz, 4H),3.90 (s, 6H), 3.06-2.84 *(br,* 3H), 2.74 (s, 3H), 2.50-2.16 *(br,* 6H), 1.69-1.50 *(br,* 2H).

**Example 138**

**N-(5-((5-Cyano-4-(3-methyl-1H-indazol-1-yl)pyrimidin-2-yl)amino)-2-((2-(dimethy lamino)ethyl)(methyl)amino)-4-methoxyphenyl)acrylamide**

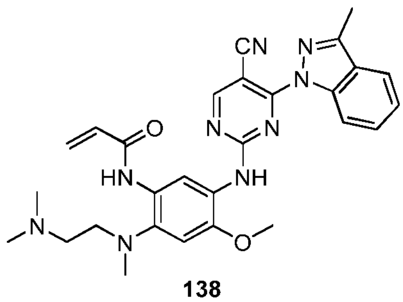

**138**

The compound of Example 138 was prepared by referring to the following synthetic route.

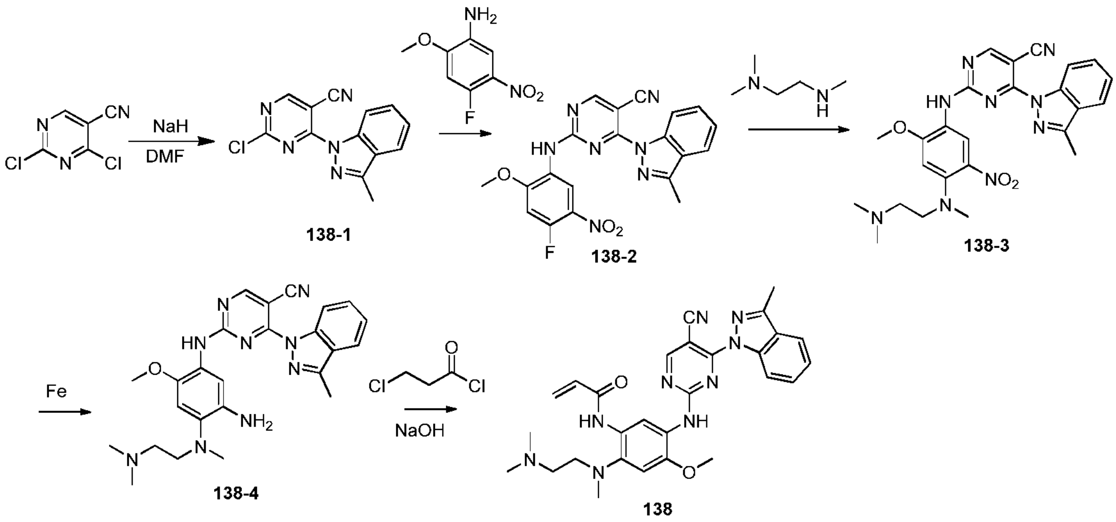


MS m/z (ESI): 526.3[M+H]+.

$^1$H NMR (400 MHz, $CDCl_3$) δ 10.44 - 9.56 (m, 1H), 9.13 (s, 1H), 8.67 (s, 1H), 8.57 - 8.27 (m, 1H), 7.80 - 7.57 (m, 1H), 7.57 - 7.43 (m, 1H), 7.44 - 7.24 (m, 3H), 6.96 - 6.67 (m, 1H), 6.57 - 6.16 (m, 1H), 5.81 - 5.50 (m, 1H), 3.86 (s, 3H), 3.45 - 2.08 (m, 16H).

**Example 139**

**N-(5-((5-Cyano-4-(1-cyclopropyl-1H-pyrrolo[2,3-b]pyridin-3-yl)pyrimidin-2-yl)ami no)-2-((2-(dimethylamino)ethyl)(methyl)amino)-4-methoxyphenyl)acrylamide**

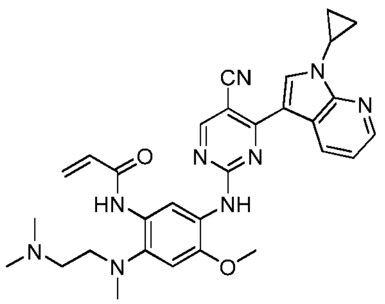

139

Step 1: 1-Cyclopropyl-1H-pyrrolo[2,3-b]pyridine

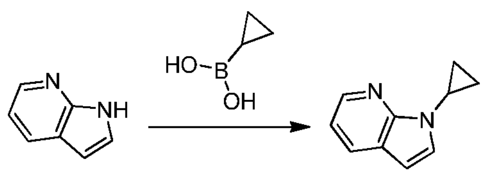

139-1

Copper acetate (6.2 g, 34 mmol) was added to a mixture of 7-azaindole (4 g, 34 mmol), cyclopropylboronic acid (5.8 g, 68 mmol), 2,2'-bipyridine (5.3 g, 34 mmol) and sodium carbonate (7.2 g, 34 mmol) in 1,2-dichloroethane (100 ml). The reaction solution was heated to 85°C under a nitrogen atmosphere, and reacted for 12 hours. The reaction solution was cooled, followed by the addition of water (150 ml) and filtration. The solid phase was rinsed with dichloromethane (60 ml), and the liquid phase was separated. The aqueous phase was extracted with dichloromethane (30 ml*2). The organic phases were combined, dried over anhydrous sodium sulfate, and concentrated to dryness to obtain the crude product. The crude product was purified by column chromatography (petroleum ether: dichloromethane = 10:1) to obtain 1-cyclopropyl-1H-pyrrolo[2,3-b]pyridine (2.7 g, yield: 50.4%).

MS m/z (ESI): 158.0[M+H]$^+$.

Step 2: 2-Chloro-4-(1-cyclopropyl-1H-pyrrolo[2,3-b]pyridin-3-yl)pyrimidine-5-carbonitrile

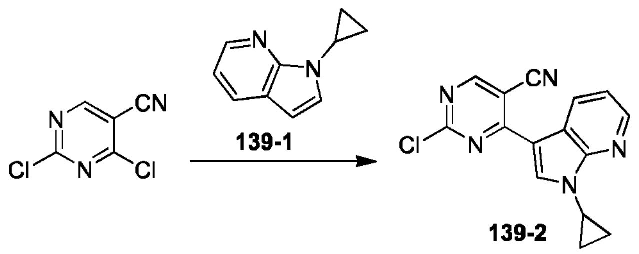


Aluminum trichloride (1.07 g, 8.1 mmol) was added to a mixture of 2,4-dichloropyrimidine-5-carbonitrile (700 mg, 4.0 mmol) and 1-cyclopropyl-1H-pyrrolo[2,3-b]pyridine (637 mg, 4.0 mmol) in batches at 0°C under a nitrogen atmosphere. The reaction was directly heated to 100°C and stirred for 0.5 hour. The reaction solution was cooled to 0°C, to which methanol (10 ml) and water (30 ml) were added slowly. The solution was stirred at room temperature for 30 minutes, and extracted with dichloromethane (30 ml*2). The organic phases were combined, dried over anhydrous sodium sulfate, and concentrated to dryness to obtain the crude product. The crude product was purified by column chromatography (petroleum ether: dichloromethane = 1:1) to obtain 2-chloro-4-(1-cyclopropyl-1H-pyrrolo[2,3-b]pyridin-3-yl)pyrimidine-5-carbonitrile (735 mg, yield: 62%).

MS m/z (ESI): 296.0[M+H]$^+$.

Step 3: 4-(1-Cyclopropyl-1H-pyrrolo[2,3-b]pyridin-3-yl)-2-((4-fluoro-2-methoxy-5-nitrophenyl )amino)pyrimidine-5-carbonitrile

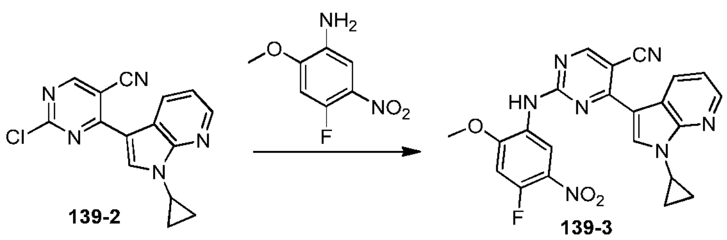


P-toluenesulfonic acid monohydrate (643 mg, 3.38 mmol) was added to a solution of 4-fluoro-2-methoxy-5-nitroaniline (189 mg, 1.01 mmol) and 2-chloro-4-(1-cyclopropyl-1H-pyrrolo[2,3-b]pyridin-3-yl)pyrimidine-5-carbonitrile (250 mg, 0.85 mmol) in 2-butanol (15 ml). The reaction solution was heated to 90°C for 12 hours. The reaction solution was cooled, and concentrated to dryness. Saturated aqueous sodium bicarbonate solution was added to adjust the pH to 9, and the solution was extracted with dichloromethane (35 ml*2). The organic phase was dried over anhydrous sodium sulfate, and concentrated to dryness to obtain the crude product. The crude product was purified by column chromatography (dichloromethane: ethyl acetate = 5:1) to obtain the product 4-(1-cyclopropyl-1H-pyrrolo[2,3-b]pyridin-3-yl)-2-((4-fluoro-2-methoxy-5-nitrophenyl) amino)pyrimidine-5-carbonitrile (230 mg, yield: 61%).

MS m/z (ESI): 446.1 [M+H]$^+$.

Step 4: 4-(1-Cyclopropyl-1H-pyrrolo[2,3-b]pyridin-3-yl)-2-((4-((2-(dimethylamino)ethyl)(meth yl)amino)-2-methoxy-5-nitrophenyl)amino)pyrimidine-5-carbonitrile

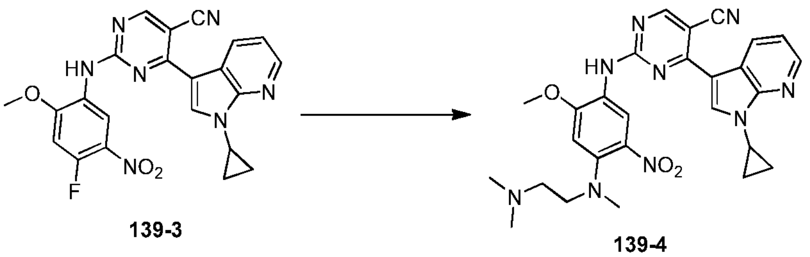

139-3

139-4

Potassium carbonate (214 mg, 1.55 mmol) was added to a solution of N,N,N'-trimethylethylenediamine (58 mg, 0.57 mmol) and 4-(1-cyclopropyl-1H-pyrrolo[2,3-b]pyridin-3-yl)-2-((4-fluoro-2-methoxy-5-nitrophenyl) amino)pyrimidine-5-carbonitrile (230 mg, 0.52 mmol) in acetonitrile (10 mL). The reaction solution was heated to 80°C and reacted for 2 hours. The reaction solution was cooled, and concentrated to dryness. The resulting residues were dissolved in dichloromethane (30 ml), and washed with brine (20 ml*3). The organic phase was dried over anhydrous sodium sulfate, and concentrated to dryness to obtain the crude product 4-(1-cyclopropyl-1H-pyrrolo[2,3-b]pyridin-3-yl)-2-((4-((2-(dimethylamino)ethyl)(meth yl)amino)-2-methoxy-5-nitrophenyl)amino)pyrimidine-5-carbonitrile (270 mg). The crude product was used directly in the next step.

MS m/z (ESI): 528.2[M+H]$^+$.

Step 5: 2-((5-Amino-4-((2-(dimethylamino)ethyl)(methyl)amino)-2-methoxyphenyl)amino)-4-( 1-cyclopropyl-1H-pyrrolo[2,3-b]pyridin-3-yl)pyrimidine-5-carbonitrile

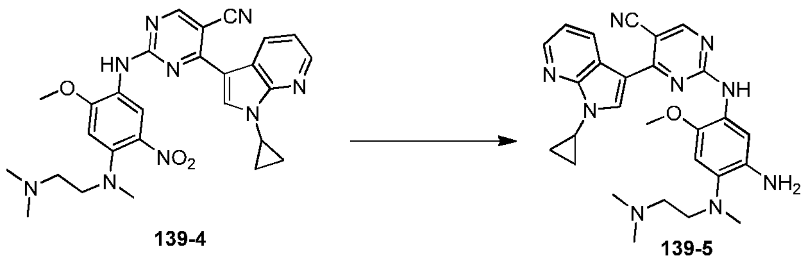

139-4

139-5

Iron powder (288 mg, 5.2 mmol) was added to a mixture of 4-(1-cyclopropyl-1H-pyrrolo[2,3-b]pyridin-3-yl)-2-((4-((2-(dimethylamino)ethyl)(meth yl)amino)-2-methoxy-5-nitrophenyl)amino)pyrimidine-5-carbonitrile (270 mg, 0.51 mol), ethanol (10 ml) and saturated aqueous ammonium chloride solution (4 ml). The reaction solution was heated to 80°C and reacted for 2 hours. The reaction solution was filtered when it was still hot, the solid was rinsed with dichloromethane, and the liquid phase was concentrated to dryness to obtain the crude product. Dichloromethane (30 ml) was added to the crude product, and the solution was washed with water (15 ml*2). The organic phase was dried over anhydrous sodium sulfate, and concentrated to dryness to obtain the crude product 2-((5-amino-4-((2-(dimethylamino)ethyl)(methyl)amino)-2-methoxyphenyl)amino)-4-(1 -cyclopropyl-1H-pyrrolo[2,3-b]pyridin-3-yl)pyrimidine-5-carbonitrile (250 mg), which was used directly in the next step.

MS m/z (ESI): 498.3[M+H]$^+$.

Step 6: 3-Chloro-N-(5-((5-cyano-4-(1-cyclopropyl-1H-pyrrolo[2,3-b]pyridin-3-yl)pyrimidin-2-yl)amino)-2-((2-(dimethylamino)ethyl)(methyl)amino)-4-methoxyphenyl)propanamide

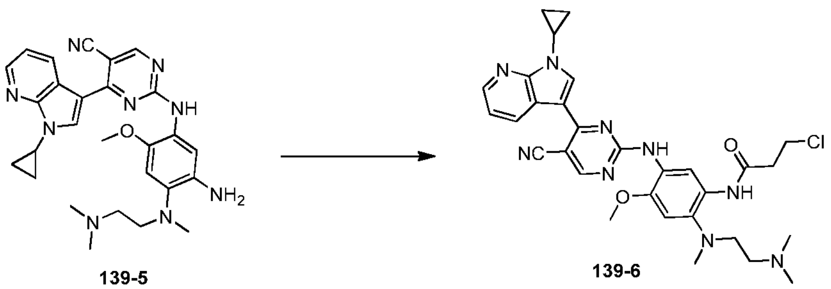

139-5 139-6

3-Chloropropionyl chloride (77 mg, 0.60 mmol) was added to a solution of 2-((5-amino-4-((2-(dimethylamino)ethyl)(methyl)amino)-2-methoxyphenyl)amino)-4-(1 -cyclopropyl-1H-pyrrolo[2,3-b]pyridin-3-yl)pyrimidine-5-carbonitrile (250 mg, 0.50 mmol) and triethylamine (0.21 ml, 1.51 mmol) in dichloromethane (10 ml) at 0°C. The reaction solution was reacted at 0°C for 30 minutes. The reaction solution was concentrated to dryness to obtain the crude product 3-chloro-N-(5-((5-cyano-4-(1-cyclopropyl-1H-pyrrolo[2,3-b]pyridin-3-yl)pyrimidin-2-y l)amino)-2-((2-(dimethylamino)ethyl)(methyl)amino)-4-methoxyphenyl)propanamide (250 mg), which was used directly in the next step.

MS m/z (ESI): 588.3[M+H]+.

Step 7: N-(5-((5-Cyano-4-(1-cyclopropyl-1H-pyrrolo[2,3-b]pyridin-3-yl)pyrimidin-2-yl)amino) -2-((2-(dimethylamino)ethyl)(methyl)amino)-4-methoxyphenyl)acrylamide

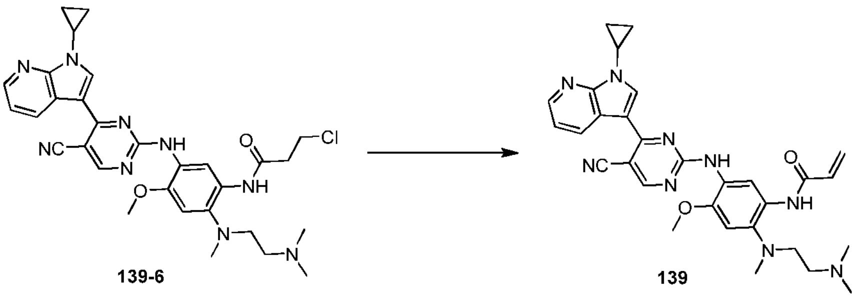

139-6 139

Aqueous sodium hydroxide solution (containing sodium hydroxide (200 mg, 5 mmol) and water (1 ml)) was added to a solution of the crude 3-chloro-N-(5-((5-cyano-4-(1-cyclopropyl-1H-pyrrolo[2,3-b]pyridin-3-yl)pyrimidin-2-y l)amino)-2-((2-(dimethylamino)ethyl)(methyl)amino)-4-methoxyphenyl)propanamide (290 mg, 0.50 mmol) in acetonitrile (10 ml). The reaction solution was stirred at room temperature for 16 hours. The reaction solution was concentrated to dryness to obtain the crude product. The crude product was dissolved in N,N-dimethylformamide (2 ml), and purified by preparative HPLC to obtain the product N-(5-((5-cyano-4-(1-cyclopropyl-1H-pyrrolo[2,3-b]pyridin-3-yl)pyrimidin-2-yl)amino)-2-((2-(dimethylamino)ethyl)(methyl)amino)-4-methoxyphenyl)acrylamide (55 mg, yield: 20%).

MS m/z (ESI): 552.3[M+H]+.

$^1$H NMR (400 MHz, $CDCl_3$) δ 10.32 - 9.88 (m, 1H), 9.59 - 9.18 (m, 1H), 8.92 - 8.52 (m, 3H), 8.52 - 8.26 (m, 1H), 7.96 - 7.61 (m, 1H), 7.24 - 6.99 (m, 1H), 6.82 (s, 1H), 6.55 - 6.15 (m, 2H), 5.85 - 5.52 (m, 1H), 3.89 (s, 3H), 3.72 - 3.58 (m, 1H), 3.01 - 2.83 (m, 2H), 2.74 (s, 3H), 2.51 - 2.19 (m, 8H), 1.30 - 1.04 (m, 4H).

**Example 140**

**N-(5-((5-Cyano-4-(1-cyclopropyl-1H -indol-3-yl)pyrimidin-2-yl)amino)-2-(4-(3-etho xyazetidin-1-yl)piperidin-1-yl)-4-methoxyphenyl)acrylamide**

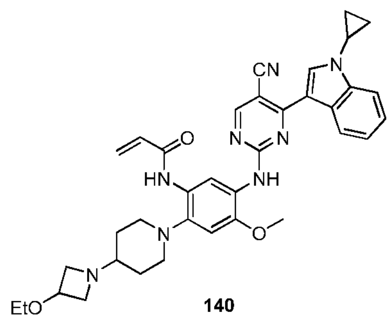

**140**

The compound of Example 140 was prepared by referring to the method of Example 67.

MS m/z (ESI): 634.4[M+H]+.

$^1$H NMR (400 MHz, $CDCl_3$) δ 12.90 (s, 1H), 9.35 (s, 1H), 8.67 (s, 1H), 8.48 (s, 1H), 8.38 - 8.27 (m, 1H), 7.79 - 7.71 (m, 1H), 7.62 (d, J = 8.1 Hz, 1H), 7.36 - 7.28 (m, 2H), 6.88 - 6.79 (m, 1H), 6.41 - 6.27 (m, 2H), 5.75 (d, J = 9.6 Hz, 1H), 4.63 - 4.47 (m, 2H), 3.91 (s, 3H), 3.78 - 3.62 (m, 2H), 3.55 - 3.43 (m, 3H), 3.30 - 3.03 (m, 4H), 2.77 - 2.68 (m, 2H), 2.24 - 1.97 (m, 4H), 1.23 (t, J = 7.0 Hz, 3H), 1.20 - 1.06 (m, 4H).

## Example 141

**N-(5-((5-Cyano-4-(1-cyclopropyl-1H -indol-3-yl)pyrimidin-2-yl)amino)-2-((3aR,6aS )-5-cyclopropylhexahydropyrrolo[3,4-c]pyrrol-2(1H)-yl)-4-methoxyphenyl)acrylam ide**

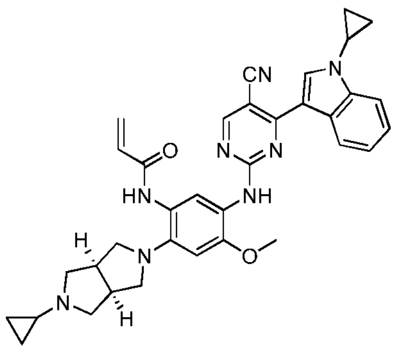

**141**

The compound of Example 141 was prepared by referring to the method of Example 67.

MS m/z (ESI): 601.2 [M+H]+.

$^1$H NMR (400 MHz, $CDCl_3$) δ 9.20 (400 MHz, CDCl9.35 (s, 1H), 8.67 (s, 1H), 8.8.41 (m, 2H), 7.84 8.27 (m, 1H), 7.79 - 7.71 (m, 1H), 7.62 (d, J = 8.1 Hz, 1H), 7.36 - 7.28 (m, 2H), 6.88 - 6.79 (m, 1H), 6.41 - 6.27 (m, 2H), 5.75 (d, J = 9.6 Hz, 1H),3.85 (m, 3H), 3.55 - 3.41 (m, 2H), 3.38 - 3.18 (m, 2H), 3.15 - 2.92 (m, 5H), 2.83 - 2.60 (m, 2H), 2.07 - 1.96 (m, 1H), 1.32 - 1.23 (m, 4H), 1.21 - 1.10 (m, 4H).

## Example 142

**2-((5-Acrylamido-4-((2-(dimethylamino)ethyl)(methyl)amino)-2-methoxyphenyl)a mino)-N-(4-fluorobenzyl)-4-(1-methyl-1H-indol-3-yl)pyrimidine-5-carboxamide**

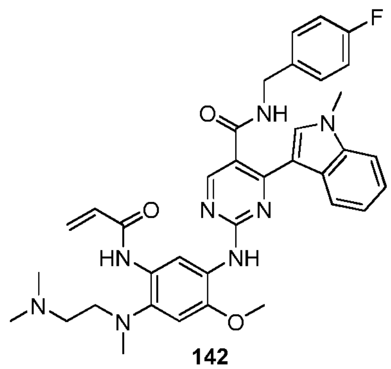

142

The compound of Example 142 was prepared by referring to the method of Example 5.
MS m/z (ESI): 651.3 $[M+H]^+$.
$^1H$ NMR (400 MHz, DMSO-$d_6$) δ 10.17 - 10.05 (m, 1H), 9.05 - 8.94 (m, 1H), 8.75 - 8.64 (m, 1H), 8.47 - 8.39 (m, 1H), 8.39 - 8.26 (m, 2H), 8.18 - 8.00 (m, 1H), 7.75 (s, 1H), 7.49 - 7.40 (m, 1H), 7.38 - 7.28 (m, 1H), 7.25 - 7.09 (m, 3H), 7.09 - 6.92 (m, 2H), 6.49 - 6.30 (m, 1H), 6.28 - 6.15 (m, 1H), 5.79 - 5.66 (m, 1H), 4.44 - 4.28 (m, 2H), 3.79 (s, 3H), 3.70 (s, 3H), 2.96 - 2.87 (m, 2H), 2.73 (s, 3H), 2.41 - 2.30 (m, 2H), 2.22 (s, 6H).

## Biological Assay and Evaluation

The present invention is further illustrated below in combination with the following test examples, which are not intended to limit the scope of the present invention.

### 1. Enzymology Experiments

### Test Example 1. Determination of the inhibitory effect of the compound of the present invention on the kinase activity of EGFR exon 20 insertion mutation

Experimental objective: The objective of this test example is to determine the inhibitory effect of the compound on the kinase activity of EGFR exon 20 insertion mutation.
Experimental instruments: Centrifuge (Eppendorf 5810R), microplate reader (BioTek Synergy HI), pipette (Eppendorf & Rainin).
Experimental process: The experiment applied the TR-FRET (time-resolved fluorescence resonance energy transfer) method to study the inhibitory effect of the compound on the kinase activity of EGFR exon 20 insertion mutation. The experiment was carried out in a 384-well plate. Experimental buffer (50mM HEPES, 1mM EGTA, 10mM $MgCl_2$, 2mM DTT, 0.01% Tween-20) was formulated. The compound was diluted in gradient to different concentrations with the experimental buffer. 2.5 μL of the solution was added to each well of the 384-well plate. 2.5 μL of diluted EGFR kinase solution (0.001-0.5 nM) was added, and the plate was incubated at room temperature for 10 minutes. 5 μL of ULight-poly GT/ATP mixed solution was added, and the plate was incubated at room temperature for 30 to 60 minutes. 5 μL of EDTA stop solution and 5 μL of Eu-labeled antibody detection solution were added, and the plate was incubated at room temperature for 1 hour. The fluorescence signal value of each well at 665 nm was measured by the microplate reader.
Experimental data processing method:
The inhibition rate (((positive control well value - sample well value)/(positive control well value - negative control well value)) *100%) was calculated using the fluorescence signal value at 665 nm. The concentration and inhibition rate were fitted to a nonlinear regression curve by Graphpad Prism software to obtain the $IC_{50}$ value. The specific data is shown in Table 1 below:

Table 1 $IC_{50}$ of the inhibitory effect of the compound on the kinase activity of EGFR exon 20 insertion mutation

| Example No. | EGFR D770_N77 1 insNPG $IC_{50}$ (nM) |
|---|---|
| AZD9291 | 2.89 |
| 24 | 0.62 |
| 26 | 0.36 |

(continued)

| Example No. | EGFR D770_N77 1 insNPG $IC_{50}$ (nM) |
|---|---|
| 35 | 0.43 |
| 37 | 0.56 |
| 38 | 0.45 |
| 40 | 0.55 |
| 43 | 0.29 |
| 45 | 1.00 |
| 52 | 0.79 |
| 53 | 0.50 |
| 57 | 0.82 |
| 63 | 0.62 |
| 64 | 0.39 |
| 65 | 0.29 |
| 67 | 0.14 |
| 68 | 0.17 |
| 74 | 0.64 |
| 77 | 0.91 |
| 78 | 0.50 |
| 81 | 0.47 |
| 85 | 0.27 |
| 109 | 0.78 |
| 110 | 0.53 |
| 111 | 0.18 |
| 112 | 0.17 |
| 113 | 0.70 |
| 114 | 0.61 |
| 118 | 0.55 |
| 119 | 0.49 |
| 127 | 0.18 |
| 137 | 0.16 |
| 139 | 0.68 |

Experimental conclusion:
The above experiment demonstrates that the compounds of the examples of the present invention have a good inhibitory effect in the EGFR exon 20 insertion mutation kinase activity inhibition experiment.

**Test Example 2. Determination of the inhibitory effect of the compound of the present invention on the kinase activity of wild type EGFR**

Experimental objective: The objective of this test example is to determine the inhibitory effect of the compound on the kinase activity of wild type EGFR.
Experimental instruments: Centrifuge (Eppendorf 5810R), microplate reader (BioTek Synergy HI), pipette (Eppendorf & Rainin).

Experimental process: The experiment applied the TR-FRET (time-resolved fluorescence resonance energy transfer) method to study the inhibitory effect of the compound on the kinase activity of wild type EGFR. The experiment was carried out in a 384-well plate. Experimental buffer (50mM HEPES, 1mM EGTA, 10mM $MgCl_2$, 2mM DTT, 0.01% Tween-20) was formulated. The compound was diluted in gradient to different concentrations with the experimental buffer. 2 μL of the solution was added to each well of the 384-well plate. 4 μL of diluted EGFR kinase solution (0.001-0.5 nM) was added, and the plate was incubated at room temperature for 10 minutes. 4 μL of ULight-poly GT/ATP mixed solution was added, and the plate was incubated at room temperature for 30 to 60 minutes. 5 μL of EDTA stop solution and 5 μL of Eu-labeled antibody detection solution were added, and the plate was incubated at room temperature for 1 hour. The fluorescence signal value of each well at 665 nm was measured by the microplate reader.

Experimental data processing method:

The inhibition rate (((positive control well value - sample well value)/(positive control well value - negative control well value)) *100%) was calculated using the fluorescence signal value at 665 nm. The concentration and inhibition rate were fitted to a nonlinear regression curve by Graphpad Prism software to obtain the $IC_{50}$ value. The specific data is shown in Table 2 below:

Table 2 $IC_{50}$ of the inhibitory effect of the compound on the kinase activity of wild type EGFR

| Example No. | EGFR WT $IC_{50}$ (nM) |
|---|---|
| AZD9291 | 1.95 |
| 25 | 89.75 |
| 27 | 121.50 |
| 28 | 63.32 |
| 30 | 453.00 |
| 31 | >1000 |
| 32 | 82.87 |
| 33 | 133.10 |
| 46 | 72.75 |
| 66 | 50.76 |
| 116 | 1324.00 |
| 120 | >1000 |
| 136 | 250.00 |
| 138 | >1000 |
| 142 | 133.10 |

Experimental conclusion:

The above experiment demonstrates that the compounds of the examples of the present invention have a weak inhibitory effect in the EGFR wild type kinase activity inhibition experiment.

**Test Example 3. Determination of the inhibitory effect of the compound of the present invention on the proliferation of Ba/F3 EGFR mutant cell line and A431 cell line**

Experimental objective: The objective of this test example is to determine the inhibitory effect of the compound on the proliferation of Ba/F3 EGFR mutant cell line and A431 cell line

Experimental instruments: Microplate reader (BioTek Synergy HI), pipette (Eppendorf & Rainin).

Experimental process:

Ba/F3 EGFR mutant cells were cultured, and harvested after reaching a suitable density. The cells were adjusted to an appropriate cell concentration with the complete medium. The cell suspension were spread on a 96-well plate (90 μL per well), and the plate was placed in a 37°C, 5% $CO_2$ incubator overnight to let the cells adhere to the wall. Solutions of the compound at different concentrations were formulated with DMSO and culture medium. Solvent control was set. The solution of the compound was added to the 96-well plate (10 μL per well), and the plate was incubated in the 37°C,

5% $CO_2$ incubator for 72h to 144h. CellTiter- Glo solution was added and mixed well by shaking, and the plate was incubated in the dark for 10 minutes. The values were measured by BioTek Synergy H1 microplate reader.

Experimental process:

A431 cells were cultured, and harvested after reaching a suitable density. The cells were adjusted to an appropriate cell concentration with the complete medium. The cell suspension were spread on a 96-well plate (90 μL per well), and the plate was placed in a 37°C, 5% $CO_2$ incubator overnight to let the cells adhere to the wall. Solutions of the compound at different concentrations were formulated with DMSO and culture medium. Solvent control was set. The solution of the compound was added to the 96-well plate (10 μL per well), and the plate was incubated in the 37°C, 5% $CO_2$ incubator for 72h. CellTiter- Glo solution was added and mixed well by shaking, and the plate was incubated in the dark for 10 minutes. The values were measured by BioTek Synergy H1 microplate reader.

Experimental data processing method:

The inhibition rate was calculated using the fluorescence signal value. The concentration and inhibition rate were fitted to a nonlinear regression curve by Graphpad Prism software to obtain the $IC_{50}$ value. The specific data is shown in Table 3 below:

Table 3 $IC_{50}$ of the inhibitory effect of the compound on the proliferation of Ba/F3 EGFR mutant cell line and A431 cell line

| Example No. | Ba/F3 EGFR-D770-N771ins_SVD $IC_{50}$ (nM) | A431 $IC_{50}$ (nM) |
|---|---|---|
| AZD9291 | 212.70 | 156.30 |
| 1 | 76.00 | 536.80 |
| 24 | 14.97 | 123.80 |
| 35 | 24.75 | 161.50 |
| 36 | 27.12 | 439.30 |
| 37 | 16.62 | 66.00 |
| 38 | 34.65 | 145.70 |
| 40 | 34.65 | 133.30 |
| 43 | 21.00 | NA |
| 45 | 53.44 | 209.80 |
| 47 | 33.13 | 51.73 |
| 52 | 34.88 | 306.50 |
| 53 | 21.65 | 182.20 |
| 56 | 45.32 | 144.40 |
| 57 | 28.08 | 113.30 |
| 63 | 14.97 | NA |
| 64 | 17.40 | 28.94 |
| 65 | 15.12 | NA |
| 67 | 10.41 | 94.54 |
| 68 | 15.40 | NA |
| 69 | 40.03 | NA |
| 70 | 47.36 | NA |
| 71 | 54.92 | 238.10 |
| 78 | 48.22 | 257.60 |
| 81 | 22.87 | 66.81 |
| 85 | 17.38 | NA |

(continued)

| Example No. | Ba/F3 EGFR-D770-N771ins_SVD $IC_{50}$ (nM) | A431 $IC_{50}$ (nM) |
|---|---|---|
| 109 | 49.14 | 167.90 |
| 111 | 21.17 | 34.08 |
| 112 | 26.69 | NA |
| 114 | 42.47 | NA |
| 118 | 45.09 | 200.60 |
| 119 | 45.44 | NA |
| 127 | 13.39 | 46.56 |
| 137 | 9.78 | 18.24 |

Experimental conclusion:
The above experiment demonstrates that the compounds of the examples of the present invention have a good inhibitory effect in the Ba/F3 EGFR mutant cell proliferation inhibition experiment, but showed a poor inhibitory effect on A431 cell. By comparison, it can be seen that the compounds of the examples of the present invention have a high selectivity for the inhibition of Ba/F3 EGFR mutant cell proliferation.

**Test Example 4. *In vivo* pharmacodynamic study of the compound of the present invention in the mouse pro-B cell Ba/F3 EGFR-D770-N771ins_SVD xenograft model**

4.1 Experimental objective:

The *in vivo* efficacy of the compound in the mouse pro-B cell Ba/F3 EGFR-D770-N771ins_SVD xenograft model was evaluated.
4.2 Experimental instruments and reagents:

4.2.1 Instruments:

1. Biological safety cabinet (BSC-1300II A2, Medical equipment factory of Shanghai Boxun Industrial Co., Ltd.)
2. Clean bench (CJ-2F, Suzhou Fengshi Laboratory Animal Equipment Co., Ltd.)
3. $CO_2$ incubator (Thermo-311, Thermo)
4. Centrifuge (Centrifuge 5720R, Eppendorf)
5. Automatic cell counter (Countess II, Life Technologies)
6. Vernier calipers (CD-6"AX, Mitutoyo, Japan)
7. Cell culture flask (T75/T225, Corning)
8. Electronic balance (CPA2202S, Sartorius)
9. Electronic balance (BSA2202S-CW, Sartorius)

4.2.2 Reagents:

1. RPMI-1640 medium (22400-089, Gibco)
2. Fetal bovine serum (FBS) (10099-141C, Gibco)
3. Phosphate buffered saline (PBS) (10010-023, Gibco)
4. Tween 80 (30189828, Sinopharm Chemical Reagent Co., Ltd.)
5. Sodium carboxymethyl cellulose (30036365, Sinopharm Chemical Reagent Co., Ltd.)

4.3 Experimental process and data processing:

4.3.1 Animals
BALB/cA nude mice (6-8 weeks old, female) were purchased from Shanghai Sino-British SIPPR/BK Lab Animal Co., Ltd.

4.3.2 Cell culture and preparation of cell suspension

a. A Ba/F3 EGFR-D770-N771ins_SVD cell line was taken from the cell bank, and the cells were resuscitated with the RPMI-1640 medium (RPMI-1640 + 10% FBS). The resuscitated cells were placed in the cell culture flasks (cell type, date, the name of the culturing person and the like were marked on the flask wall), and incubated in a $CO_2$ incubator (the incubator temperature was 37°C, and the $CO_2$ concentration was 5%).
b. The cells were passaged every three days. After passage, the cells were further incubated in the $CO_2$ incubator. The process was repeated until the number of cells met the requirements of the *in vivo* pharmacodynamic study.
c. The cultured cells were collected and counted by the automatic cell counter. According to the counting results, the cells were resuspended in PBS to prepare a cell suspension (density: $2 \times 10^7$ cells/mL), which was placed in an ice box for later use.

4.3.3 Cell inoculation

a. The nude mice were marked before inoculation with disposable universal ear tags for mouse and rat.
b. At the time of inoculation, the cell suspension was mixed well, 0.1 to 1 mL of the cell suspension was taken with a 1 mL syringe, air bubbles were removed, and the syringe was placed on an ice bag for later use.
c. The nude mice were bound with the operator's left hand. The right shoulder of the right back of the nude mouse (inoculation site) was disinfected with 75% alcohol. The nude mice were inoculated 30 seconds later.
d. The test nude mice were inoculated in turn (0.1 mL of cell suspension per mouse).

4.3.4 Tumor volume measurement, grouping and administration in the tumor-bearing mouse

a. Tumor was measured on Day 8 to Day 14 after inoculation depending on the tumor growth, and tumor size was calculated.

Tumor volume calculation: tumor volume ($mm^3$) = length (mm)×width (mm)×width (mm)/2

b. According to the body weight and tumor size of the tumor-bearing mouse, the mice were randomly grouped (5 mice per group).
c. According to the grouping results, the test drug was administered (administration method: oral administration; administration frequency: 1 time/day; administration period: 14 days; vehicle: 0.5% CMC/1% Tween 80).
d. The tumor was measured and the mouse was weighed twice a week after the test drug was administrated.
e. The animals were euthanized at the end of the test.
f. The data were processed with software such as Excel.

Calculation of the tumor growth inhibition rate TGI (%) of the compound:

TGI(%) = [(1-average tumor volume at the end of the administration in a certain treatment group)/average tumor volume at the end of the administration in the solvent control group]×100%.

TGI≥60% indicates that the compound is effective in this model;
TGI<60% indicates that the compound is ineffective in this model.

4.4 The test results are shown in Table 4 below:

Table 4 Pharmacodynamic parameters of the compound in xenograft mouse

| Group | Administration dose | Tumor volume (mm3, Mean ± SD) | T/C(%) | TGI (%) |
|---|---|---|---|---|
| | | Day14 | Day 14 | Day 14 |
| ADZ9291 | 40 mg/kg | 935±176 (1932±123) | 48.40 | 51.60 |
| Example 37 | 40 mg/kg | 499±151 (1938±197) | 25.75 | 74.25 |
| Example 67 | 40 mg/kg | 212±44 (1938±197) | 10.94 | 89.06 |
| Example 81 | 40 mg/kg | 334±121 (1938±197) | 17.23 | 82.77 |

Note: The data in parentheses refer to the tumor volume of the corresponding Vehicle QD × 2w group (*i.e.,* the control group) at the corresponding time.

4.5 Experimental results

The above data demonstrates that: after 14 days of continuous oral administration, AZD9291 40mpk is ineffective in the Ba/F3 EGFR-D770-N771ins_SVD pharmacodynamic model, while the compounds of Examples 37, 67 and 81 are effective at the same dose of 40 mpk.

**Test Example 5. Plasma stability test**

5.1 Experimental objective

The objective of this experiment is to determine the stability of the compounds of the examples in the plasma of mouse, rat and human.

5.2 Experimental steps

5.2.1 Solution formulation

1. Plasma preparation: Animal or human whole blood was collected, placed into a test tube containing anticoagulant, and centrifuged at 3500 rpm for 10 min. The light yellow plasma in the upper layer was collected.
2. 1 mM test compound (m/M/V=C), the compound was weighed and formulated into stock solution with DMSO.

5.2.2 Experimental process:

1. 398 μL of plasma and 2 μL of 1 mM compound (test compound) were added successively to a 96-well plate, and incubated at 37°C.
2. 50 μL of solution was taken out at 0, 15, 30, 60, 120 min, to which 400 μL of methanol stop solution containing internal standard was added.
3. The solution was centrifuged (3220 g, 30 min). 50 μL of supernatant was taken out, to which 50 μL of $DDH_2O$ was added to dilute. The resulting solution was injected into a LC-MS/MS.

5.3 Chromatography conditions

Instrument: Shimadzu LC-30AD (Nexera X2)
Column: XSelect HSS T3 $C_{18}$ (50*2.1 mm, particle size: 2.5 μm);

Mobile phases:

A: 0.1% formic acid solution,
B: Acetonitrile containing 0.1% formic acid,
0~0.3 min: 95% A→95% A;
0.3~0.8 min: 95%A→5% A;
Flow rate: 0.5 mL/min;
Running time: 2.0 min;
Injection volume: 3 μL.

5.4 Mass spectrometry conditions
Instrument:

API4000 Liquid Chromatography-Mass Spectrometer, AB Company, USA;
The ion source was an electrospray ionization source (ESI);
The drying gas ($N_2$) temperature was 500°C;
The electrospray voltage was 5500V;
The detection method was positive ion detection;
The scanning method was the selective reaction monitoring (MRM) method.

Table 5: Results of plasma stability of the compounds of the examples

| Species | No. | Residual rate (%) | | | | | $t_{1/2}$ (min) |
|---|---|---|---|---|---|---|---|
| | | 0 min | 15 min | 30 min | 60 min | 120 min | |
| Human | Example 67 | 100.00 | 93.86 | 85.71 | 67.74 | 37.38 | 82.71 |
| Rat | Example 67 | 100.00 | 90.61 | 81.14 | 59.66 | 33.25 | 74.04 |
| Mouse | Example 67 | 100.00 | 88.69 | 69.92 | 36.29 | 9.06 | 39.89 |

5.5 Experimental conclusion:
The above data demonstrate that the compounds of the examples of the present invention have high plasma stability and small differences between species.

**Claims**

**1.** A compound of formula (Ia), a stereoisomer thereof or a pharmaceutically acceptable salt thereof:

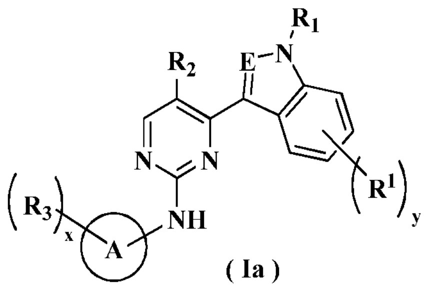
(Ia)

wherein:

E is selected from the group consisting of $CR_{aa}$ and N;
ring A is selected from the group consisting of heterocyclyl, aryl and heteroaryl;
$R_1$ is selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl are each optionally further substituted;
$R_2$ is selected from the group consisting of hydrogen, deuterium, alkyl, deuterated alkyl, haloalkyl, alkoxy, haloalkoxy, halogen, amino, nitro, hydroxy, cyano, azido, alkoxycarbonyl, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}C(O)R_{bb}$, $-C(O)NR_{aa}(CH_2)_nR_{bb}$, $-NR_{aa}C(O)NR_{bb}R_{cc}$, $-NR_{aa}C(O)NR_{bb}(CH_2)_nR_{cc}$, $-C{\equiv}CR_{aa}$, $-NR_{aa}C(O)CH{=}CH(CH_2)_nR_{bb}$, $-NR_{aa}C(O)C{\equiv}CR_{bb}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$, $-(CH_2)_nNR_{aa}S(O)_mR_{bb}$ and

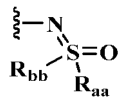

, wherein the alkyl, haloalkyl, cycloalkyl, heterocyclyl, aryl and heteroaryl are each optionally further substituted; each $R_3$ is independently selected from the group consisting of hydrogen, deuterium, alkyl, deuterated alkyl, haloalkyl, alkoxy, haloalkoxy, halogen, amino, nitro, hydroxy, cyano, azido, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}(CH_2)_nR_{bb}$, $-NR_{aa}(CH_2)_nNR_{bb}R_{cc}$, $-NR_{aa}C(O)R_{bb}$, $-NR_{aa}C(O)NR_{bb}R_{cc}$, $-NR_{aa}C(O)NR_{bb}(CH_2)_nR_{cc}$, $-NR_{aa}C(O)C{\equiv}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}R_{bb}$, $-C(O)OR_{aa}$, $-NR_{aa}S(O)_mR_{bb}$, $-O(CH_2)_nR_{aa}$, $-O(CH_2)_nNR_{aa}R_{bb}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$, wherein the alkyl, haloalkyl, cycloalkyl, heterocyclyl, aryl and heteroaryl are each optionally further substituted;

each $R^1$ is independently selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl are each optionally further substituted;

or, any two of $R^1$ are bonded to form a cycloalkyl, heterocyclyl, aryl or heteroaryl, wherein the cycloalkyl, heterocyclyl, aryl and heteroaryl are each optionally further substituted;

$R_{aa}$, $R_{bb}$ and $R_{cc}$ are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are each optionally further substituted;

x is an integer from 0 to 4;

y is an integer from 0 to 4;

m is an integer from 0 to 2;

n is an integer from 0 to 4.

2. A compound of formula (I), a stereoisomer thereof or a pharmaceutically acceptable salt thereof:

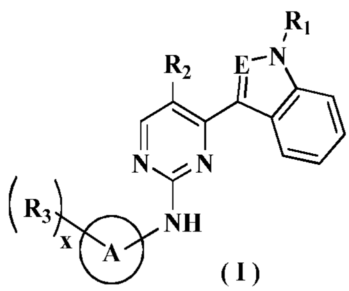

(I)

wherein:

E is selected from the group consisting of $CR_{aa}$ and N;

ring A is selected from the group consisting of heterocyclyl, aryl and heteroaryl;

$R_1$ is selected from the group consisting of hydrogen, deuterium, alkyl, deuterated alkyl, haloalkyl, alkoxy, haloalkoxy, halogen, amino, nitro, hydroxy, cyano, alkenyl, alkynyl, cycloalkyl, heterocyclyl, aryl, heteroaryl and $-(CH_2)_nR_{aa}$, wherein the alkyl, haloalkyl, cycloalkyl, heterocyclyl, aryl and heteroaryl are each optionally further substituted;

$R_2$ is selected from the group consisting of hydrogen, deuterium, alkyl, deuterated alkyl, haloalkyl, alkoxy, haloalkoxy, halogen, amino, nitro, hydroxy, cyano, azido, alkoxycarbonyl, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}C(O)R_{bb}$, $-C(O)NR_{aa}(CH_2)_nR_{bb}$, $-NR_{aa}C(O)NR_{bb}R_{cc}$ $-NR_{aa}C(O)NR_{bb}(CH_2)_nR_{cc}$, $-C{\equiv}CR_{aa}$, $-NR_{aa}C(O)CH{=}CH(CH_2)_nR_{bb}$, $-NR_{aa}C(O)C{\equiv}CR_{bb}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$, wherein the alkyl, haloalkyl, cycloalkyl, heterocyclyl, aryl and heteroaryl are each optionally further substituted;

each $R_3$ is independently selected from the group consisting of hydrogen, deuterium, alkyl, deuterated alkyl, haloalkyl, alkoxy, haloalkoxy, halogen, amino, nitro, hydroxy, cyano, azido, alkenyl, alkynyl, cycloalkyl, heterocyclyl, aryl, heteroaryl, $-(CH_2)_nOR_{aa}$, $-(CH_2)_nNR_{aa}R_{bb}$, $-NR_{aa}(CH_2)_nR_{bb}$, $-NR_{aa}(CH_2)_nNR_{bb}R_{cc}$, $-NR_{aa}C(O)R_{bb}$, $-NR_{aa}C(O)NR_{bb}R_{cc}$, $-NR_{aa}C(O)NR_{bb}(CH_2)_nR_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}R_{bb}$, $-C(O)OR_{aa}$, $-NR_{aa}S(O)_mR_{bb}$, $-O(CH_2)_nR_{aa}$, $-(CH_2)_nP(O)R_{aa}R_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$, wherein the alkyl, haloalkyl, cycloalkyl, heterocyclyl, aryl and heteroaryl are each optionally further substituted;

$R_{aa}$, $R_{bb}$ and $R_{cc}$ are each independently selected from the group consisting of hydrogen, deuterium, alkyl, deuterated alkyl, haloalkyl, alkoxy, hydroxyalkyl, haloalkoxy, halogen, cyano, nitro, hydroxy, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are each optionally further substituted;

x is an integer from 0 to 4;

m is an integer from 0 to 2;

n is an integer from 0 to 4.

3. The compound, a stereoisomer thereof or a pharmaceutically acceptable salt thereof according to claim 1 or 2, **characterized in that** ring A is selected from the group consisting of $C_{6-14}$ aryl and 5 to 14 membered heteroaryl; preferably selected from the group consisting of phenyl and benzoheteroaryl; and more preferably selected from the group consisting of phenyl, quinolyl and quinoxalyl.

4. The compound, a stereoisomer thereof or a pharmaceutically acceptable salt thereof according to claim 1 or 2, **characterized in that** ring A is selected from the group consisting of 3 to 12 membered heterocyclyl and 5 to 14 membered heteroaryl; preferably selected from the group consisting of 3 to 8 membered heterocyclyl and 5 to 10 membered heteroaryl; and more preferably selected from the group consisting of 1,4-dihydropyridyl and pyridyl.

5. The compound, a stereoisomer thereof or a pharmaceutically acceptable salt thereof according to claim 1, **characterized in that** $R_1$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-(CH_2)_nR_{aa}$; preferably selected from the group consisting of hydrogen, $C_{1-6}$ alkyl, $C_{3-8}$ cycloalkyl and $-(CH_2)_nR_{aa}$; and more preferably selected from the group consisting of methyl, cyclopropyl and $-(CH_2)_nR_{aa}$.

6. The compound, a stereoisomer thereof or a pharmaceutically acceptable salt thereof according to claim 1 or 2, **characterized in that** $R_1$ is selected from the group consisting of hydrogen, substituted or unsubstituted $C_{1-6}$ alkyl, substituted or unsubstituted $C_{3-8}$ cycloalkyl and $-(CH_2)_nR_{aa}$, preferably selected from the group consisting of hydrogen, substituted or unsubstituted $C_{1-3}$ alkyl, substituted or unsubstituted $C_{3-6}$ cycloalkyl and $-(CH_2)_nR_{aa}$, more preferably selected from the group consisting of hydrogen, $C_{1-3}$ alkyl substituted by one or more of deuterium, fluorine, chlorine, bromine and hydroxy, $C_{3-6}$ cycloalkyl substituted by one or more of deuterium, fluorine, chlorine, bromine and hydroxy, and $-(CH_2)_nR_{aa}$, and further preferably selected from the group consisting of hydrogen, methyl, ethyl, propyl, butyl, tri-deuterated methyl, methyl substituted by one to three of fluorine, methyl substituted by one to three of chlorine, methyl substituted by one to three of bromine, cyclopropyl, cyclobutyl, cyclopentyl, cyclohexyl,

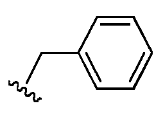,
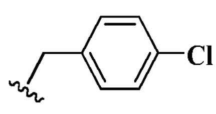

and

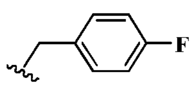.

7. The compound, a stereoisomer thereof or a pharmaceutically acceptable salt thereof according to claim 1 or 2, **characterized in that** $R_1$ is substituted or unsubstituted 3 to 12 membered heterocyclyl, preferably substituted or unsubstituted $C_{3-8}$ heterocyclyl, more preferably $C_{3-6}$ heterocyclyl, and further preferably oxetane.

**8.** The compound, a stereoisomer thereof or a pharmaceutically acceptable salt thereof according to claim 1 or 2, **characterized in that** $R_2$ is selected from the group consisting of hydrogen, deuterium, halogen, cyano, amino, nitro, hydroxy, azido, alkoxycarbonyl, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-C{\equiv}CR_{aa}$, $-NR_{aa}C(O)CH{=}CH(CH_2)_nR_{bb}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$; and preferably selected from the group consisting of hydrogen, halogen, cyano, azido, alkoxycarbonyl, $C_{1-6}$ alkyl, $C_{2-6}$ alkenyl, $C_{2-6}$ alkynyl, 3 to 12 membered heterocyclyl, 5 to 14 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$ and $-(CH_2)_nP(O)R_{aa}R_{bb}$.

**9.** The compound, a stereoisomer thereof or a pharmaceutically acceptable salt thereof according to claim 1 or 2, **characterized in that** $R_2$ is selected from the group consisting of hydrogen, halogen, cyano, azido, 1 to 6 membered alkoxycarbonyl, $C_{1-6}$ alkyl, $C_{1-6}$ haloalkyl, $C_{2-6}$ alkenyl, $C_{2-6}$ alkynyl, substituted or unsubstituted 3 to 12 membered heterocyclyl, substituted or unsubstituted $C_{6-14}$ aryl, substituted or unsubstituted 5 to 14 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)_nS(O)_mR_{aa}$ and $-(CH_2)_nP(O)R_{aa}R_{bb}$, preferably selected from the group consisting of hydrogen, halogen, cyano, azido, 1 to 6 membered alkoxycarbonyl, $C_{1-3}$ alkyl, $C_{1-3}$ haloalkyl, $C_{2-5}$ alkenyl, $C_{2-5}$ alkynyl, substituted or unsubstituted 3 to 10 membered heterocyclyl, substituted or unsubstituted $C_{6-12}$ aryl, substituted or unsubstituted 5 to 12 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)_nS(O)_mR_{aa}$ and $-(CH_2)_nP(O)R_{aa}R_{bb}$, more preferably selected from the group consisting of hydrogen, halogen, cyano, azido, 1 to 3 membered alkoxycarbonyl, $C_{1-3}$ alkyl, $C_{1-3}$ haloalkyl, $C_{2-5}$ alkenyl, $C_{2-5}$ alkynyl, substituted or unsubstituted 3 to 8 membered heterocyclyl, substituted or unsubstituted $C_{6-10}$ aryl, substituted or unsubstituted 5 to 10 membered heteroaryl, $-NR_{aa}C(O)NR_{bb}(CH_2)_nR_{cc}$, $-C{\equiv}CR_{aa}$, $-NR_{aa}C(O)CH{=}CHR_{bb}$, $-C(O)NR_{aa}(CH_2)_nR_{bb}$, $-C(O)OR_{aa}$, $-O(CH_2)_nR_{aa}$, $-(CH_2)_nS(O)_mR_{aa}$ and $-(CH_2)_nP(O)R_{aa}R_{bb}$, and further preferably selected from the group consisting of hydrogen, fluorine, chlorine, bromine, azido, ethynyl, propynyl, propargyl, cyano, methyl, ethyl, propyl, trifluoromethyl, $-S(O)_2CH_3$, $-S(O)_2CH(CH_3)_2$, $-P(O)(CH_3)_2$, $-C(O)N(CH_3)_2$, $-C(O)OCH_3$, $-C(O)OCH_2CH_3$, $-C(O)OCH(CH_3)_2$,

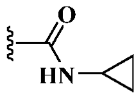, 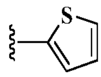,

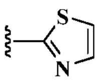, 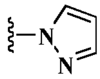, 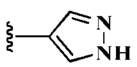, 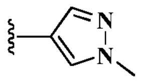, 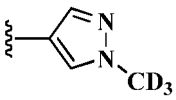, 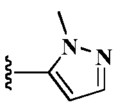, 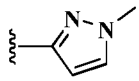,

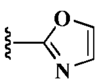, 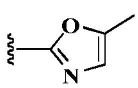, 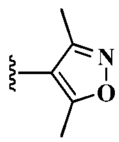, 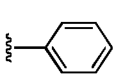, 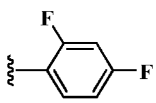, 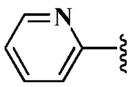, 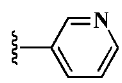,

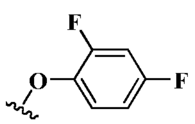, 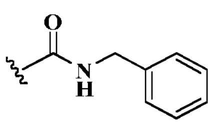, 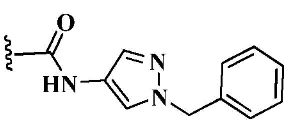, 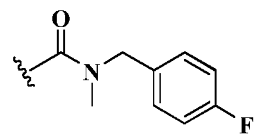,

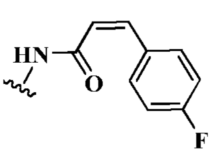, 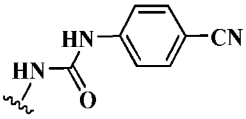

and

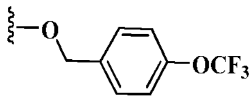

.

**10.** The compound, a stereoisomer thereof or a pharmaceutically acceptable salt thereof according to claim 1 or 2, **characterized in that** $R_2$ is selected from the group consisting of $C_{1-6}$ hydroxyalkyl, substituted or unsubstituted 5 to 12 membered heteroaryl and

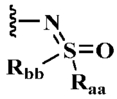

; preferably selected from the group consisting of $C_{1-3}$ hydroxyalkyl, substituted or unsubstituted 5 to 10 membered heteroaryl and

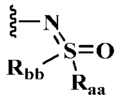

;

and more preferably selected from the group consisting of

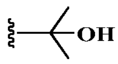

,

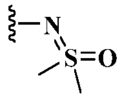

,

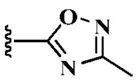

and

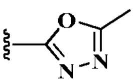

.

**11.** The compound, a stereoisomer thereof or a pharmaceutically acceptable salt thereof according to claim 1 or 2, **characterized in that** $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}(CH_2)_nR_{bb}$, $-NR_{aa}(CH_2)_nNR_{bb}R_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$; and preferably selected from the group consisting of hydrogen atom, $C_{1-6}$ alkyl, $C_{2-6}$ alkenyl, $C_{2-6}$ alkynyl, $C_{1-6}$ alkoxy, 3 to 12 membered heterocyclyl, 5 to 14 membered heteroaryl, $-NR_{aa}(CH_2)_nR_{bb}$, $-NR_{aa}(CH_2)_nNR_{bb}R_{cc}$, $-NR_{aa}C(O)C\equiv CR_{bb}$, $-NR_{aa}C(O)CH=CH(CH_2)_nR_{bb}$, $-NR_{aa}C(O)CH=CH(CH_2)_nNR_{bb}R_{cc}$ and $-O(CH_2)_nR_{aa}$.

**12.** The compound, a stereoisomer thereof or a pharmaceutically acceptable salt thereof according to claim 1 or 2, **characterized in that** $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-6}$ cycloalkyl, 3 to 12 membered heterocyclyl, $C_{6-14}$ aryl, 5 to 14 membered heteroaryl, $-NR_eC(O)C\equiv CR_f$ and $-NR_eC(O)CH=CH(CH_2)_nR_f$, preferably, $R_3$ is selected from the group consisting of $C_{1-3}$ alkoxy, $C_{1-3}$ alkoxy substituted by halogen, $-NHC(O)C\equiv CCH_3$, $-NHC(O)CH=CH_2$, $-NHC(O)CH=CHCH_3$, $-NHC(O)CH=CHCH_2N(CH_3)_2$,

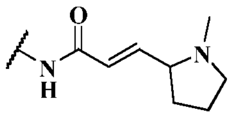, 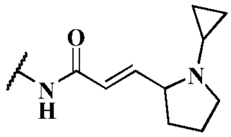,

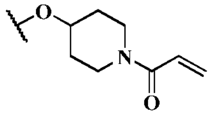, 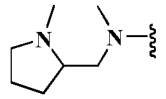, 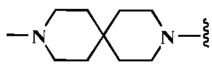, 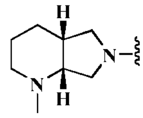, 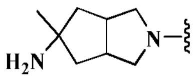,

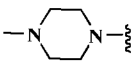, 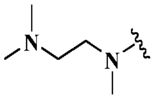, 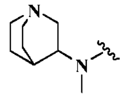, 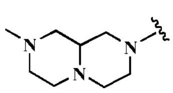, 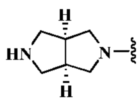, 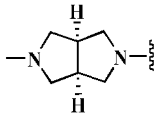,

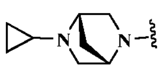, 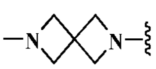, 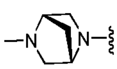, 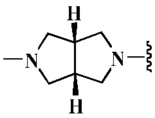, 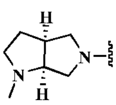, 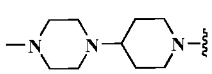,

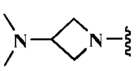, 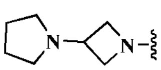, 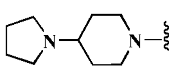, 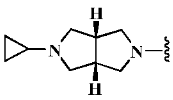, 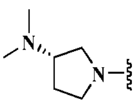, 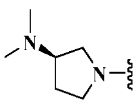,

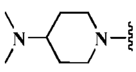

and

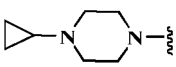;

wherein, $R_e$ and $R_f$ are each independently selected from the group consisting of hydrogen, deuterium atom, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom.

**13.** The compound, a stereoisomer thereof or a pharmaceutically acceptable salt thereof according to claim 1 or 2, **characterized in that** $R_3$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-6}$ cycloalkyl, 3 to 12 membered heterocyclyl, $C_{6-14}$ aryl, 5 to 14 membered heteroaryl, $-OR_e$, $-NR_e(CH_2)_nR_f$, $-NR_eC(O)C{=}CR_f$, $-NHC(O)CR_e{=}CH(CH_2)_nR_f$ and $-NR_eC(O)CH{=}CH(CH_2)_nR_f$, preferably selected from the group consisting of hydrogen, deuterium, halogen, cyano, amino, nitro, hydroxy, azido, $C_{1-3}$ alkyl, $C_{2-5}$ alkenyl, $C_{2-5}$ alkynyl, $C_{1-3}$ alkoxy, $C_{1-3}$ hydroxyalkyl, $C_{3-6}$ cycloalkyl, 3 to 10 membered heterocyclyl, $C_{6-12}$ aryl, 5 to 12 membered heteroaryl, $-OR_e$, $-NR_e(CH_2)_nR_f$, $-NR_eC(O)C{\equiv}CR_f$, $-NHC(O)CR_e{=}CH(CH_2)_nR_f$ and $-NR_eC(O)CH{=}CH(CH_2)_nR_f$, more preferably selected from the group consisting of hydrogen, deuterium, halogen, cyano, amino, nitro, hydroxy, azido, $C_{1-3}$ alkyl, $C_{2-5}$ alkenyl, $C_{2-5}$ alkynyl, $C_{1-3}$ alkoxy, $C_{1-3}$ hydroxyalkyl, $C_{3-6}$ cycloalkyl, 3 to 8 membered heterocyclyl, $C_{6-10}$ aryl, 5 to 10 membered heteroaryl, $-OR_e$, $-NR_e(CH_2)_nR_f$, $-NR_eC(O)C{=}CR_f$, $-NHC(O)CR_e{=}CH(CH_2)_nR_f$ and $-NR_eC(O)CH{=}CH(CH_2)_nR_f$, and further preferably selected from the group consisting of methyl, ethyl, propyl, methoxy, ethoxy, propoxy, methoxy substituted by 1 to 3 fluorine, methoxy substituted by 1 to 3 chlorine, methoxy substituted by 1 to 3 bromine, $-NHC(O)C{=}CH$, $-NHC(O)C{\equiv}CCH_3$, $-NHC(O)CH{=}CH_2$, $-NHC(O)CF{=}CH_2$, $-NHC(O)C(CH_3){=}CH_2$, $-NHC(O)CH{=}CHCH_3$, $-NHC(O)CH{=}CHCH_2N(CH_3)_2$,

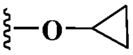, 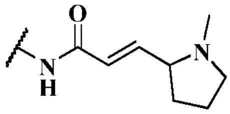, 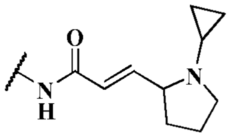, 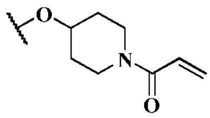, 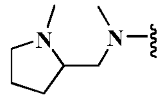,

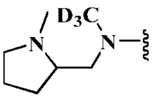, 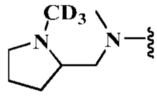, 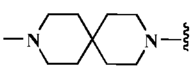, 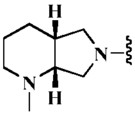, 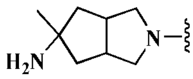, 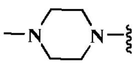,

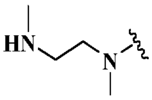, 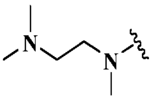, 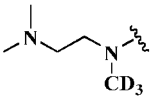, 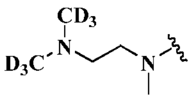, 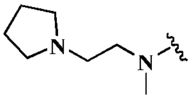, 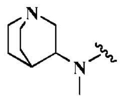,

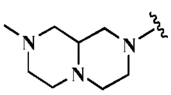, 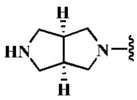, 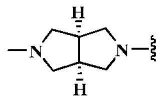, 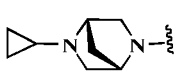, 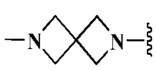, 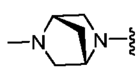,

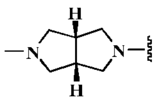, 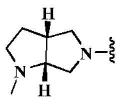, 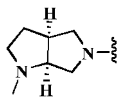, 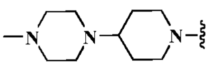, 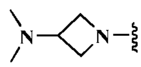, 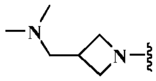,

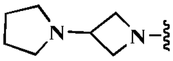, 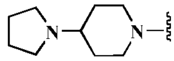, 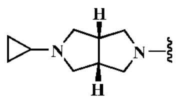, 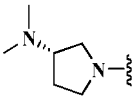, 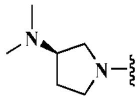, 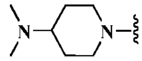,

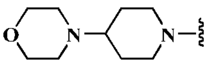, 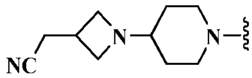, 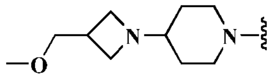

and

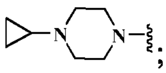;

wherein, $R_e$ and $R_f$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom.

**14.** The compound, a stereoisomer thereof or a pharmaceutically acceptable salt thereof according to claim 1 or 2, **characterized in that** $R_3$ is selected from the group consisting of substituted or unsubstituted 3 to 12 membered heterocyclyl, $-O(CH_2)_nR_e$ and $-O(CH_2)_nNR_eR_f$; preferably selected from the group consisting of substituted or unsubstituted 3 to 8 membered heterocyclyl, $-O(CH_2)_nR_e$ and $-O(CH_2)_nNR_eR_f$; and more preferably selected from the group consisting of

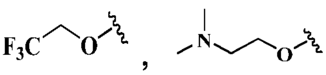

and

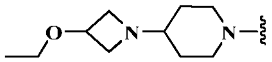

;

$R_e$ and $R_f$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl containing 1 to 2 nitrogen atom; wherein the $C_{1-6}$ alkyl, $C_{1-6}$ alkoxy, $C_{3-6}$ cycloalkyl and $C_{3-6}$ heterocyclyl containing 1 to 2 nitrogen atom are each optionally substituted by one or more substituents selected from the group consisting of hydrogen, deuterium, halogen, cyano, amino, nitro, hydroxy, $C_{1-6}$ alkyl, $C_{1-6}$ alkoxy, $C_{1-6}$ hydroxyalkyl, $C_{3-6}$ cycloalkyl, $C_{3-6}$ heterocyclyl and $-C(O)CH=CR_gR_h$;

$R_g$ and $R_h$ are each independently selected from the group consisting of hydrogen, deuterium, halogen, cyano, amino, nitro, hydroxy, azido, $C_{1-6}$ alkyl, $C_{1-6}$ alkoxy, $C_{1-6}$ hydroxyalkyl, $C_{3-6}$ cycloalkyl and $C_{3-6}$ heterocyclyl.

**15.** The compound, a stereoisomer thereof or a pharmaceutically acceptable salt thereof according to claim 1 or 2, **characterized in that** $R_3$ is selected from the group consisting of substituted or unsubstituted 3 to 12 membered heterocyclyl; preferably selected from the group consisting of substituted or unsubstituted 3 to 8 membered heterocyclyl; and more preferably is

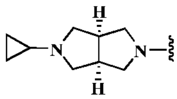

.

**16.** The compound, a stereoisomer thereof or a pharmaceutically acceptable salt thereof according to claim 1 or 2, **characterized in that** $R^1$ is selected from the group consisting of hydrogen, deuterium, halogen, amino, nitro, hydroxy, cyano, $C_{1-6}$ alkyl, $C_{2-6}$ alkenyl, $C_{2-6}$ alkynyl, deuterated $C_{1-6}$ alkyl, $C_{1-6}$ haloalkyl, $C_{1-6}$hydroxyalkyl, $C_{1-6}$ alkoxy, $C_{1-6}$ haloalkoxy, $C_{3-8}$ cycloalkyl, 3 to 12 membered heterocyclyl, $C_{6-14}$ aryl and 5 to 14 membered heteroaryl; preferably selected from the group consisting of hydrogen, deuterium, halogen, amino, hydroxy, cyano, oxo, thioxo, $C_{1-3}$ alkyl, $C_{2-5}$ alkenyl, $C_{2-5}$ alkynyl, deuterated $C_{1-3}$ alkyl, $C_{1-3}$ haloalkyl, $C_{1-3}$ hydroxyalkyl, $C_{1-3}$ alkoxy, $C_{1-3}$ haloalkoxy, $C_{3-6}$ cycloalkyl, 3 to 10 membered heterocyclyl, $C_{6-12}$ aryl and 5 to 12 membered heteroaryl; more preferably selected from the group consisting of hydrogen, deuterium, halogen, amino, hydroxy, cyano, oxo, thioxo, $C_{1-3}$ alkyl, $C_{2-5}$ alkenyl, $C_{2-5}$ alkynyl, deuterated $C_{1-3}$ alkyl, $C_{1-3}$ haloalkyl, $C_{1-3}$ hydroxyalkyl, $C_{1-3}$ alkoxy, $C_{1-3}$ haloalkoxy, $C_{3-6}$ cycloalkyl, 3 to 8 membered heterocyclyl containing 1 to 3 atoms selected from the group consisting of N, O or and S, $C_{6-10}$ aryl and 5 to 10 membered heteroaryl containing 1 to 3 atoms selected from the group consisting of N, O or and S; and further preferably selected from the group consisting of hydrogen, deuterium, fluorine, chlorine, bromine, amino, hydroxy, cyano, oxo, thioxo, methyl, ethyl, propyl, vinyl, propenyl, allyl, ethynyl, propynyl, propargyl, deuterated methyl, deuterated ethyl, deuterated propyl, fluoromethyl, fluoroethyl, fluoropropyl, chloromethyl, chloroethyl, chloropropyl, bromomethyl, bromoethyl, bromopropyl, hydroxymethyl, hydroxyethyl, hydroxypropyl, methoxy, ethoxy, propoxy, fluoromethoxy, fluoroethoxy, fluoropropoxy, chloromethoxy, chloroethoxy, chloropropoxy, cyclopropyl, cyclobutyl, cyclopentyl, cyclohexyl, cycloheptyl, cyclopropoxy, cyclobutoxy, cyclopentyloxy, cyclohexyloxy, cycloheptyloxy, aziridinyl, azetidinyl, azacyclopentyl, azacyclohexyl, azacycloheptyl, thienyl, pyrrolyl, pyridyl, pyranyl, piperazinyl, phenyl and naphthyl;

or, any two of $R^1$ are bonded to form a $C_{3-8}$ cycloalkyl, 3 to 12 membered heterocyclyl, $C_{6-14}$ aryl or 5 to 14 membered heteroaryl; preferably a 3 to 8 membered heterocyclyl or 5 to 10 membered heteroaryl; more preferably a 3 to 8 membered heterocyclyl; and further preferably

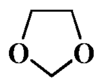

or

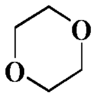

;

**17.** The compound, a stereoisomer thereof or a pharmaceutically acceptable salt thereof according to claim 1 or 2,

**characterized in that** $R_{aa}$, $R_{bb}$ and $R_{cc}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

**18.** The compound, a stereoisomer thereof or a pharmaceutically acceptable salt thereof according to claim 1 or 2, **characterized in that** $R_{aa}$, $R_{bb}$ and $R_{cc}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more of deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, substituted or unsubstituted 3 to 12 membered heterocyclyl, $C_{6-14}$ aryl, 5 to 14 membered heteroaryl, $-O(CH_2)_nR_{AA}$ and $-C(O)CH=CHR_{AA}R_{BB}$; $R_{AA}$ and $R_{BB}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

**19.** The compound, a stereoisomer thereof or a pharmaceutically acceptable salt thereof according to claim 1, **characterized in that** the formula (Ia) is further shown as formula (IIa):

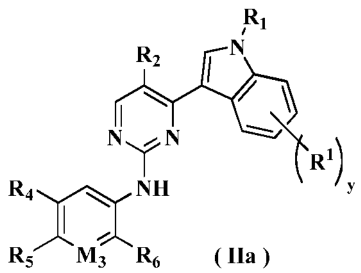

( IIa )

wherein:

$M_3$ is selected from the group consisting of $CR_{13}$ and N;
$R_4$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)R_{bb}$, $-NR_{aa}C(O)NR_{bb}(CH_2)_nR_{cc}$, $-NR_{aa}C(O)C{\equiv}CR_{bb}$, $-NR_{aa}C(O)CH=CH(CH_2)_nR_{bb}$, $-NR_{aa}C(O)CH=CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)_nS(O)_mNR_{aa}R_{bb}$, $-(CH_2)_nC(O)R_{aa}$, $-NR_{aa}C(O)OR_{bb}$, $-(CH_2)_nS(O)_mR_{aa}$ and $-(CH_2)_nNR_{aa}S(O)_mR_{bb}$; and preferably selected from the group consisting of $-NR_{aa}C(O)R_{bb}$, $-NR_{aa}C(O)C{\equiv}CR_{bb}$, $-NR_{aa}C(O)CH=CH(CH_2)_nR_{bb}$ and $-NR_{aa}C(O)CH=CH(CH_2)_nNR_{bb}R_{cc}$;
$R_5$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$, $-O(CH_2)_nNR_{aa}R_{bb}$ and $-NR_{aa}(CH_2)_nNR_{bb}R_{cc}$, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of hydrogen, deuterium, halogen, hydroxy, thiol, amino, $C_{1-4}$ alkyl, $C_{1-4}$ haloalkyl, $C_{1-4}$ alkoxy, $C_{1-4}$ haloalkoxy, $C_{2-6}$ alkenylcarbonyl, $C_{1-4}$ hydroxyalkyl, $C_{3-8}$ cycloalkyl, substituted or unsubstituted 3 to 12 membered heterocyclyl and $-(CH_2)_nNR_{dd}R_{ee}$; and preferably $R_5$ is selected from the group consisting of hydrogen, deuterium, halogen, cyano, amino, nitro, hydroxy, $C_{1-3}$ alkyl, $C_{2-4}$ alkenyl, $C_{2-4}$ alkynyl, $C_{1-3}$ alkoxy, $C_{1-3}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$, $-O(CH_2)_nNR_{aa}R_{bb}$ and $-NR_{aa}(CH_2)_nNR_{bb}R_{cc}$, wherein the $C_{1-3}$ alkyl, $C_{2-4}$ alkenyl, $C_{2-4}$ alkynyl, $C_{1-3}$ alkoxy, $C_{1-3}$

hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of hydrogen, deuterium, halogen, hydroxy, thiol, amino, $C_{1-3}$ alkyl, $C_{1-3}$ haloalkyl, $C_{1-3}$ alkoxy, $C_{1-3}$ haloalkoxy, $C_{2-4}$ alkenylcarbonyl, $C_{1-3}$ hydroxyalkyl, $C_{3-8}$ cycloalkyl, substituted or unsubstituted 3 to 12 membered heterocyclyl and $-(CH_2)_nNR_{dd}R_{ee}$;

$R_6$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-O(CH_2)_nR_{aa}$, preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 10 membered heterocyclyl, $C_{6-12}$ aryl, 5 to 12 membered heteroaryl and $-O(CH_2)_nR_{aa}$, and more preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 8 membered heterocyclyl, $C_{6-10}$ aryl, 5 to 10 membered heteroaryl and $-O(CH_2)_nR_{aa}$;

$R_{13}$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl, preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 10 membered heterocyclyl, $C_{6-12}$ aryl and 5 to 12 membered heteroaryl, and more preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 8 membered heterocyclyl, $C_{6-10}$ aryl and 5 to 10 membered heteroaryl;

$R_{dd}$ and $R_{ee}$ are each independently selected from the group consisting of hydrogen atom, deuterium atom, halogen, cyano, amino, nitro, hydroxy, $C_{1-6}$ alkyl, $C_{1-6}$ alkoxy, $C_{1-6}$ hydroxyalkyl, $C_{1-6}$ cycloalkyl, 3 to 12 membered heterocyclyl, $C_{6-14}$ aryl and 5 to 14 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

**20.** The compound, a stereoisomer thereof or a pharmaceutically acceptable salt thereof according to claim 2, **characterized in that** the formula (I) is further shown as formula (II):

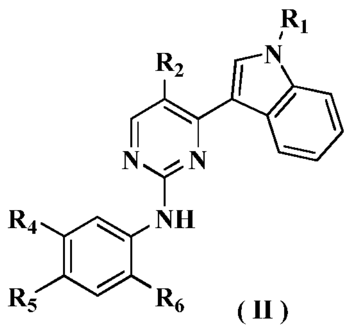

( II )

wherein:

$R_4$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{=}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)nS(O)_mNR_{aa}R_{bb}$, $-(CH_2)_nC(O)R_{aa}$, $-NR_{aa}C(O)OR_{bb}$, $-(CH_2)nS(O)_mR_{aa}$ and $-(CH_2)_nNR_{aa}S(O)_mR_{bb}$;

$R_5$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more

substituents selected from the group consisting of halogen, hydroxy, thiol, $C_{1-4}$ alkyl, $C_{1-4}$ haloalkyl, $C_{1-4}$ alkoxy, $C_{1-4}$ hydroxyalkyl and $C_{3-8}$ cycloalkyl;

$R_6$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl.

**21.** The compound, a stereoisomer thereof or a pharmaceutically acceptable salt thereof according to claim 1, 2, 19 or 20, **characterized in that** $R_2$ is selected from the group consisting of hydrogen, halogen, cyano, azido, alkoxycarbonyl, $C_{1-6}$ alkyl, halo$C_{1-6}$ alkyl, $C_{2-6}$ alkenyl, $C_{2-6}$ alkynyl, 3 to 12 membered heterocyclyl, $C_{6-14}$ aryl, substituted or unsubstituted 5 to 14 membered heteroaryl, $-NR_aC(O)NR_c(CH_2)_nR_b$, $-C{\equiv}CR_a$, $-NR_aC(O)CH{=}CHR_b$, $-C(O)NR_a(CH_2)_nR_b$, $-C(O)OR_a$, $-O(CH_2)_nR_a$, $-(CH_2)_nS(O)_mR_a$, $-(CH_2)_nS(O)_mNR_aR_b$ and $-(CH_2)_nP(O)R_aR_b$, wherein the heterocyclyl is selected from 5 to 8 membered heteroaryl containing 1 to 2 atoms selected from the group consisting of nitrogen, oxygen and sulfur atom, which is optionally substituted by hydroxy, halogen, $C_{1-6}$ alkyl or $C_{1-6}$ alkoxy, further selected from the group consisting of:

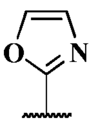 , 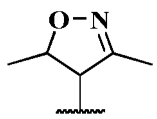 , 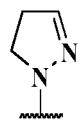 , 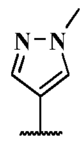 , 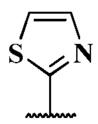 , 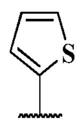 , 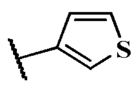 , 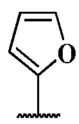 ,

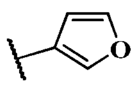 , 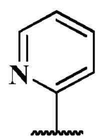 , 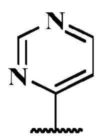

and

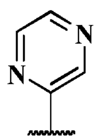 ;

$R_a$, $R_b$ and $R_c$ are each independently selected from the group consisting of hydrogen, $C_{1-6}$ alkyl, $C_{3-6}$ cycloalkyl, 3 to 12 membered heterocyclyl, $C_{6-14}$ aryl and 5 to 14 membered heteroaryl, which is optionally further substituted by one or more of halogen, hydroxy, $C_{1-6}$ alkyl, $C_{3-6}$ cycloalkyl, 3 to 12 membered heterocyclyl, $C_{6-14}$ aryl and 5 to 14 membered heteroaryl.

**22.** The compound, a stereoisomer thereof or a pharmaceutically acceptable salt thereof according to claim 19 or 20, **characterized in that** $R_4$ is selected from the group consisting of $-NR_cC(O)R_d$, $-NR_cC(O)C{=}CR_d$ and $-NR_cC(O)CH{=}CH(CH_2)_nR_d$; wherein $R_c$ and $R_d$ are each independently selected from the group consisting of hydrogen, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-6}$ cycloalkyl, 3 to 12 membered heterocyclyl, $C_{6-14}$ aryl and 5 to 14 membered heteroaryl, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl.

**23.** The compound, a stereoisomer thereof or the pharmaceutically acceptable salt thereof according to claim 19 or 20, **characterized in that** $R_4$ is selected from the group consisting of $-NR_cC(O)R_d$, $-NR_cC(O)C{=}CR_d$, $-NR_cC(O)CH{=}CH(CH_2)_nR_d$ and $-NR_cC(O)CR_c{=}CH(CH_2)_nR_d$; and preferably selected from the group consisting of $-NHC(O)CH{=}CH_2$, $-NHC(O)CF{=}CH_2$, $-NHC(O)C(CH_3){=}CH_2$, $-NHC(O)C{\equiv}CH$, $-NHC(O)C{=}CCH_3$, $-NHC(O)CH{=}CHCH_3$, $-NHC(O)CH{=}CHCH_2N(CH_3)_2$ and

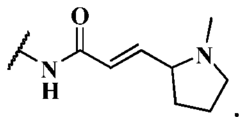.

**24.** The compound, a stereoisomer thereof or a pharmaceutically acceptable salt thereof according to claim 19 or 20, **characterized in that** $R_5$ is selected from the group consisting of hydrogen, $C_{1-6}$ alkyl, 3 to 12 membered heterocyclyl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl and 3 to 12 membered heterocyclyl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl;

$R_5$ is preferably selected from the group consisting of:

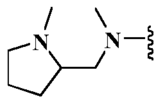, 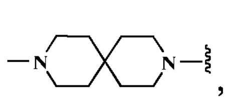, 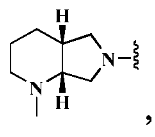, 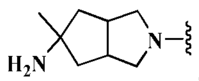, 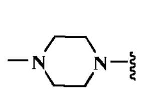, 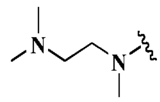,

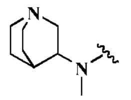, 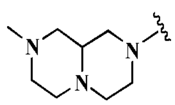, 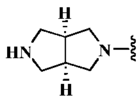, 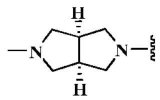, 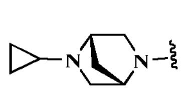, 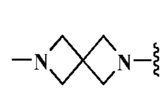,

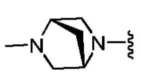, 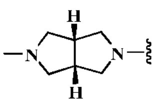, 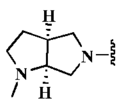, 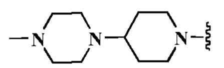, 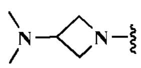, 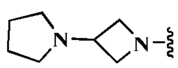,

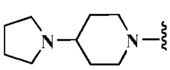, 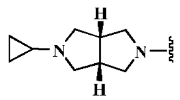, 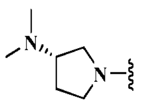, 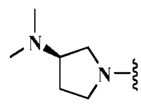, 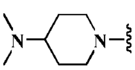, 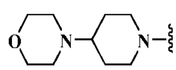

and

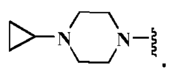.

**25.** The compound, a stereoisomer thereof or a pharmaceutically acceptable salt thereof according to claim 19 or 20, **characterized in that** $R_5$ is selected from the group consisting of hydrogen, $C_{1-6}$ alkyl, 3 to 12 membered heterocyclyl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl and 3 to 12 membered heterocyclyl are each optionally further substituted by deuterium, halogen, amino, cyano, $C_{1-4}$ alkyl, $C_{1-6}$ alkoxy, $C_{1-6}$ haloalkoxy, $C_{1-6}$ haloalkyl, $C_{2-6}$ alkenylcarbonyl, $C_{1-6}$ hydroxyalkyl, $C_{3-8}$ cycloalkyl, 3 to 12 membered heterocyclyl, $C_{6-14}$ aryl or 5 to 14 membered heteroaryl; and further selected from the group consisting of: methyl,

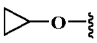, 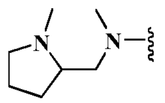, 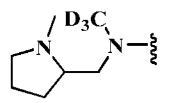, 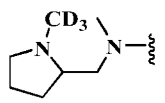, 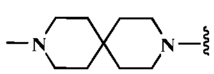,

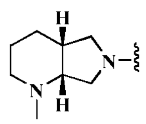, 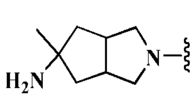, 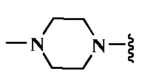, 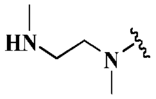, 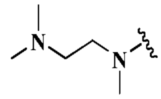, 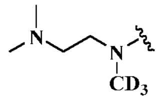,

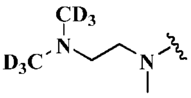, 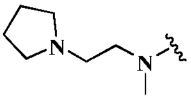, 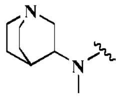, 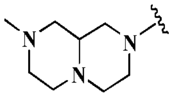, 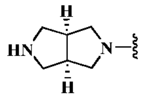, 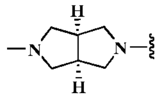,

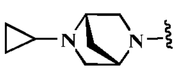, 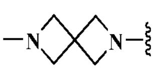, 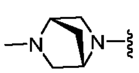, 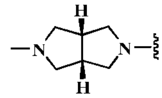, 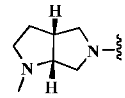, 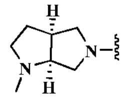,

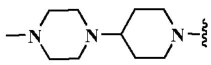, 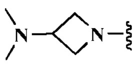, 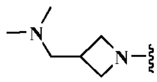, 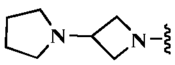, 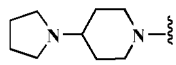, 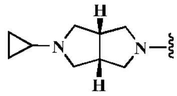,

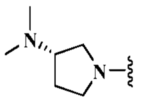, 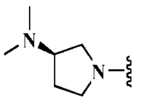, 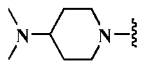, 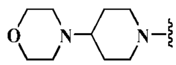, 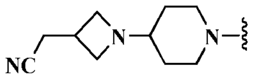,

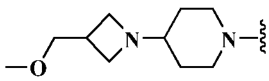

and

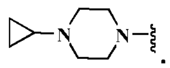.

**26.** The compound, a stereoisomer thereof or a pharmaceutically acceptable salt thereof according to claim 19 or 20, **characterized in that** $R_5$ is selected from the group consisting of substituted or unsubstituted 3 to 12 membered heterocyclyl, and more preferably selected from the group consisting of

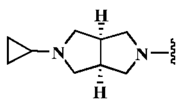

and

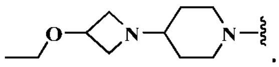.

**27.** The compound, a stereoisomer thereof or a pharmaceutically acceptable salt thereof according to claim 19 or 20, **characterized in that** $R_6$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl and 5 to 14 membered heteroaryl.

**28.** The compound, a stereoisomer thereof or a pharmaceutically acceptable salt thereof according to claim 19 or 20, **characterized in that** $R_6$ is selected from the group consisting of hydrogen, deuterium, halogen, cyano, amino, hydroxy, $C_{1-6}$ alkyl, $C_{2-6}$ alkenyl, $C_{2-6}$ alkynyl, $C_{1-6}$ alkoxy, $C_{1-6}$ haloalkoxy, $C_{1-6}$ hydroxyalkyl, $C_{3-8}$ cycloalkyl, 3 to 12 membered heterocyclyl, $C_{6-14}$ aryl, 5 to 14 membered heteroaryl and $-O(CH_2)_nR_{aa}$, preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl, $C_{3-6}$ cycloalkyl, 3 to 10 membered heterocyclyl, $C_{6-12}$ aryl, 5 to 12 membered heteroaryl and $-O(CH_2)_nR_{aa}$, more preferably selected from the group consisting of hydrogen, deuterium, halogen, cyano, amino, hydroxy, $C_{1-3}$ alkyl, $C_{2-5}$ alkenyl, $C_{2-5}$ alkynyl, $C_{1-3}$ alkoxy, $C_{1-3}$ haloalkoxy, $C_{1-3}$ hydroxyalkyl,

$C_{3-6}$ cycloalkyl, 3 to 8 membered heterocyclyl, $C_{6-10}$ aryl, 5 to 10 membered heteroaryl and $-O(CH_2)_nR_{aa}$, and further preferably selected from the group consisting of hydrogen, deuterium, methyl, ethyl, propyl, methoxy, ethoxy, propoxy, $-OCH_2F$, $-OCHF_2$ and $-OCF_3$.

**29.** The compound, a stereoisomer thereof or a pharmaceutically acceptable salt thereof according to claim 2, **characterized in that** the formula (I) is further shown as formula (III):

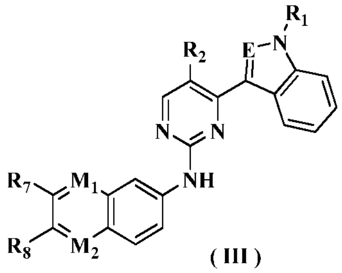

(III)

wherein:

$M_1$ and $M_2$ are each independently selected from the group consisting of $CR_9$ and N;
$R_7$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{=}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)_nP(O)R_{aa}R_{bb}$, $-NR_{aa}S(O)_mR_{bb}$, $-(CH_2)nS(O)_mNR_{aa}R_{bb}$, $-(CH_2)_nC(O)R_{aa}$, $-NR_{aa}C(O)OR_{bb}$, $-(CH_2)nS(O)_mR_{aa}$ and $-(CH_2)_nNR_{aa}S(O)_mR_{bb}$;
Rg is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-O(CH_2)_nR_{aa}$, $-NR_{aa}(CH_2)_nR_{bb}$ and $-NR_{aa}(CH_2)_nNR_{bb}R_{cc}$, wherein the $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl and 5 to 14 membered heteroaryl are each optionally further substituted by one or more substituents selected from the group consisting of halogen, hydroxy, thiol, $C_{1-6}$ alkyl, $C_{1-6}$ haloalkyl, $C_{1-6}$ alkoxy, $C_{1-6}$ hydroxyalkyl and $C_{3-8}$ cycloalkyl;
$R_9$ is selected from the group consisting of hydrogen, deuterium, halogen, cyano, amino, nitro, hydroxy, azido, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-NR_{aa}C(O)NR_{bb}(CH_2)_nR_{cc}$, $-NR_{aa}C(O)C{=}CR_{bb}$, $-NR_{aa}C(O)CH{=}CH(CH_2)_nR_{bb}$, $-NR_{aa}C(O)CH{=}CH(CH_2)_nNR_{bb}R_{cc}$, $-C(O)NR_{aa}(CH_2)_nR_{bb}$, $-C(O)OR_{aa}$, $-O(CH_2)_nR_{aa}$, $-(CH_2)nP(O)R_{aa}R_{bb}$, $-NR_{aa}S(O)_mR_{bb}$, $-(CH_2)nS(O)_mNR_{aa}R_{bb}$, $-(CH_2)_nC(O)R_{aa}$, $-NR_{aa}C(O)OR_{bb}$, $-(CH_2)nS(O)_mR_{aa}$ and $-(CH_2)_nNR_{aa}S(O)_mR_{bb}$.

**30.** The compound, a stereoisomer thereof or a pharmaceutically acceptable salt thereof according to claim 2, **characterized in that** the formula (I) is further shown as formula (IV):

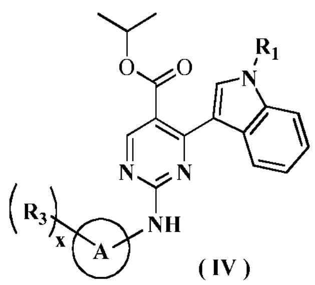

(IV) .

**31.** The compound, a stereoisomer thereof or a pharmaceutically acceptable salt thereof according to claim 20, **characterized in that** the formula (I) is further shown as shown formula (V):

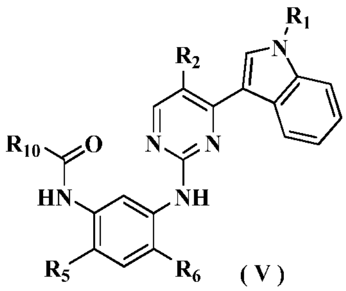

( V )

wherein:

$R_{10}$ is selected from the group consisting of hydrogen, deuterium, halogen, cyano, amino, nitro, hydroxy, $C_{1-6}$ alkyl, $C_{2-6}$ alkenyl, $C_{2-6}$ alkynyl, $C_{1-6}$ alkoxy, $C_{1-6}$ hydroxyalkyl, $C_{3-8}$ cycloalkyl, 3 to 12 membered heterocyclyl, $C_{6-14}$ aryl, 5 to 14 membered heteroaryl, $-C\equiv CR_{aa}$, $-CH=CH(CH_2)_nR_{aa}$, $-CR_{aa}=CH(CH_2)_nR_{bb}$ and $-CH=CH(CH_2)_nNR_{aa}R_{bb}$.

**32.** The compound, a stereoisomer thereof or a pharmaceutically acceptable salt thereof according to claim 20, **characterized in that** the formula (I) is further shown as formula (VI):

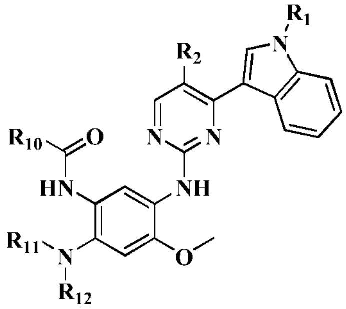

( VI )

wherein:

$R_{11}$ and $R_{12}$ are each independently selected from the group consisting of hydrogen, deuterium, halogen, cyano, amino, nitro, hydroxy, substituted or unsubstituted $C_{1-6}$ alkyl, $C_{2-6}$ alkenyl, $C_{2-6}$ alkynyl, $C_{1-6}$ alkoxy, $C_{1-6}$ hydroxyalkyl, substituted or unsubstituted $C_{3-8}$ cycloalkyl, 3 to 12 membered heterocyclyl, $C_{6-14}$ aryl, 5 to 14 membered heteroaryl, $-(CH_2)_nR_{aa}$ and $-(CH_2)_nNR_{aa}R_{bb}$, preferably selected from the group consisting of hydrogen, deuterium, halogen, cyano, amino, nitro, hydroxy, substituted or unsubstituted $C_{1-3}$alkyl, $C_{2-5}$ alkenyl, $C_{2-5}$ alkynyl, $C_{1-3}$ alkoxy, $C_{1-3}$ hydroxyalkyl, substituted or unsubstituted $C_{3-6}$ cycloalkyl, 3 to 10 membered heterocyclyl, $C_{6-12}$ aryl, 5 to 12 membered heteroaryl, $-(CH_2)_nR_{aa}$ and $-(CH_2)_nNR_{aa}R_{bb}$, more preferably selected from the group consisting of hydrogen, deuterium, halogen, cyano, amino, nitro, hydroxy, $C_{1-3}$ alkyl substituted by one or more substituents selected from the group consisting of deuterium, fluorine, chlorine and bromine atom, $C_{2-5}$ alkenyl, $C_{2-5}$ alkynyl, $C_{1-3}$ alkoxy, $C_{1-3}$ hydroxyalkyl, $C_{3-6}$ cycloalkyl substituted by one or more substituents selected from the group consisting of deuterium, fluorine, chlorine and bromine atom, 3 to 10 membered heterocyclyl containing 1 to 3 atoms selected from the group consisting of N, O or and S, $C_{6-12}$ aryl, 5 to 12 membered heteroaryl containing 1 to 3 atoms selected from the group consisting of N, O or and S, $-(CH_2)_nR_{aa}$ and $-(CH_2)_nNR_{aa}R_{bb}$, and further preferably selected from the group consisting of hydrogen, methyl, ethyl, propyl, tri-deuterated methyl, $-CH_2F$, $-CHF_2$, $-CF_3$,

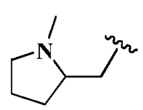 , 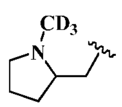 , 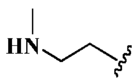 ,

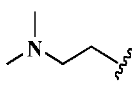 , 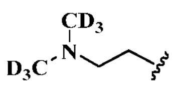 , 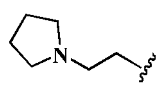

and

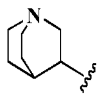;

or, $R_{11}$ and $R_{12}$, together with the nitrogen atom to which they are attached, form a 3 to 12 membered heterocyclyl or 5 to 10 membered heteroaryl, wherein the 3 to 12 membered heterocyclyl or 5 to 10 membered heteroaryl is optionally substituted by one or more substituents selected from the group consisting of deuterium, halogen, amino, hydroxy, cyano, nitro, oxo, $C_{1-6}$ alkyl, $C_{2-6}$ alkenyl, $C_{2-6}$ alkynyl, deuterated $C_{1-6}$ alkyl, $C_{1-6}$ haloalkyl, $C_{1-6}$ alkoxy, $C_{1-6}$ haloalkoxy, $C_{1-6}$ hydroxyalkyl, $C_{3-12}$ cycloalkyl, substituted or unsubstituted 3 to 12 membered heterocyclyl, $C_{6-14}$ aryl, 5 to 14 membered heteroaryl and $-(CH_2)_nNR_{aa}R_{bb}$; and preferably further form a group selected from the group consisting of:

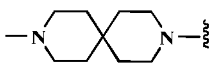, 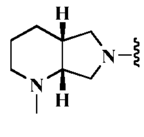, 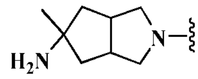, 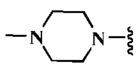,

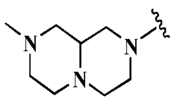, 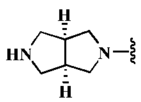, 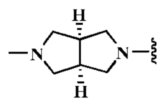, 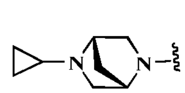, 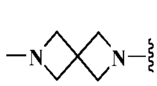, 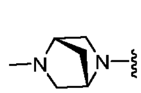,

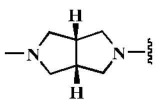, 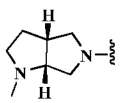, 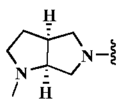, 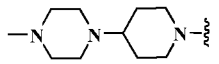, 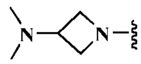, 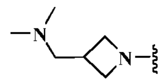,

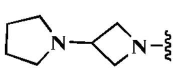, 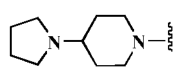, 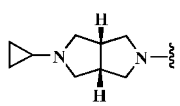, 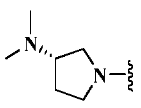, 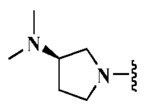, 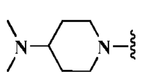,

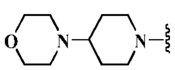, 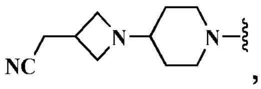, 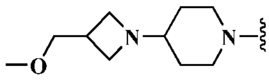

and

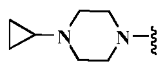.

**33.** The compound, a stereoisomer thereof or a pharmaceutically acceptable salt thereof according to claim 19, **characterized in that** the compound is further shown as formula (VII):

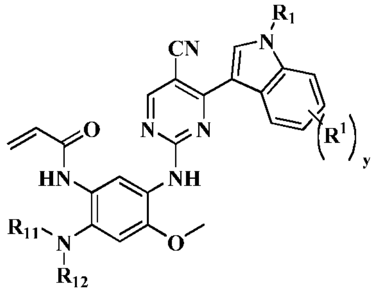

(VII)

wherein:

$R_1$ is selected from the group consisting of hydrogen, deuterium, halogen, amino, nitro, hydroxy, cyano, $C_{1-6}$ alkyl, $C_{3-8}$ cycloalkyl and 3 to 6 membered heterocyclyl, which is optionally further substituted by one or more substituents selected from the group consisting of deuterium, halogen, amino, nitro, hydroxy, cyano, $C_{1-6}$ alkyl, $C_{3-8}$ cycloalkyl and 3 to 6 membered heterocyclyl; preferably, $R_1$ is selected from the group consisting of $C_{1-3}$ alkyl, $C_{3-6}$ cycloalkyl and 3 to 6 membered heterocyclyl containing 1 to 2 atoms selected from the group consisting of N, O and S, which is optionally further substituted by one or more substituents selected from the group consisting of deuterium, halogen, amino, nitro, hydroxy, cyano, $C_{1-3}$ alkyl, $C_{3-6}$ cycloalkyl and 3 to 6 membered heterocyclyl; and more preferably, $R_1$ is selected from the group consisting of methyl, ethyl, propyl, isopropyl, cyclopropyl, cyclobutyl, cyclopentyl, cyclohexyl, cyclopropoxy, cyclobutoxy, tetrahydrofuranyl, pyranyl, aziridinyl, azetidinyl, pyrrolyl, piperidinyl and tetrahydrothienyl, which is optionally further substituted by one or more substituents selected from the group consisting of deuterium, fluorine, chlorine, bromine, amino, nitro, hydroxy, cyano, methyl, ethyl, propyl and isopropyl;

$R_{11}$ and $R_{12}$ are each independently selected from the group consisting of hydrogen, deuterium, halogen, cyano, amino, nitro, hydroxy, $C_{1-6}$ alkyl and $-(CH_2)_nNR_{aa}R_{bb}$;

$R_{aa}$ and $R_{bb}$ are each independently selected from the group consisting of hydrogen, deuterium, halogen, cyano, amino, nitro, hydroxy, $C_{1-6}$ alkyl, $C_{1-6}$ alkoxy and 3 to 6 membered heterocyclyl; and preferably, $R_{aa}$ and $R_{bb}$ are each independently selected from the group consisting of hydrogen, deuterium, halogen, cyano, amino, nitro, hydroxy, methyl, ethyl, propyl, isopropyl, methoxy, ethoxy, propoxy, isopropoxy and 5 to 6 membered heterocyclyl containing 1 to 2 atoms selected from the group consisting of N and O atom;

n is an integer of 0, 1,2 or 3;

or, $R_{11}$ and $R_{12}$, together with the nitrogen atom to which they are attached, form a 4 to 12 membered heterocyclyl, which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;

each $R^1$ is independently selected from the group consisting of hydrogen, deuterium, halogen, amino, nitro, hydroxy, cyano, $C_{1-6}$ alkyl, $C_{1-6}$ alkoxy, $C_{1-6}$ hydroxyalkyl and $C_{3-8}$ cycloalkyl; and preferably, $R^1$ is selected from the group consisting of hydrogen, deuterium, fluorine, chlorine, bromine, amino, nitro, hydroxy, cyano, methyl, ethyl, propyl, isopropyl, hydroxymethyl, hydroxyethyl, hydroxypropyl, isohydroxypropyl, methoxy, ethoxy, propoxy, isopropoxy, cyclopropyl, cyclobutyl, cyclopentyl and cyclohexyl;

y is an integer of 0, 1,2 or 3.

**34.** The compound, a stereoisomer thereof or a pharmaceutically acceptable salt thereof according to claim 19, **characterized in that** the compound is further shown as formula (VIII):

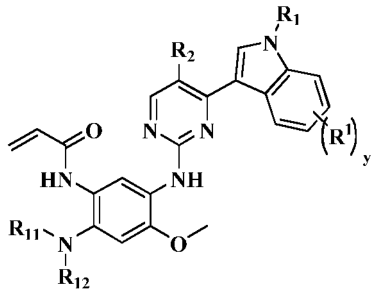

( VIII)

wherein:

$R_2$ is selected from the group consisting of $C_{6-12}$ aryl, 4 to 7 membered heterocyclyl and 4 to 7 membered heteroaryl, which is optionally further substituted by one or more substituents selected from the group consisting of hydroxy, amino, halogen, $C_{1-6}$ alkyl, $C_{1-6}$ hydroxyalkyl and $C_{1-6}$ alkoxy; preferably, $R_2$ is selected from the group consisting of phenyl, 5 to 6 membered heterocyclyl containing 1 to 2 atoms selected from the group consisting of N, O and S and 5 to 6 membered heteroaryl containing 1 to 2 atoms selected from the group consisting of N, O or and S, which is optionally further substituted by one or more substituents selected from the group consisting of hydroxy, amino, halogen, $C_{1-3}$ alkyl, $C_{1-3}$ hydroxyalkyl and $C_{1-3}$ alkoxy; and more preferably, $R_2$ is selected from the group consisting of:

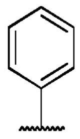, 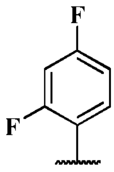, 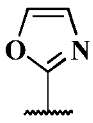, 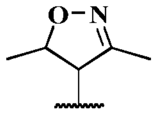, 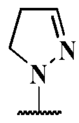, 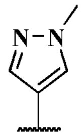, 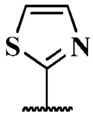, 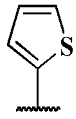, 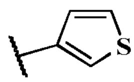,

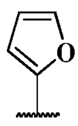, 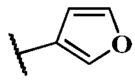, 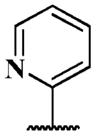, 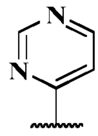 and 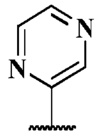,

which is optionally further substituted by one or more substituents selected from the group consisting of hydroxy, amino, halogen, methyl, ethyl, propyl, isopropyl, hydroxymethyl, hydroxyethyl, hydroxypropyl, hydroxyisopropyl, methoxy, ethoxy, propoxy and isopropoxy;

$R_1$ is selected from the group consisting of hydrogen, deuterium, halogen, amino, nitro, hydroxy, cyano, $C_{1-6}$ alkyl, $C_{3-8}$ cycloalkyl and 3 to 6 membered heterocyclyl, which is optionally further substituted by one or more substituents selected from the group consisting of deuterium, halogen, amino, nitro, hydroxy, cyano, $C_{1-6}$ alkyl, $C_{3-8}$ cycloalkyl and 3 to 6 membered heterocyclyl; preferably, $R_1$ is selected from the group consisting of $C_{1-3}$ alkyl, $C_{3-6}$ cycloalkyl and 3 to 6 membered heterocyclyl containing 1 to 2 atoms selected from the group consisting of N, O or and S, which is optionally further substituted by one or more substituents selected from the group consisting of deuterium, halogen, amino, nitro, hydroxy, cyano, $C_{1-3}$ alkyl, $C_{3-6}$ cycloalkyl and 3 to 6 membered heterocyclyl; and more preferably, $R_1$ is selected from the group consisting of methyl, ethyl, propyl, isopropyl, cyclopropyl, cyclobutyl, cyclopentyl, cyclohexyl, cyclopropoxy, cyclobutoxy, tetrahydrofuranyl, pyranyl, aziridinyl, azetidinyl, pyrrolyl, piperidinyl and tetrahydrothienyl, which is optionally further substituted by one or more substituents selected from the group consisting of deuterium, fluorine, chlorine, bromine, amino, nitro, hydroxy, cyano, methyl, ethyl, propyl and isopropyl;
$R_{11}$ and $R_{12}$ are each independently selected from the group consisting of hydrogen, deuterium, halogen, cyano, amino, nitro, hydroxy, $C_{1-6}$ alkyl and $-(CH_2)_nNR_{aa}R_{bb}$;
$R_{aa}$ and $R_{bb}$ are each independently selected from the group consisting of hydrogen, deuterium, halogen, cyano, amino, nitro, hydroxy, $C_{1-6}$ alkyl, $C_{1-6}$ alkoxy and 3 to 6 membered heterocyclyl; and preferably, $R_{aa}$ and $R_{bb}$ are each independently selected from the group consisting of hydrogen, deuterium, halogen, cyano, amino, nitro, hydroxy, methyl, ethyl, propyl, isopropyl, methoxy, ethoxy, propoxy, isopropoxy and 5 to 6 membered heterocyclyl containing 1 to 2 atoms selected from the group consisting of N and O;
n is an integer of 0, 1,2 or 3;
or, $R_{11}$ and $R_{12}$, together with the nitrogen atom to which they are attached, form a 4 to 12 membered heterocyclyl, which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;
each $R^1$ is independently selected from the group consisting of hydrogen, deuterium, halogen, amino, nitro, hydroxy, cyano, $C_{1-6}$ alkyl, $C_{1-6}$ alkoxy, $C_{1-6}$ hydroxyalkyl and $C_{3-8}$ cycloalkyl; and preferably, $R^1$ is selected from the group consisting of hydrogen, deuterium, fluorine, chlorine, bromine, amino, nitro, hydroxy, cyano, methyl, ethyl, propyl, isopropyl, hydroxymethyl, hydroxyethyl, hydroxypropyl, isohydroxypropyl, methoxy, ethoxy, propoxy, isopropoxy, cyclopropyl, cyclobutyl, cyclopentyl and cyclohexyl.

**35.** The compound, a stereoisomer thereof or a pharmaceutically acceptable salt thereof according to any one of claims 32 to 34, **characterized in that** $R_{11}$ and $R_{12}$, together with the nitrogen atom to which they are attached, form a 4 to 10 membered heterocyclyl, which is optionally substituted by one or more substituents selected from the group consisting of halogen, hydroxy, amino, $C_{1-6}$ alkyl, $C_{1-6}$ alkoxy, amino substituted by $C_{1-6}$ alkyl, $C_{3-12}$ cycloalkyl and substituted or unsubstituted 3 to 12 membered heterocyclyl;

preferably, $R_{11}$ and $R_{12}$, together with the nitrogen atom to which they are attached , form a 4 to 8 membered heterocyclyl containing 1 to 2 nitrogen atom, which is optionally substituted by one or more substituents selected from the group consisting of halogen, hydroxy, amino, $C_{1-3}$ alkyl, $C_{1-3}$ alkoxy, amino substituted by $C_{1-3}$ alkyl, $C_{3-6}$ cycloalkyl and substituted or unsubstituted 3 to 6 membered monocyclic heterocyclyl;
preferably further form a group selected from the group consisting of:

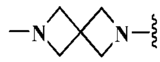,

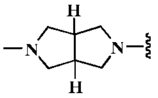, 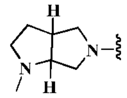, 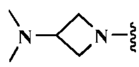, 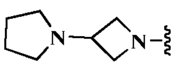, 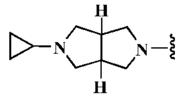 and

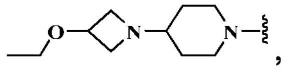,

which is optionally substituted by one or more substituents selected from the group consisting of halogen, hydroxy, amino, methyl, ethyl, propyl, isopropyl, methoxy, ethoxy, propoxy, isopropoxy, methylamino, dimethylamino, ethylamino, diethylamino, propylamino, dipropylamino, isopropylamino, diisopropylamino, cyclopropyl, cyclobutyl, cyclopentyl, cyclohexyl and substituted or unsubstituted 3 to 6 membered monocyclic heterocyclyl containing one nitrogen atom.

**36.** The compound, a stereoisomer thereof or a pharmaceutically acceptable salt thereof according to any one of claims 1 to 35, **characterized in that** the compound has the following structure:

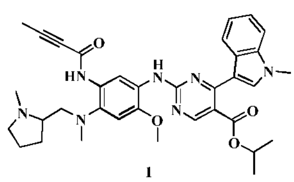
1

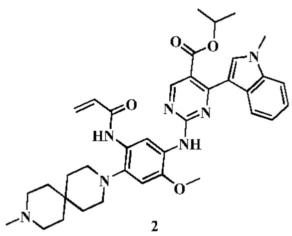
2

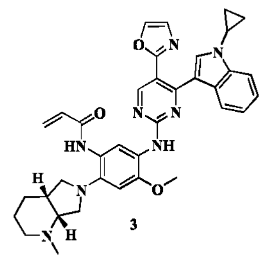
3

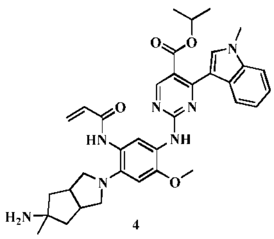
4

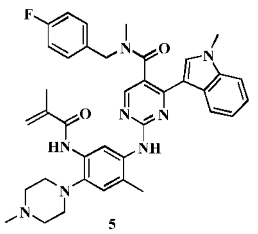
5

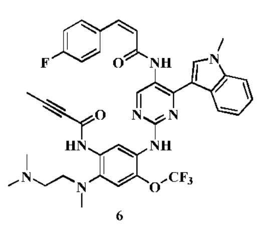
6

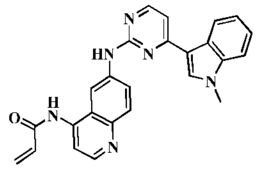
7

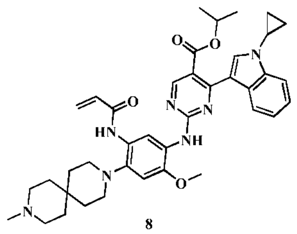
8

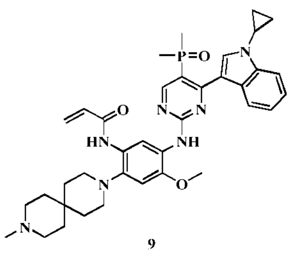
9

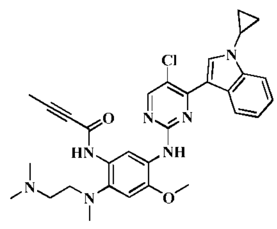
10

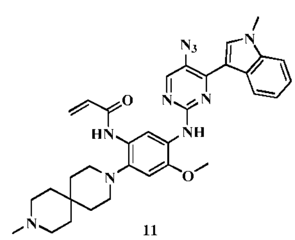
11

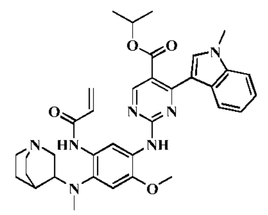
12

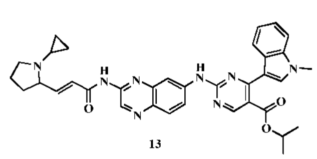
13

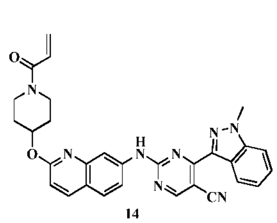
14

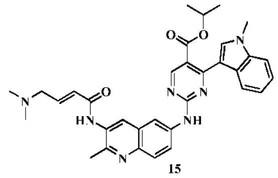
15

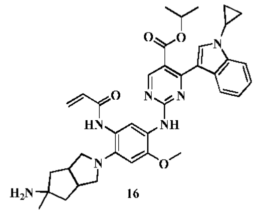
16

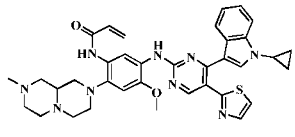
17

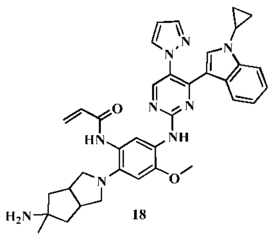
18

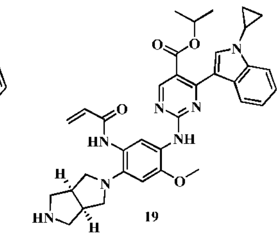
19

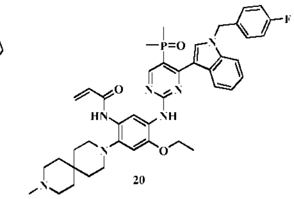
20

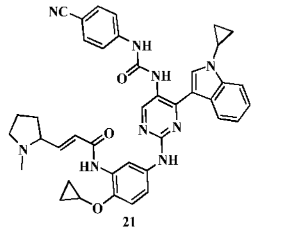
21

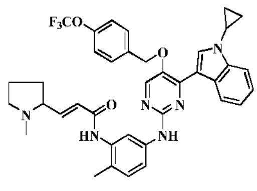
22

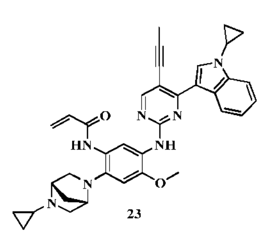
23

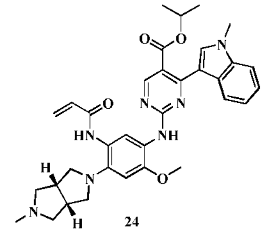
24

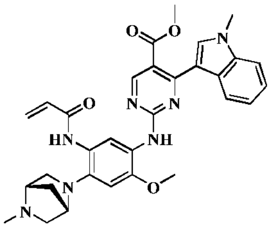
25

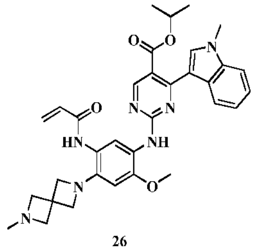
26

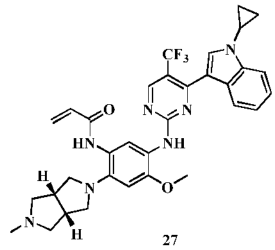
27

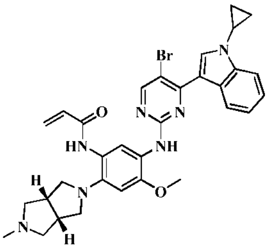
28

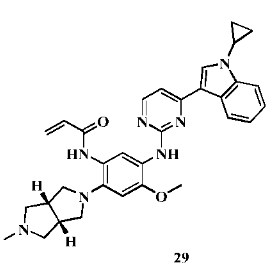
29

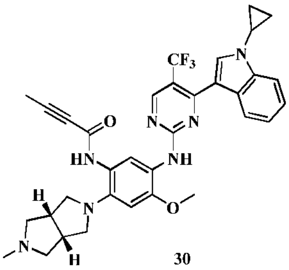
30

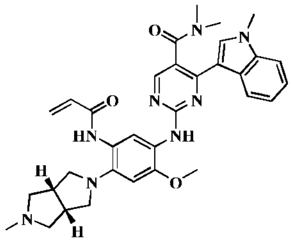
31

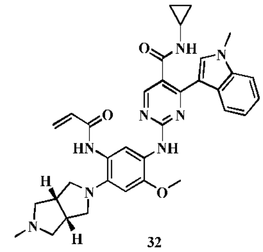
32

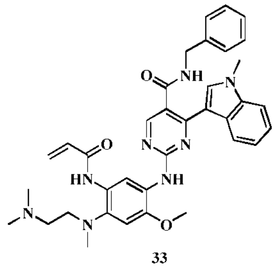
33

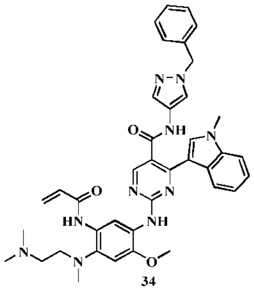
34

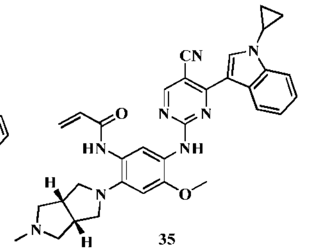
35

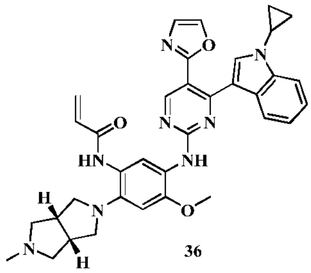
36

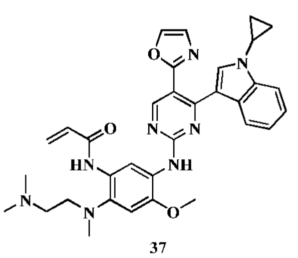
37

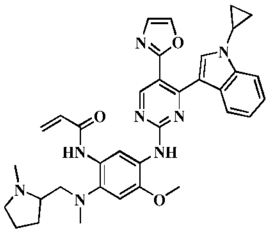
38

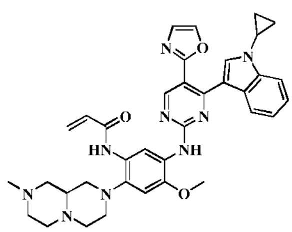
39

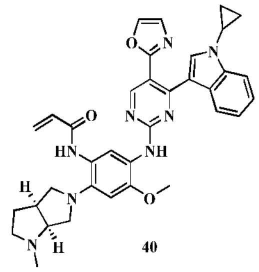
40

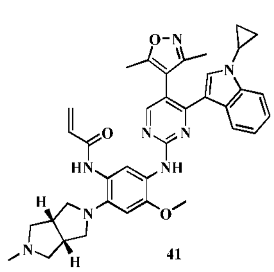
41

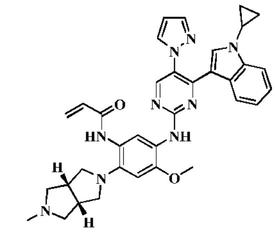
42

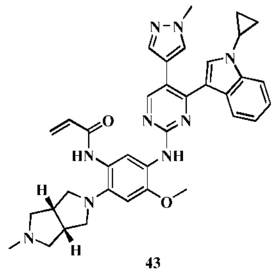
43

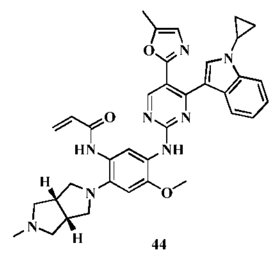
44

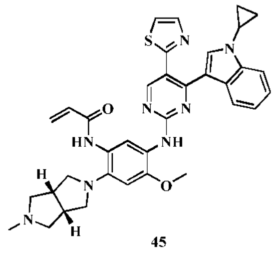
45

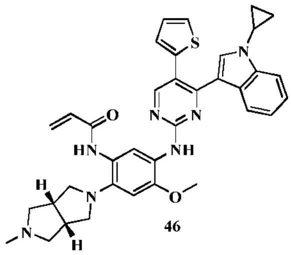
46

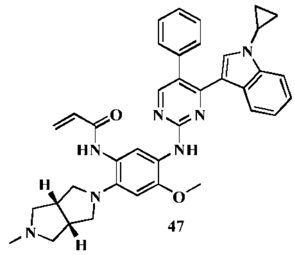
47

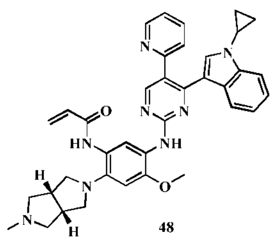
48

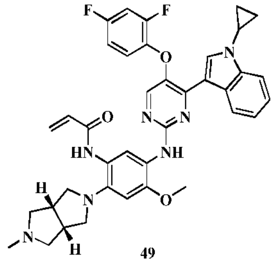
49

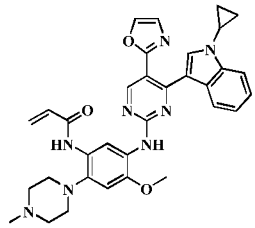
50

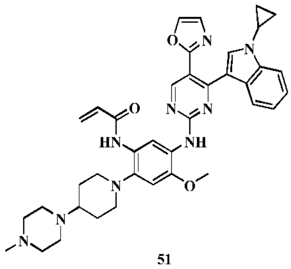
51

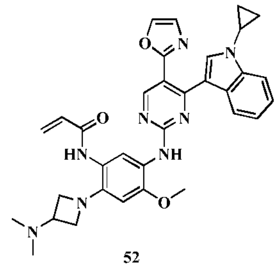
52

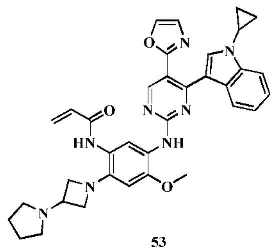
53

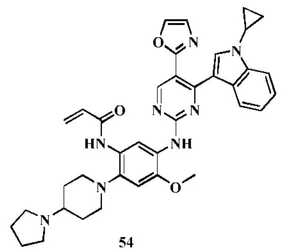
54

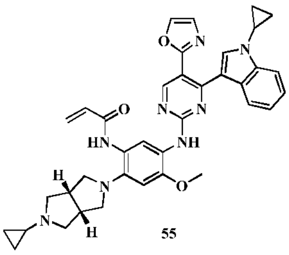
55

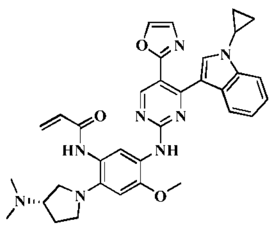
56

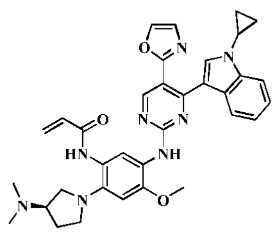
57

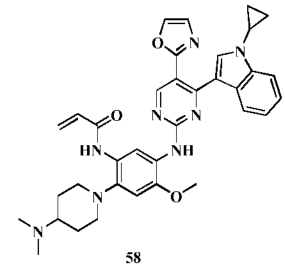
58

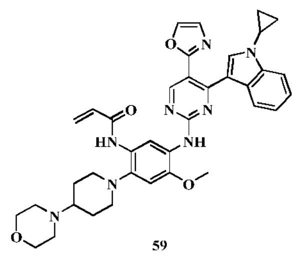
59

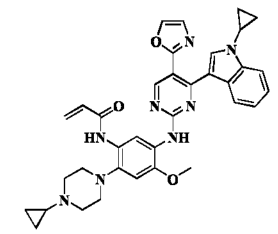
60

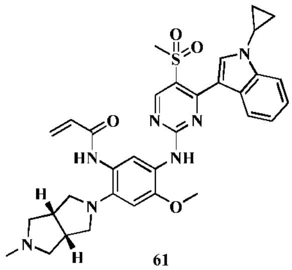
61

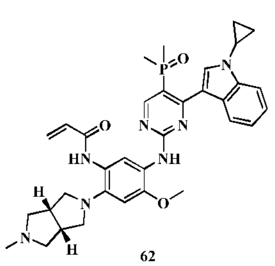
62

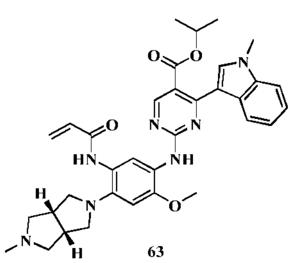
63

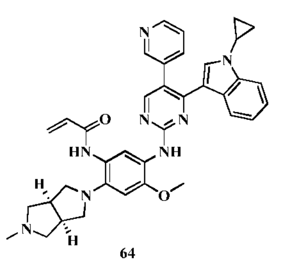
64

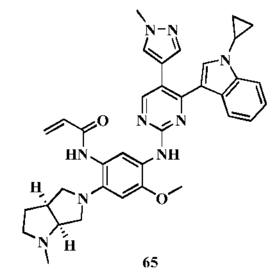
65

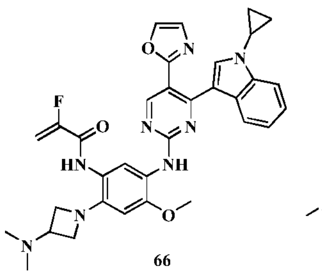
66

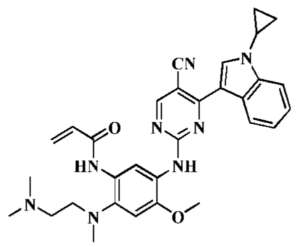
67

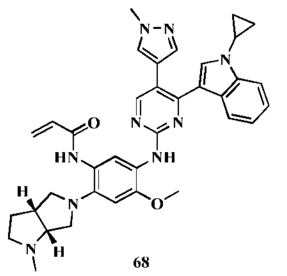
68

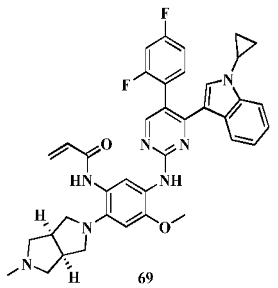
69

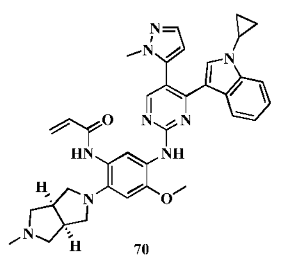
70

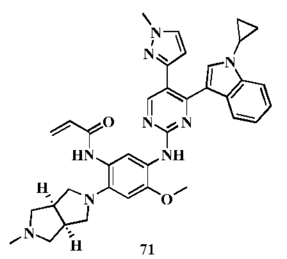
71

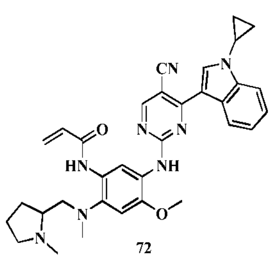
72

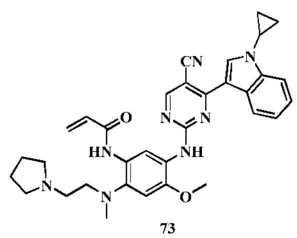
73

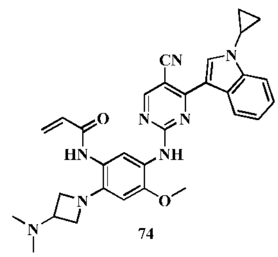
74

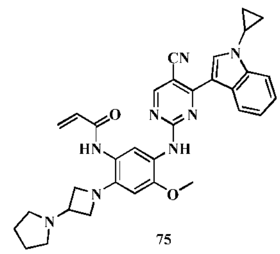
75

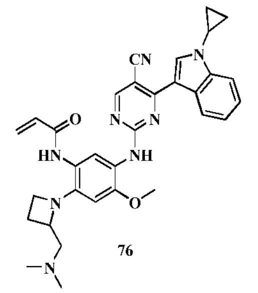
76

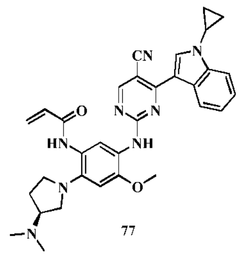
77

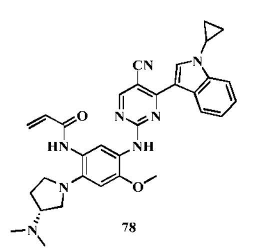
78

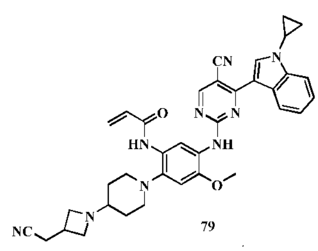
79

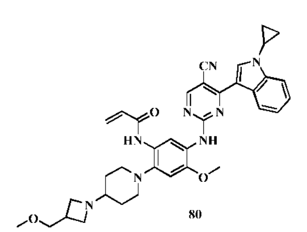
80

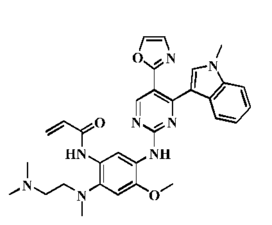

81

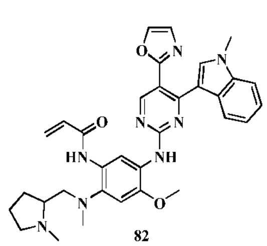

82

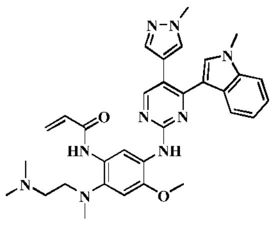

83

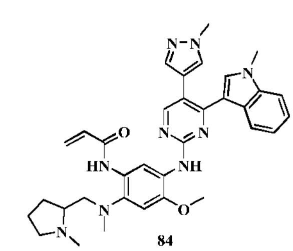

84

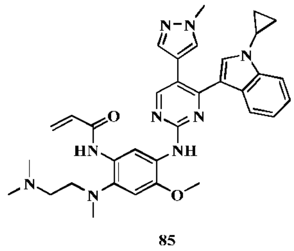

85

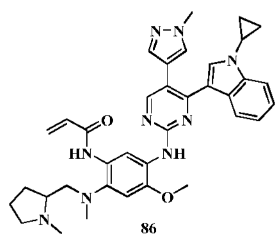

86

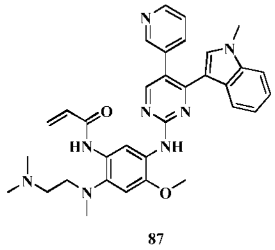

87

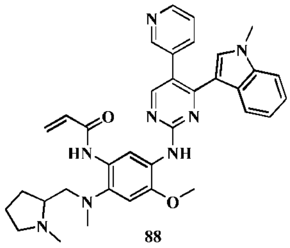

88

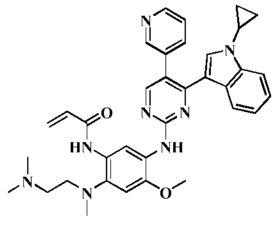

89

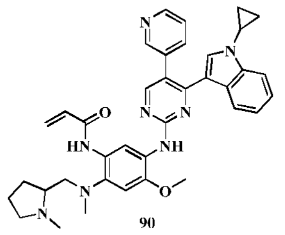

90

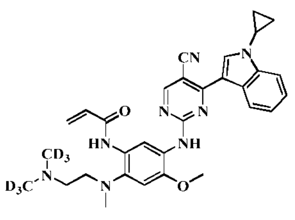

91

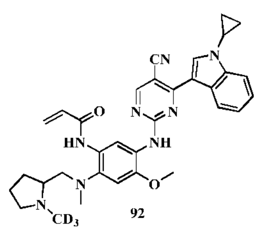

92

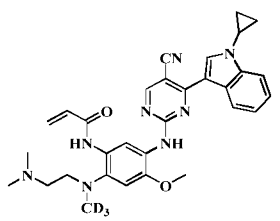

93

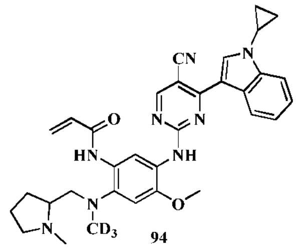

94

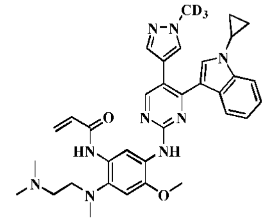

95

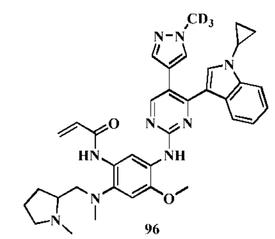

96

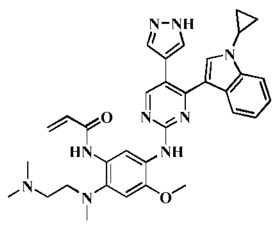

97

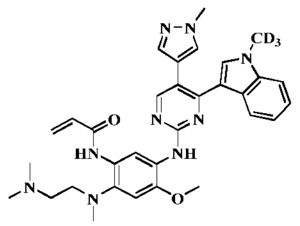

98

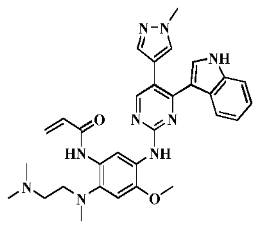

99

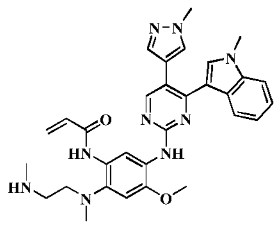

100

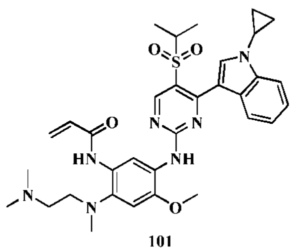

101

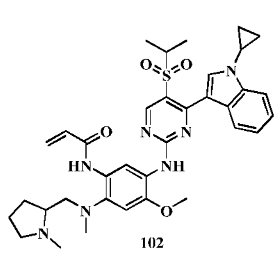

102

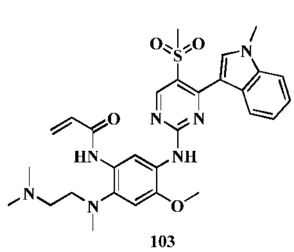

103

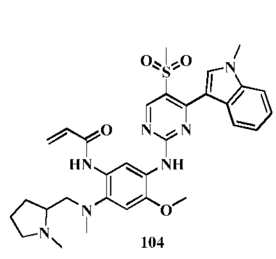

104

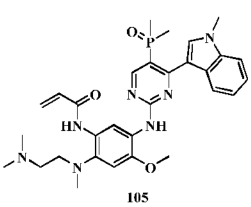
105

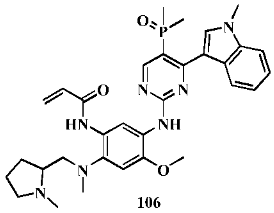
106

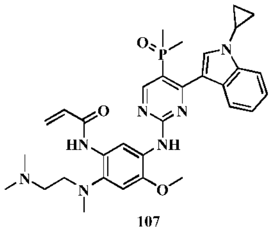
107

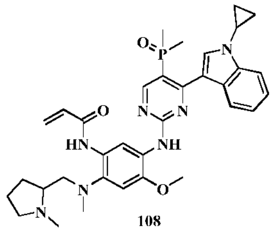
108

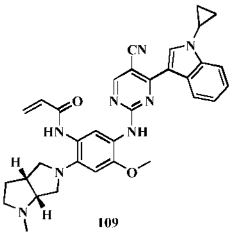
109

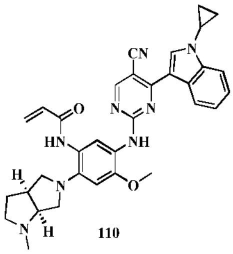
110

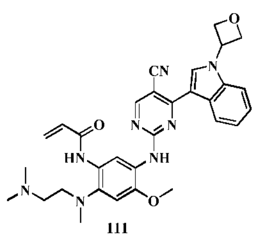
111

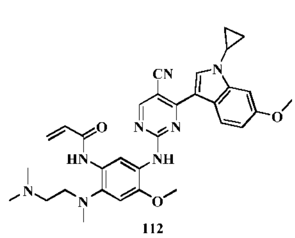
112

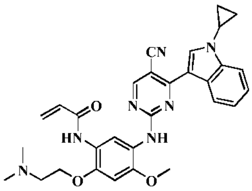
113

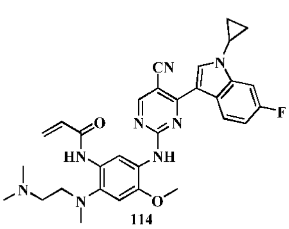
114

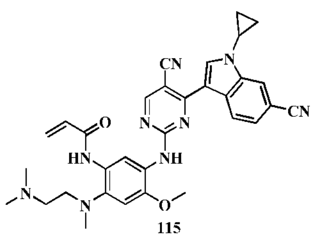
115

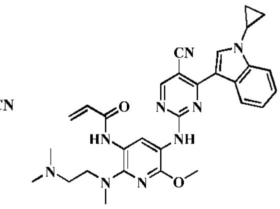
116

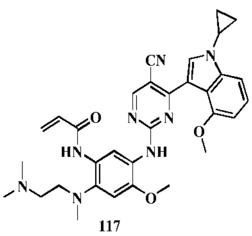
117

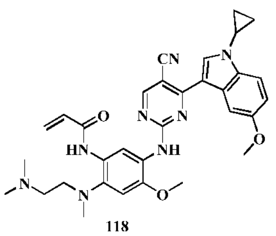
118

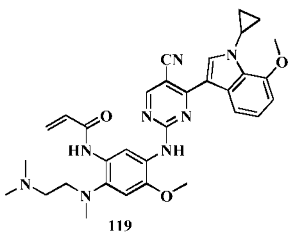
119

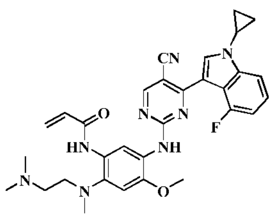
120

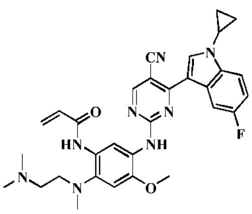
121

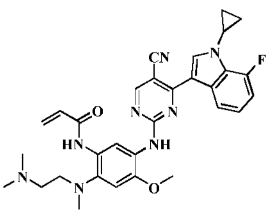
122

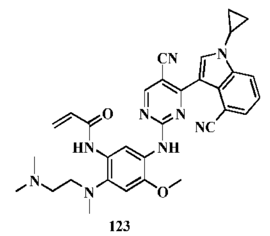
123

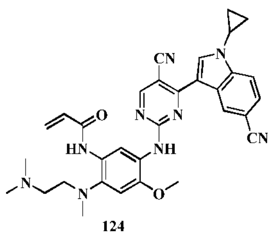
124

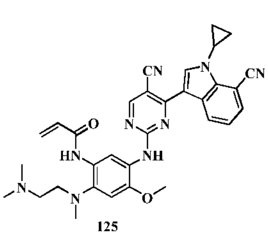
125

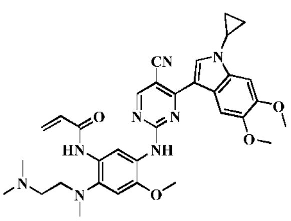
126

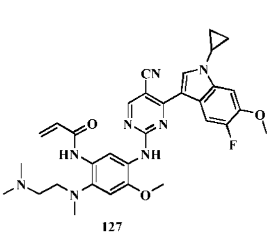
127

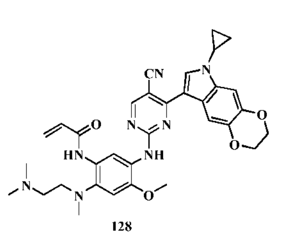
128

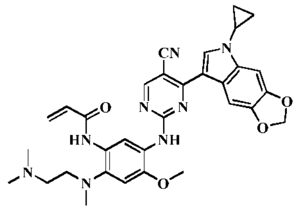
129

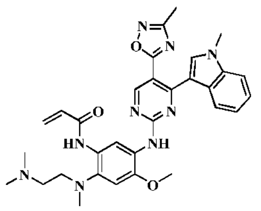
130

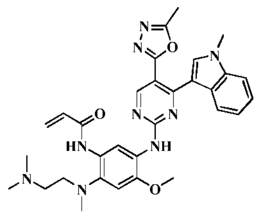
131

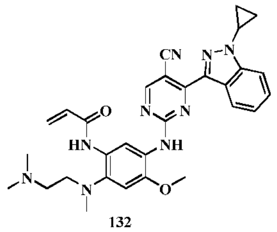
132

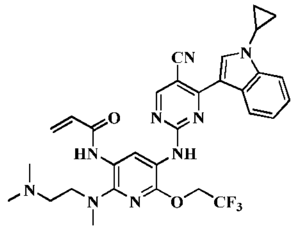
133

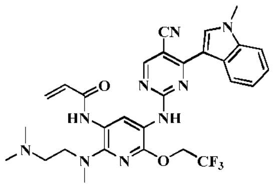
134

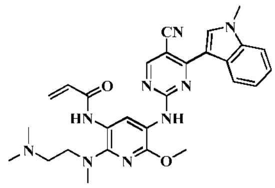
135

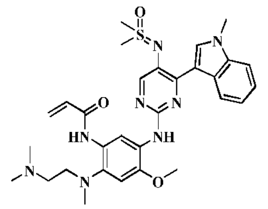
136

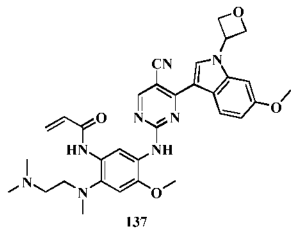
137

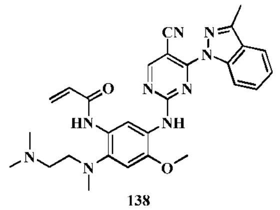
138

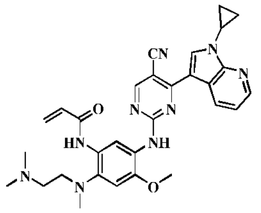
139

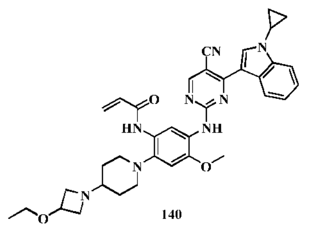
140

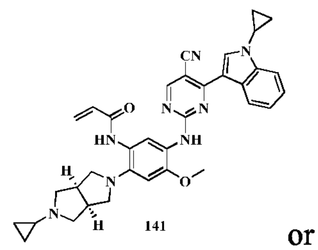
141

or

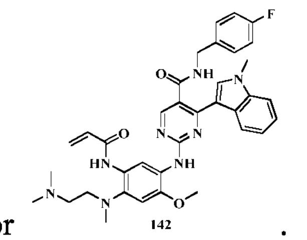
142

.

**37.** A method for preparing the compound of formula (V), a stereoisomer thereof or a pharmaceutically acceptable salt thereof according to claim 31, **characterized by** comprising the following steps of:

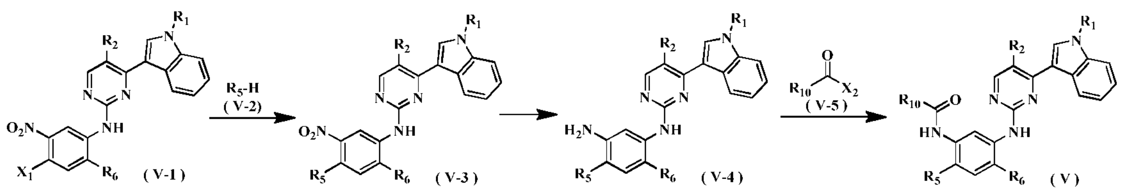


reacting a compound of formula (V-1) with a compound of formula (V-2) to obtain a compound of formula (V-3); then subjecting the compound of formula (V-3) to a reduction reaction to obtain a compound of formula (V-4); then reacting the compound of formula (V-4) with a compound of formula (V-5) to obtain the compound of formula (V), a stereoisomer thereof or a pharmaceutically acceptable salt thereof;
wherein:

$X_1$ is selected from the group consisting of halogen; preferably fluorine, chlorine, bromine or iodine; and more preferably fluorine;
$X_2$ is selected from the group consisting of halogen; preferably fluorine, chlorine, bromine or iodine; and more preferably chlorine;
$R_1$, $R_2$, $R_5$, $R_6$ and $R_{10}$ are as defined in claim 31.

**38.** The method for preparing the compound of formula (V), a stereoisomer thereof or a pharmaceutically acceptable salt thereof according to claim 37, **characterized by** comprising the following step of:

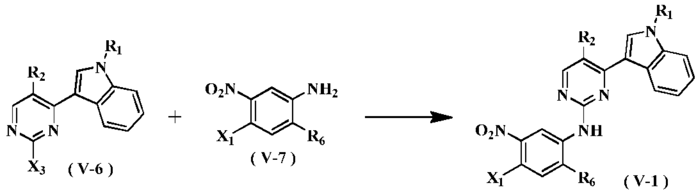


reacting a compound of formula (V-6) with a compound of formula (V-7) to obtain the compound of formula (V-1); then subjecting the compound of formula (V-1) to reactions to obtain the compound of formula (V), a stereoisomer thereof or a pharmaceutically acceptable salt thereof;
wherein:
$X_3$ is selected from the group consisting of halogen; preferably fluorine, chlorine, bromine or iodine; and more preferably chlorine.

**39.** A method for preparing the compound of formula (VII), a stereoisomer thereof or a pharmaceutically acceptable salt thereof according to claim 33, **characterized by** comprising the following steps of:

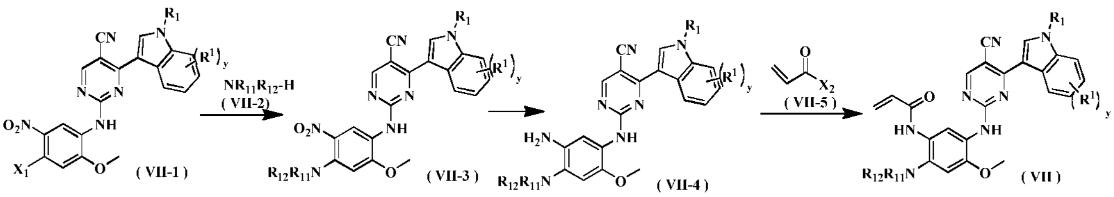


reacting a compound of formula (VII-1) with a compound of formula (VII-2) to obtain a compound of formula (VII-3); then subjecting the compound of formula (VII-3) to a reduction reaction to obtain a compound of formula (VII-4); then reacting the compound of formula (VII-4) with a compound of formula (VII-5) to obtain the compound of formula (VII), a stereoisomer thereof or a pharmaceutically acceptable salt thereof;
wherein:

$X_1$ is selected from the group consisting of halogen; preferably fluorine, chlorine, bromine or iodine; and more preferably fluorine;
$X_2$ is selected from the group consisting of halogen; preferably fluorine, chlorine, bromine or iodine; and more preferably chlorine;
$R^1$, $R_1$, $R_{11}$, $R_{12}$ and y are as defined in claim 33.

**40.** A method for preparing the compound of formula (VIII), a stereoisomer thereof or a pharmaceutically acceptable salt thereof according to claim 34, **characterized by** comprising the following steps of:

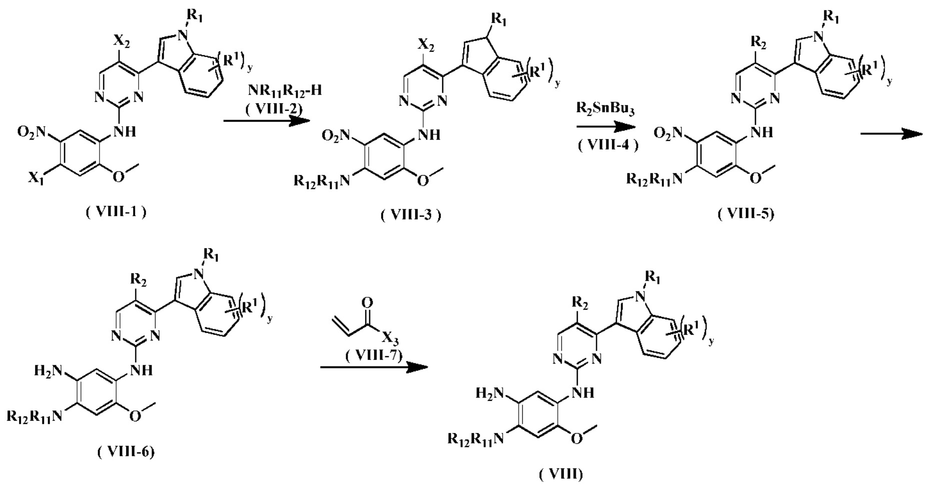


( VIII-1 ) ( VIII-3 ) ( VIII-5) ( VIII-6) ( VIII)

wherein:

$X_1$ is selected from the group consisting of halogen; preferably fluorine, chlorine, bromine or iodine; and more preferably fluorine;

$X_2$ and $X_3$ are each independently selected from the group consisting of halogen; preferably fluorine, chlorine, bromine or iodine; and more preferably bromine;

$R^1$, $R_1$, $R_2$, $R_{11}$, $R_{12}$ and y are as defined in claim 34.

**41.** A pharmaceutical composition comprising a therapeutically effective dose of the compound, a stereoisomer thereof or a pharmaceutically acceptable salt thereof according to claims 1 to 36, and one or more pharmaceutically acceptable carriers, diluents or excipients.

**42.** Use of the compound, a stereoisomer thereof or a pharmaceutically acceptable salt thereof according to any one of claims 1 to 36, or the pharmaceutical composition according to claim 41 in the preparation of a kinase inhibitor medicament.

**43.** The use according to claim 42, wherein the kinase inhibitor is a receptor tyrosine kinase inhibitor, preferably a HER2 inhibitor, EGFR inhibitor, EGFR monoclonal antibody or combined medicament thereof, and more preferably a HER2 exon 20 mutant inhibitor, EGFR exon 20 mutant inhibitor, EGFR exon 20 mutant monoclonal antibody or combined medicament thereof.

**44.** Use of the compound, a stereoisomer thereof or a pharmaceutically acceptable salt thereof according to any one of claims 1 to 36, or the pharmaceutical composition according to claim 39 in the preparation of medicaments for the treatment of cancer, inflammation, chronic liver disease, diabetes, cardiovascular disease or AIDS-related disease, wherein the cancer, inflammation, chronic liver disease, diabetes, cardiovascular disease or AIDS-related disease is preferably a disease mediated by HER2 exon 20 mutation and/or EGFR exon 20 mutation.

**45.** The use according to claim 44, wherein the cancer is selected from the group consisting of breast cancer, cervical cancer, colon cancer, lung cancer, stomach cancer, rectal cancer, pancreatic cancer, brain cancer, liver cancer, solid tumor, glioma, glioblastoma, leukemia, lymphoma, myeloma and non-small cell lung cancer.

**INTERNATIONAL SEARCH REPORT**

International application No. **PCT/CN2020/091558**

**A. CLASSIFICATION OF SUBJECT MATTER**

C07D 403/04(2006.01)i； C07D 403/12(2006.01)i； C07F 9/53(2006.01)i； C07D 491/10(2006.01)i； C07D 405/14(2006.01)i； A61K 31/506(2006.01)i； A61K 31/505(2006.01)i； A61K 31/675(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; C07F; A61K; A61P

Documentation searched other than minimum documentation to the extent that such documents are included in the fields searched

Electronic data base consulted during the international search (name of data base and, where practicable, search terms used)

DWPI; SIPOABS; CNABS; CNTXT; STNext: 上海翰森; 江苏豪森; 王峰; 外显子突变; exons mutation; EGFR; HER2; TAK-788

**C. DOCUMENTS CONSIDERED TO BE RELEVANT**

| Category* | Citation of document, with indication, where appropriate, of the relevant passages | Relevant to claim No. |
|---|---|---|
| X | WO 2015195228 A1 (ARIAD PHARMACEUTICALS, INC. et al.) 23 December 2015 (2015-12-23)<br>abstract, claims 1, 98, 160, embodiments 8-24, compounds | 1-36, 41-45 |
| X | WO 2016054987 A1 (SHANGHAI HANSOH BIOMEDICAL CO., LTD. et al.) 14 April 2016 (2016-04-14)<br>abstract, claims 1, 7-8, 12, 22, 27, 29, 38 | 1-45 |
| X | CN 108017633 A (TIANJIN UNIVERSITY) 11 May 2018 (2018-05-11)<br>claims 1-10, embodiments 15, 17-30 | 1-36, 41-45 |
| X | CN 103702990 A (ASTRAZENECA AB) 02 April 2014 (2014-04-02)<br>claims 1-16 | 1-36, 41-45 |
| X | CN 109280048 A (JIANGXI SCIENCE & TECHNOLOGY NORMAL UNIVERSITY) 29 January 2019 (2019-01-29)<br>embodiments 1-34 | 1-36, 41-45 |
| X | CN 106995437 A (QILU PHARMACEUTICAL CO., LTD.) 01 August 2017 (2017-08-01)<br>claims 1-9 | 1-36, 41-45 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 August 2020** | **19 August 2020** |

Name and mailing address of the ISA/CN

**China National Intellectual Property Administration (ISA/CN)**
**No. 6, Xitucheng Road, Jimenqiao Haidian District, Beijing 100088**
**China**

Facsimile No. **(86-10)62019451**

Authorized officer

Telephone No.

Form PCT/ISA/210 (second sheet) (January 2015)

**INTERNATIONAL SEARCH REPORT**

International application No. **PCT/CN2020/091558**

**C. DOCUMENTS CONSIDERED TO BE RELEVANT**

| Category* | Citation of document, with indication, where appropriate, of the relevant passages | Relevant to claim No. |
|---|---|---|
| PX | CN 110652514 A (SHANGHAI HANSOH BIOMEDICAL CO., LTD. et al.) 07 January 2020 (2020-01-07)<br>1-11 | 1-36, 41-45 |

Form PCT/ISA/210 (second sheet) (January 2015)

**INTERNATIONAL SEARCH REPORT**
**Information on patent family members**

International application No.
**PCT/CN2020/091558**

| Patent document cited in search report | | | Publication date (day/month/year) | Patent family member(s) | | | Publication date (day/month/year) |
|---|---|---|---|---|---|---|---|
| WO | 2015195228 | A1 | 23 December 2015 | HR | P20190407 | T1 | 03 May 2019 |
| | | | | JP | 6546630 | B2 | 17 July 2019 |
| | | | | ZA | 201608224 | B | 24 April 2019 |
| | | | | AP | 201709690 | A0 | 31 January 2017 |
| | | | | EA | 034691 | B1 | 06 March 2020 |
| | | | | JP | 2018012712 | A | 25 January 2018 |
| | | | | IL | 248859 | D0 | 31 January 2017 |
| | | | | CN | 106559991 | A | 05 April 2017 |
| | | | | AU | 2015277786 | A1 | 22 December 2016 |
| | | | | PE | 02682017 | A1 | 21 April 2017 |
| | | | | CR | 20170011 | A | 04 April 2017 |
| | | | | KR | 20170016861 | A | 14 February 2017 |
| | | | | LT | 3157916 | T | 10 April 2019 |
| | | | | CN | 106559991 | B | 20 September 2019 |
| | | | | SG | 11201610517 P | A | 27 January 2017 |
| | | | | DK | 3157916 | T3 | 18 March 2019 |
| | | | | CN | 110526912 | A | 03 December 2019 |
| | | | | EP | 3157916 | A1 | 26 April 2017 |
| | | | | RS | 58541 | B1 | 30 April 2019 |
| | | | | EA | 201692261 | A1 | 31 May 2017 |
| | | | | US | 10227342 | B2 | 12 March 2019 |
| | | | | CA | 2949793 | A1 | 23 December 2015 |
| | | | | GE | P20197011 | B | 12 August 2019 |
| | | | | SI | 3157916 | T1 | 31 May 2019 |
| | | | | AP | 201709690 | D0 | 31 January 2017 |
| | | | | TN | 2016000560 | A1 | 04 April 2018 |
| | | | | GE | AP202014706 | A | 10 February 2020 |
| | | | | JP | 2019194217 | A | 07 November 2019 |
| | | | | MA | 40240 | B1 | 29 March 2019 |
| | | | | ES | 2715500 | T3 | 04 June 2019 |
| | | | | CU | 20160185 | A7 | 10 May 2017 |
| | | | | PT | 3157916 | T | 25 March 2019 |
| | | | | EP | 3157916 | A4 | 07 March 2018 |
| | | | | MX | 2016016766 | A | 27 April 2017 |
| | | | | SG | 10201913753V | A | 30 March 2020 |
| | | | | CL | 2016003222 | A1 | 13 October 2017 |
| | | | | PH | 12016502453 | A1 | 06 March 2017 |
| | | | | BR | 112016029662 | A2 | 24 October 2017 |
| | | | | MX | 361802 | B | 14 December 2018 |
| | | | | US | 9796712 | B2 | 24 October 2017 |
| | | | | US | 2017197962 | A1 | 13 July 2017 |
| | | | | EP | 3409669 | A1 | 05 December 2018 |
| | | | | JP | 6230205 | B2 | 15 November 2017 |
| | | | | TR | 201903322 | T4 | 21 March 2019 |
| | | | | AU | 2019206024 | A1 | 01 August 2019 |
| | | | | AU | 2015277786 | B2 | 18 April 2019 |
| | | | | JP | 2017521394 | A | 03 August 2017 |
| | | | | HU | E042390 | T2 | 28 June 2019 |
| | | | | US | 2017253594 | A1 | 07 September 2017 |
| WO | 2016054987 | A1 | 14 April 2016 | JP | 2017532326 | A | 02 November 2017 |

Form PCT/ISA/210 (patent family annex) (January 2015)

**INTERNATIONAL SEARCH REPORT**
**Information on patent family members**

International application No.
**PCT/CN2020/091558**

| Patent document cited in search report | | | Publication date (day/month/year) | Patent family member(s) | | | Publication date (day/month/year) |
|---|---|---|---|---|---|---|---|
| | | | | RU | 2702631 | C2 | 09 October 2019 |
| | | | | US | 2017313714 | A1 | 02 November 2017 |
| | | | | US | 2019100528 | A1 | 04 April 2019 |
| | | | | EP | 3205650 | A1 | 16 August 2017 |
| | | | | TW | 201613875 | A | 16 April 2016 |
| | | | | BR | 112017006713 | A2 | 26 December 2017 |
| | | | | CN | 111170999 | A | 19 May 2020 |
| | | | | US | 10259820 | B2 | 16 April 2019 |
| | | | | CN | 111171000 | A | 19 May 2020 |
| | | | | CN | 106661000 | A | 10 May 2017 |
| | | | | CN | 111187221 | A | 22 May 2020 |
| | | | | RU | 2017114687 | A | 20 November 2018 |
| | | | | AU | 2015330506 | A1 | 06 April 2017 |
| | | | | CN | 106661000 | B | 09 April 2019 |
| | | | | US | 10428081 | B2 | 01 October 2019 |
| | | | | ZA | 201701465 | B | 28 August 2019 |
| | | | | RU | 2017114687 | A3 | 31 January 2019 |
| | | | | JP | 6691281 | B2 | 28 April 2020 |
| | | | | EP | 3205650 | A4 | 26 September 2018 |
| | | | | KR | 20170118681 | A | 25 October 2017 |
| | | | | CA | 2959194 | A1 | 14 April 2016 |
| | | | | MX | 2017004234 | A | 08 June 2017 |
| | | | | AU | 2015330506 | B2 | 30 January 2020 |
| CN | 108017633 | A | 11 May 2018 | None | | | |
| CN | 103702990 | A | 02 April 2014 | DK | 3009431 | T3 | 08 January 2018 |
| | | | | PT | 3009431 | T | 26 December 2017 |
| | | | | HK | 1192554 | A1 | 10 June 2016 |
| | | | | CA | 2843109 | C | 16 June 2015 |
| | | | | EP | 3333161 | B1 | 19 February 2020 |
| | | | | CY | 1120072 | T1 | 12 December 2018 |
| | | | | CA | 2881991 | A1 | 31 January 2013 |
| | | | | US | 2019092746 | A1 | 28 March 2019 |
| | | | | GT | 201300288 | A | 09 October 2017 |
| | | | | BR | 112014001768 | B1 | 05 February 2019 |
| | | | | TW | 201443040 | A | 16 November 2014 |
| | | | | EA | 201391491 | A1 | 29 August 2014 |
| | | | | PE | 20141700 | A1 | 20 November 2014 |
| | | | | PE | 17002014 | A1 | 20 November 2014 |
| | | | | SI | EP2736895 | T1 | 31 March 2016 |
| | | | | AU | 2012288626 | B2 | 07 May 2015 |
| | | | | WO | 2013014448 | A1 | 31 January 2013 |
| | | | | CA | 2843109 | A1 | 31 January 2013 |
| | | | | SG | 10201910986 Q | A | 30 January 2020 |
| | | | | CA | 2881987 | A1 | 31 January 2013 |
| | | | | PH | 12015501326 | B1 | 14 September 2015 |
| | | | | HU | E037645 | T2 | 28 September 2018 |
| | | | | CR | 20130629 | A | 12 March 2014 |
| | | | | EA | 201690328 | A1 | 30 November 2016 |
| | | | | IL | 242278 | A | 29 December 2016 |
| | | | | CY | 1117431 | T1 | 26 April 2017 |

Form PCT/ISA/210 (patent family annex) (January 2015)

**INTERNATIONAL SEARCH REPORT**
**Information on patent family members**

International application No.
**PCT/CN2020/091558**

| Patent document cited in search report | | | Publication date (day/month/year) | Patent family member(s) | | | Publication date (day/month/year) |
|---|---|---|---|---|---|---|---|
| | | | | CA | 2881991 | C | 25 October 2016 |
| | | | | HR | P20171957 | T1 | 09 February 2018 |
| | | | | KR | 20140062181 | A | 22 May 2014 |
| | | | | TW | 201319057 | A | 16 May 2013 |
| | | | | SG | 10201910984 X | A | 30 January 2020 |
| | | | | JP | 2013544273 | A | 12 December 2013 |
| | | | | TW | I465445 | B | 21 December 2014 |
| | | | | IL | 242286 | A | 31 July 2016 |
| | | | | IL | 242278 | D0 | 30 November 2015 |
| | | | | AU | 2012288626 | A8 | 26 September 2013 |
| | | | | HK | 1192549 | A1 | 26 August 2016 |
| | | | | CN | 105175396 | B | 16 January 2018 |
| | | | | NI | 201300134 | A | 12 February 2014 |
| | | | | SM | T201600070 | B | 29 April 2016 |
| | | | | EP | 2736895 | A1 | 04 June 2014 |
| | | | | BR | 122014026150 | A2 | 28 May 2019 |
| | | | | CN | 105175396 | A | 23 December 2015 |
| | | | | US | 2013053409 | A1 | 28 February 2013 |
| | | | | IL | 242279 | D0 | 30 November 2015 |
| | | | | PL | 3009431 | T3 | 28 February 2018 |
| | | | | JP | 2014094930 | A | 22 May 2014 |
| | | | | TW | I555743 | B | 01 November 2016 |
| | | | | BR | 122014026114 | A2 | 20 August 2019 |
| | | | | CA | 2882018 | A1 | 31 January 2013 |
| CN | 109280048 | A | 29 January 2019 | None | | | |
| CN | 106995437 | A | 01 August 2017 | None | | | |
| CN | 110652514 | A | 07 January 2020 | None | | | |

Form PCT/ISA/210 (patent family annex) (January 2015)